# EUROPEAN PATENT APPLICATION

(11) **EP 2 019 142 A2**
(43) Date of publication of application: **28.01.2009**
(21) Application number: 08013335.8
(22) Date of filing: 05.12.2005
(51) Int. Cl.: C12N 15/82, C07K 14/415

(54) **Isoforms of elF-5A and methods of using same**

(30) Priority: 03.12.2004 US 632567 P; 14.06.2005 US 690160 P; 25.08.2005 US 710999 P; 21.10.2005 US 728737 P
(62) Divisional of application: 05852821.7
(71) Applicant: Senesco Technologies, Inc., New Brunswick, NJ 08901 (US)
(72) Inventor: Thompson, John, E., Waterloo Ontario N2K 4N1 (CA); McNamara, Linda, Waterloo Ontario N2V 1C3 (CA)
(74) Representative: Vossius & Partner

(57) **Abstract**

The present invention relates to unique isoforms of eukaryotic initiation Factor 5A ("eIF-5A"): senescence-induced eIF-5A; wounding/pathogen induced eIF-5A; stress eIF-5A and growth eIF-5A, as well as polynucleotides that encode these three factors. The present invention also relates to methods involving modulating the expression of these factors. The present invention also relates to deoxyhypusine synthase ("DHS"), polynucleotides that encode DHS, and methods involving modulating the expression of DHS.

## Description

This application claims priority to and incorporates by reference U.S. provisional application 60/632,567, filed on Dec. 3, 2004, U.S. provisional 60/690,160 filed on June 14, 2005, U.S. provisional 60/710,999 filed on August 25, 2005 and U.S. provisional 60/728,737 filed on October 21, 2005. This application is a CIP of U.S. application 10/862,440, filed June 8, 2004, which is a continuation-in-part application of Serial No. 09/725,019, filed Nov. 29, 2000 (now U.S. patent 6,878,860), which is a continuation-in-part of 09/597,771, filed June 19, 2000 (now U.S. Patent 6,538,182), which is a continuation in part of 09/348,675, filed July 6, 1999, now abandoned, all of which are herein incorporated by reference in their entireties.

### FIELD OF THE INVENTION

The present invention relates to unique isoforms of eukaryotic initiation Factor 5A ("eIF-5A") and polynucleotides that encode eIF-5A and deoxyhypusine synthase ("DHS"), and polynucleotides that encode DHS, and methods involving modulating the expression of the isoforms eIF-5A and DHS.

### DESCRIPTION OF THE PRIOR ART

Senescence is the terminal phase of biological development in the life of a plant. It presages death and occurs at various levels of biological organization including the whole plant, organs, flowers and fruit, tissues and individual cells.

The onset of senescence can be induced by different factors both internal and external. Senescence is a complex, highly regulated developmental stage in the life of a plant or plant tissue, such as fruit, flowers and leaves. Senescence results in the coordinated breakdown of cell membranes and macromolecules and the subsequent mobilization of metabolites to other parts of the plant.

In addition to the programmed senescence which takes place during normal plant development, death of cells and tissues and ensuing remobilization of metabolites occurs as a coordinated response to external, environmental factors. External factors that induce premature initiation of senescence, which is also referred to as necrosis or apoptosis, include environmental stresses such as temperature, drought, poor light or nutrient supply, as well as pathogen attack. Plant tissues exposed to environmental stress also produce ethylene, commonly known as stress ethylene (Buchanan-Wollaston, V., 1997, J. Exp. Botany, 48:181-199; Wright, M., 1974, Plant, 120:63-69). Ethylene is known to cause senescence in some plants.

Senescence is not a passive process, but, rather, is an actively regulated process that involves coordinated expression of specific genes. During senescence, the levels of total RNA decrease and the expression of many genes is switched off (Bate et al., 1991, J. Exper. Botany, 42, 801-11; Hensel et al., 1993, The Plant Cell, 5, 553-64). However, there is increasing evidence that the senescence process depends on *de novo* transcription of nuclear genes. For example, senescence is blocked by inhibitors of mRNA and protein synthesis and enucleation. Molecular studies using mRNA from senescing leaves and green leaves for *in vitro* translation experiments show a changed pattern of leaf protein products in senescing leaves (Thomas et al., 1992, J. Plant Physiol., 139, 403-12). With the use of differential screening and subtractive hybridization techniques, many cDNA clones representing senescence-induced genes have been identified from a range of different plants, including both monocots and dicots, such as Arabidopsis, maize, cucumber, asparagus, tomato, rice and potato. Identification of genes that are expressed specifically during senescence is hard evidence of the requirement for *de novo* transcription for senescence to proceed.

The events that take place during senescence appear to be highly coordinated to allow maximum use of the cellular components before necrosis and death occur. Complex interactions involving the perception of specific signals and the induction of cascades of gene expression must occur to regulate this process. Expression of genes encoding senescence related proteins is probably regulated via common activator proteins that are, in turn, activated directly or indirectly by hormonal signals. Little is known about the mechanisms involved in the initial signaling or subsequent co-ordination of the process.

Coordinated gene expression requires factors involved in transcription and translation, including initiation factors. Translation initiation factor genes have been isolated and characterized in a variety of organisms, including plants. Translation initiation factors can control the rate at which mRNA populations are moved out of the nucleus, the rate at which they are associated with a ribosome and to some extent can affect the stability of specific mRNAs. Zuk, et al., EMBO J. 17:2914 -2925 (1998). Indeed, one such translation initiation factor, which is not required for global translation activity, is believed to shuttle specific subsets of mRNAs from the nucleus to the cytoplasm for translation. Jao, et al., J. Cell. Biochem. 86: 590-600, (2002); Wang et al., J Biol Chem 276:17541-17549 (2001); Rosorius et al., J.Cell Sci., 112, 2369-2380 (1999). This translation factor is known as the eukaryotic initiation factor 5A (eIF-5A), and is the only protein known to contain the amino acid hypusine. Park, et al., J Biol Chem 263:15264-15269 (1988).

Eukaryotic translation initiation factor 5A (eIF-5A) is an essential protein factor approximately 17 KDa in size, which is involved in the initiation of eukaryotic cellular protein synthesis. It is characterized by the presence of hypusine [N-(4-amino-2-hydroxybutyl) lysine], a unique modified amino acid, known to be present only in eIF-5A. Hypusine is formed post-translationally via the transfer and hydroxylation of the butylamino group from the polyamine, spermidine, to the side chain amino group of a specific lysine residue in eIF-5A. Activation of eIF-5A involves transfer of the butylamine residue of spermidine to the lysine of eIF-5A, forming hypusine and activating eIF-5A. In eukaryotes, deoxyhypusine synthase (DHS) mediates the post-translational synthesis of hypusine in eIF-5A. The hypusine modification has been shown to be essential for eIF-5A activity *in vitro* using a methionyl-puromycin assay.

Hypusine is formed on eIF-5A post-translationally through the conversion of a conserved lysine residue by the action of deoxyhypusine synthase (DHS; EC 1.1.1.249) and deoxyhypusine hydroxylase (DHH; EC 1.14.99.29). DHS has been isolated from several plant species, including tomato (GenBank Accession Number AF296077), *Arabidopsis thaliana* (AT-DHS; GenBank Accession Number AF296078), tobacco (Ober and Hartmann, 1999), carnation (GenBank Accession Number AF296079) and banana (GenBank Accession Number AF296080), whereas the gene for DHH has not been recognized.

DHS converts a conserved lysine residue of eIF-5A to deoxyhypusine through the addition of a butylamine group derived from spermidine. This intermediate form of eIF-5A is then hydroxylated by DHH to become hypusine. Park et al., Biol. Signals 6, 115-123 (1997). Both the deoxyhypusine and the hypusine form of eIF-5A are able to bind mRNA *in vitro.* Liu et al., Biol Signals 6:166-174 (1997). Although the function of eIF-5A is not fully understood, there is some evidence that it may regulate cell division (Park et al., J Biol Chem 263:15264-15269 (1998); Tome et al., Biol Signals 6:150- 156, (1997)) and senescence. (Wang et al., J. Biol. Chem. 276(20): 17541-17549 (2001)). *See also* U.S. patent 6,538,182 and U.S. application 09/725,019, which are herein incorporated by reference in their entirety. It appears that several organisms are known to have more than one isoform of eIF-5A, which would suit the premise that each isoform is a specific shuttle to specific suites of mRNAs that are involved in such processes as cell division and senescence.

Wang *et al.* demonstrated that an increased level of DHS mRNA correlates with fruit softening and natural and stress-induced leaf senescence of tomato. Wang et al., J. Biol. Chem. 276(20): 17541-17549 (2001). Furthermore when the expression of DHS was suppressed in transgenic tomato plants by introducing a DHS antisense cDNA fragment under the regulation of a constitutive promoter, the tomato fruit from these transgenic plants exhibited dramatically delayed senescence as evidenced by delayed fruit softening and spoilage. *See* U.S. patent 6,538,182 and U.S. application 09/725,019, filed November 29, 2003, incorporated herein by reference in their entirety. Since DHS is known to activate eIF-5A, these data suggest that the hypusine-modified eIF-5A (active eIF-5A) may regulate senescence through selective translation of mRNA species required for senescence. This is further demonstrated through the down-regulation of DHS in *Arabidopsis thaliana* ("AT") by antisense polynucleotides of the full length or 3'UTR cDNA under the control of a constitutive promoter. By down regulating *Arabidopsis* thaliana DHS ("AT-DHS") expression and making it less available for eIF-5A activation, senescence was delayed by approximately 2 weeks (*See* U.S. 6,538,182). Not only was senescence delayed, but also an increase in seed yield, an increase in stress tolerance and an increase in biomass were observed in the transgenic plants, where the extent of each phenotype was determined by the extent of the down-regulation of DHS. Since tomato and *Arabidopsis thaliana* only have one copy of DHS in their genome, as shown by Southern blot (Wang *et al.,* 2001) and BLAST analysis, in order to target the specific eIF-5A isoform responsible for shuttling of senescence transcripts out of the nucleus, the senescence specific isoform of eIF-5A must be identified and specifically down-regulated through the antisense constructs of senescence-induced eIF-5A (of the 3' UTR) or by taking advantage of the plant's natural ability for down-regulation of an over expressed gene (*i.e.* creating over-expression through the use of sense polynucleotides).

Plants lack immune systems and thus, have a unique way of dealing with viruses - called co-suppression, which results in sequence-specific degradation of the viral RNA. When a transgene is under a strong constitutive promoter, like the cauliflower mosaic virus double 35S promoter, it appears as a viral transcript to the plant and sequence-specific degradation occurs, but not just of the transgene, but also the endogenous gene. (reviewed in Fagard and Vaucheret, Annual Review. Plant Physiol. Plant Mol. Biol., June; 51:167-194 (2000). There is some evidence that co-suppression may be as effective, if not more effective, than antisense suppression of expression for the down-regulation of an endogenous gene.

### SUMMARY OF THE INVENTION

The present invention provides isolated isoforms of eukaryotic initiation Factor 5A ("eIF-5A"): senescence-induced eIF-5A; wounding/pathogen-induced eIF-5A; growth eIF-5A; and stress eIF-5A as well as polynucleotides that encode these isoforms. The present invention provides antisense and sense polynucleotides of the eIF-5A isoforms. The invention also provide expression vectors comprising sense and antisense polynucleotides of the eIF-5A isoforms. The present invention also relates to methods involving modulating (increasing/up-regulating or inhibiting/down-regulating) the expression of these factors, in addition to plants and their plant parts and progeny having modified expression of eIF-5A. Further, the present invention contemplates knock down mutants of these isoforms of eIF-5A.

The present invention also relates to isolated deoxyhypusine synthase ("DHS") and polynucleotides that encode DHS. The present invention also provides antisense and sense polynucleotides of DHS. The invention also provide expression vectors comprising sense and antisense polynucleotides of DHS. The present invention also relates to methods involving modulating (increasing/up-regulating or inhibiting/down-regulating) the expression of DHS in addition to plants and their plant parts and progeny having modified expression of DHS. Further, the present invention contemplates knock down mutants of DHS.

### BRIEF DESCRIPTION OF THE DRAWINGS

Figure 1 shows the alignment of three isoforms of eIF-5A isolated *Arabidopsis thaliana* senescence-induced eIF-5A (line 1)(previously described in U.S. patent 6,538,182 and pending application, 09/725,019); wounding/pathogen induced eIF-5A (line 2); and growth eIF-5A (line 3).
Figure 2 shows the alignment of the coding regions of these three *Arabidopsis thaliana* isoforms. Line 1 is senescence-induced eIF-5A. Line 2 is wounding/pathogen induced eIF-5A. Line 3 is growth eIF-5A.
Figures 3 provides the genomic sequence of the senescence-induced eIF-5A of *Arabidopsis thaliana.*
Figure 4 provides the genomic sequence of the wounding/pathogen induced eIF5A of *Arabidopsis thaliana.*
Figure 5 provides the genomic sequence of the growth eIF5A of *Arabidopsis thaliana.*
Figure 6 is a map of binary vector pKYLX71-35S².
Figure 7 is a map of binary vector pGEM®-T Easy Vector.
Figure 8 shows Western blots of all three isoforms of eIF-5A in different tissues of *Arabidopsis thaliana* wild type of the Columbia ecotype.
Figure 9 are Western blots for the senescence-induced eIF-5A and the wounding/pathogen induced eIF-5A of infected leaves after 72 hours of *Arabidopsis thaliana* wild type of the Columbia ecotype.
Figure 10 are Northern blots for the three isoforms of eIF-5A in wounded leaves after 72 hours of *Arabidopsis thaliana* wild type of the Columbia ecotype.
Figure 11 depicts PCR products from genomic DNA of *Arabidopsis* senescence-induced eIF-5A (ATeIF-5A), wounding/pathogen-induced AteIF-5A, and growth AteIF-5A in lanes 1, 2 and 3 respectively.
Figure 12 shows an agarose gel with senescence-induced AteIF-5A, wounding/pathogen induced AteIF-5A, and growth AteIF-5A genomic sequences in pGEM.
Figure 13 shows an agarose gel with wounding/pathogen induced AteIF-5A, growth AteIF-5A, genomic sequences in pKYLX71.
Figure 14 is a picture of a T1 plate for plants transformed with a construct having sense wounding/pathogen induced AteIF-5A.
Figure 15 is a picture of T1 plants transformed with sense wounding/pathogen-induced AteIF-5A at 4 weeks of age.
Figure 16 is a picture of T1 plants transformed with sense wounding/pathogen induced AteIF-5A at 5.5 weeks of age.
Figure 17 is a picture of T2 plants transformed with sense wounding/pathogen induced AteIF-5A at 10 days post seeding.
Figure 18 is a picture of T1 plants transformed with sense growth AteIF-5A at 10 days post seeding.
Figure 19 is a picture of T1 plants transformed with sense growth AteIF-5A at 4 weeks of age.
Figure 20 is a Western blot of T2 plants transformed with sense growth AteIF-5A lines.
Figure 21 are T2 plants transformed with sense growth AteIF-5A (Lines 1A-1D) at 4 weeks of age (top), 5 weeks of age (bottom left) and 6 weeks of age (bottom right).
Figure 22 are T2 plants transformed with sense growth AteIF-5A (Lines 2A-1D) at 4 weeks of age (top), 5 weeks of age (bottom left) and 6 weeks of age (bottom right).
Figure 23 are T2 plants transformed with sense growth AteIF-5A (Lines 4A-D) at 4 weeks of age (top), 5 weeks of age (bottom left) and 6 weeks of age (bottom right).
Figure 24 are T2 plants transformed with sense growth AteIF-5A (Lines 15A-D) at 4 weeks of age (top), 5 weeks of age (bottom left) and 6 weeks of age (bottom right).
Figure 25 are T2 plants transformed with sense growth AteIF-5A (Lines 8A-D) at 4 weeks of age (top), 5 weeks of age (bottom left) and 6 weeks of age (bottom right).
Figure 26 are T2 plants transformed with sense growth AteIF-5A (Lines 9E-H) at 4 weeks of age (top), 5 weeks of age (bottom left) and 6 weeks of age (bottom right).
Figure 27 are T2 plants transformed with sense growth AteIF-5A (Lines 11A-D) at 4 weeks of age (top), 5 weeks of age (bottom left) and 6 weeks of age (bottom right).
Figure 28 are T2 plants transformed with sense growth AteIF-5A (Lines 16A-D) at 4 weeks of age (top), 5 weeks of age (bottom left) and 6 weeks of age (bottom right).
Figure 29 are photographs of *Arabidopsis thaliana* seeds from various plant lines (including wild type control and plant lines having been transformed with sense growth AteIF-5A).
Figure 30 is a bar graph of average seed size for each plant subline having been transformed with sense growth AteIF-5A.
Figure 31 is a bar graph of individual seed weight for each plant subline having been transformed with sense growth AteIF-5A.

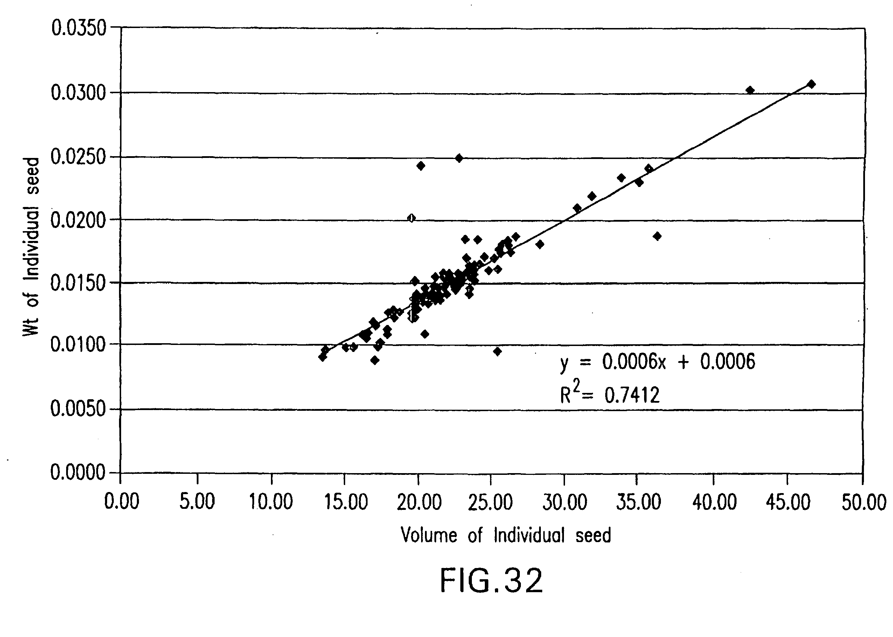

Figure 32 is a graph showing the proportional relationship between the weight of the individual seeds versus the volume of individual seeds.

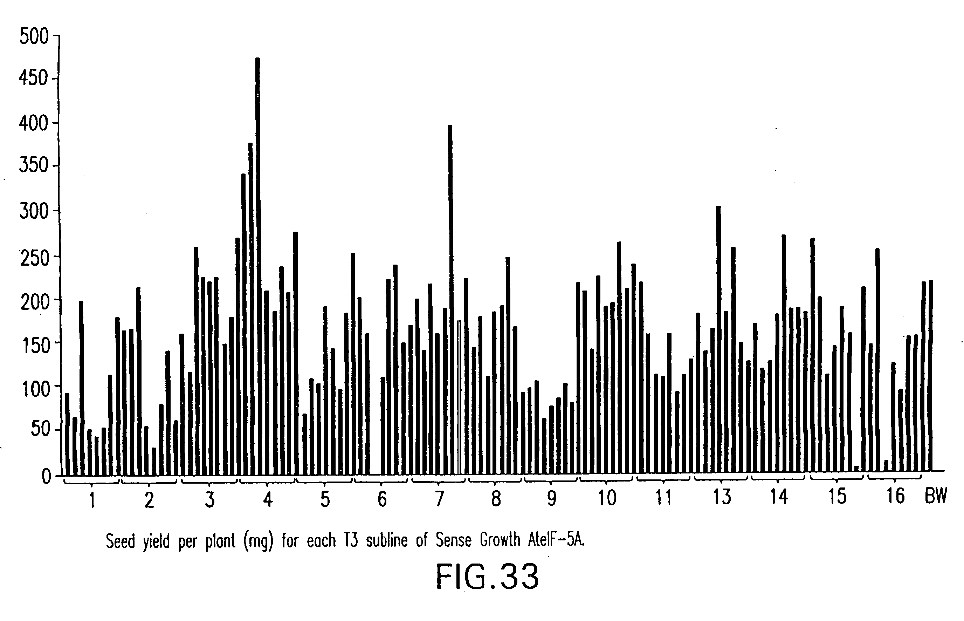

Figure 33 is a bar graph showing seed yield per plant for each plant subline having been transformed with sense growth AteIF-5A.

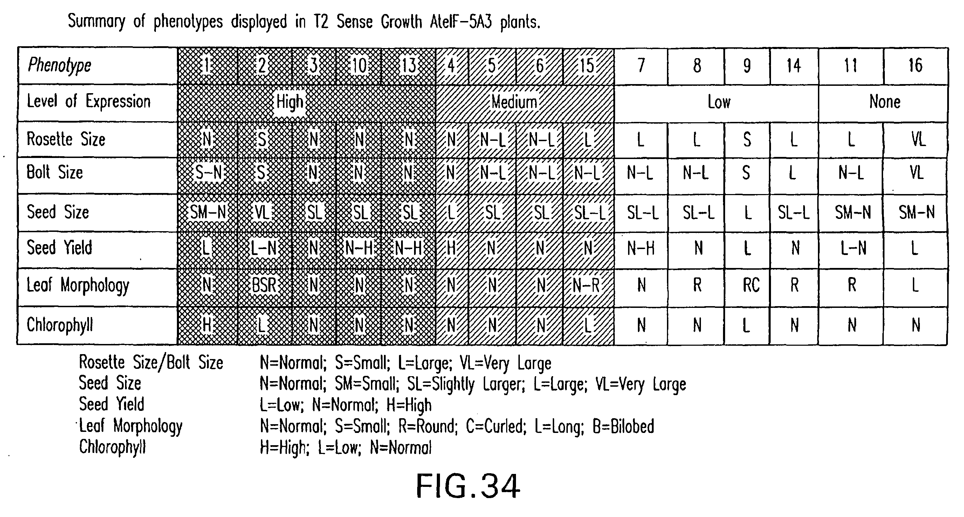

Figure 34 is a summary of phenotypes displayed in sense growth AteIF-5A plants.
Figure 35 shows a comparison of transgenic arabidopsis plant (transformed with antisense full length senescence-induced eIF-5A) with a wild type plant. The transgenic plant exhibits delayed senescence.

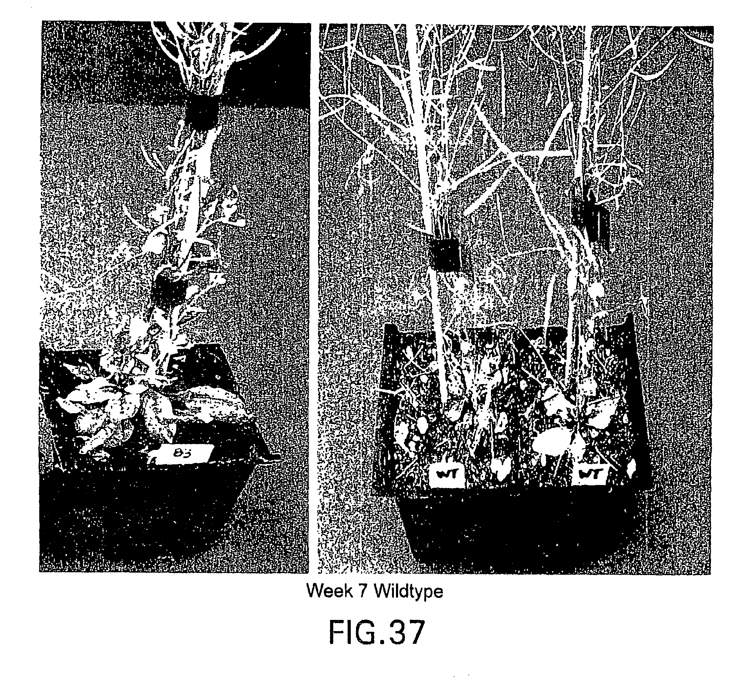

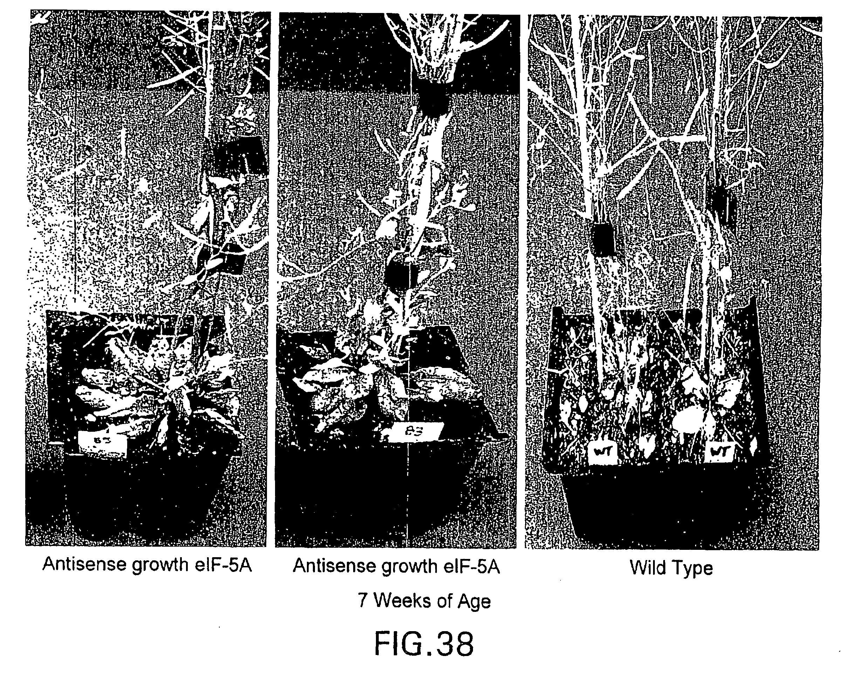

Figures 36-38 show photographs of a plant (transformed with antisense growth eIF-5A).

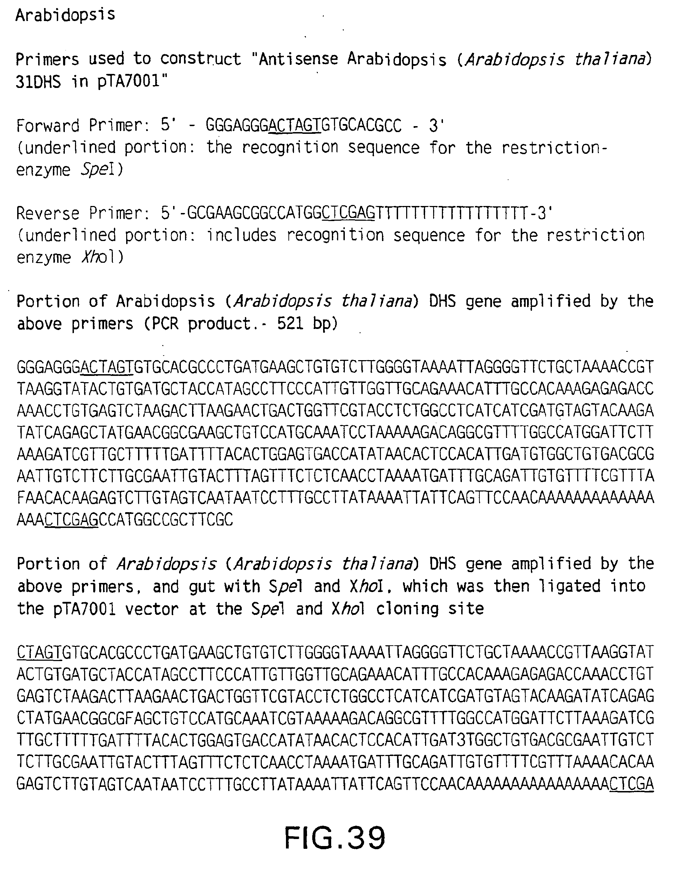

Figure 39 shows the primers used to construct the vector for generating antisense arabidopsis thaliana 3' DHS.

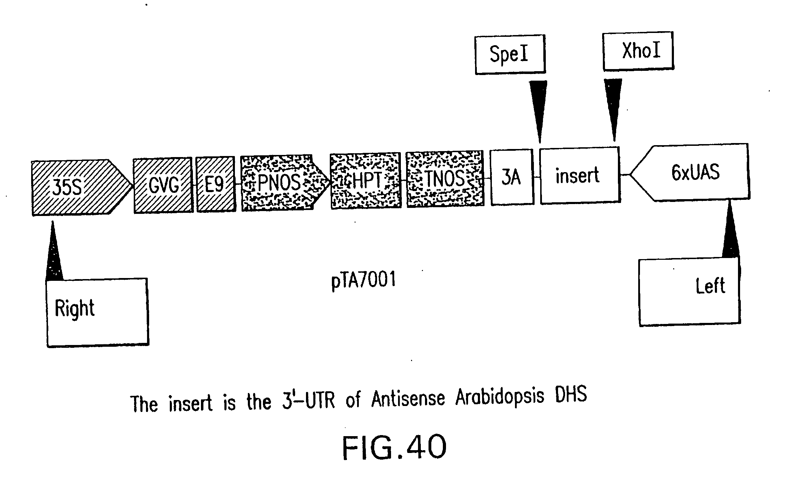

Figure 40 shows the vector construct.
Figure 41 shows the sequence for wounding/pathogen factor eIF-5A isolated from arabidopsis and the location of the antisense construct.

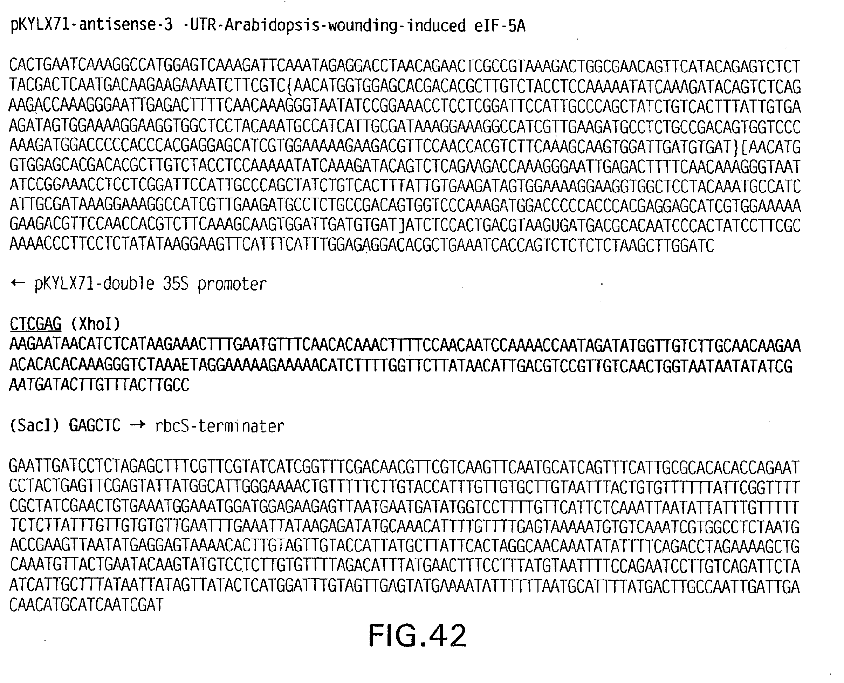

Figure 42 shows the vector construct.
Figure 43 shows plate counts of leaf discs inoculated with pseudomonas.
Figure 44 shows a graph of CFUs in antisense transgenic plants versus wild-type.
Figure 45 depict the nucleotide sequence of the tomato leaf DHS cDNA sequence (SEQ ID NO:1) and the derived amino acid sequence (SEQ ID NO. 2) obtained from a tomato leaf cDNA library.
Figure 46 depicts the nucleotide sequence of an *Arabidopsis* DHS gene obtained by aligning the tomato DHS sequence with unidentified genomic sequences in the *Arabidopsis* gene bank (SEQ ID NO:5). The gaps between amino acid sequences are predicted introns. Figure 46B depicts the derived *Arabidopsis* DHS amino acid sequence (SEQ ID NO:6). Figure 46C depicts the nucleotide sequence of a 600 base pair *Arabidopsis* DHS cDNA obtained by PCR. Figure 46D depicts the derived amino acid sequence of the *Arabidopsis* DHS cDNA fragment.
Figure 47 is an alignment of the derived full length tomato leaf DHS amino acid sequence (SEQ ID NO: 2) and the derived full length *Arabidopsis* senescence-induced DHS amino acid sequence with sequence of DHS proteins of human, yeast, fungi, and *Archaeobacteria.* Identical amino acids among three or four of the sequences are boxed.

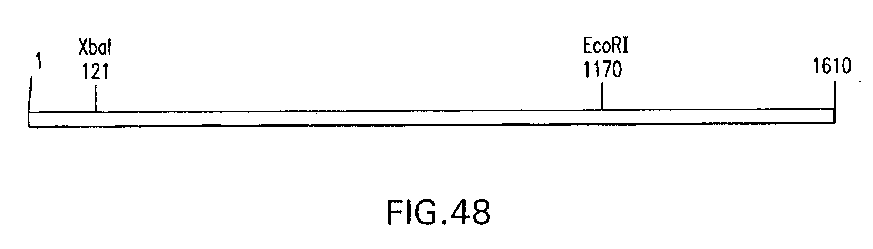

Figure 48 is a restriction map of the tomato DHS cDNA.
Figure 49 is a Southern blot of genomic DNA isolated from tomato leaves and probed with ³²P-dCTP-labeled full length tomato DHS cDNA.
Figure 50 is a Northern blot of RNA isolated from tomato flowers at different stages of development. The top panel is the ethidium bromide stained gel of total RNA. Each lane contains 10 µg RNA. The bottom panel is an autoradiograph of the Northern blot probed with ³²P-dCTP-labeled full length tomato DHS cDNA.
Figure 51 is a Northern blot of RNA isolated from tomato fruit at various stages of ripening that was probed with ³²P-dCTP-labeled full length tomato DHS cDNA. Each lane contains 10 µg RNA.
Figure 52 is a Northern blot of RNA isolated from tomato leaves that had been drought-stressed by treatment with 2 M sorbitol for six hours. Each lane contains 10 µg RNA. The blot was probed with ³²P-dCTP-labeled full length tomato DHS cDNA.
Figure 53 is a Northern blot of RNA isolated from tomato leaves that had been exposed to chilling temperature. Figure 53A is the ethidium bromide stained gel of total RNA. Each lane contained 10 µg RNA. Figure 53B is an autoradiograph of the Northern blot probed with ³²P-dCTP-labeled full length tomato DHS cDNA. Figure 53C shows corresponding leakage data measured as conductivity of leaf diffusates.
Figure 54 is the carnation DHS full-length (1384 base pairs) cDNA clone nucleotide sequence (SEQ ID NO: 9) not including the PolyA tail and 5' end non-coding region. The derived amino acid sequence is shown below the nucleotide sequence (373 amino acids). (SEQ ID NO:10)
Figure 55 is a Northern blot of total RNA from senescing *Arabidopsis* leaves probed with ³²P-dCTP-labeled full-length *Arabidopsis* DHS cDNA. The autoradiograph is at the top, the ethidium stained gel below.
Figure 56 is a Northern blot of total RNA isolated from petals of carnation flowers at various stages. The blot was probed with ³²P-dCTP-labeled full-length carnation DHS cDNA. The autoradiograph is at the top, the ethidium stained gel below.
Figure 57 is the nucleotide (top) (SEQ ID NO:11) and derived amino acid (bottom) (SEQ ID NO:12) sequence of the tomato fruit senescence-induced eIF-5A gene.
Figure 58 is the nucleotide (top) (SEQ ID NO:13) and derived amino acid (bottom) (SEQ ID NO: 14) sequence of the carnation senescence-induced eIF-5A gene.
Figure 59 is the nucleotide (top) (SEQ ID NO:15) and derived amino acid (bottom) (SEQ ID NO: 16) sequence of the *Arabidopsis* senescence-induced eIF-5A gene.

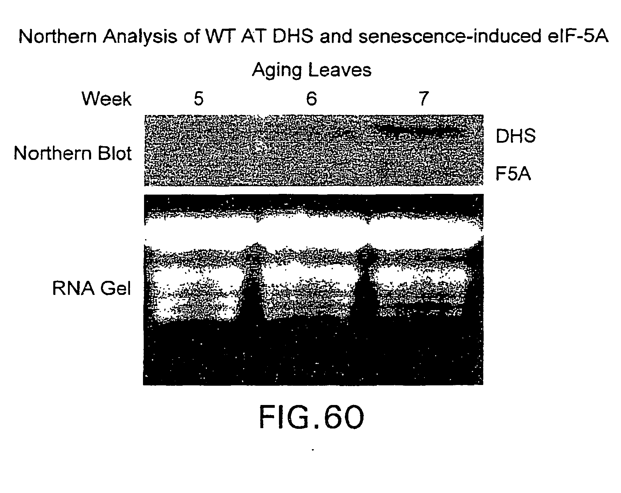

Figure 60 is a Northern blot of total RNA isolated from leaves of *Arabidopsis* plants at various developmental stages. The blot was probed with ³²P-dCTP-labeled full-length *Arabidopsis* DHS cDNA and full-length senescence-induced eIF-5A. The autoradiograph is at the top, the ethidium stained gel below.
Figure 61 is a Northern blot of total RNA isolated from tomato fruit at breaker (BK), red-firm (RF) and red-soft (RS) stages of development. The blot was probed with ³²P-dCTP-labeled full-length DHS cDNA and full-length senescence-induced eIF-5A. DHS and eIF-5A are up-regulated in parallel in red-soft fruit coincident with fruit ripening. The autoradiograph is at the top, the ethidium stained gel below.
Figure 62 is a Northern blot of total RNA isolated from leaves of tomato that were treated with sorbitol to induce drought stress. C is control; S is sorbitol treated. The blot was probed with ³²P-dCTT-labeled full-length DHS cDNA and full-length senescence-induced eIF-5A. Both eIF-5A and DHS are up-regulated in response to drought stress. The autoradiograph is at the top, the ethidium stained gel below.
Figure 63 is a Northern blot of total RNA isolated from flower buds and open senescing flowers of tomato plants. The blot was probed with ³²P-dCTP-labeled full-length senescence-induced DHS cDNA and full-length senescence-induced eIF-5A. Both eIF-5A and DHS are up-regulated in open/senescing flowers. The autoradiograph is at the top, the ethidium stained gel below.

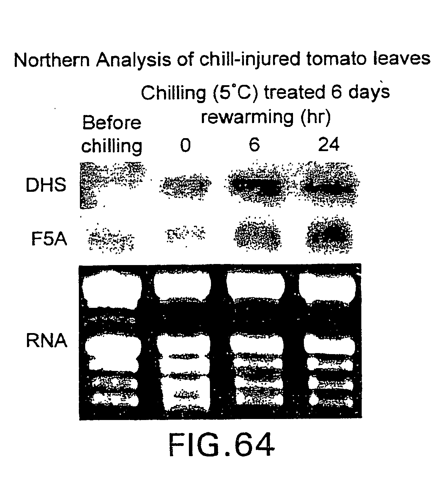

Figure 64 is a Northern blot of total RNA isolated from chill-injured tomato leaves. The blot was probed with ³²P-dCTP-labeled full-length DHS cDNA and full-length senescence-induced eIF-5A. Both eIF-5A and DHS are up-regulated with the development of chilling injury during rewarming The autoradiograph is at the top, the ethidium stained gel below.
Figure 65 is a photograph of 3.1 week old *Arabidopsis* wild-type (left) and transgenic plants expressing the 3'-end of the DHS gene (sequence shown in Figure 80) in antisense orientation showing increased leaf size in the transgenic plants.
Figure 66 is a photograph of 4.6 week old *Arabidopsis* wild-type (left) and transgenic plants expressing the 3'-end of the DHS gene (sequence shown in Figure 80) in antisense orientation showing increased leaf size in the transgenic plants.
Figure 67 is a photograph of 5.6 week old *Arabidopsis* wild-type (left) and transgenic plants expressing the 3'-end of the DHS gene (sequence shown in Figure 80) in antisense orientation showing increased leaf size in the transgenic plants.
Figure 68 is a photograph of 6.1 week old *Arabidopsis* wild-type (left) and transgenic plants expressing the 3'-end of the DHS gene (sequence shown in Figure 80) in antisense orientation showing increased size of transgenic plants.
Figure 69 is a graph showing the increase in seed yield from three T₁ transgenic *Arabidopsis* plant lines expressing the DHS gene in antisense orientation. Seed yield is expressed as volume of seed. SE for n=30 is shown for wild-type plants.
Figure 70 is a photograph of transgenic tomato plants expressing the 3'-end of the DHS gene (sequence shown in Figure 80) in antisense orientation (left) and wild-type plants (right) showing increased leaf size and increased plant size in the transgenic plants. The photograph was taken 18 days after transfer of the plantlets to soil.
Figure 71 is a photograph of transgenic tomato plants expressing the 3'-end of the DHS gene (sequence shown in Figure 36) in antisense orientation (left) and wild-type plants (right) showing increased leaf size and increased plant size in the transgenic plants. The photograph was taken 32 days after transfer of the plantlets to soil.
Figures 72 through 79 are photographs of tomato fruit from wild-type (top panels) and transgenic plants expressing the full-length DHS gene in antisense orientation (bottom panels). Fruit were harvested at the breaker stage of development and ripened in a growth chamber. Days after harvest are indicated in the upper left corner of each panel.
Figure 80 is the nucleotide (top) (SEQ ID NO:30) and derived amino acid (bottom) sequence of the 3'-end of the *Arabidopsis* senescence-induced DHS gene used in antisense orientation to transform plants.
Figure 81 is the nucleotide (top) (SEQ ID NO:31) and derived amino acid (bottom) sequence of the 3'-end of the tomato DHS gene used in antisense orientation to transform plants.
Figure 82 is the nucleotide (top) (SEQ ID NO:26) and derived amino acid (bottom) sequence of a 600 base pair *Arabidopsis* DHS probe used to isolate the full-length *Arabidopsis* gene.
Figure 83 is the nucleotide (top) (SEQ ID NO:27) and derived amino acid (bottom) sequence of the 483 base pair carnation DHS probe used to isolate the full-length carnation gene.
Figure 84 (a) and (b) are photographs of tomato fruits from transgenic tomato plants expressing the 3'-end of the DHS gene (sequence shown in Figure 81) in antisense orientation (right) and tomato fruits from wild-type plants (left). While the wild-type fruit exhibits blossom end rot, the transgenic fruit does not.
Figure 85 shows the alignment of various isoforms of eIF-5A from several plant species. It also provides alignment of the hypusine conserved region.
Figure 86 provides tomato senescence-induced eIF-5A polynucleotide and amino acid sequences.

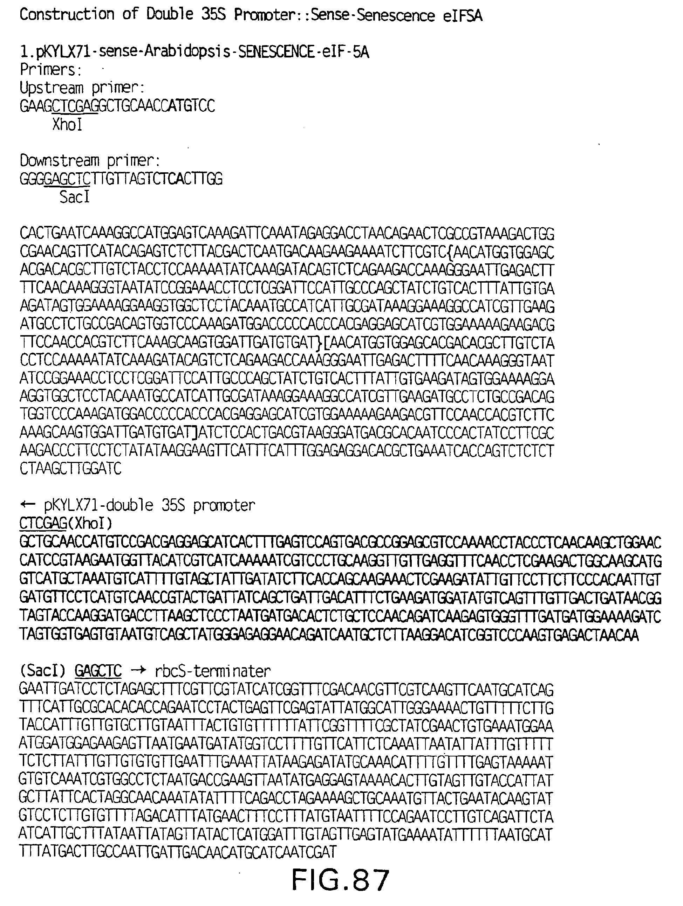

Figure 87 provides Arabidopsis senescence-induced eIF-5A and the construction of pKYLX71-sense senescence-induced eIF-5A.

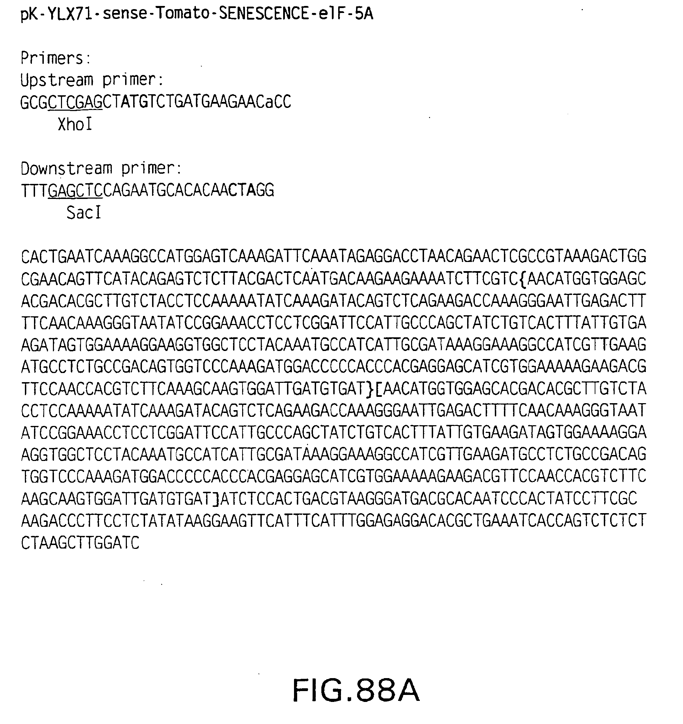

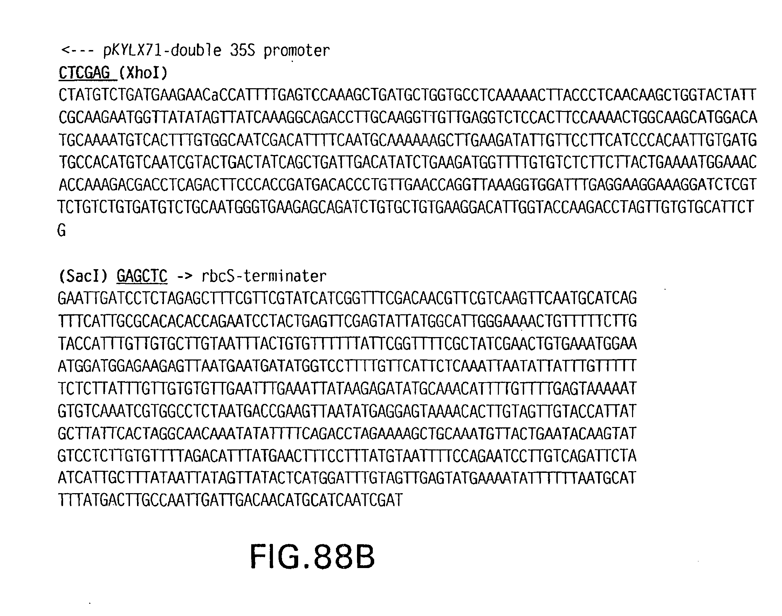

Figure 88 provides tomato senescence-induced eIF-5A and the construction of pKYLX71-sense senescence-induced eIF-5A.
Figure 89 provides photographs of a comparison of *Arabidopsis thaliana* control and transgenic plants comprising a sense polynucleotide senescence-induced eIF-5A. The transgenic plant has thicker inflorescence stems over that of the control plant.
Figures 90 and 91 shows that transgenic plants comprising an sense polynucleotide senescence-induced eIF-5A (Figure 90 - arabidopsis and figure 91 - tomato) have increased xylogenesis as indicated by the increased xylem in the transgenic plant.
Figure 92 provides photographs of a comparison of *Arabidopsis thaliana* control and *Arabidopsis thaliana* transgenic plants comprising a sense polynucleotide senescence-induced eIF-5A. A tomato sense polynucleotide senescence-induced eIF-5A was used in *Arabidopsis thaliana.* The transgenic plant has thicker inflorescence stems over that of the control plant.
Figures 93 and 94 are bar graphs that show increased xylogenesis in transgenic plants comprising a sense polynucleotide senescence-induced eIF-5A. Figure 94 concerned tomato sense polynucleotide senescence-induced eIF-5A.
Figure 95 provides canola growth eIF-5A amino acid and polynucleotide sequences.

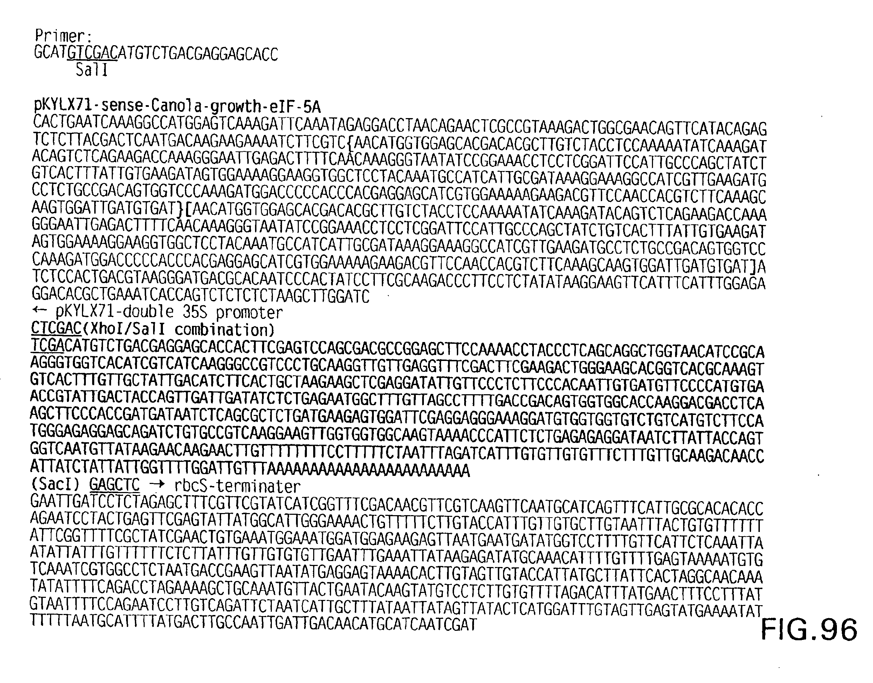

Figure 96 provides canola growth eIF-5A and the construction of pKYLX71-sense growth eIF-5A.
Figure 97 provides canola DHS amino acid and polynucleotide sequences.

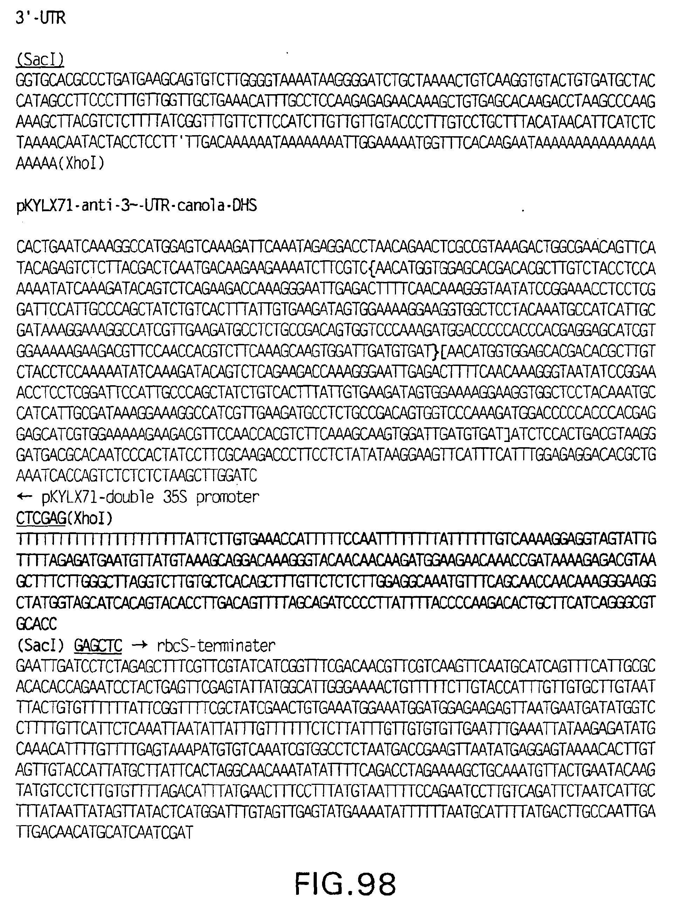

Figure 98 provides canola DHS and the construction of pKYLX71-sense DHS.
Figure 99 shows in bar graph form that inhibition of DHS expression increases seed yield in canola.
Figure 100 shows in bar graph form that up regulation of growth isoforms of eIF-5A from left to right arabidopsis, canola, tomato, and up regulation of tomato DHS.
Figure 101 provides tomato growth eIF-5A amino acid and polynucleotide sequences.

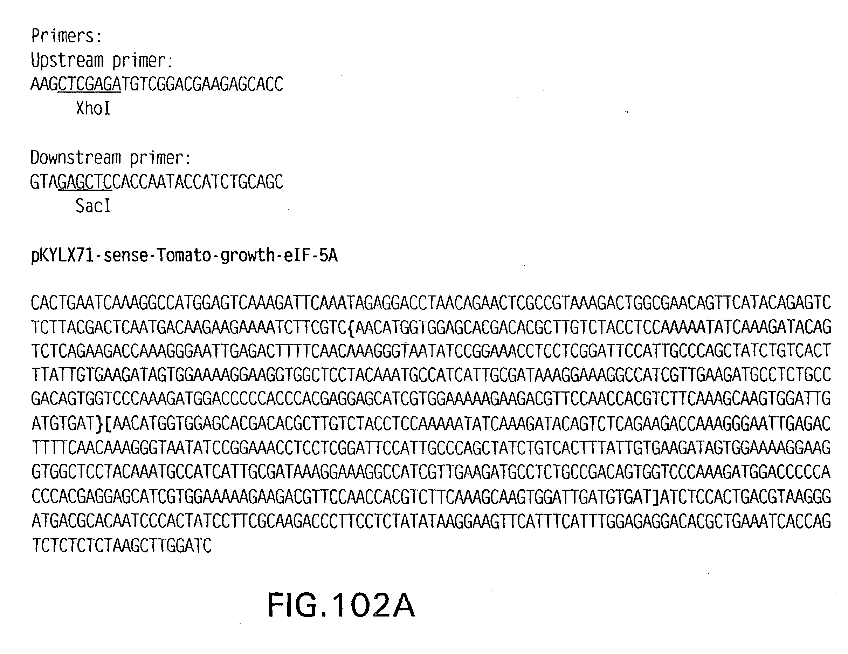

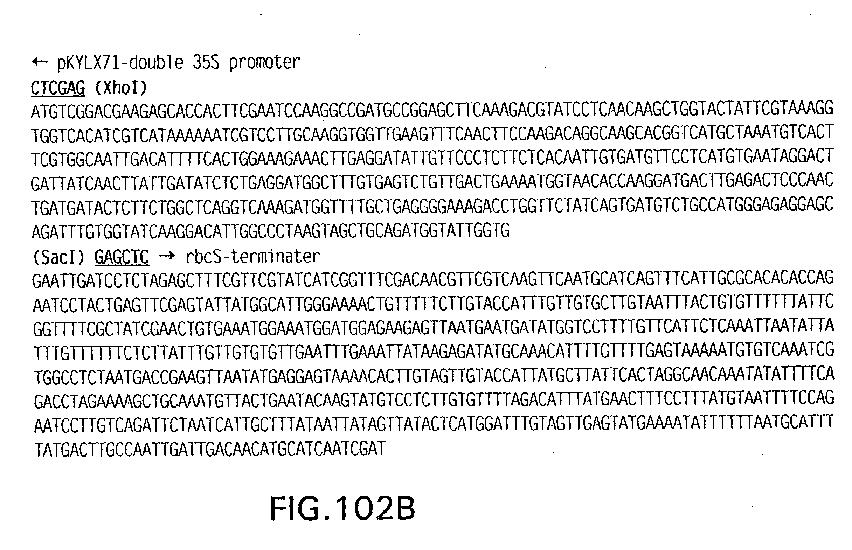

Figure 102 provides tomato growth eIF-5A and the construction of pKYLX71-sense tomato growth eIF-5A.
Figure 103 provides tomato wounding/pathogen induced eIF-5A amino acid and polynucleotide sequences.

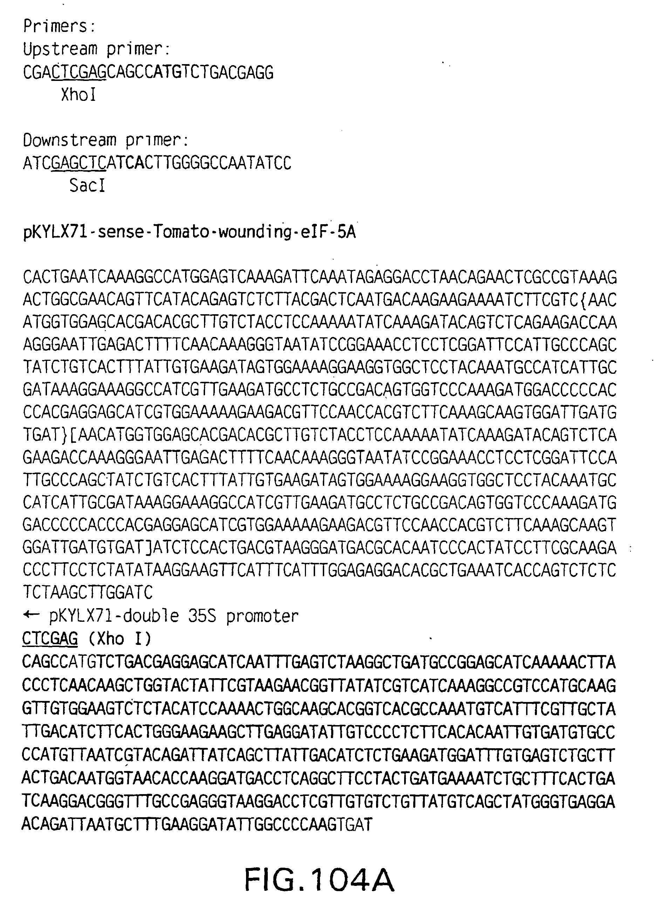

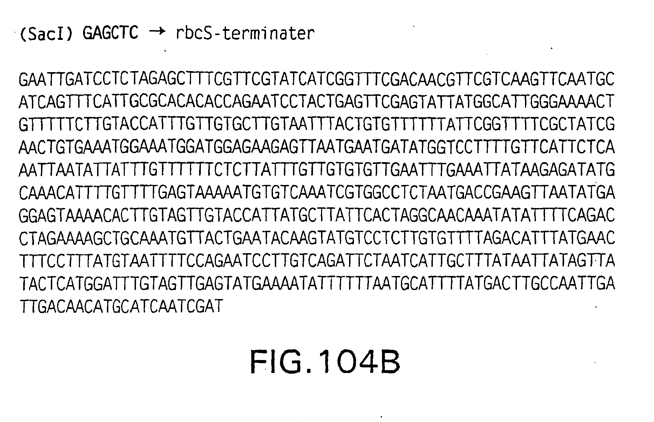

Figure 104a and b provides tomato wounding/pathogen induced eIF-5A and the construction of pKYLX71-sense tomato wounding/pathogen induced eIF-5A.
Figure 105 provides portions of lettuce DHS polynucleotide sequences.

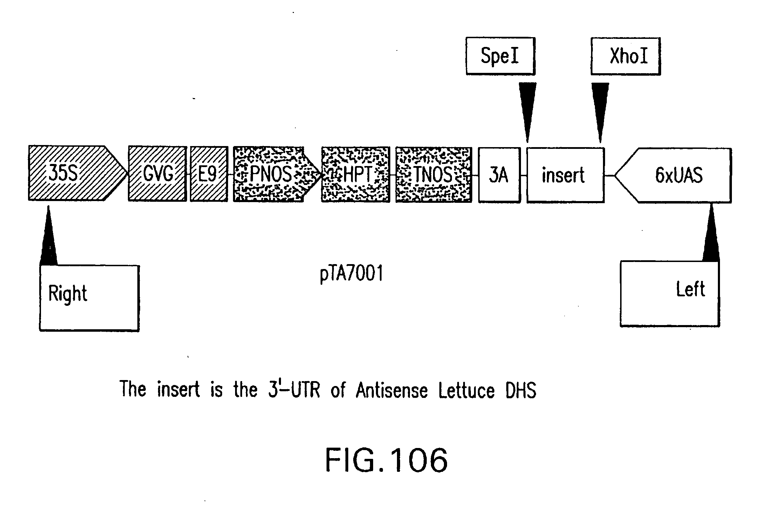

Figure 106 provides the construct of pTA7001-3'UTR antisense lettuce DHS.
Figure 107A and B provides alfalfa DHS amino acid and polynucleotide sequences.
Figure 108 A and B provides banana DHS amino acid and polynucleotide sequences.
Figure 109 A and B provides cottonwood DHS amino acid and polynucleotide sequences.
Figure 110 provides partial mycosphaerella fijiensis DHS amino acid and polynucleotide sequences.
Figure 111 shows a schematic of the activation of eIF-5A.
Figure 112 is a Northern analysis of carnation aging and expression of eIF-5A.
Figure 113 shows that transgenic carnations (antisense senescence-induced eIF-5A) show longer shelf life of cut flowers.
Figure 114 shows delayed spoilage of bananas (transgenic plants with antisense DHS).
Figure 115 shows delayed spoilage of tomatoes (transgenic plants with antisense DHS).
Figure 116 shows expression of *arabidopsis* senescence-induced eIF-5A (AteIF-5A1) in cotyledons, leaves and flowers.
Figure 117 shows delayed senescence of Arabidopsis leaves in transgenic *arabidopsis* (*arabidopsis* antisense senescence-induced eIF-5A)(AteIF-5Al).
Figure 118 shows expression of *arabidopsis* senescence-induced eIF-5A::GUS in first true leaf pair.
Figure 119 shows transgenic plants (antisense senescence-induced eIF-5A) have less xylem than wild type plants.
Figure 120 shows enhanced xylem formation in transgenic plants (sense constructs of senescence-induced eIF-5A).
Figure 121 shows resistance to drought in transgenic plants (reduced expression of stress-induced eIF-5A through antisense constructs).
Figure 122 is a bar graph showing survival in wild type and transgenic Arabidopsis under drought stress. Transgenic plants (reduced expression of stress-induced eIF-5A through antisense constructs) have increased survival.
Figure 123 shows a comparison of wild type canola and transgenic canola (reduced expression of stress-induced eIF-5A through antisense constructs) and shows that transgenic canola has an increased biomass.
Figure 124 shows transgenic canola lines (reduced expression of stress-induced eIF-5A through antisense constructs) have an increase in seed yield over wild type plants.
Figure 125 indicates that wounding/pathogen induced eIF-5A is up-regulated after infection with *P. syringae.*
Figure 126 shows that antisense suppression of wounding/pathogen-induced isoform of eIF-5A inhibits infection by virulent pathogens. Transgenic plants showed a greater than 99% increase in an inhibition of infection over control plants.
Figures 127-130 show that transgenic canola (antisense wounding/pathogen-induced isoform of eIF-5A) shows a resistance to infection by *Sclerotinia sclerotiorum.* "WT" is a leaf from a wild type plant. "TG-1" and "TG-5" are leaves from transgenic plants.
Figure 131 shows a photograph of transgenic bananas (antisense DHS)(left side) and wild type bananas (right side) and shows that transgenic banana plants have a larger biomass than the wild type control.
Figure 132 shows transgenic tomatoes (growth eIF-5A in sense orientation)(left side) have an increased biomass over wild type control plants (right side).
Figure 133 shows transgenic canola (growth eIF-5A in sense orientation) have an increased biomass over wild type control plants.
Figure 134 shows transgenic canola (growth eIF-5A in sense orientation) have an increased seed yield over wild type control plants.

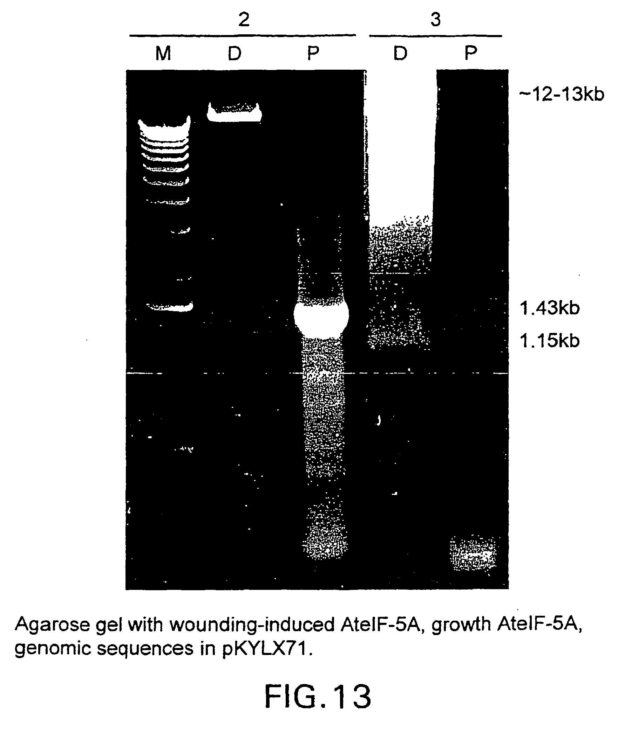

Figure 13 5 provides lettuce DHS sequences. The nucleic acid is SEQ ID NO: __ and the amino acid sequence is SEQ ID NO:__.

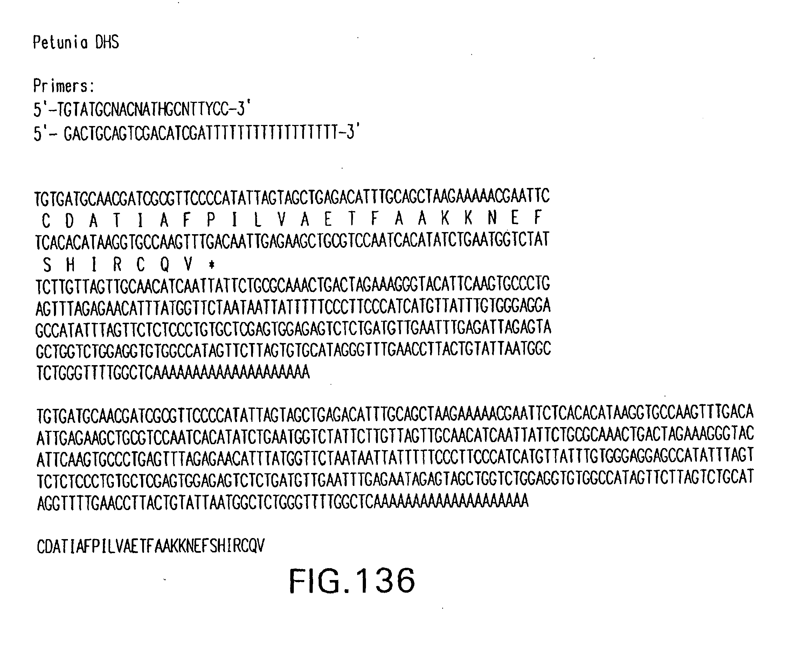

Figure 136 provides a partial petunia DHS nucleotide (SEQ ID NO: _) and amino acid sequence (SEQ ID NO:__) as well as two primers used to isolate the DHS sequences (SEQ ID NOs:__)

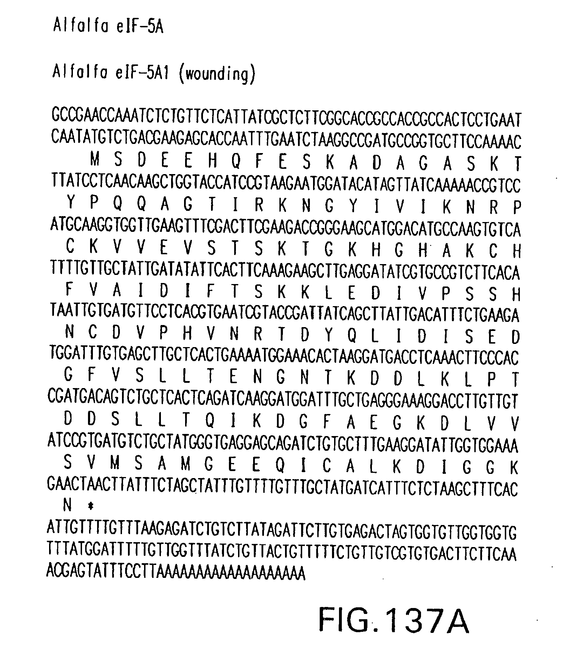

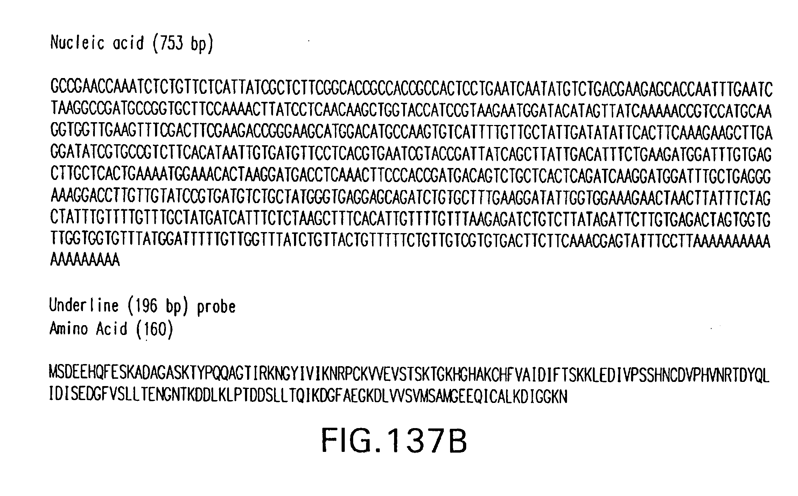

Figure 137 provides alfalfa nucleic acid sequence for wounding/pathogen eIF-5A (SEQ ID NO:__) and the amino acid sequence (SEQ ID NO:__).

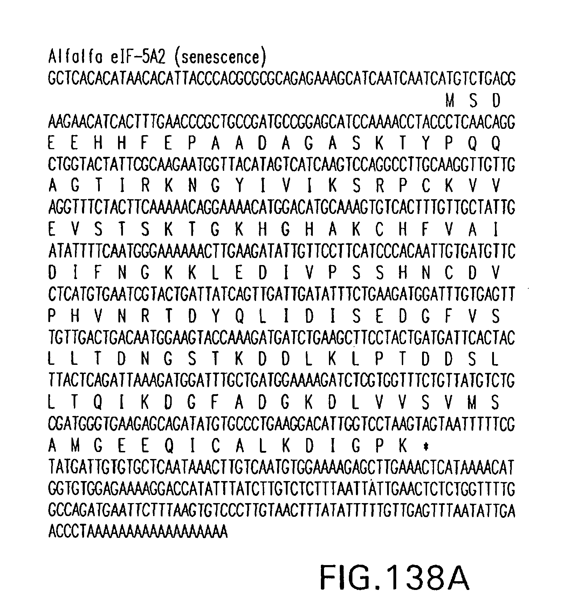

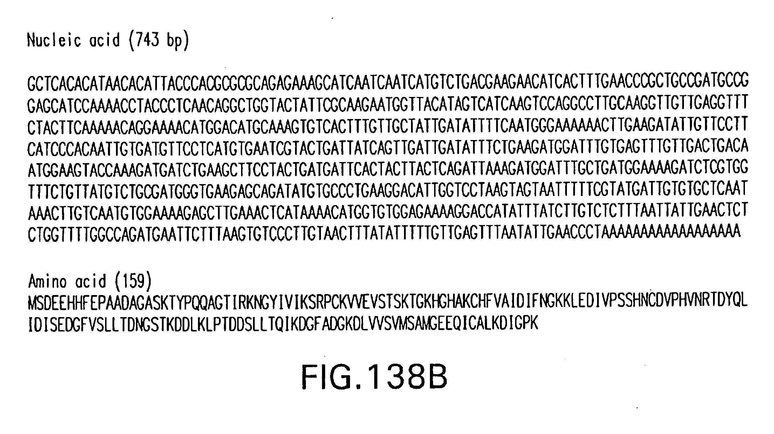

Figure 138 provides alfalfa nucleic acid sequence for senescence eIF-5A (SEQ ID NO:_) and the amino acid sequence (SEQ ID NO:_).

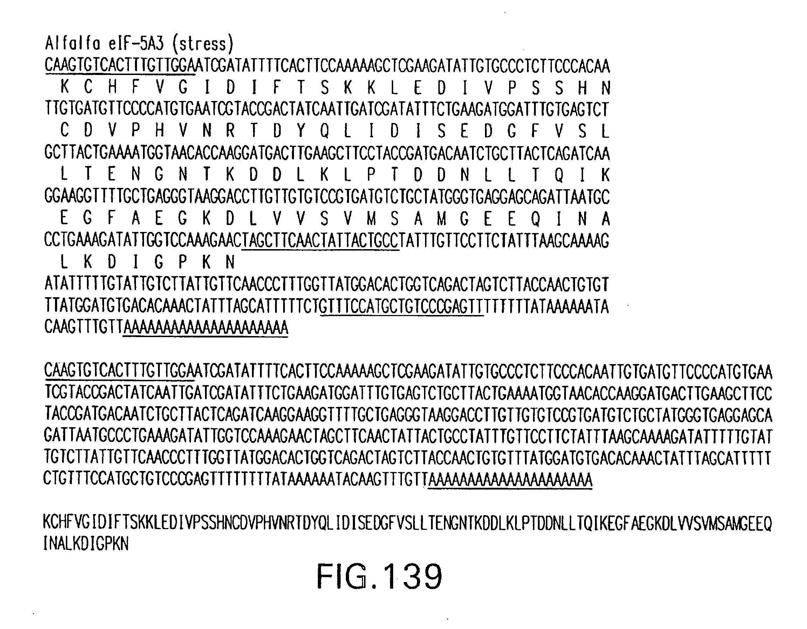

Figure 139 provides alfalfa nucleic acid sequence for stress eIF-5A (SEQ ID NO:_) and the amino acid sequence (SEQ ID NO:_).

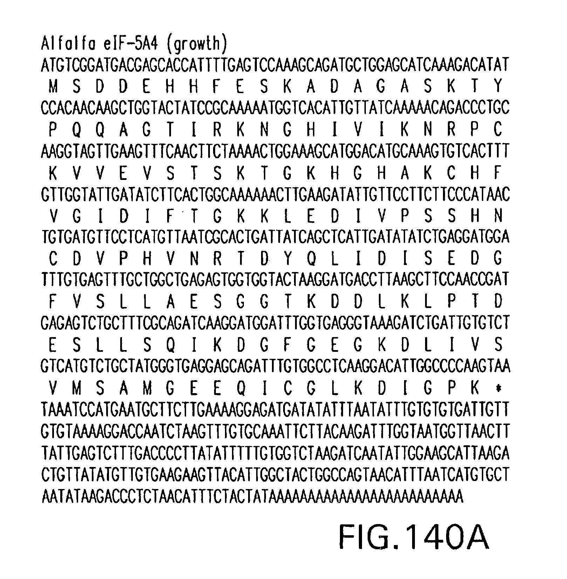

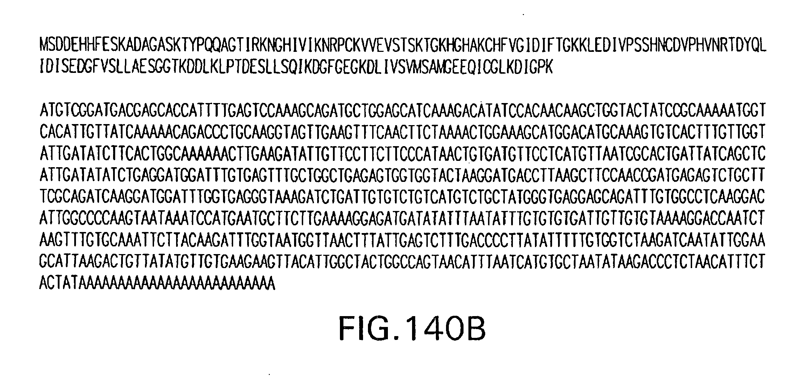

Figure 140 provides alfalfa nucleic acid sequence for growth eIF-5A (SEQ ID NO:_) and the amino acid sequence (SEQ ID NO:_).

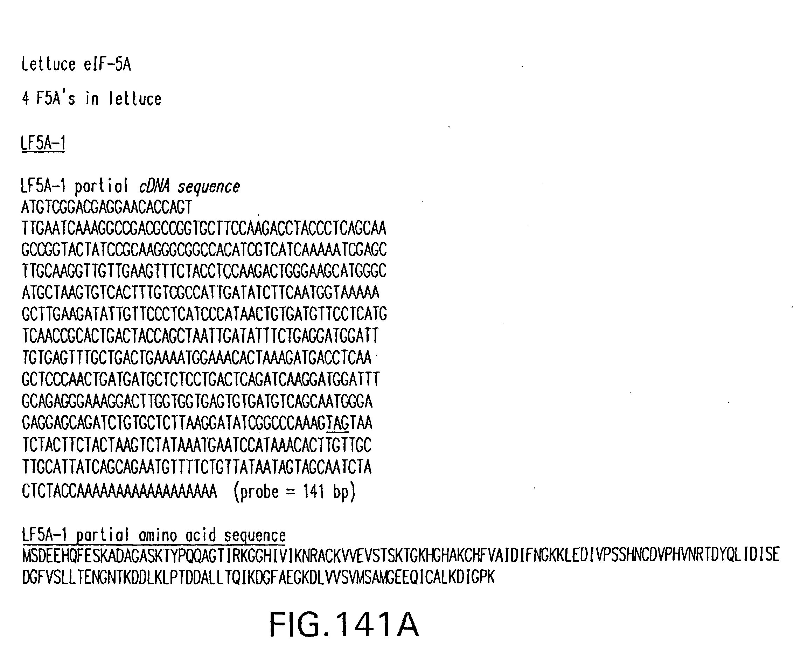

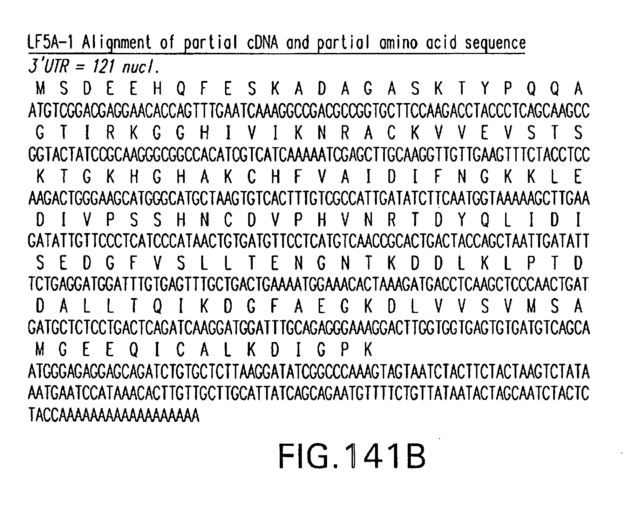

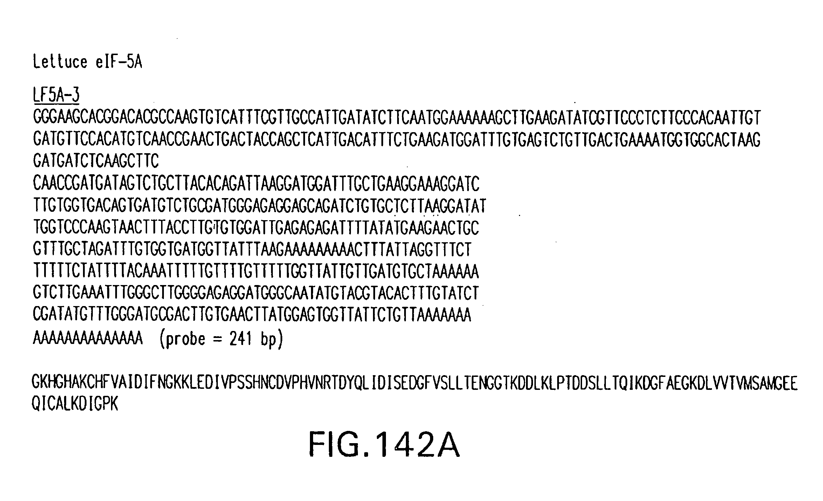

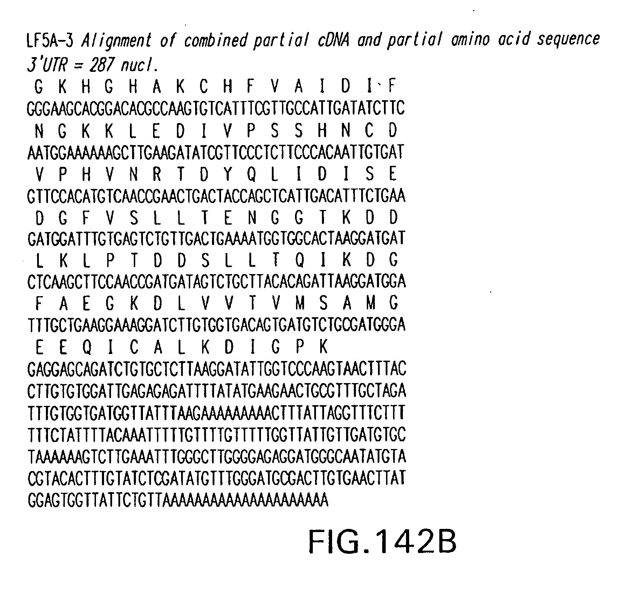

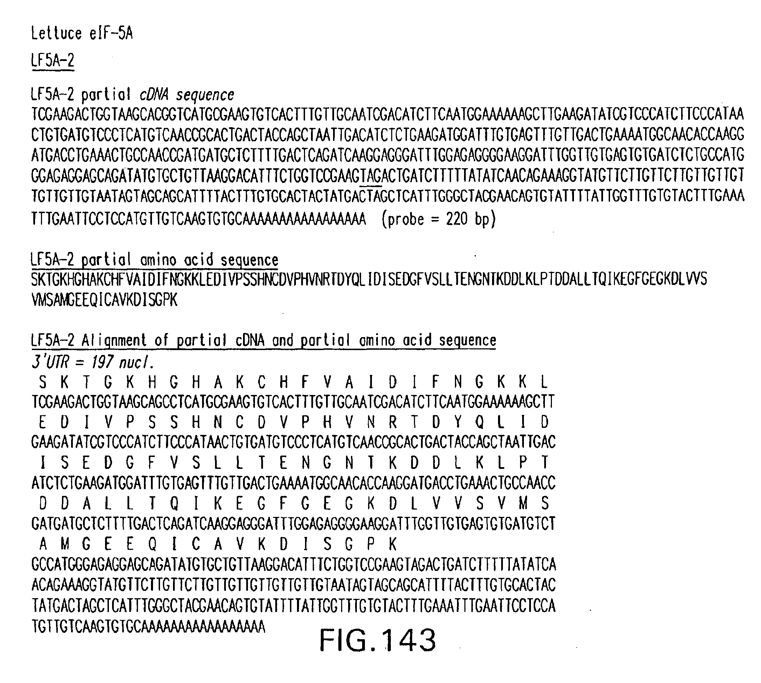

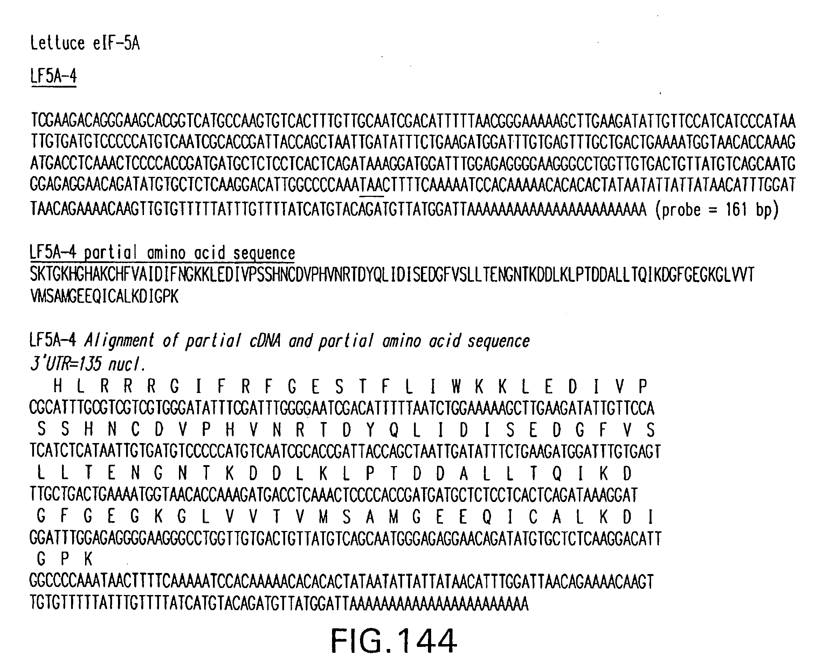

Figures 141-144 provide partial sequences for different isoforms of eIF-5A in lettuce (LF5A-1, LF5A-3, LFA-2, and LFA-1).

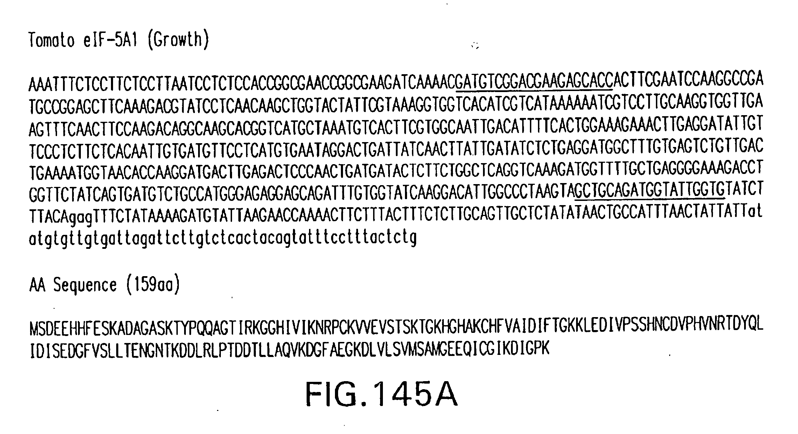

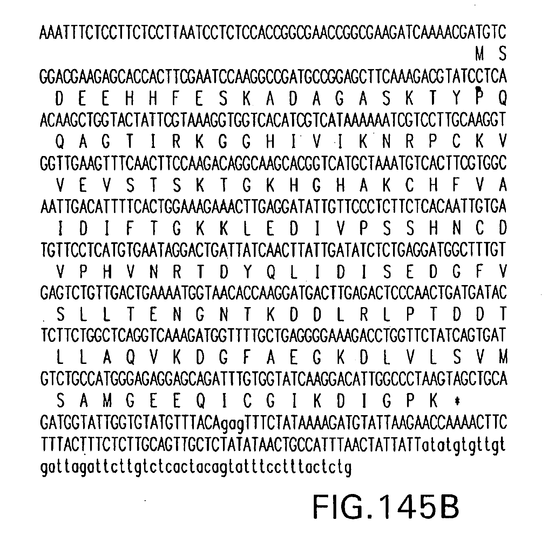

Figure 145 provides the sequences for growth eIF-5A sequences for tomato (nucleic acid - SEQ ID NO:__) and amino acid (SEQ ID NO: __).

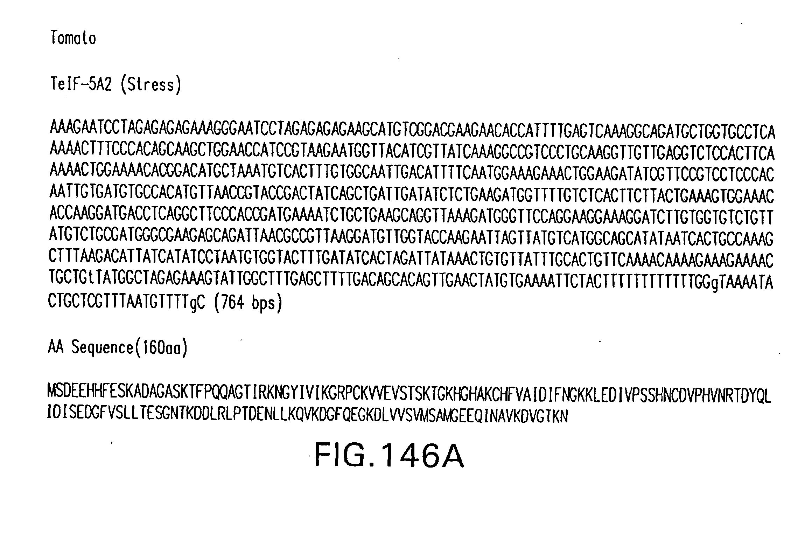

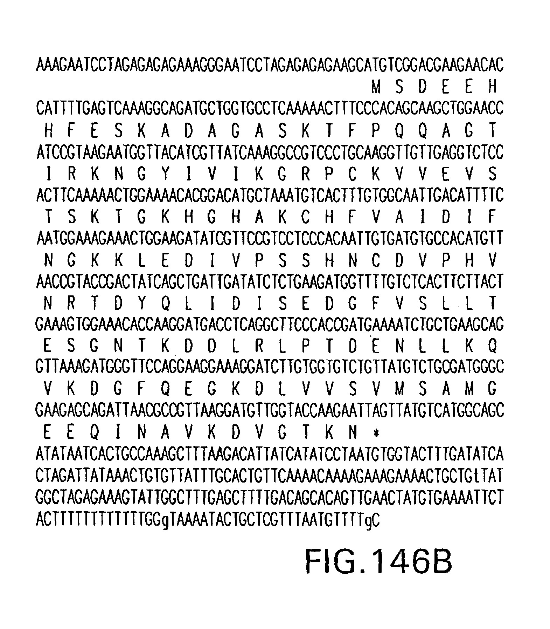

Figure 146 provides the sequences for stress eIF-5A sequences in tomato (nucleic acid - SEQ ID NO:__) and amino acid (SEQ ID NO: __).

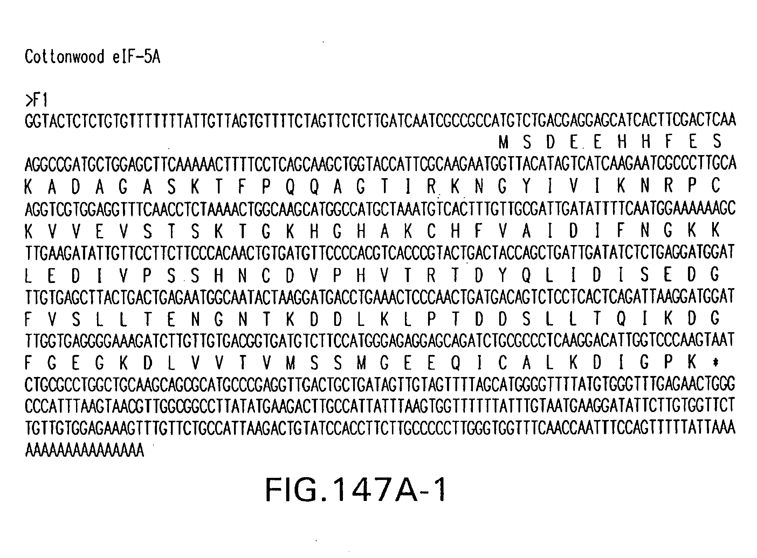

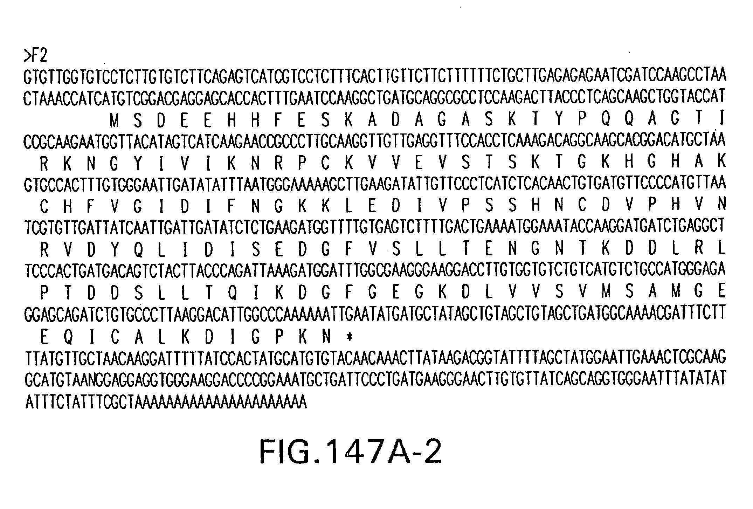

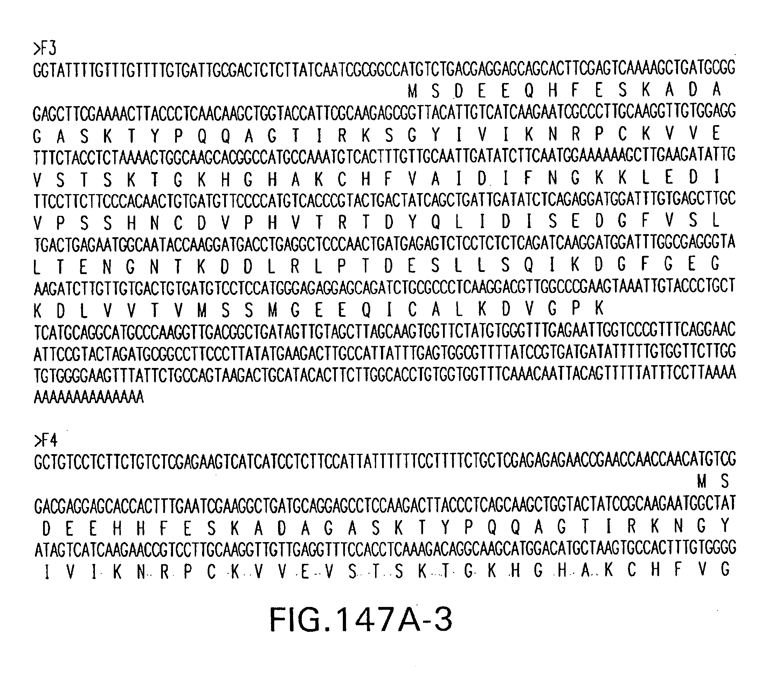

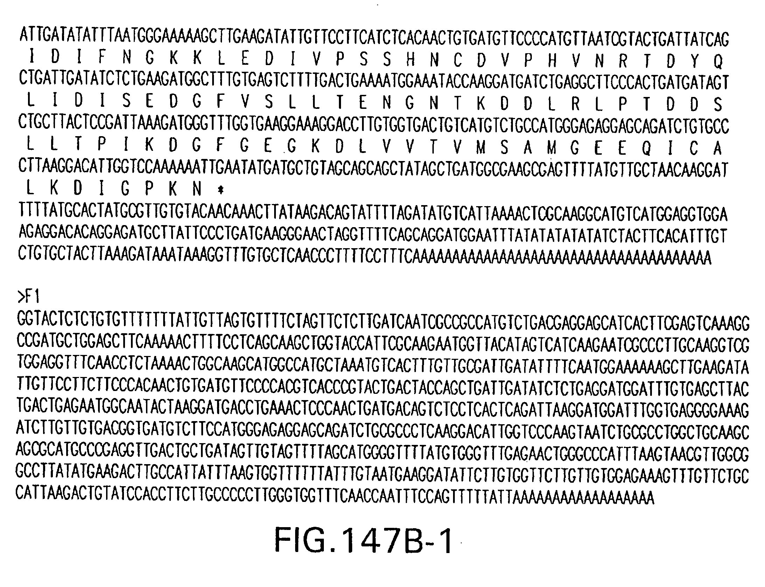

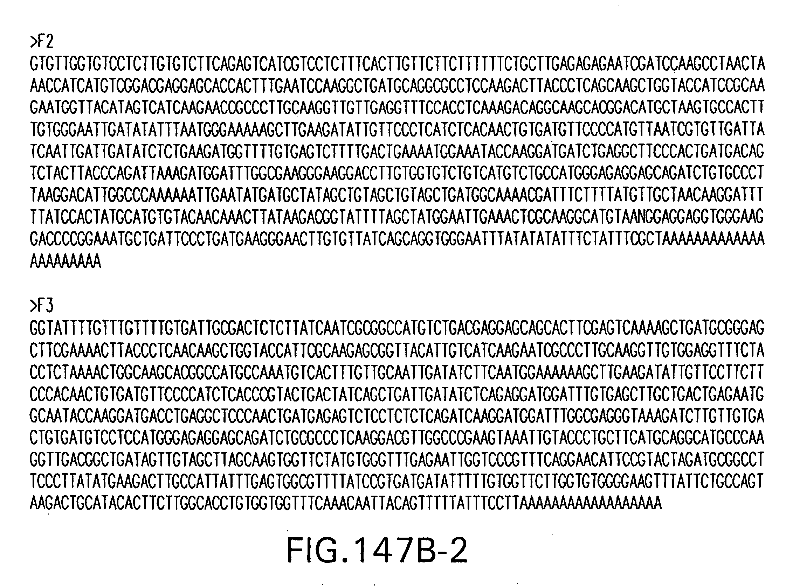

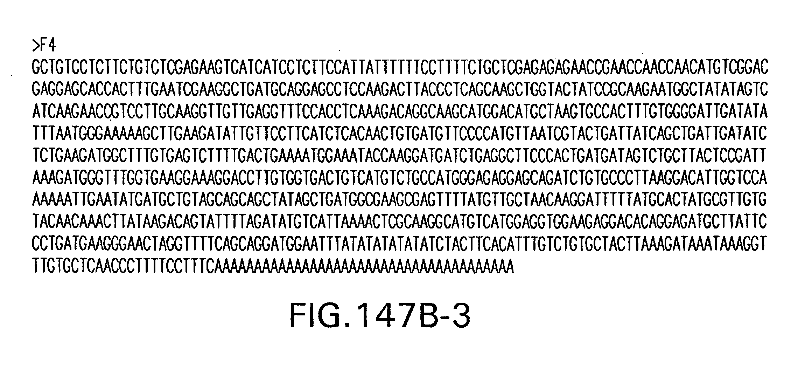

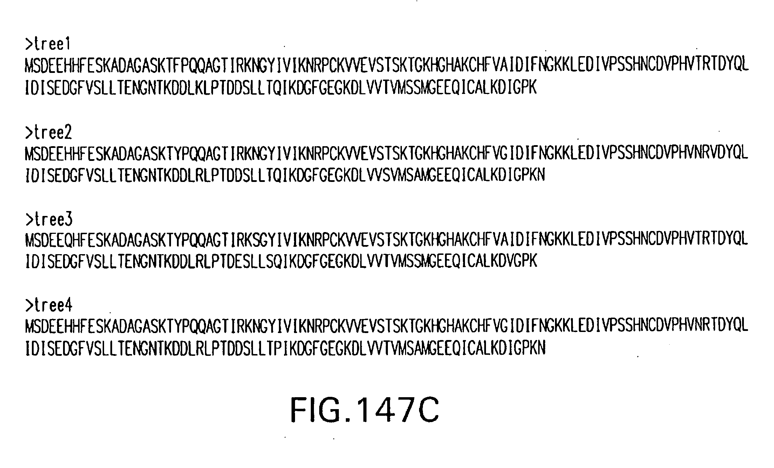

Figures 147A- C provides nucleic acid and amino acid eIF-5A seqeunces isolated from four eIF-5A from cottonwood trees ("F1," "F3," "F3" and "F4").

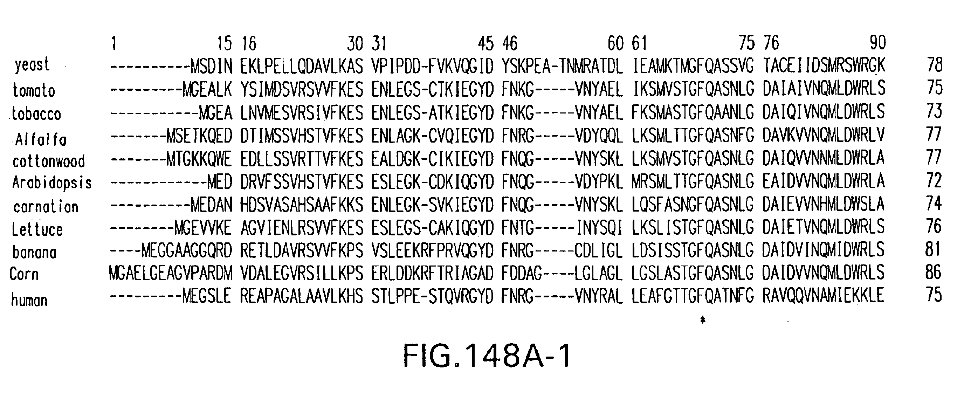

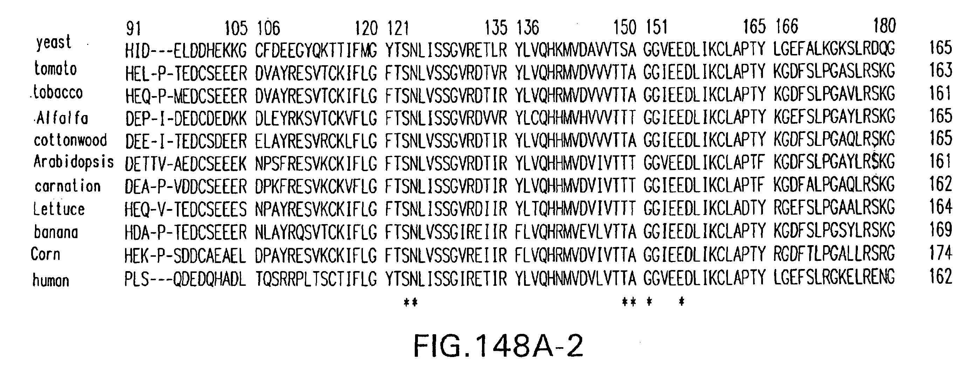

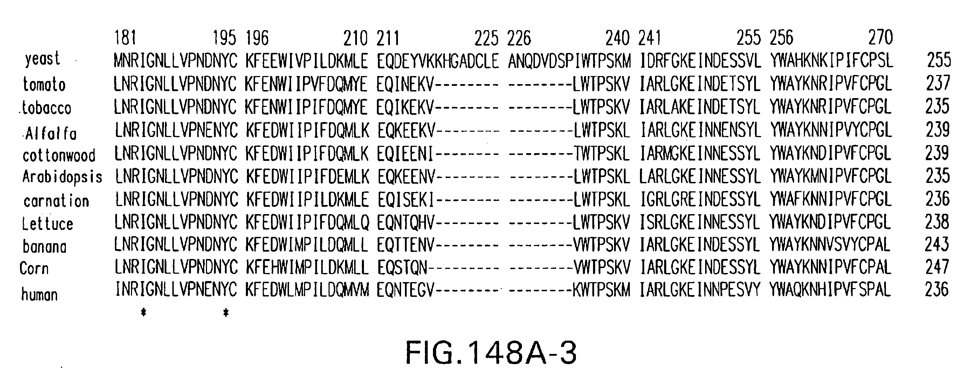

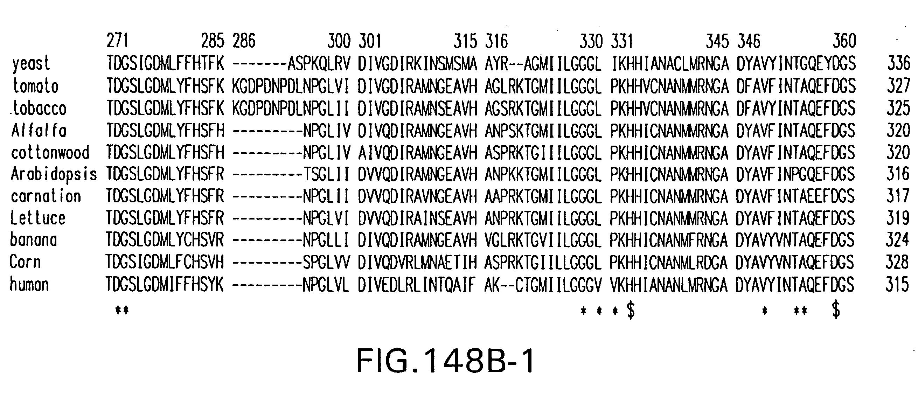

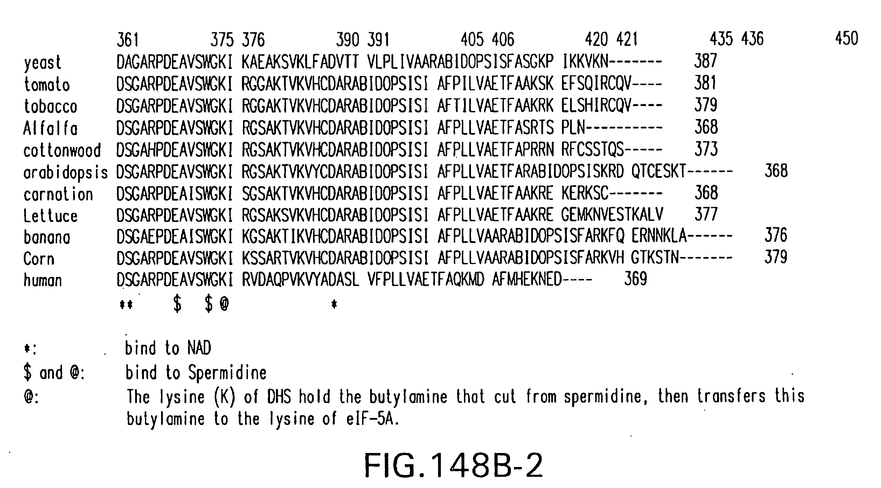

Figure 148 (a) and (b) provides an amino acid alignment of the various DHS showing the highly conserved nature of DHS across species.

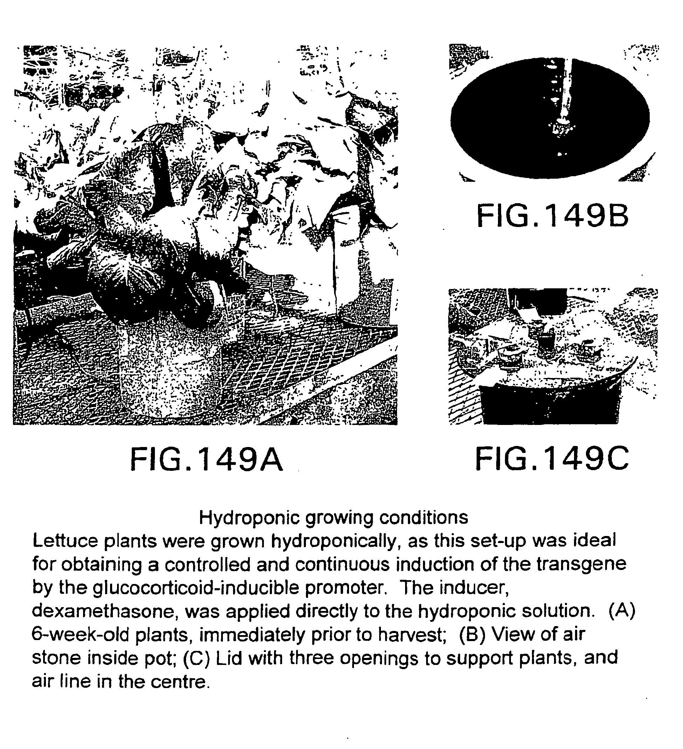

Figure 149 shows the hydroponic conditions used for growing lettuce having a down-regulation of DHS.

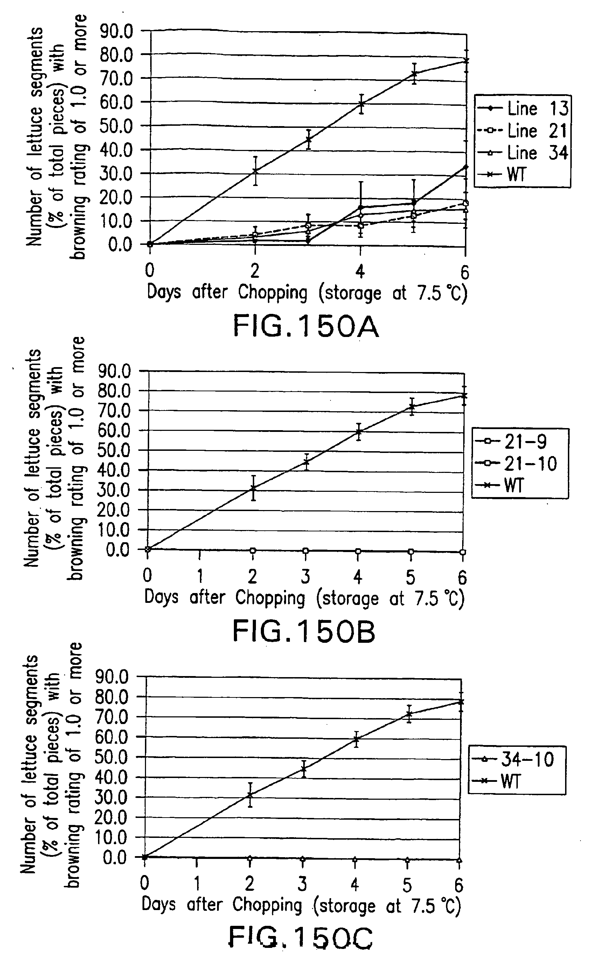

Figure 150 shows delayed browning in cut lettuce having expression of lettuce DHS down-regulated.

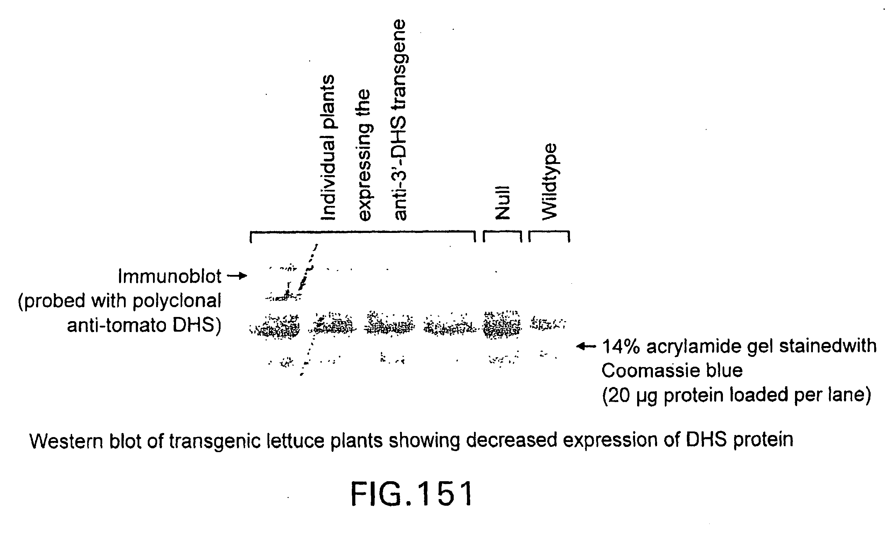

Figure 151 shows a Western blot indicating that transgenic lettuce having antisense 3'end of lettuce DHS exhibit a decreased expression of DHS.

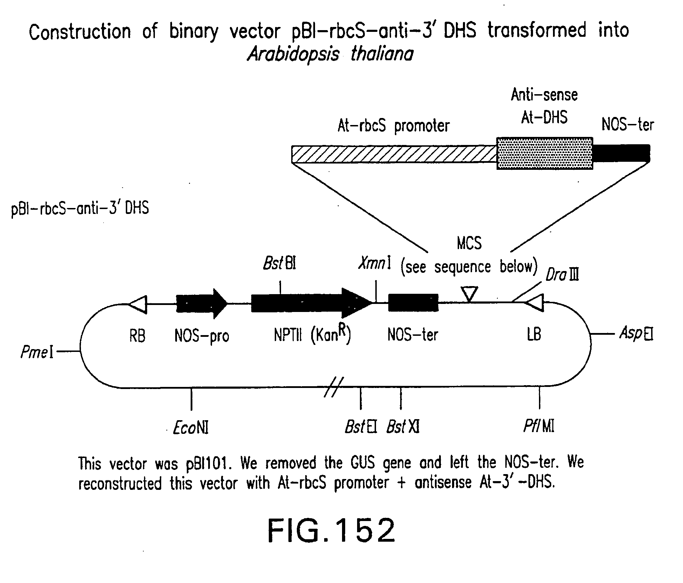

Figure 152 shows a vector constructed used to transform Arabidposis with antisense 3'end of DHS.

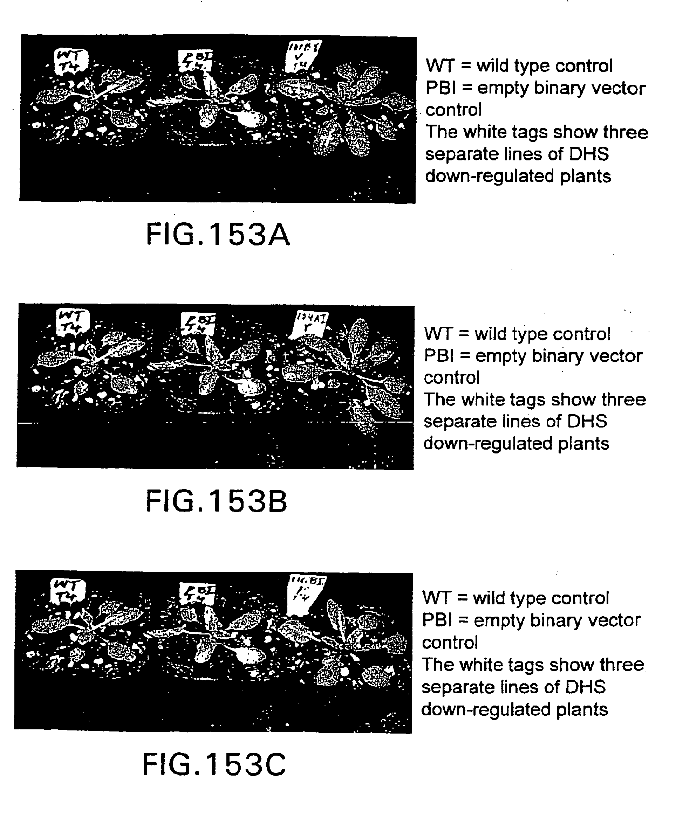

Figure 153 shows arabidopsis plants having DHS down regulated have an increased growth rate/biomass over wild type plants (transgenic arabidopsis generated with the vector shown in figure 152).

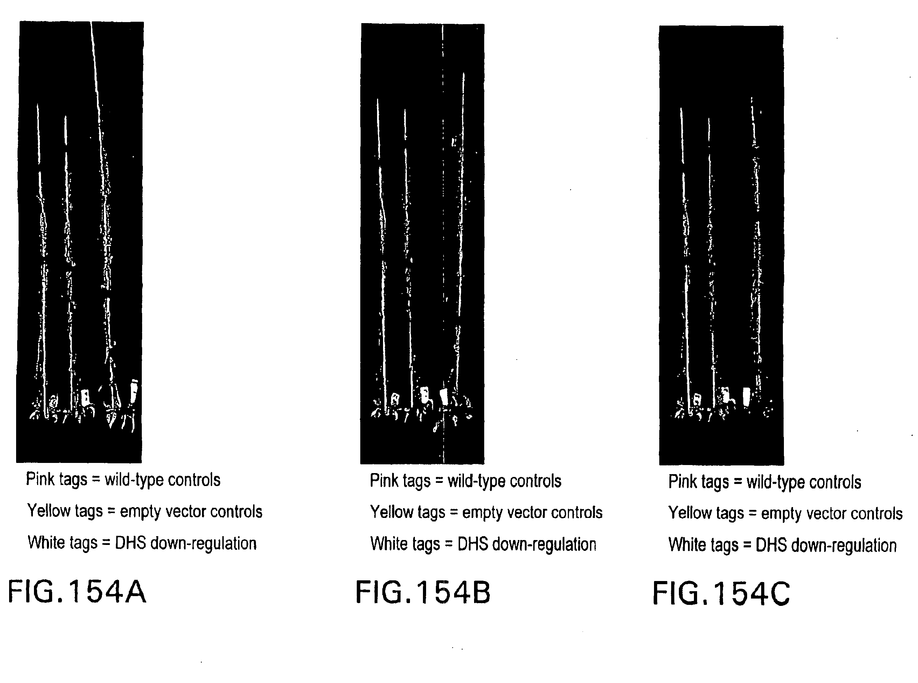

Figure 154 shows arabidopsis plants having DHS down regulated have an increased growth rate/biomass over wild type plants (transgenic arabidopsis generated with the vector shown in figure 152).

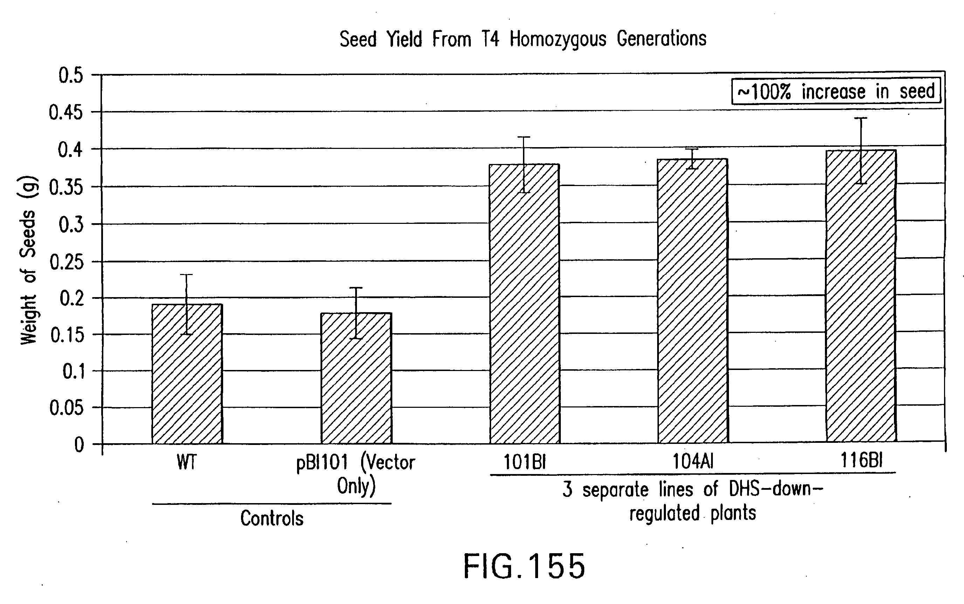

Figure 155 shows arabidopsis plants having DHS down regulated have an increased seed yield over wild type plants (transgenic arabidopsis generated with the vector shown in figure 152).

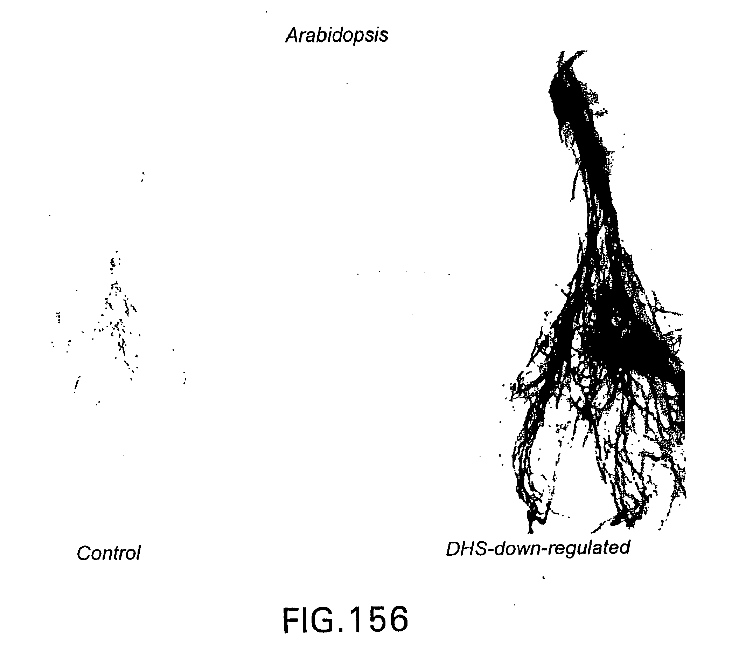

Figure 156 shows arabidopsis plants having DHS down regulated have an increased root development over wild type plants (transgenic arabidopsis generated with the vector shown in figure 152).

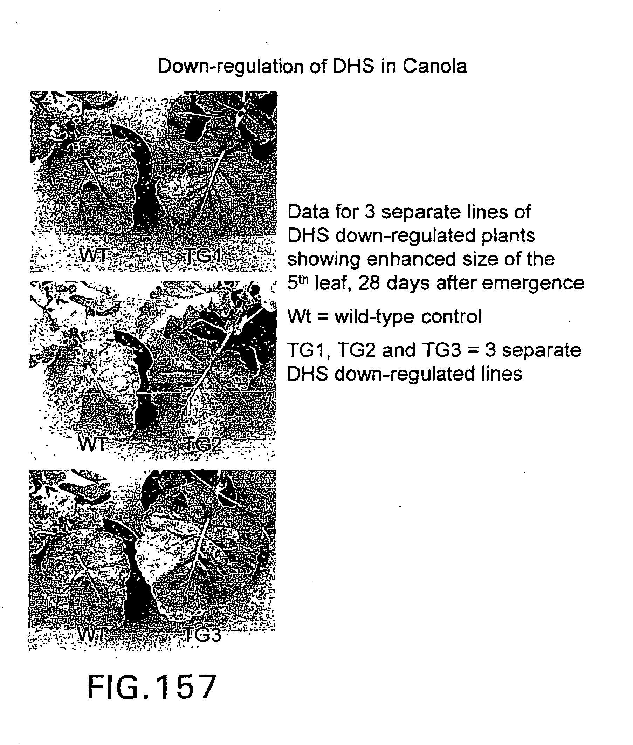

Figure 157 shows canola plants having DHS down regulated have an increased growth rate/biomass over wild type plants.

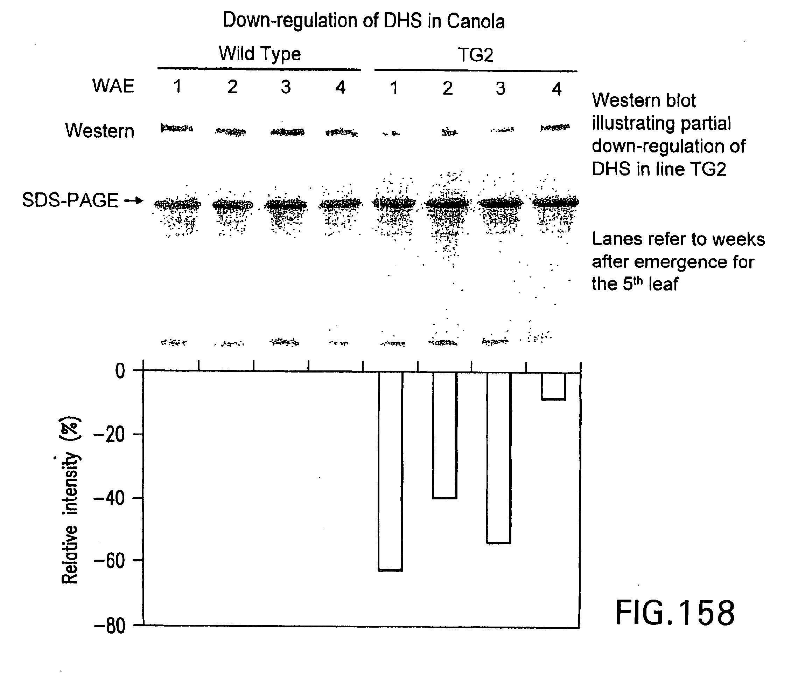

Figure 158 shows a Western blot indicating that antisense DHS has caused a decreased expression of DHS.

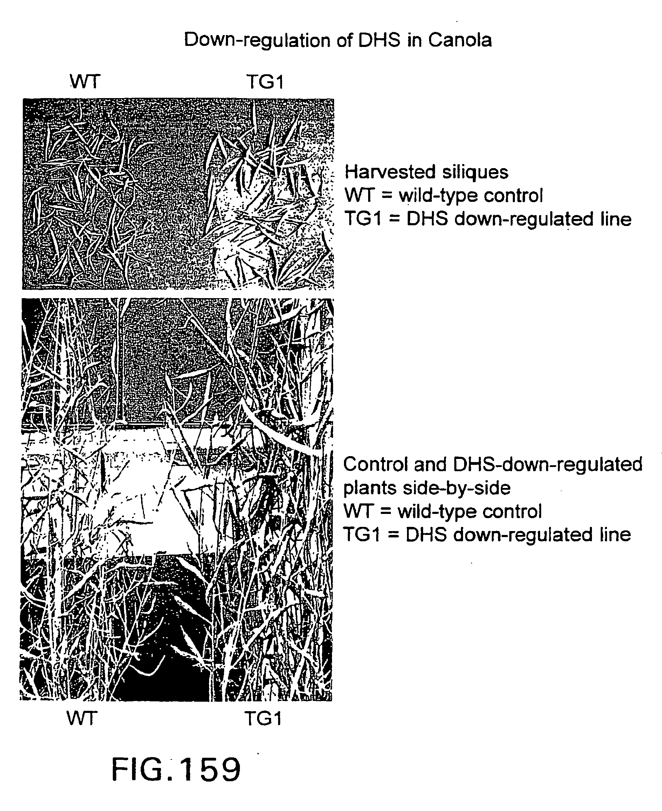

Figure 159 shows canola plants having DHS down regulated have an increase in silique size over wild type plants.

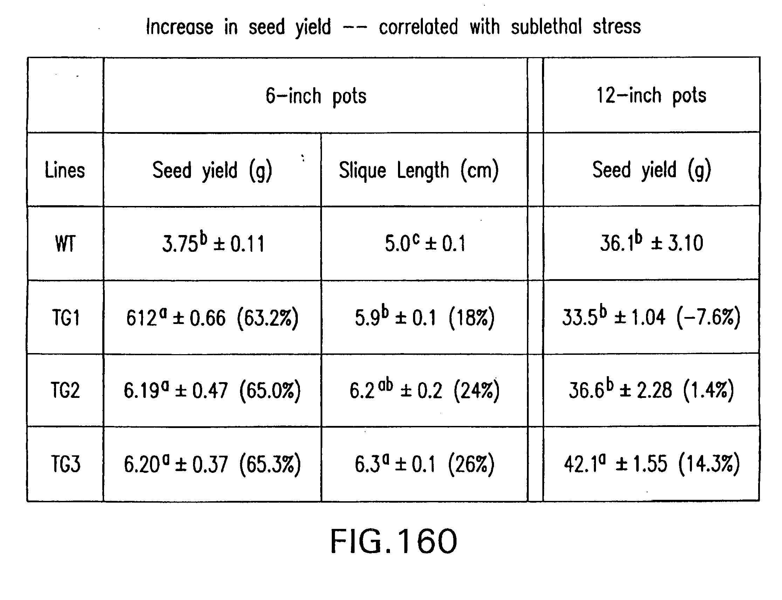

Figure 160 shows canola plants having DHS down regulated have an increase in seed yield over wild type plants.

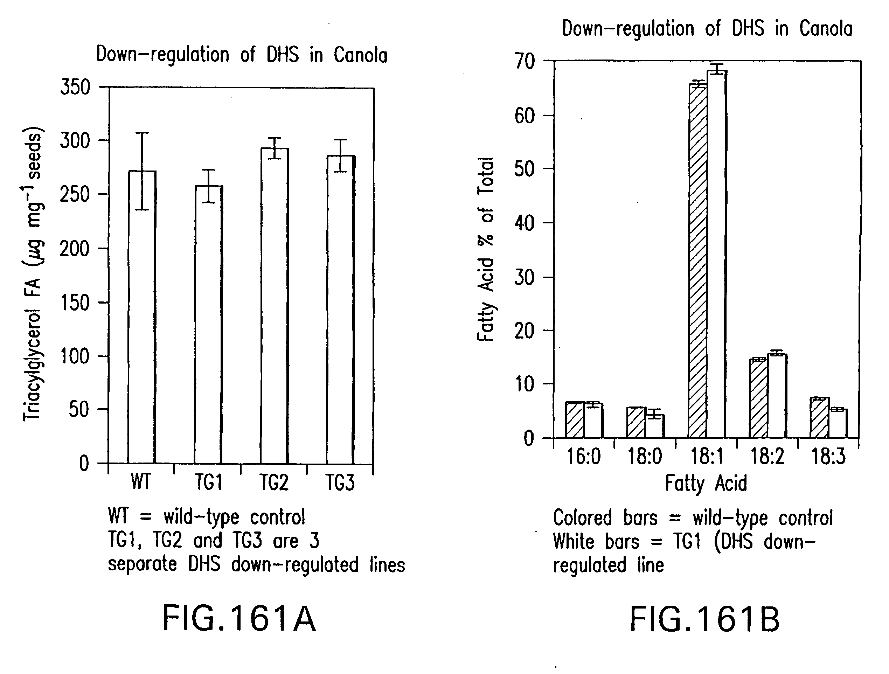

Figures 161 (a) and (b) shows that in canola plants having DHS down regulated, there is no change in the quantity of oil per seed and no change is the fatty acid composition of the oil.

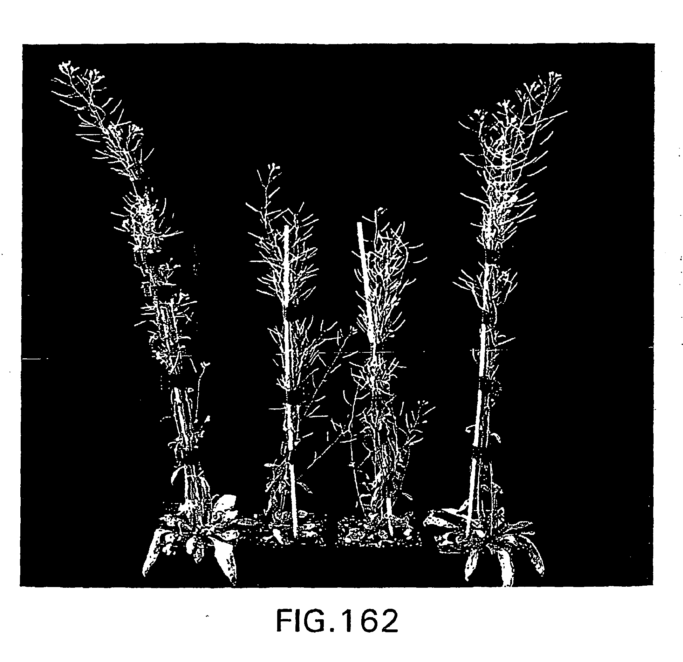

Figure 162 shows the effect of introducing cottonwood growth eIF-5A into arabidopsis in sense orientation to achieve up-regulation of growth eIF-5A. The resulting plants have an increased growth rate/biomass.

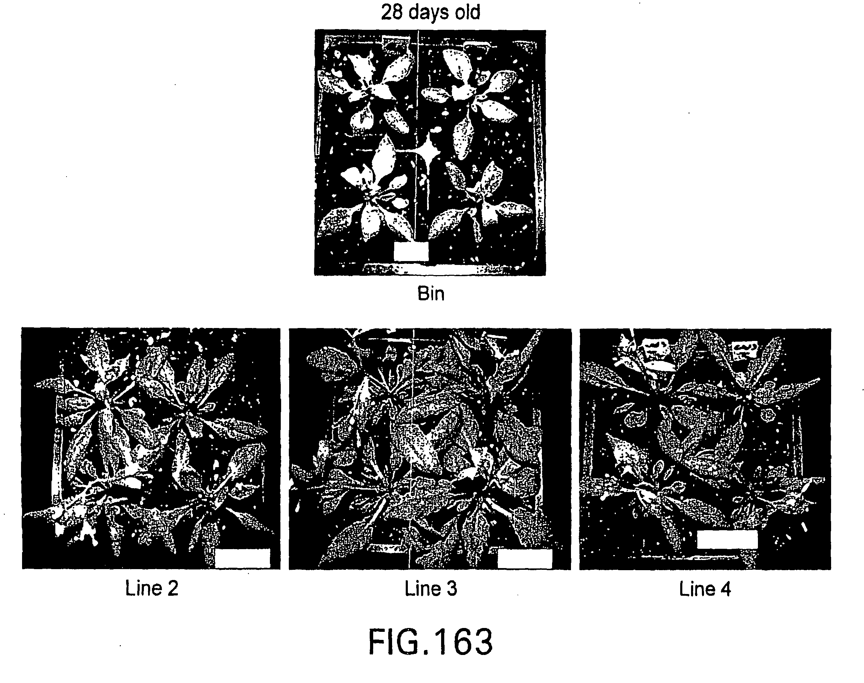

Figure 163 shows the effect of introducing cottonwood growth eIF-5A into arabidopsis in sense orientation to achieve up-regulation of growth eIF-5A. The resulting plants have an increased growth rate/biomass.

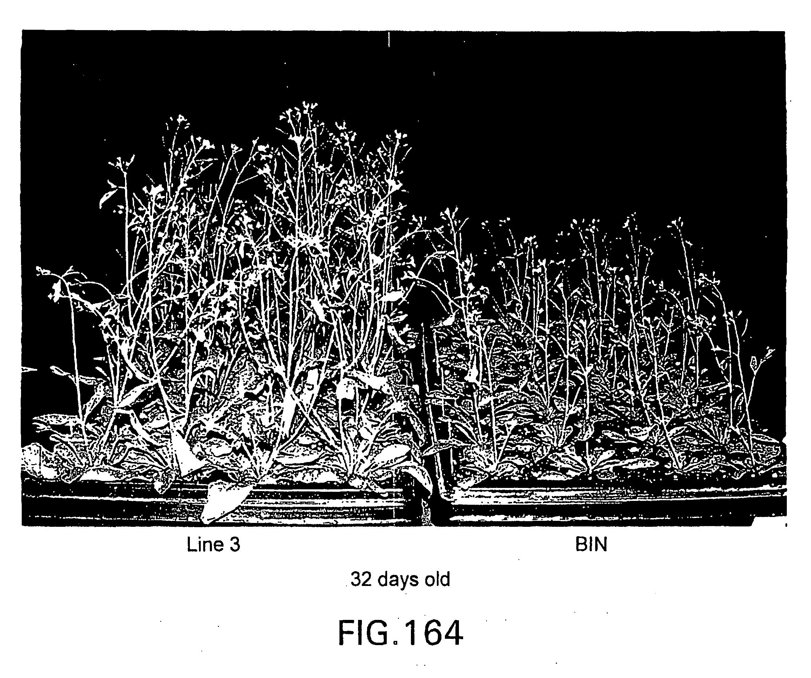

Figure 164 shows the effect of introducing cottonwood growth eIF-5A into arabidopsis in sense orientation to achieve up-regulation of growth eIF-5A. The resulting plants have an increased growth rate/biomass.

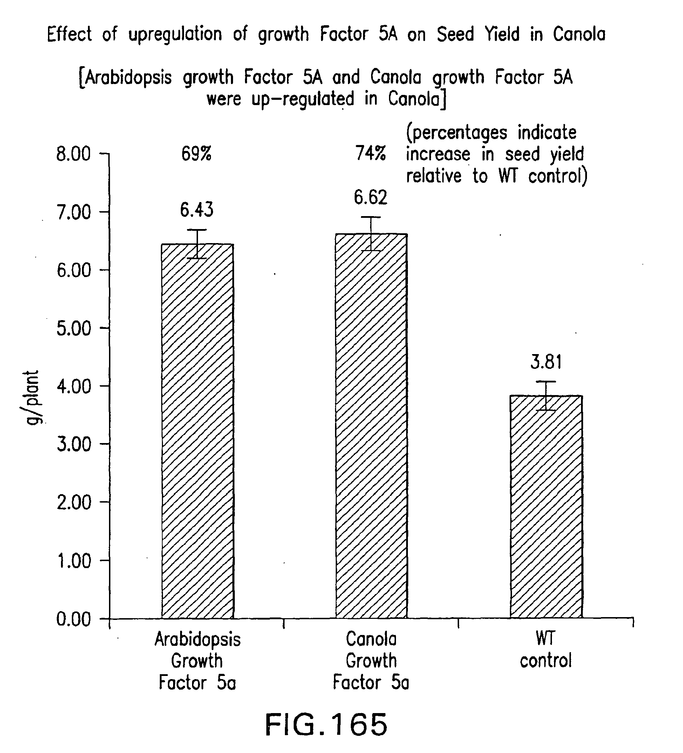

Figure 165 show that there is an increase in seed yield in canola plants when either a canola or arabisopsis growth eIF-5A is used in sense orientation in canola plants.

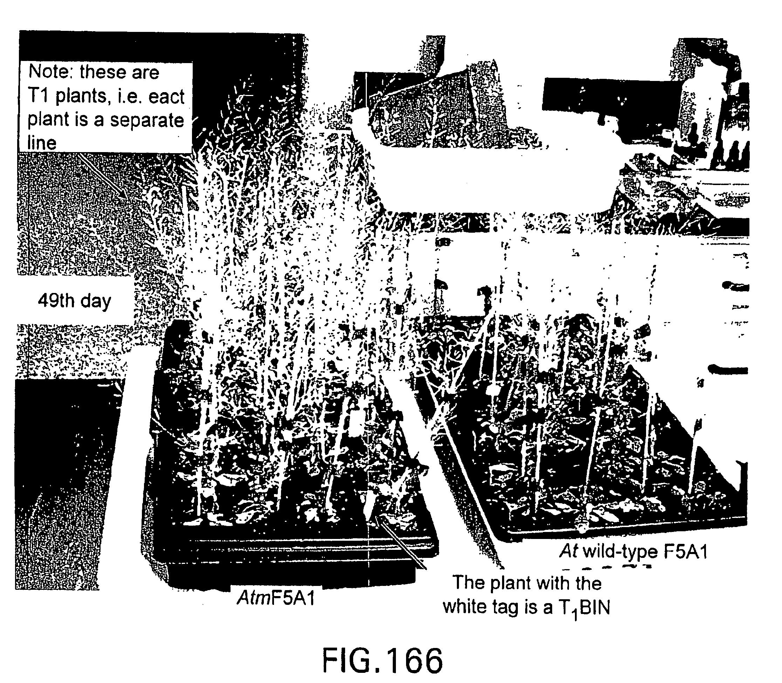

Figure 166 shows that a transgenic arabidopsis having been transformed with a mutant senescence eIF-5A (not able to be hypusinated) has increased biomass over wild type arabidopsis.

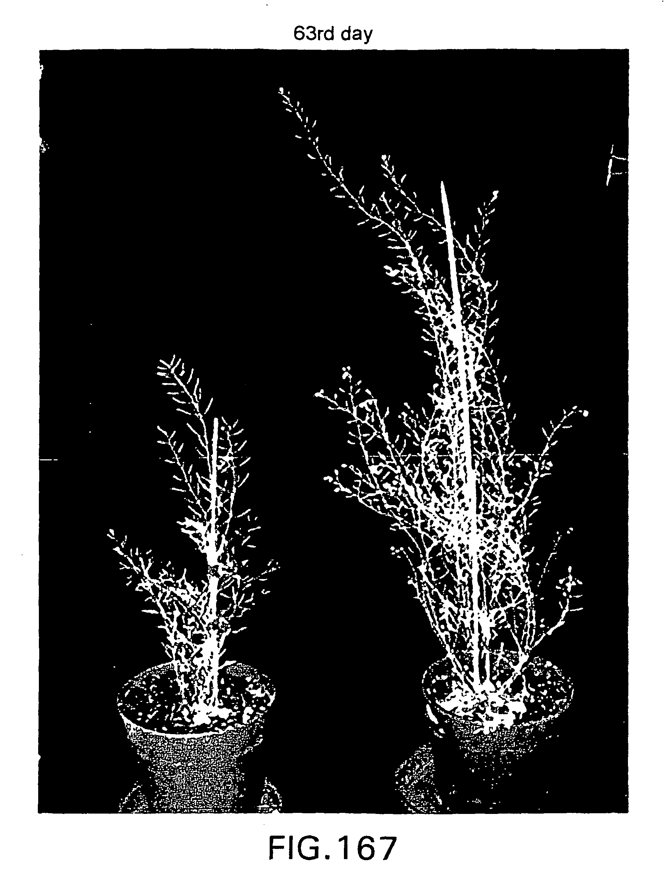

Figure 167 shows that a transgenic arabidopsis having been transformed with a mutant senescence eIF-5A (not able to be hypusinated) has increased biomass over wild type arabidopsis.

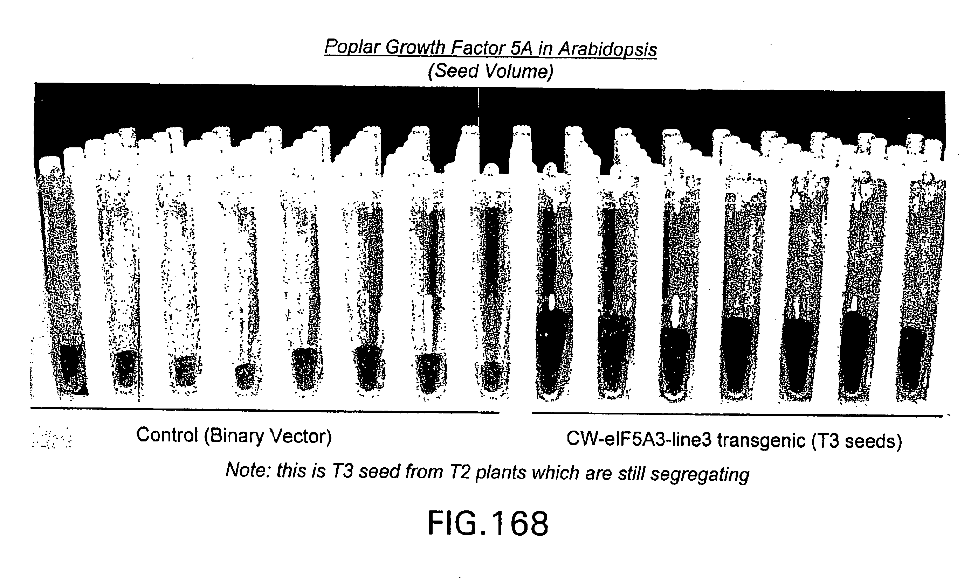

Figure 168 shows that growth eIF5A isolated from cottonwood (populas deltoides) expressed in sense orientation in arabidopsis, results in an increase in seed yield over the control plants.

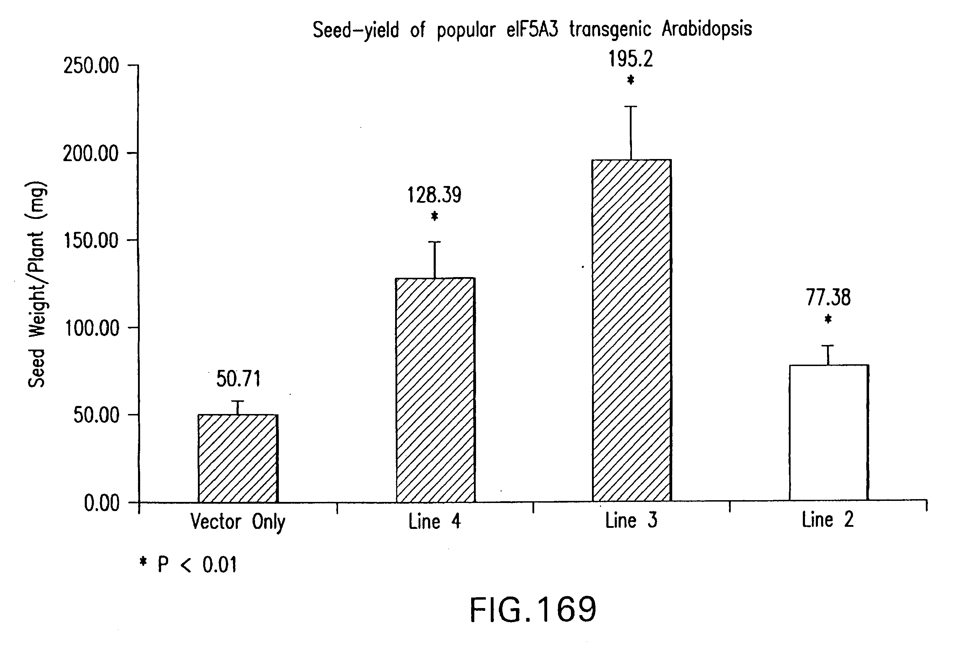

Figure 169 compares the seed yield from three different transgenic arabidopsis lines having cottonwood growth factor eIF5A expressed in sense orientation, showing that the three lines all had an increase of seed yield.

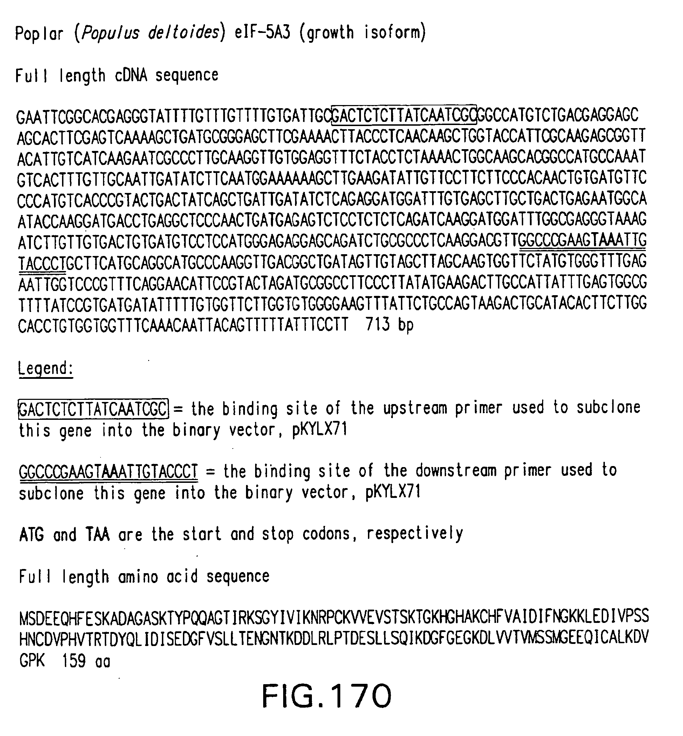

Figure 170 provides the full length cDNA of populus deltoides (cottonwood) growth eIF-5A and the full length amino acid sequence of the protein.

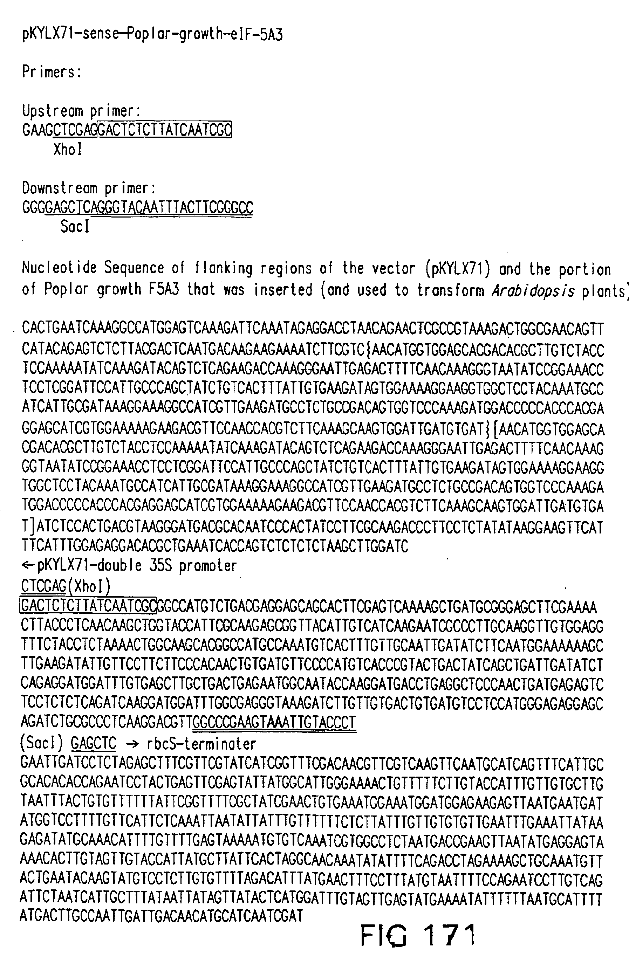

Figure 171 provides the vector construct used to transform arabidopsis with cottonwood growth eIF5A in the sense orientation.

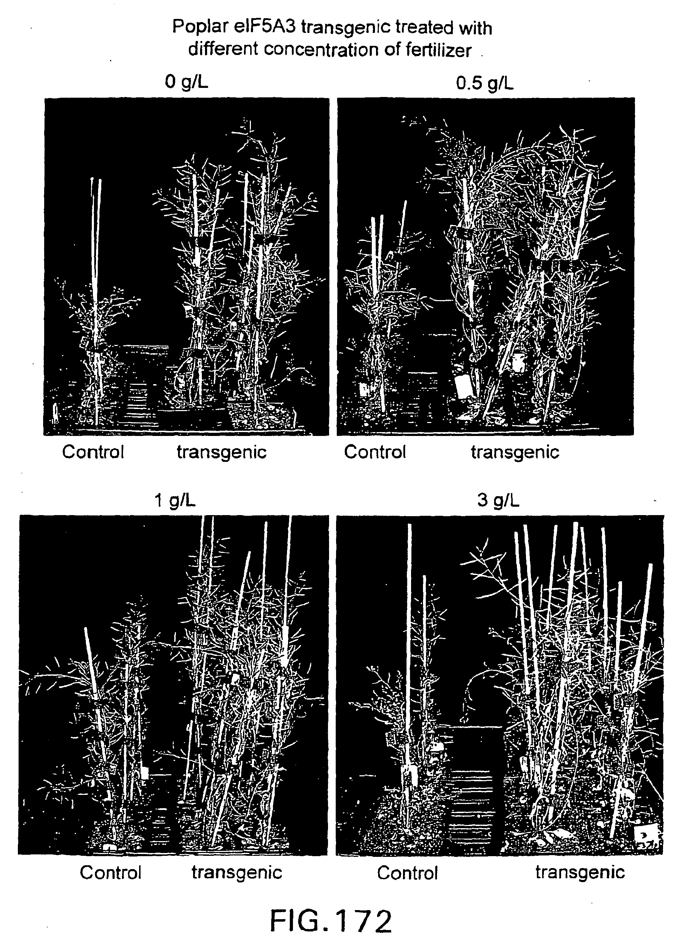

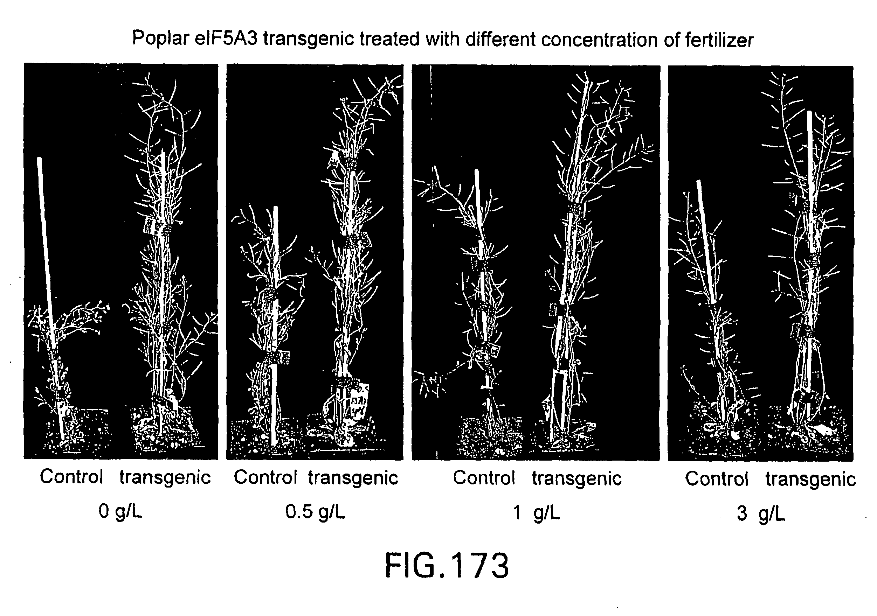

Figures 172 and 173 show that transgenic arabidopsis plants (having been transformed with the vector shown in figure 171) have increased biomass over control plants when treated with different levels of fertilizer, including optimal, suboptimal, and supraoptimal concentrations.

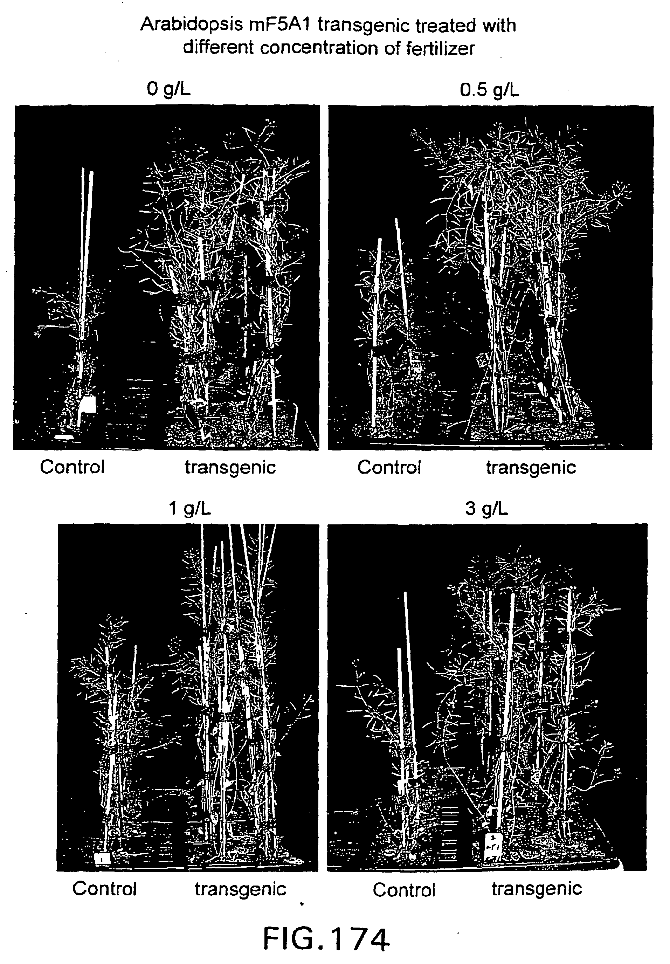

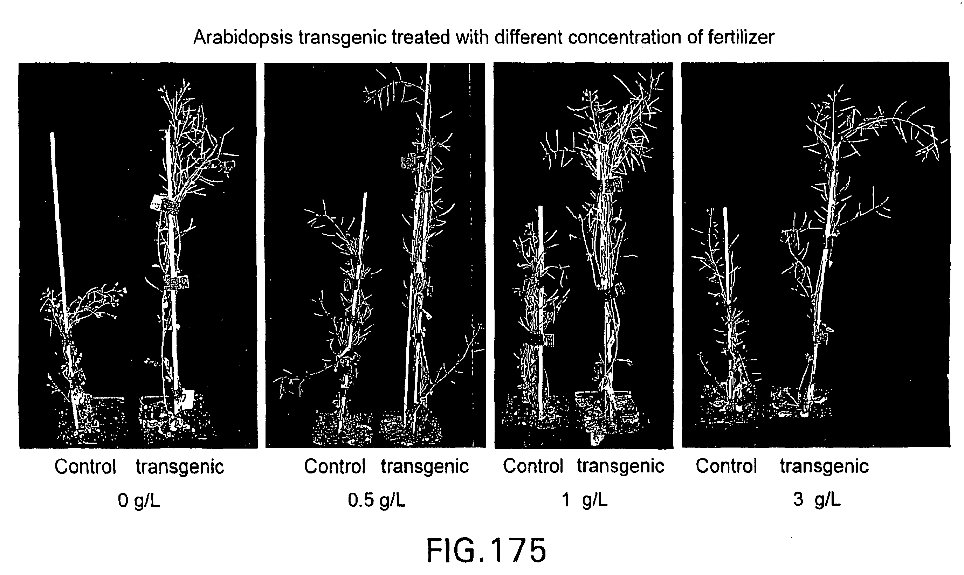

Figures 174 and 175 show that transgenic arabidopsis plants (having been transformed with a mutant senescence-induced eIF-5a) have increased biomass over control plants when treated with different levels of fertilizer, including optimal, suboptimal, and supraoptimal concentrations.

### DETAILED DESCRIPTION

As used herein, the term "plant" refers to either a whole plant, a plant part, a plant cell or a group of plant cells. The type of plant which can be used in the methods of the invention is not limited and includes, for example, ethylene-sensitive and ethylene-insensitive plants; fruit bearing plants such as apricots, apples, oranges, bananas, grapefruit, pears, tomatoes, strawberries, avocados, etc.; vegetables such as carrots, peas, lettuce, cabbage, turnips, potatoes, broccoli, asparagus, etc.; flowers such as carnations, roses, mums, etc.; agronomic crop plants such as corn, rice, soybean, alfalfa and the like, and forest species such as deciduous trees, conifers, evergreens, etc., and in general, any plant that can take up and express DNA molecules of the present invention. It may include plants of a variety of ploidy levels, including haploid, diploid, tetraploid and polyploid. The plant may be either a monocotyledon or dicotyledon.

A transgenic plant is defined herein as a plant which is genetically modified in some way, including but not limited to a plant which has incorporated heterologous or homologous senescence-induced eIF-5A, wounding/pathogen induced eIF-5A, growth eIF-5A, stress eIF-5A or DHS into its genome. The altered genetic material may encode a protein, comprise a regulatory or control sequence, or may be or include an antisense sequence or sense sequence or encode an antisense RNA or sense RNA which is antisense or sense to senescence-induced eIF-5A, wounding/pathogen induced eIF-5A, growth eIF-5A, stress elf-5A or DHS DNA or mRNA sequence or portion thereof of the plant.

The term "hybridization" as used herein is generally used to mean hybridization of nucleic acids at appropriate conditions of stringency as would be readily evident to those skilled in the art depending upon the nature of the probe sequence and target sequences. Conditions of hybridization and washing are well known in the art, and the adjustment of conditions depending upon the desired stringency by varying incubation time, temperature and/or ionic strength of the solution are readily accomplished. See, for example, Sambrook, J. et al., Molecular Cloning: A Laboratory Manual, 2nd edition, Cold Spring Harbor Press, Cold Spring Harbor, New York, 1989. The choice of conditions is dictated by the length of the sequences being hybridized, in particular, the length of the probe sequence, the relative G-C content of the nucleic acids and the amount of mismatches to be permitted. Low stringency conditions are preferred when partial hybridization between strands that have lesser degrees of complementarity is desired. When perfect or near perfect complementarity is desired, high stringency conditions are preferred. What is meant herein as high stringency conditions is as follows: the hybridization solution contains 6X S.S.C., 0.01 M EDTA, 1X Denhardt's solution and 0.5% SDS. Hybridization is carried out at about 68°C for about 3 to 4 hours for fragments of cloned DNA and for about 12 to about 16 hours for total eukaryotic DNA. For lower stringencies the temperature of hybridization is reduced to about 42°C below the melting temperature (T_{M}) of the duplex. The T_{M} is known to be a function of the G-C content and duplex length as well as the ionic strength of the solution.

As used herein, the term "substantial sequence identity" or "substantial homology" is used to indicate that a nucleotide sequence or an amino acid sequence exhibits substantial structural or functional equivalence with another nucleotide or amino acid sequence. Any structural or functional differences between sequences having substantial sequence identity or substantial homology will be *de minimis;* that is, they will not affect the ability of the sequence to function as indicated in the desired application. Differences may be due to inherent variations in codon usage among different species, for example. Structural differences are considered *de minimis* if there is a significant amount of sequence overlap or similarity between two or more different sequences or if the different sequences exhibit similar physical characteristics even if the sequences differ in length or structure. Such characteristics include, for example, ability to hybridize under defined conditions, or in the case of proteins, immunological crossreactivity, similar enzymatic activity, etc. Each of these characteristics can readily be determined by the skilled practitioner by art known methods.

Additionally, two nucleotide sequences are "substantially complementary" if the sequences have at least about 70 percent, more preferably, 80 percent and most preferably about 90 percent sequence similarity between them. Two amino acid sequences are substantially homologous if they have at least 70% similarity between the active portions of the polypeptides.

As used herein, the phrase "hybridizes to a corresponding portion" of a DNA or RNA molecule means that the molecule that hybridizes, e.g., oligonucleotide, polynucleotide, or any nucleotide sequence (in sense or antisense orientation) recognizes and hybridizes to a sequence in another nucleic acid molecule that is of approximately the same size and has enough sequence similarity thereto to effect hybridization under appropriate conditions. For example, a 100 nucleotide long antisense molecule from the 3' coding or non-coding region of tomato wounding/pathogen induced eIF-5A will recognize and hybridize to an approximately 100 nucleotide portion of a nucleotide sequence within the 3' coding or non-coding region, respectively of AT wounding/pathogen induced eIF-5A gene or any other plant wounding/pathogen induced eIF-5A gene so long as there is about 70% or more sequence similarity between the two sequences. It is to be understood that the size of the "corresponding portion" will allow for some mismatches in hybridization such that the "corresponding portion" may be smaller or larger than the molecule which hybridizes to it, for example 20-30% larger or smaller, preferably no more than about 12-15 % larger or smaller.

The term "functional derivative" of a nucleic acid (or polynucleotide) as used herein means a fragment, variant, homolog, or analog of the gene or nucleotide sequence encoding senescence-induced eIF-5A, wounding/pathogen induced eIF-5A, growth eIF-5A or DHS. A functional derivative retains at least a portion of the function of the senescence-induced eIF-5A, wounding/pathogen induced eIF-5A, growth eIF-5A, stress eIF-5A or DHS encoding DNA, which permits its utility in accordance with the invention. Such function may include the ability to hybridize under high stringency conditions with native isolated senescence-induced eIF-5A, wounding/pathogen induced eIF-5A, growth eIF-5A, stress eIF-5A or DHS or substantially homologous DNA from another plant or an mRNA transcript thereof, and which senescence-induced eIF-5A, wounding/pathogen induced eIF-5A, growth eIF-5A, stress eIF-5A or DHS in antisense orientation inhibits expression of senescence-induced eIF-5A, wounding/pathogen induced eIF-5A, growth eIF-5A, stress eIF-5A or DHS.

A "fragment" of the gene or DNA sequence refers to any subset of the molecule, e.g., a shorter polynucleotide or oligonucleotide. A "variant" refers to a molecule substantially similar to either the entire gene or a fragment thereof, such as a nucleotide substitution variant having one or more substituted nucleotides, but which maintains the ability to hybridize with the particular gene or to encode mRNA transcript which hybridizes with the native DNA. A "homolog" refers to a fragment or variant sequence from a different plant genus or species. An "analog" refers to a non-natural molecule substantially similar to or functioning in relation to either the entire molecule, a variant or a fragment thereof.

By "modulating expression" it is meant that either the expression is inhibited or up-regulated. "Inhibition of expression" refers to the absence or detectable decrease in the level of protein and/or mRNA product from a target gene, such as senescence-induced eIF-5A, wounding/pathogen induced eIF-5A, growth eIF-5A, stress eIF-5A or DHS. "Up-regulation" or "over expression" refers to a detectable increase in the level of protein and/or mRNA product from a target gene, such as senescence-induced eIF-5A, wounding/pathogen induced eIF-5A, growth eIF-5A, stress eIF-5A or DHS. Included within the meaning of modulating expression, are decreasing the activity of DHS or any one of the isoforms of eIF-5A. For example, one may create a knock down mutant of DHS in a plant and the resulting plant will have a DHS that activates eIF-5A at a lower level or efficiency of a wild type DHS. Modulating expression also includes the use of chemicals or drugs that inhibit or decrease the activity of DHS or eIF-5A.

Isolated polynucleotides and peptides of the present invention include those isolated from natural sources, recombinantly produced or synthesized. Isolated proteins of the present invention include senescence-induced eIF-5A, wounding/pathogen induced eIF-5A, growth eIF-5A or DHS expressed as a fusion protein, preferably comprising eIF-5A or DHS fused with maltose binding protein.

"Functional derivatives" of senescence-induced eIF-5A, wounding/pathogen induced eIF-SA, growth eIF-5A, stress eIF-5A or DHS peptides of the present invention include fragments, variants, analogs, or chemical derivatives, which retain at least a portion of the activity or immunological cross reactivity with an antibody specific for the eIF-5A isoform or DHS. A fragment of eIF-5A or DHS peptide refers to any subset of the molecule. Variant peptides may be made by direct chemical synthesis, for example, using methods well known in the art. An analog of eIF-5A or DHS peptide refers to a non-natural protein substantially similar to either the entire protein or a fragment thereof. Chemical derivatives of eIF-5A or DHS contain additional chemical moieties not normally a part of the peptide or peptide fragment. Modifications may be introduced into peptides or fragments thereof by reacting targeted amino acid residues of the peptide with an organic derivatizing agent that is capable of reacting with selected side chains or terminal residues.

A eIF-5A or DHS peptide according to the invention may be produced by culturing a cell transformed with a nucleotide sequence of this invention (in the sense orientation), allowing the cell to synthesize the protein and then isolating the protein, either as a free protein or as a fusion protein, depending on the cloning protocol used, from either the culture medium or from cell extracts. Alternatively, the protein can be produced in a cell-free system. Ranu, et al., Meth. Enzymol., 60:459-484, (1979).

Preparation of plasmid DNA, restriction enzyme digestion, agarose gel electrophoresis of DNA, polyacrylamide gel electrophoresis of protein, PCR, RT-PCR, Southern blots, Northern blots, DNA ligation and bacterial transformation were carried out using conventional methods well-known in the art. See, for example, Sambrook, J. et al., Molecular Cloning: A Laboratory Manual, 2nd ed., Cold Spring Harbor Press, Cold Spring Harbor, NY, 1989. Techniques of nucleic acid hybridization are disclosed by Sambrook.

Procedures for constructing recombinant nucleotide molecules in accordance with the present invention are disclosed in Sambrook, et al., In: Molecular Cloning: A Laboratory Manual, 2nd ed., Cold Spring Harbor Press, Cold Spring Harbor, N.Y. (1989), and Maniatis, T. et al., Molecular mechanisms in the Control of Gene expression, eds., Nierlich, et al., eds., Acad. Press, N.Y. (1976), which are both incorporated herein in its entirety.

Transgenic plants made in accordance with the present invention may be prepared by DNA transformation using any method of plant transformation known in the art. Plant transformation methods include, but are not limited to, direct co-cultivation of plants, tissues or cells with *Agrobacterium tumefaciens* or direct infection (Miki, et al., Meth. in Plant Mol. Biol. and Biotechnology, (1993), p. 67-88); direct gene transfer into protoplasts or protoplast uptake (Paszkowski, et al., EMBO J., 12:2717 (1984); electroporation (Fromm, et al., Nature, 319:719 (1986); particle bombardment (Klein et al., BioTechnology, 6:559-563 (1988); injection into meristematic tissues of seedlings and plants (De LaPena, et al., Nature, 325:274-276 (1987); injection into protoplasts of cultured cells and tissues (Reich, et al., BioTechnology, 4:1001-1004 (1986)).

Generally a complete plant is obtained from the transformation process. Plants may be regenerated from protoplasts, callus, tissue parts or explants, etc. Plant parts obtained from regenerated transgenic plants in which the expression of an eIF-5A isoform or DHS is altered, such as leaves, flowers, fruit, seeds, pollen and the like are included in the definition of "plant" as used herein. Progeny, variants and mutants of the regenerated plants are also included in the definition of "plant."

### eIF-5A generally

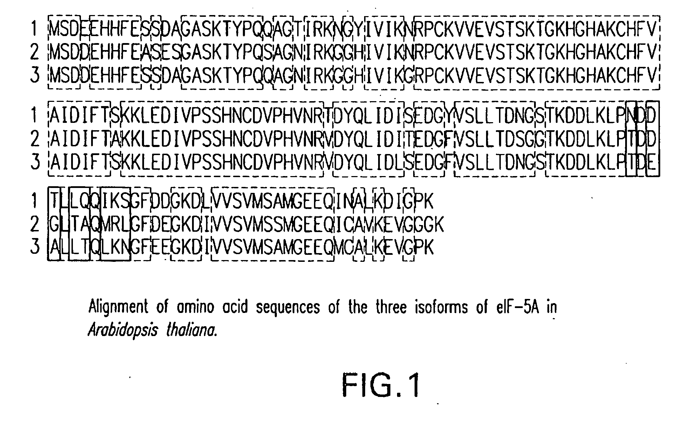

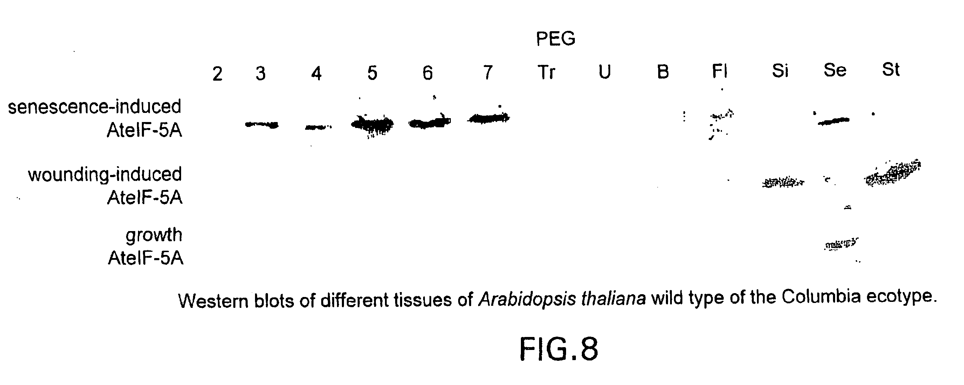

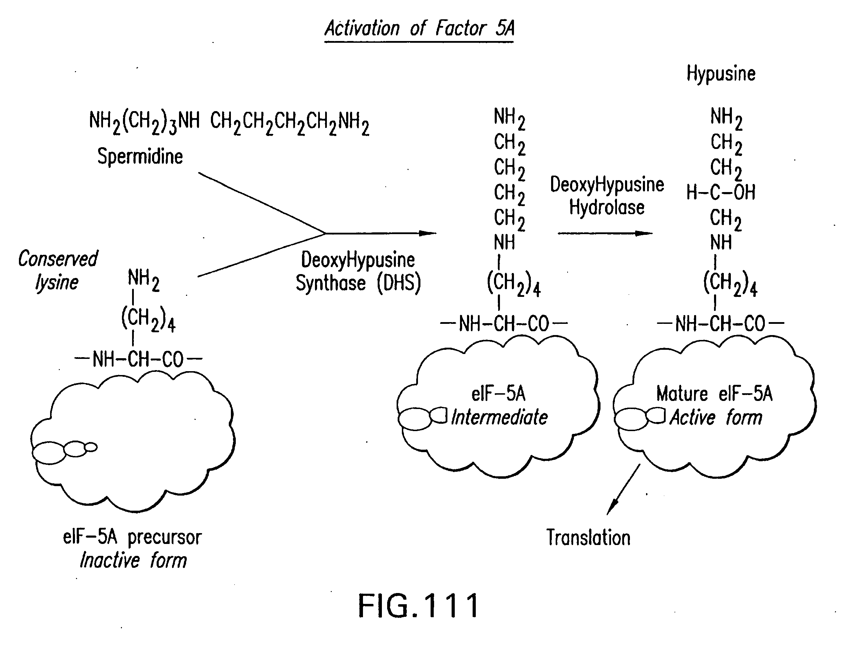

The present invention relates to eIF-5A (eukaryotic translation initiation factor 5A). eIF-5A regulates programmed cell death in natural senescence of leaves, flowers and fruit; stress-induced premature senescence, cell death associated with xylogenesis and cell death associated with pathogen ingression. eIF-5A is post translationally activated by two enzymes deoxyhypusine synthase (DHS) and deoxyhypusine hydrolase (DHH). See figure 111. eIF-5A is not a conventional translation initiation factor but rather functions as a shuttle protein. Three or four distinct isoforms of eIF-5A have been identified. The three different isoforms selectively translocate different subpopulations of mRNAs from the nucleus to ribosomes for translation. One isoform controls cell division and growth, and two or three isoforms control cell death. For example, figure 1 shows three different isoforms of eIF-5A isolated in Arabidopsis. Figures 8-10 show Western blots probed with isoform specific antibodies against eIF-5A ("At" means arabidopsis). These figures show expression levels of the three different isoforms and different life cycles and events in Arabidopsis. In plants with larger genomes, e.g. crop plants, there are four isoforms of eIF-5A. One isoform is involved in cell division and cell growth and three isoforms are involved in cell death.

The present invention relates to different isoforms of eIF-5A: senescence-induced eIF-5A; wounding induced eIF-5A; stress eIF-5A, and growth eIF-5A. Sensecence-induced eIF-5A induces senescence at the end of the lifespan of a plant or plant tissue. Stress eIF-5A is also a death isoform that induces senescence prematurely in response to environmental stress e.g. drought stress. Disease/wounding eIf-5A is a death isoform that induces cell death in response to pathogen ingression or a wounding event. Growth eIF-5A promotes plant growth. eIF-5A appears to function as a biological switch, regulating growth in one position and death in another. In the death position, one or more of the death isoforms are strongly expressed, and the growth isoform is only weakly expressed or not expressed at all. In the growth position, the growth isoform is strongly expressed, and the death isoforms are only weakly expressed or not expressed at all.

Although there are 3 to 4 isoforms of eIF-5A, it is believed that there is only one isoform of DHS, and this single isoform activates (hypusinates) all of the isoforms of eIF-5A.

The present invention provides various isoforms of eIF-5A isolated from various plant species and methods of isolating the isoforms of eIF-5A. The present invention also provides polynucleotides that encode the various isoforms of eIF-5A of the present invention. The invention also provides antisense polynucleotides of the isoforms of eIF-5A and expression vectors containing such polynucleotides or antisense polynucleotides. In some embodiments, there are provided methods of inhibiting expression of endogenous eIF-5A through the use of expression vectors containing antisense polynucleotides of the isoforms of eIF-5A to transform plants. In some embodiments, there are provided methods of up-regulating endogenous eIF-5A isoforms by providing expression vectors containing polynucleotides of the isoforms of eIF-5A in the sense orientation.

The different isoforms are naturally up or down-regulated depending upon the life stage of the plant or the plant's condition. For example in senescing tissues, the senescence-induced eIF-5A isoform is up-regulated. The senescence-induced eIF-5A is thought to participate in further senescence of the plant or plant tissues by shuttling specific subsets of mRNAs (those involved in the senescence pathway) from the nucleus to the cytoplasm for translation. By down regulating or inhibiting the expression of senescence-induced eIF-5A, senescence can be delayed in the plant and/or plant tissues. Delayed senescence is manifested in the transformed/transgenic plants by having a larger bio-mass, increased shelf life for fruit, increased shelf life of flowers, increased seed size and increased seed yield as compared to non-transformed or wild type plants.

When a plant and/or plant tissues are exposed to a wounding event, such as chilling, dehydration, or mechanical forces, or exposed to a pathogen, wounding/pathogen induced eIF-5A isoform is up-regulated. By down regulating the expression of wounding/pathogen induced eIF-5A, an increased resistance to virulent damage arising from pathogen ingression is conferred on the plants as compared to resistance to virulent damage in non-transformed or wild type plants.

When a plant is in the growth phase, growth eIF-5A isoform is up-regulated. By up-regulating growth eIF-5A, the resulting transgenic plants have an increased seed size, increased biomass and increased seed yield.

When certain plants undergo stress, stress eIF-5A isoform is up-regulated. By down regulating stress eIF-5A, the resulting transgenic plants show an increased tolerance to the stress, such as an increased tolerance to drought. Further, these plants have an increased seed yield over wild type plants.

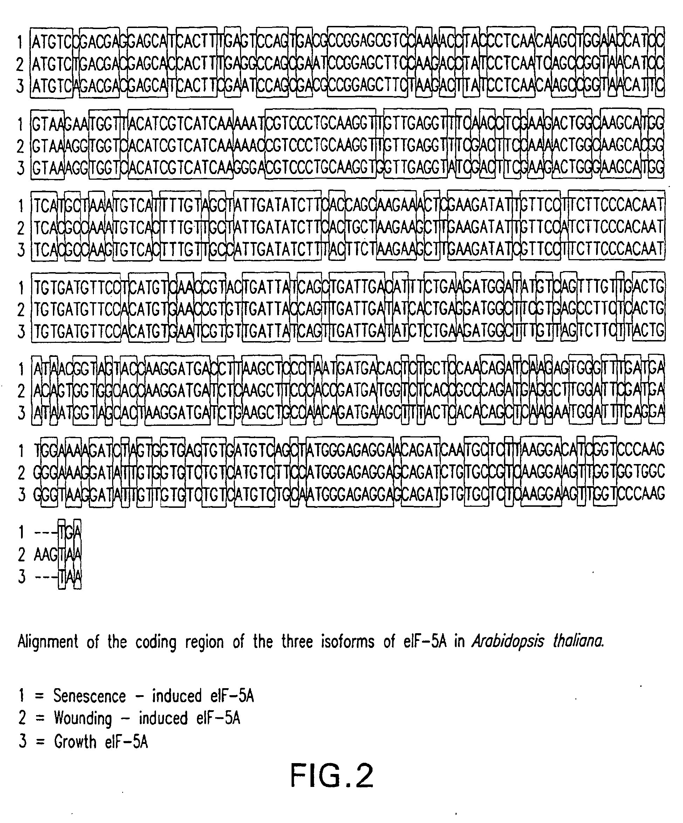

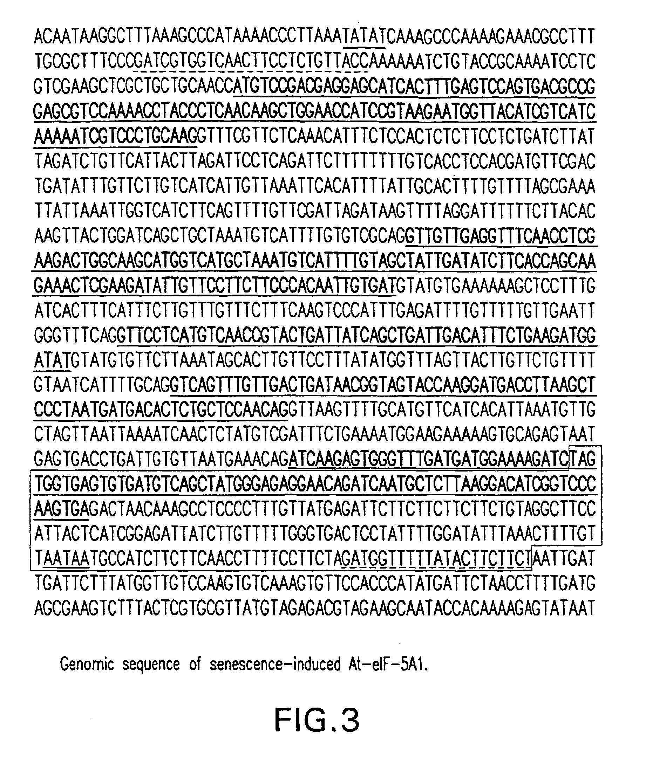

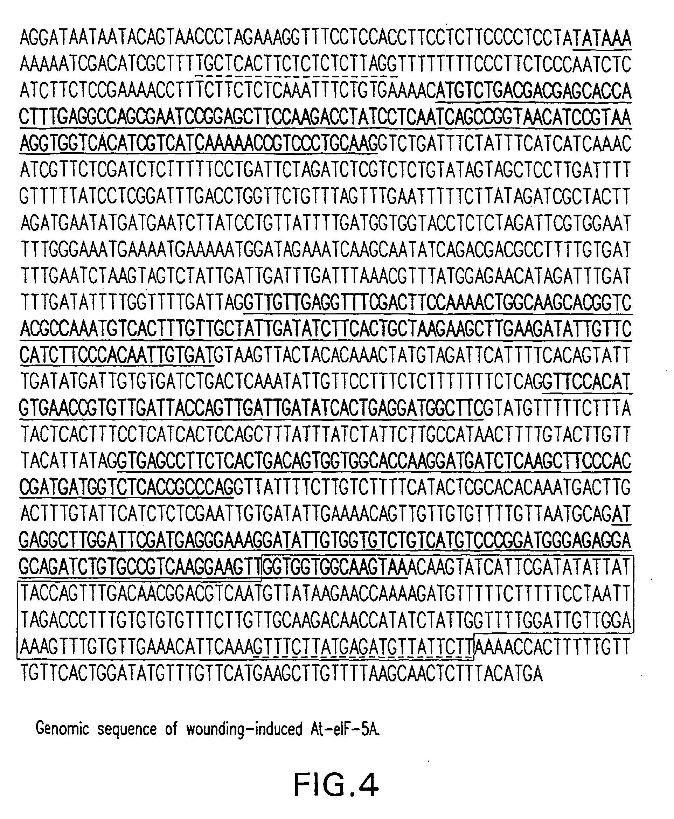

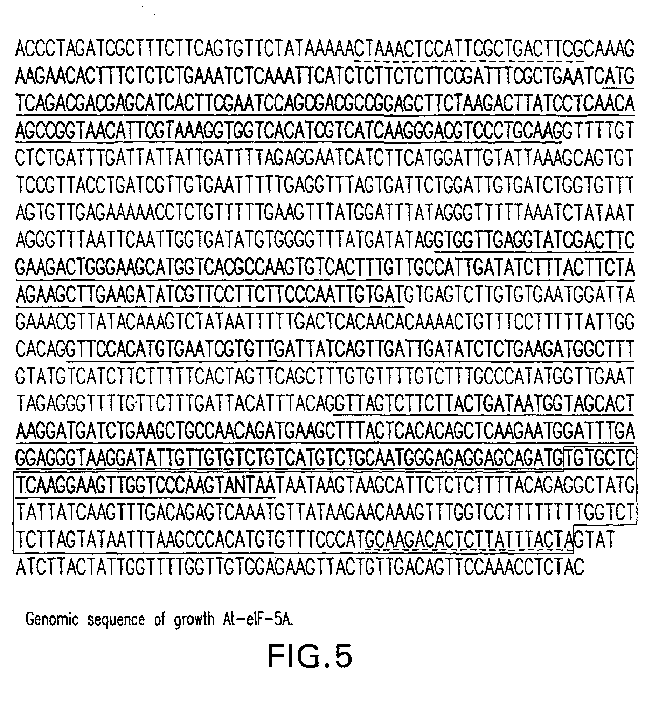

Figure 1 shows the alignment of three isoforms of eIF-5A isolated from Arabidopsis thaliana ("At"). Figure 2 shows the alignment of the coding regions of these three isoforms. Figures 3-5 provide the genomic sequence of the three isoforms.

Western blots (see Figure 8) show the expression of three isoforms of eIF-5A at different plant life stages. Figure 8 reveals that the amount of the senescence-induced factor eIF-5A isoform increases as the ages of the leaves increases. It is not seen in the unopened flower buds, siliques or stems but it is seen in the imbibed seeds. In the imbibed seeds there is cotyledon tissue as well as growing embryo. Thus, senescence-induced eIF-5A is present in the imbibed seeds because the cotyledon tissue is senescing as the embryo is growing. Growth eIF-5A is seen in the imbibed seeds because there the embryo is actively growing. The wounding/pathogen induced eIF-5A is seen in the siliques, seeds and stems as the harvesting of these tissues induces some wounding.

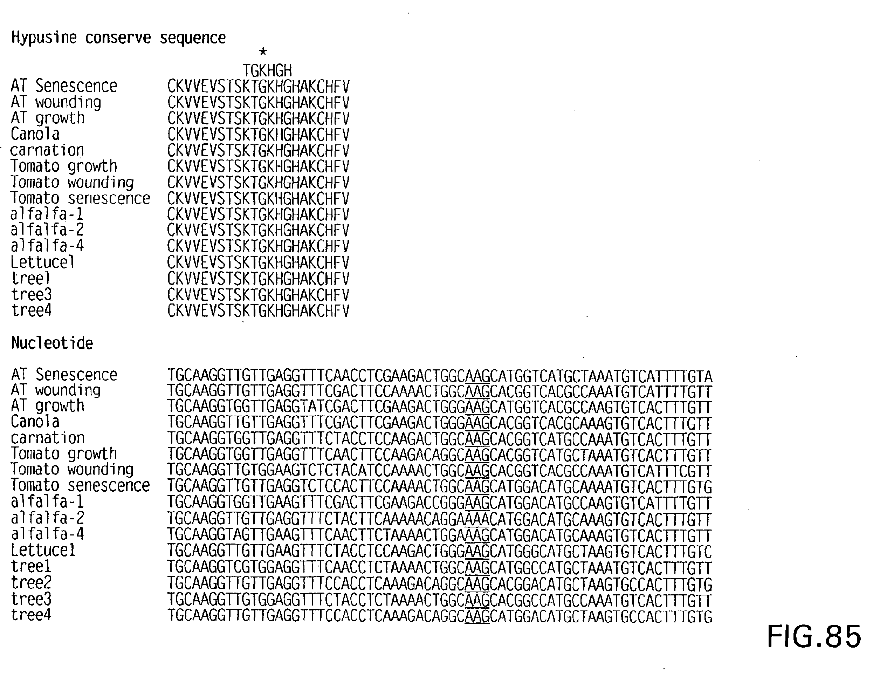

Although there is a high degree of homology (about 85%) between the different isoforms and between the isoforms in different plant species, the different isoforms vary from each other in the 3'UTR. One region that is highly conserved between the isoforms and between species as well, is the area that is believed to be the hypusine site. The hypusine site is believed to be the following amino acids: 5'-CKVVEVSTSKTGKHGHAKCHFV-3' (SEQ ID NO:_). See Figure 85 for alignment of various eIF-5A isoforms and of several plant species.

### Senescence-induced eIF-5A

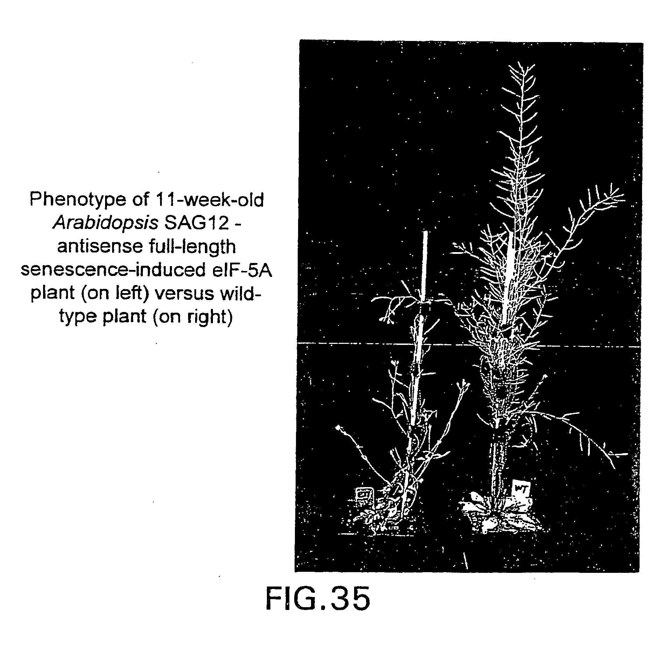

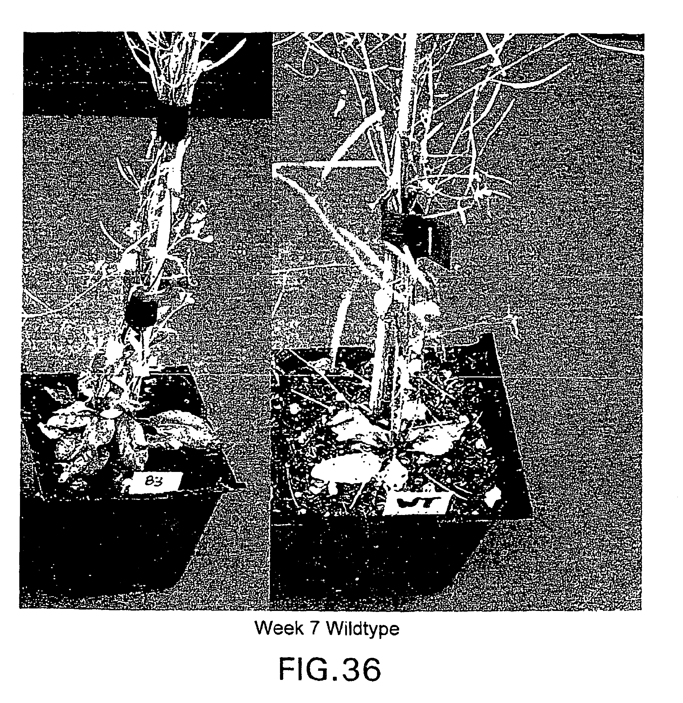

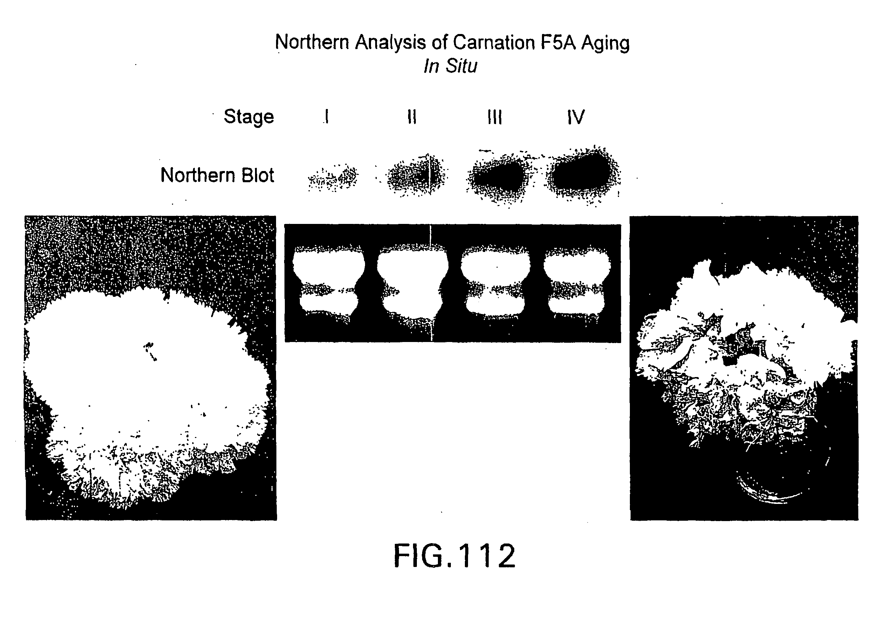

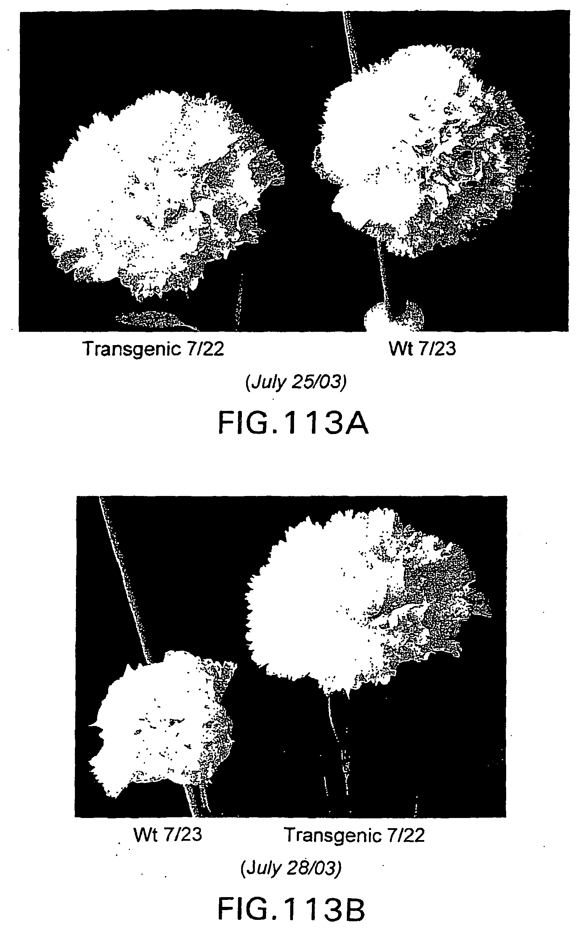

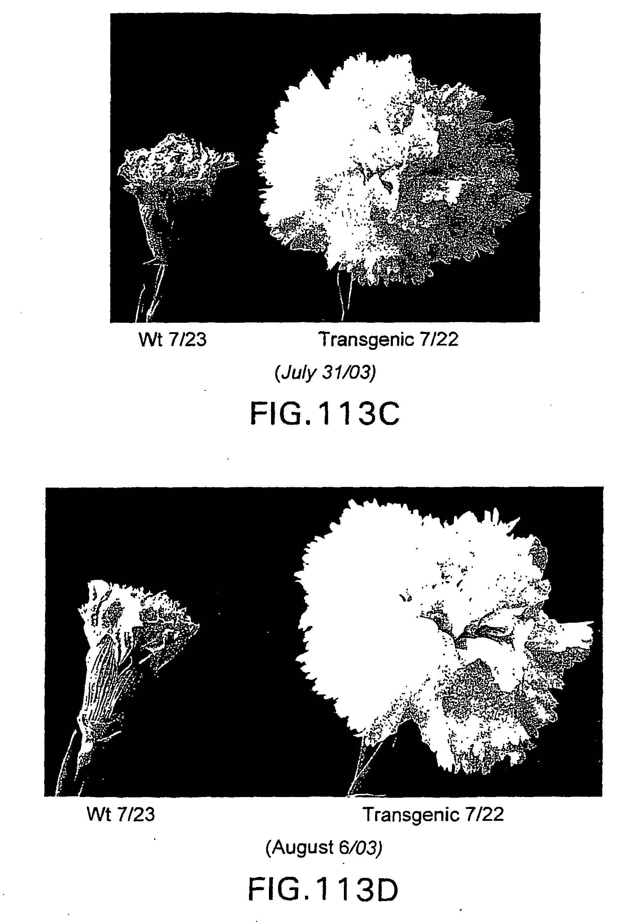

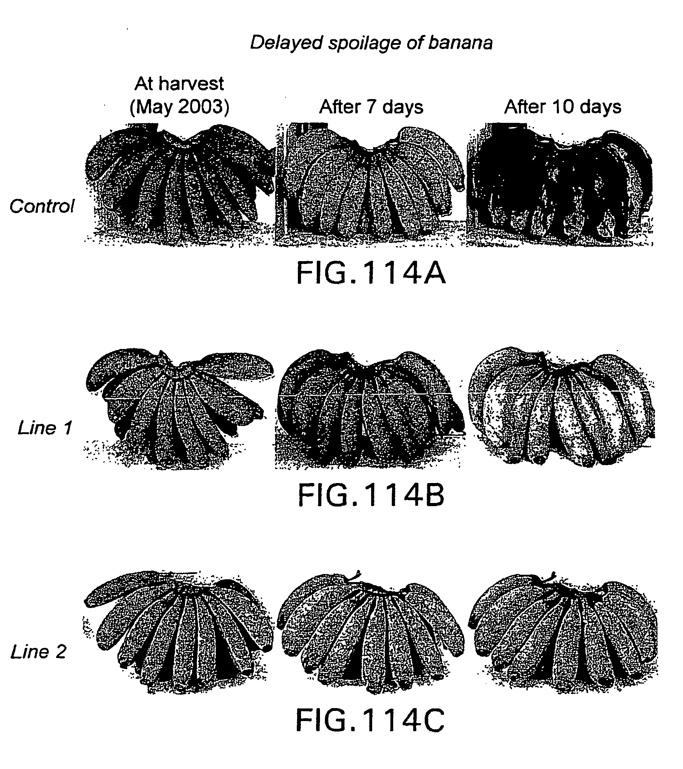

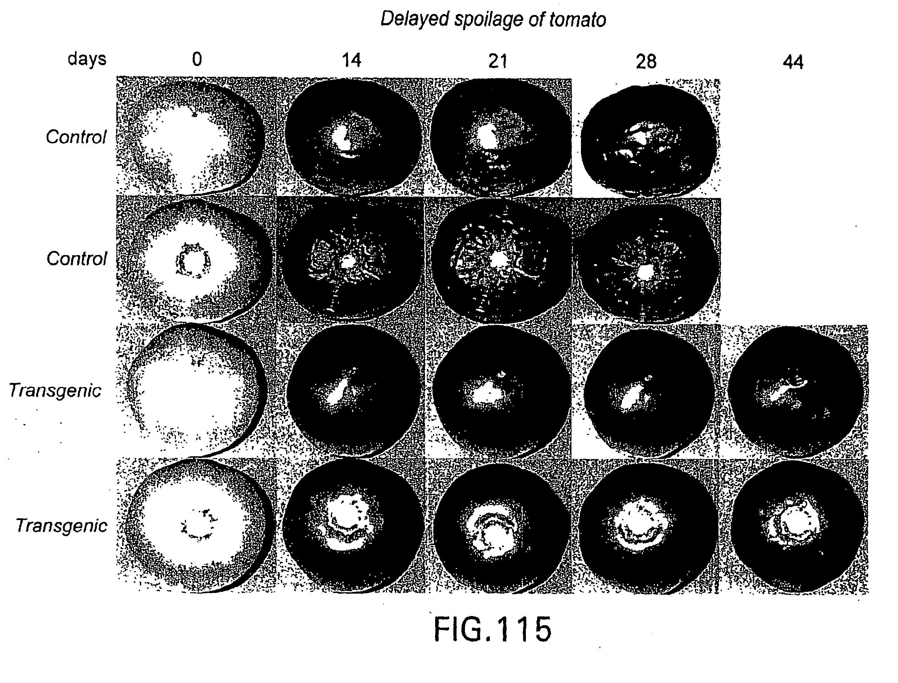

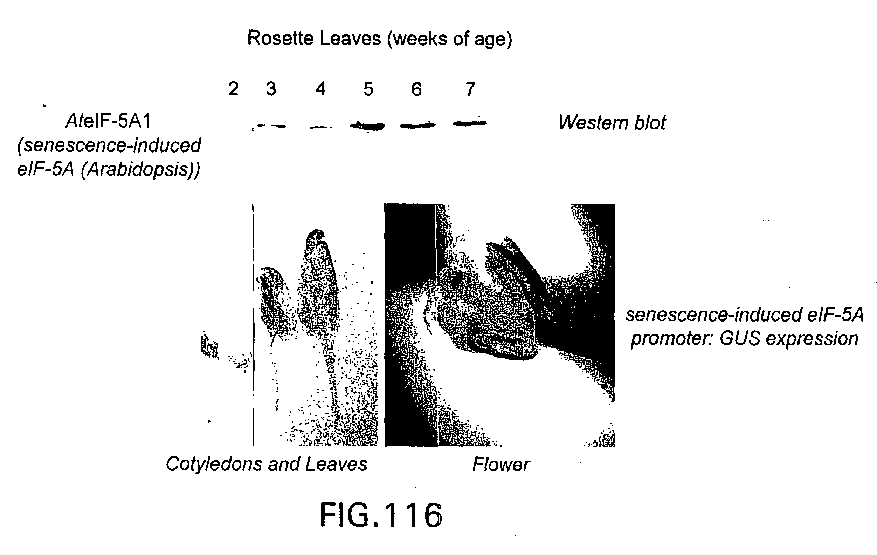

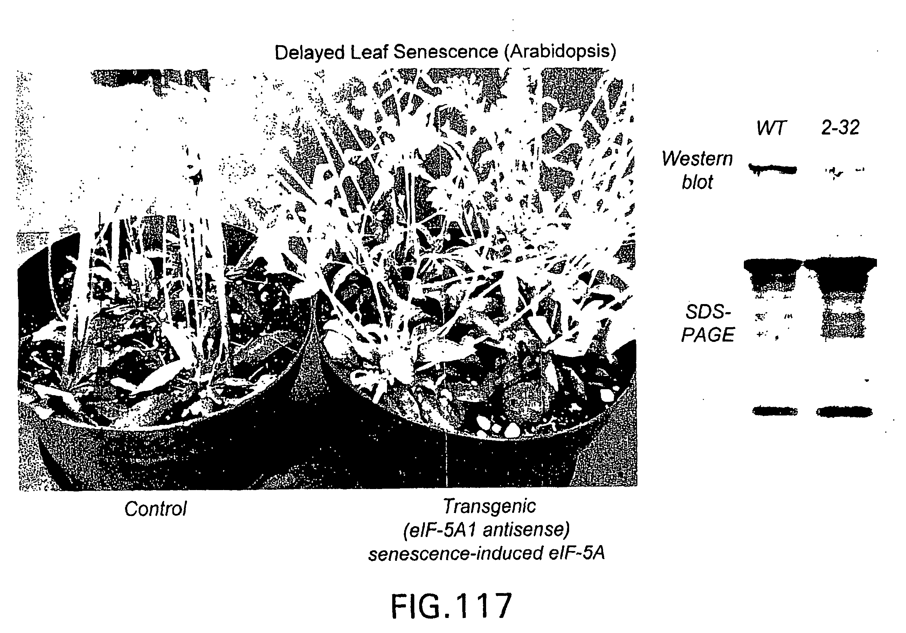

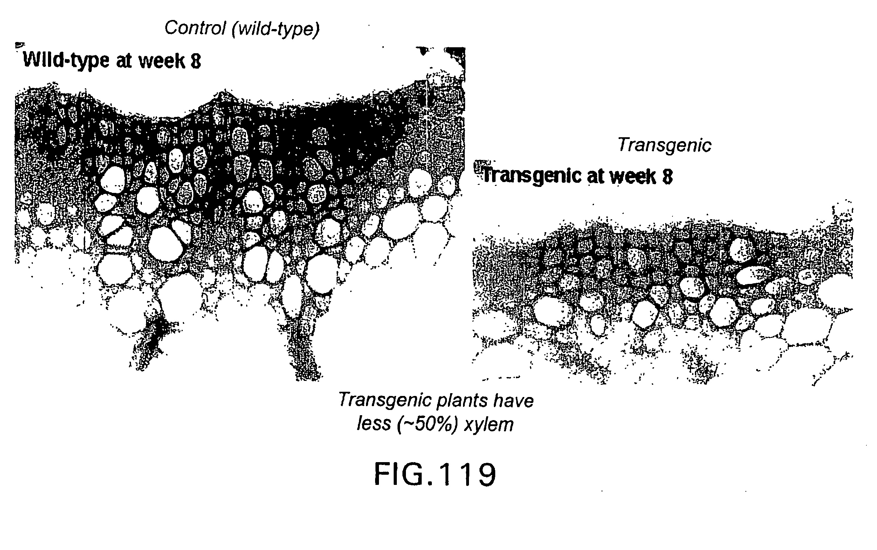

Senescence-induced eIF-5A is expressed in senescing tissues. See figure 116. The present invention relates to the discovery of senescence-induced eIF-5A in various plants including *Arabidopsis thaliana,* tomato, carnation, lettuce and alfalfa plants. Senescence-induced eIF-5A is up-regulated in senescing tissues and is involved in the induction of senescence related morphological changes in plants and plant tissues. When the senescence isoform is down regulated, natural senescence is delayed. Down regulation of senescence-induced eIF-5A (either through the use of antisense constructs of senescence-induced eIF-5A, use of sense constructs to achieve co-suppression, knock down mutants of senescence-induced eIF-5A, decreased expression of DHS (which is required to activate eIF-5A), knock down mutants of DHS, or use of inhibitors of DHS) results in various morphological changes in the transgenic plants, including increased plant bio-mass, delayed fruit softening or spoilage, delayed browning of cut flowers or plant tissues, such as lettuce leaves, increased seed yield and increased seed size. See figures 35, 36 and 117(delayed leaf senescence in Arabidopsis), figures 112 and 113 (longer shelf life of cut carnation flowers), figure 114 (delayed browning in bananas), figure 115 (delayed spoilage of tomato), and figure 119 (decreased xylem formation).

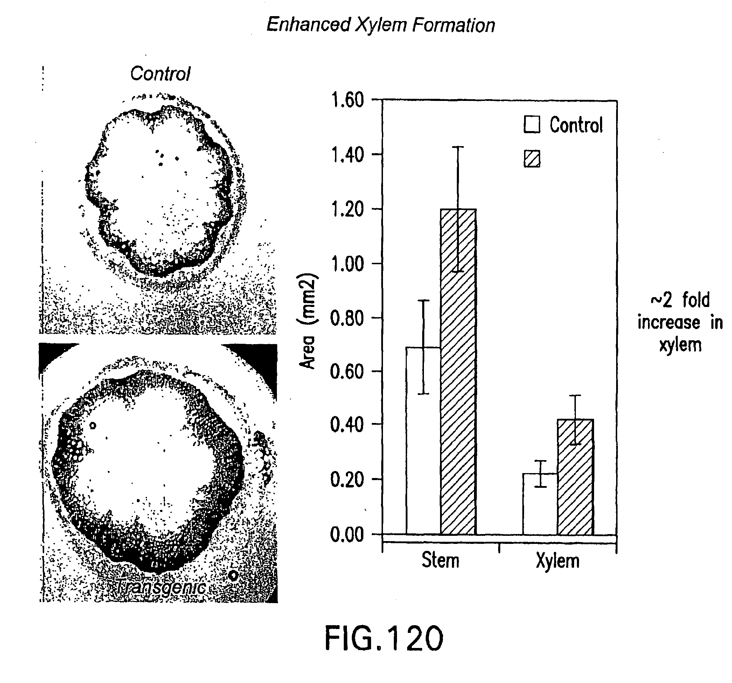

When senescence-induced eIF-5A is up-regulated with sense constructs of senescence-induced eIF-5A, a gain-of-function phenotype results. For example, an increase in the formation of xylem is noticed. See Figure 120.

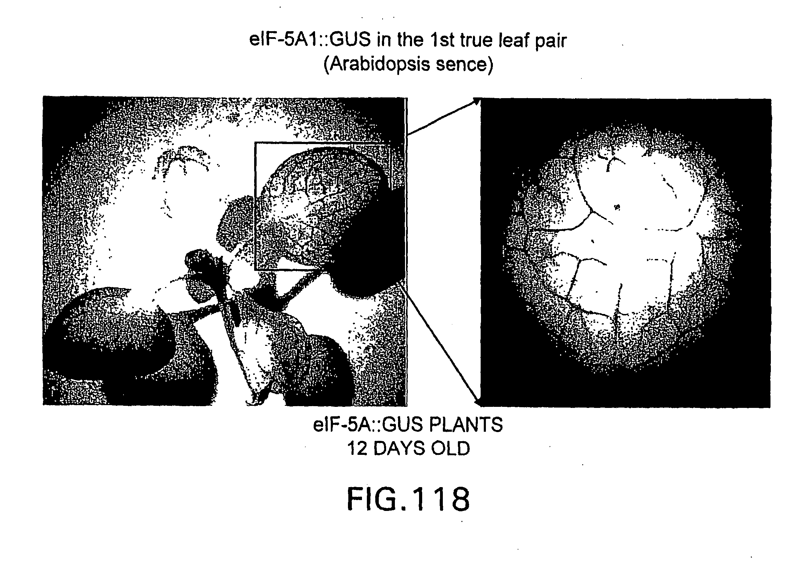

To further elucidate the roles of DHS and senescence-induced eIF-5A, the promoter activities of both DHS and senescence-induced eIF-5A were characterized during growth and development of transgenic Arabidopsis plants using GUS as a reporter gene. Figure 118 shows that senescence-induced eIF-5A is expressed in vascular tissues. The levels of DHS::GUS and senescence-induced eIF-5A::GUS expression were strongly upregulated at the onset of leaf senescence, and this high expression was maintained until leaf senescence terminated. Furthermore, and unexpectedly, DHS and senescence-induced eIF-5A were transiently and co-ordinately expressed in the vascular tissues of developing cotyledons, rosette leaves and stems. The timing and localization of the GUS expression imply a role for senescence-induced eIF-5A in xylogenesis. In addition, DHS and senescence-induced eIF-SA were co-ordinately expressed in anther tissues during the degenerative stages of pollen production. Taken together, these results suggest that DHS and senescence-induced eIF-5A regulate programmed cell death associated with xylogenesis, leaf senescence and flower senescence in Arabidopsis.

Senescence-induced eIF-5A is also expressed in developing xylem. In order to determine whether senescence-induced eIF-5A plays a role in xylem formation, its full-length cDNA was constitutively over-expressed in transgenic Arabidopsis plants. These plants had larger rosette leaves as well as taller and thicker inflorescence stems in comparison to control plants, reflecting more rapid growth. In addition, their main inflorescence stems exhibited a significant enhancement in xylem development. In both vascular and interfascicular regions, cell layers and tissue thicknesses were increased by up to 80% and 60%, respectively, depending on the line. Increased xylem growth was more pronounced in secondary xylem than in primary xylem. Moreover, constitutive antisense suppression of senescence-induced eIF-5A resulted in stunted statures and delayed leaf senescence as well as ~30% reduction in xylem tissue in comparison with control plants.

eIF5A is post-translationally modified by conversion of a conserved lysine to hypusine. To confirm that it is the hypusinated form of senescence-induced eIF-5A that regulates xylogenesis, we also generated transgenic Arabidopsis plants with constitutive overexpression of a mutant form of senescence-induced eIF-5A that cannot be hypusinated. The mutation was made by changing the codon, AAG, for the conserved lysine to GCG (for alanine), thereby disabling hypusination of senescence-induced eIF-5A. In the mutant population, there was no change in xylem development relative to control plants. Thus, senescence-induced eIF-5A appears to positively regulate xylogenesis, possibly by facilitating the programmed death of treachery elements, and such a function requires activation of the protein through hypusination.

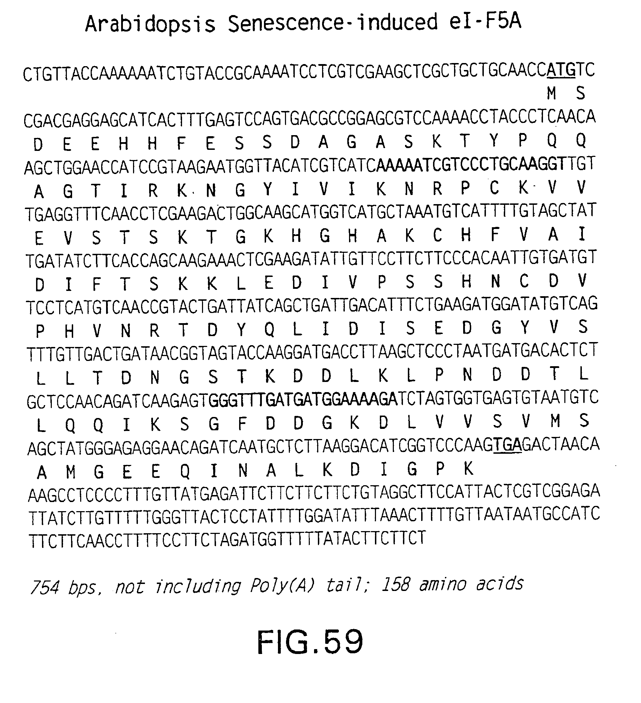

Thus, one embodiment of the present invention is isolated senescence-induced eIF-5A from *Arabidopsis thaliana.* The amino acid sequence is provided in Figure 59 and is SEQ ID NO: _. The polynucleotide encoding the amino acid is provided in Figure 59 and is SEQ ID NO: _.

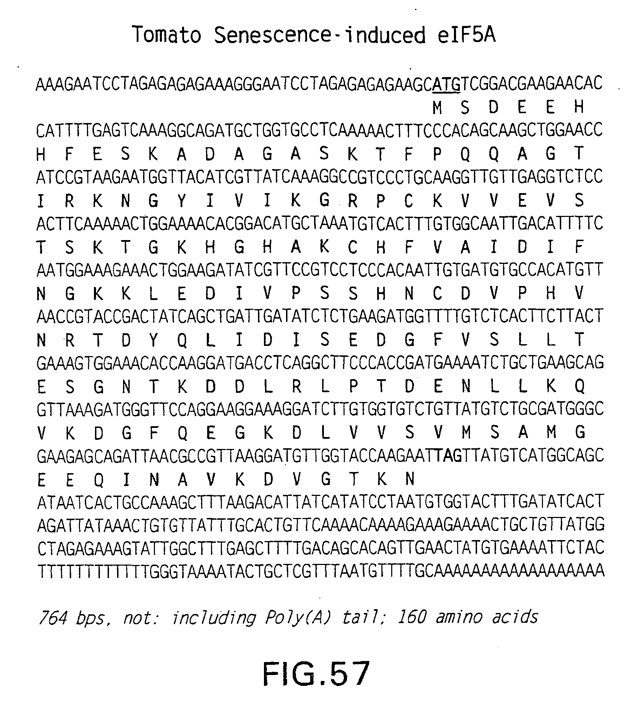

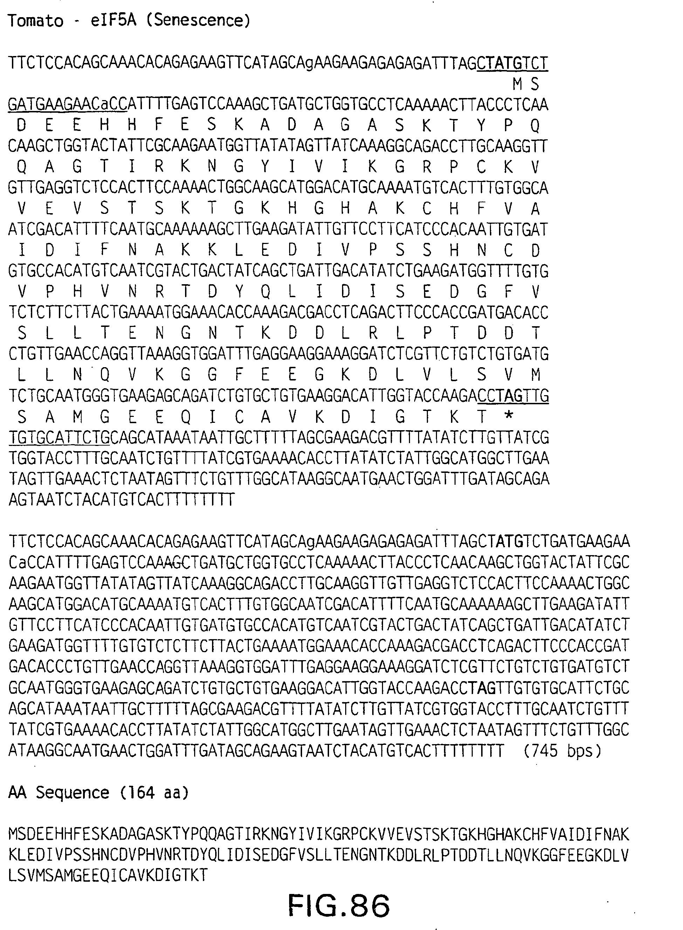

Another embodiment of the present invention is isolated senescence-induced eIF-5A from tomato. The amino acid sequence is provided in Figure 57 and 86 and is SEQ ID NO: _. The polynucleotide encoding the amino acid is provided in Figure 57 and 86 and is SEQ ID NO: _.

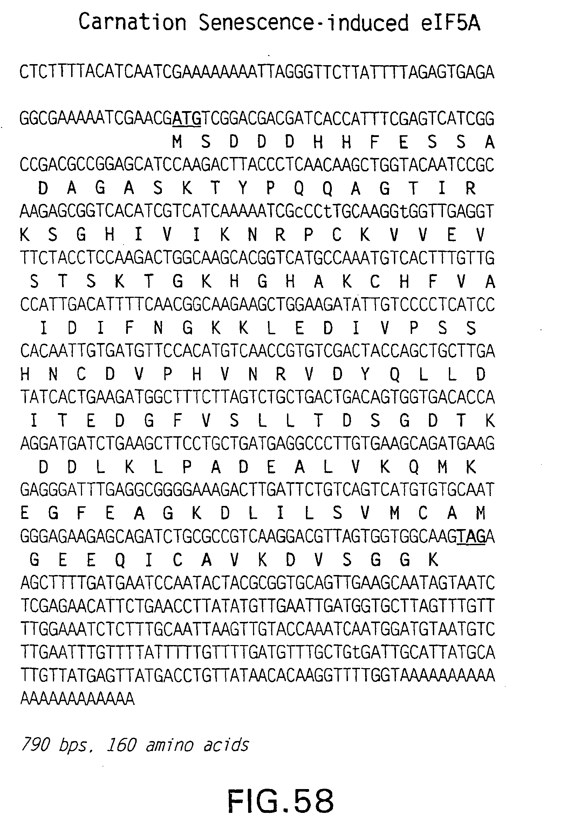

Another embodiment of the present invention is isolated senescence-induced eIF-5A from carnation The amino acid sequence is provided in Figure 58 and is SEQ ID NO: __. The polynucleotide encoding the amino acid is provided in Figure 58 and is SEQ ID NO: __.

Another embodiment of the present invention is isolated senescence-induced eIF-5A from alfalfa The amino acid sequence is provided in Figure 138 and is SEQ ID NO:__. The polynucleotide encoding the amino acid is provided in Figure 138 and is SEQ ID NO:__.

The present invention also provides isolated polynucleotides of senescence-induced eIF-5A that have 65-100% sequence homology to the above enumerated SEQ ID NOs, and hybridize under high stringency conditions to the complement of the enumerated SEQ ID NOs and which encode senescence-induced eIF-5A.

The present invention also provides senescence-induced eIF-5A isolated polypeptides of senescence-induced eIF-5A that have 65-100% amino acid sequence homology to the above enumerated SEQ ID NOs.

The present invention also provides antisense polynucleotides of the senescence-induced eIF-5As. The antisense polynucleotides may be of any length as long as they are able to inhibit expression. In some embodiments the antisense polynucleotides comprise the full length coding sequence and in other particularly preferred embodiments the antisense polynucleotides are directed at the 3'UTR since the different isoforms of eIF-5A have a higher degree of variation at the 3'UTR. In some embodiments the antisense polynucleotides are directed at the 5'- non-coding sequence. Antisense polynucleotides primarily complementary to 5'-non-coding sequences are known to be effective inhibitors of expression of genes encoding transcription factors. Branch, M.A., Molec. Cell Biol., 13:4284-4290 (1993).

The term "antisense polynucleotide of senescence-induced eIF5A" as used herein and in the claims encompasses not only those antisense polynucleotides that share 100 % homology of the complement of an enumerated SEQ ID NO but also includes those antisense polynucleotides that are a functional variants. Functional variants are those variants, either natural or man made, that have at least 80% sequence homology to and hybridizes under high stringency conditions with the corresponding portion of the senescence-induced eIF-5A. Further the variant must have the function as intended by the present invention, that is it is capable of modulating expression of endogenous senescence-induced eIF-5A when introduced into an expression vector and wherein such vector is incorporated into the genome of at least one plant cell. One skilled in the art can appreciate that insubstantial changes can be made in the sequence that would not detrimentally effect the ability of the antisense polynucleotide to bind to the transcript and reduce or inhibition expression of the gene. Thus, the term "antisense polynucleotide" encompasses those polynucleotides that are substantially complementary to the transcript and that still maintain the ability to specifically bind to the transcript and inhibit or reduce gene expression. For a general discussion of antisense see Alberts, et al., Molecular Biology of the Cell, 2nd ed., Garland Publishing, Inc. New York, New York ,1989 (in particular pages 195-196, incorporated herein by reference).

One embodiment of the present invention provides expression vectors comprising either the senescence-induced eIF-5A polynucleotides (of the present invention as described above) or antisense polynucleotides of senescence-induced eIF-5A (of the present invention as described above). Vectors can be plasmids, preferably, or maybe viral or other vectors known in the art to replicate and express genes encoded thereon in plant cells or bacterial cells. The vector becomes chromosomally integrated such that it can be transcribed to produce the desired antisense polynucleotide of senescence-induced eIF-5A RNA. Such plasmid or viral vectors can be constructed by recombinant DNA technology methods that are standard in the art. For example, the vector may be a plasmid vector containing a replication system functional in a prokaryotic host and an antisense polynucleotide according to the invention. Alternatively, the vector may be a plasmid containing a replication system functional in *Agrobacterium* and an antisense polynucleotide according to the invention. Plasmids that are capable of replicating in *Agrobacterium* are well known in the art. See, Miki, et al., Procedures for Introducing Foreign DNA Into Plants, Methods in Plant Molecular Biology and Biotechnology,, Eds. B.R. Glick and J.E. Thompson. CRC Press (1993), PP. 67-83.

The vector further comprises regulatory sequences operatively linked to the polynucleotides to allow expression of such polynucleotides. The regulatory sequences may include a promoter functional in the transformed plant cell. The promoter may be inducible, constitutive, or tissue specific. Such promoters are known by those skilled in the art.

Promoter regulatory elements that are useful in combination with the various isoforms of eIF-5A and DHS of the present invention to generate sense or antisense transcripts of the gene include any plant promoter in general, and more particularly, a constitutive promoter such as the fig wart mosaic virus 35S promoter, the cauliflower mosaic virus promoter, CaMV35S promoter, or the MAS promoter, or a tissue-specific or senescence-induced promoter, such as the carnation petal GST1 promoter or the *Arabidopsis* SAG12 promoter (See, for example, J.C. Palaqui et al., Plant Physiol., 112:1447-1456 (1996); Morton et al., Molecular Breeding, 1:123-132 (1995); Fobert et al., Plant Journal, 6:567-577 (1994); and Gan et al., Plant Physiol., 113:313 (1997), incorporated herein by reference). Preferably, the promoter used in the present invention is a constitutive promoter. The SAG12 promoter is preferably preferred when using antisense polynucleotides of senescence-induced eIF-5A. See example 23.

Expression levels from a promoter which is useful for the present invention can be tested using conventional expression systems, for example by measuring levels of a reporter gene product, e.g., protein or mRNA in extracts of the leaves, flowers, fruit or other tissues of a transgenic plant into which the promoter/reporter gene have been introduced. An exemplary reporter gene is GUS.

Optionally, the regulatory sequences include a 5' non-translated leader sequence or a polyadenylation signal or enhancers. The present invention further contemplates other regulatory sequences as known by those skilled in the art.

The invention also provides a transgenic plant cell transformed with a vector or combination of vectors of the present invention comprising polynucleotides of senescence-induced eIF-5A in sense or antisense orientation, a transgenic plantlet or mature transgenic plant generated from such a cell, or a plant part, such as a flower, fruit, leaves, seeds, etc. of the transgenic plant.

The present invention also provides methods of inhibiting expression of endogenous senescence-induced eIF-5A. These methods comprise integrating into the genome of at least one cell of a plant, expression vectors of the present invention comprising antisense polynucleotides of senescence-induced eIF-5A. The antisense polynucleotides of senescence-induced "eIF-5A are transcribed and inhibit expression of endogenous senescence-induced eIF-5A.

In another method of inhibiting expression of endogenous senescence-induced eIF-5A, an expression vector containing a senescence-induced eIF-5A polynucleotide of the present invention in a sense orientation is integrated into the genome of at least one cell of a plant. The polynucleotide of senescence-induced eIF-5A is transcribed and the resulting co-expression of exogenous senescence-induced eIF-5A causes a down-regulation or inhibition of expression of endogenous senescence-induced eIF-5A.

### Stress-induced eIF-5A

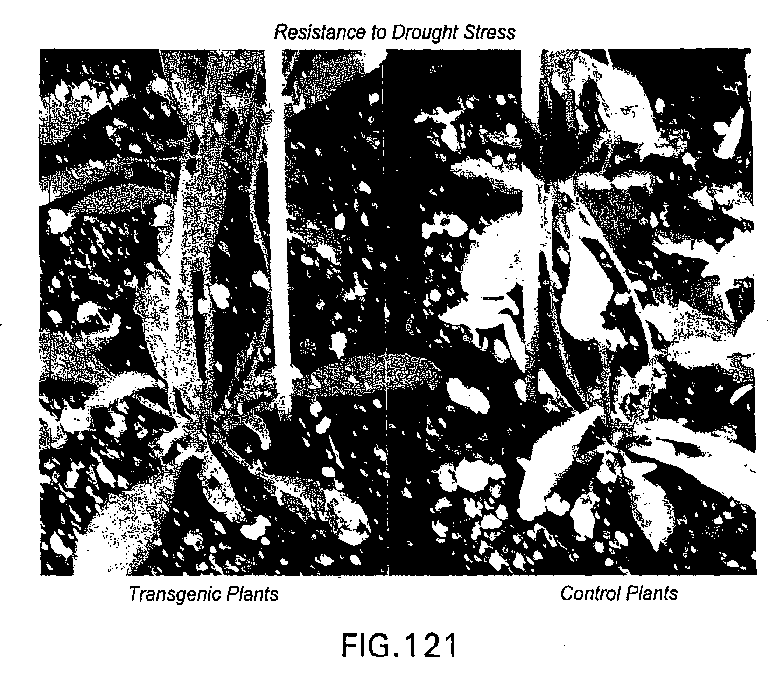

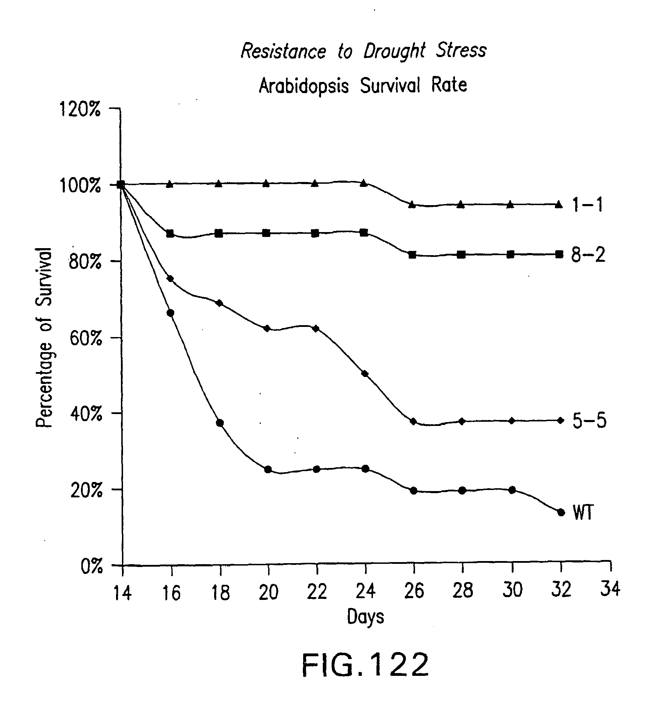

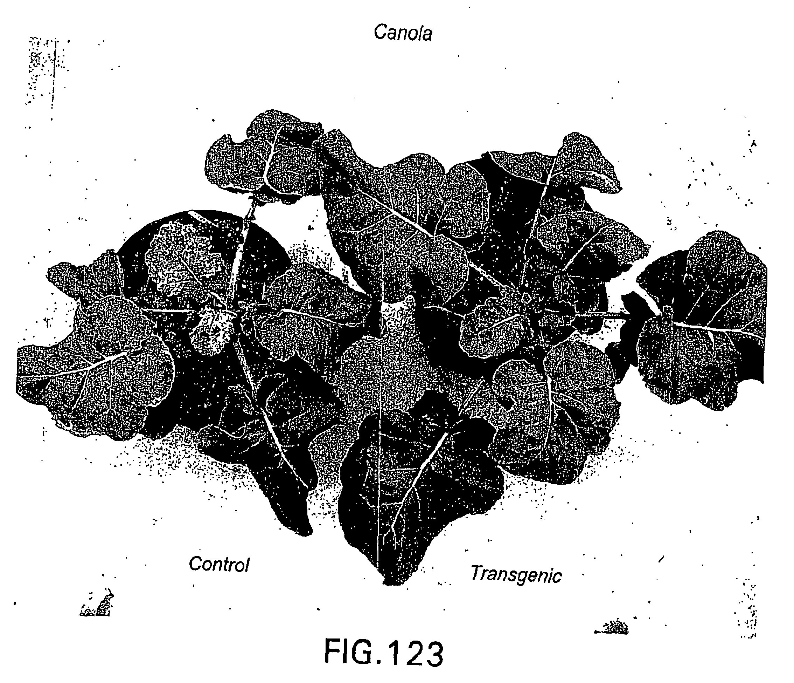

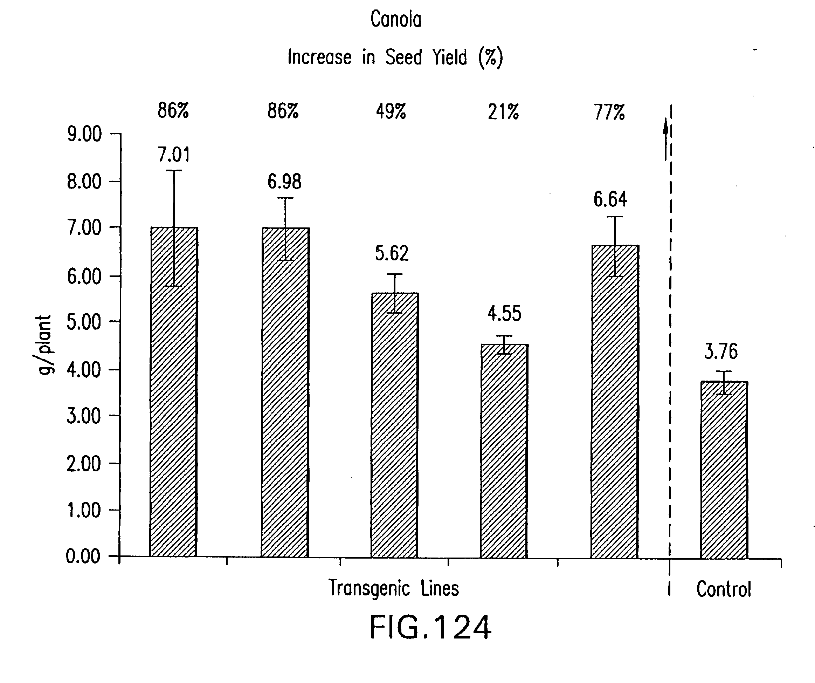

The present invention relates to the discovery of stress-induced eIf-5A in various plants including alfalfa, tomato and lettuce. Down regulating the stress isoform of eIF-5A ameliorates stress-induced senescence. Down regulation of stress-isoform eIF-5A provides an increase tolerance to stress and results in enhanced growth. Figures 121-123 shows that transgenic plants (having a reduced expression of stress-induced eIF-5A) show an increased resistance to drought. Figure 124 shows that transgenic plants (having a reduced expression of stress-induced eIF-5A) have an increased seed yield over control (wild-type) plants.

Thus, one embodiment of the present invention is isolated stress eIF-5A from alfalfa. The amino acid sequence is provided in Figure 139 and is SEQ ID NO: _. The polynucleotide encoding the amino acid is provided in Figure 139 and is SEQ ID NO: _.

Thus, one embodiment of the present invention is isolated stress eIF-5A from tomato. The amino acid sequence is provided in Figure 146 and is SEQ ID NO: _. The polynucleotide encoding the amino acid is provided in Figure 146 and is SEQ ID NO: _.

The present invention also provides isolated polynucleotides of stress eIF-5A that have 65-100% sequence homology to the above enumerated SEQ ID NOs, and hybridize under high stringency conditions to the complement of the enumerated SEQ ID NOs and which encode stress eIF-5A.

The present invention also provides stress eIF-SA isolated polypeptides of stress eIF-5A that have 65-100% amino acid sequence homology to the above enumerated SEQ ID NOs.

The present invention also provides antisense polynucleotides of the stress eIF-5As. The antisense polynucleotides may be of any length as long as they are able to inhibit expression. In some embodiments the antisense polynucleotides comprise the full length coding sequence and in other particularly preferred embodiments the antisense polynucleotides are directed at the 3'UTR since the different isoforms of eIF-5A have a higher degree of variation at the 3'UTR. In some embodiments the antisense polynucleotides are directed at the 5'- non-coding sequence. Antisense polynucleotides primarily complementary to 5'-non-coding sequences are known to be effective inhibitors of expression of genes encoding transcription factors. Branch, M.A., Molec. Cell Biol., 13:4284-4290 (1993).

The term "antisense polynucleotide of stress eIF5A" as used herein and in the claims encompasses not only those antisense polynucleotides that share 100 % homology of the complement of an enumerated SEQ ID NO but also includes those antisense polynucleotides that are a functional variants. Functional variants are those variants, either natural or man made, that have at least 80% sequence homology to and hybridizes under high stringency conditions with the corresponding portion of the stress eIF-5A. Further the variant must have the function as intended by the present invention, that is it is capable of modulating expression of endogenous stress eIF-5A when introduced into an expression vector and wherein such vector is incorporated into the genome of at least one plant cell. One skilled in the art can appreciate that insubstantial changes can be made in the sequence that would not detrimentally effect the ability of the antisense polynucleotide to bind to the transcript and reduce or inhibition expression of the gene. Thus, the term "antisense polynucleotide" encompasses those polynucleotides that are substantially complementary to the transcript and that still maintain the ability to specifically bind to the transcript and inhibit or reduce gene expression. For a general discussion of antisense *see* Alberts, et al., Molecular Biology of the Cell, 2nd ed., Garland Publishing, Inc. New York, New York ,1989 (in particular pages 195-196, incorporated herein by reference).

One embodiment of the present invention provides expression vectors comprising either the stress eIF-5A polynucleotides (of the present invention as described above) or antisense polynucleotides of stress eIF-5A (of the present invention as described above). Vectors can be plasmids, preferably, or may be viral or other vectors known in the art to replicate and express genes encoded thereon in plant cells or bacterial cells. The vector becomes chromosomally integrated such that it can be transcribed to produce the desired antisense polynucleotide of stress eIF-5A RNA. Such plasmid or viral vectors can be constructed by recombinant DNA technology methods that are standard in the art. For example, the vector may be a plasmid vector containing a replication system functional in a prokaryotic host and an antisense polynucleotide according to the invention. Alternatively, the vector may be a plasmid containing a replication system functional in *Agrobacterium* and an antisense polynucleotide according to the invention. Plasmids that are capable of replicating in *Agrobacterium* are well known in the art. See, Miki, et al., Procedures for Introducing Foreign DNA Into Plants, Methods in Plant Molecular Biology and Biotechnology,, Eds. B.R. Glick and J.E. Thompson. CRC Press (1993), PP. 67-83.

The vector further comprises regulatory sequences and promoter regulatory elements as discussed above.

The invention also provides a transgenic plant cell transformed with a vector or combination of vectors of the present invention comprising polynucleotides of stress eIF-5A in sense or antisense orientation, a transgenic plantlet or mature transgenic plant generated from such a cell, or a plant part, such as a flower, fruit, leaves, seeds, etc. of the transgenic plant.

The present invention also provides methods of inhibiting expression of endogenous stress eIF-5A. These methods comprise integrating into the genome of at least one cell of a plant, expression vectors of the present invention comprising antisense polynucleotides of stress eIF-SA. The antisense polynucleotides of stress eIF-5A are transcribed and inhibit expression of endogenous stress eIF-5A.

In another method of inhibiting expression of endogenous stress eIF-5A, an expression vector containing a stress eIF-5A polynucleotide of the present invention in a sense orientation is integrated into the genome of at least one cell of a plant. The polynucleotide of stress eIF-5A is transcribed and the resulting co-expression of exogenous stress eIF-5A causes a down-regulation or inhibition of expression of endogenous stress eIF-5A.

### Wounding/pathogen-induced eIF-5A

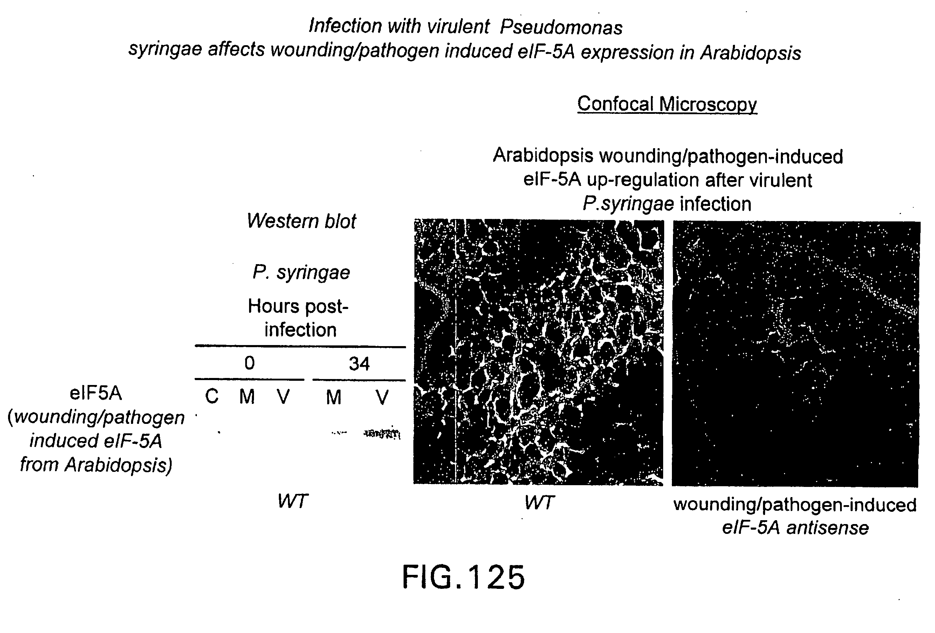

Wounding/pathogen induced eIF-5A (also referred to as eIF-5A3) is expressed in wounded tissues and infected tissues. The present invention relates to the discovery of wounding/pathogen induced eIF-5A in various plants including *Arabidopsis thaliana,* tomato, alfalfa and lettuce. The present inventors have discovered that this isoform is upregulated during a wounding event or pathogen ingression of the plant. Figure 125 indicates that wounding/pathogen induced eIF-5A is up-regulated after infection with *P. syringae.* The up-regulation occurs at the transcriptional level. Further, it is up-regulated exclusively at the protein level following virulent infection, which then gives rise to cell death, leading to the inference that wounding/pathogen induced eIF-5A is driving cell death in the event of ingression by pathogens.

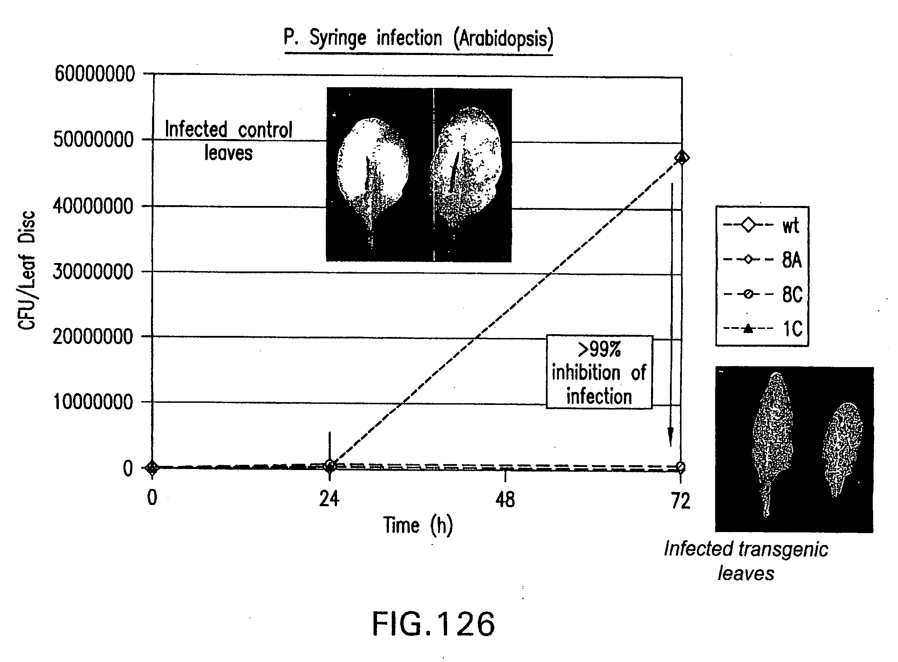

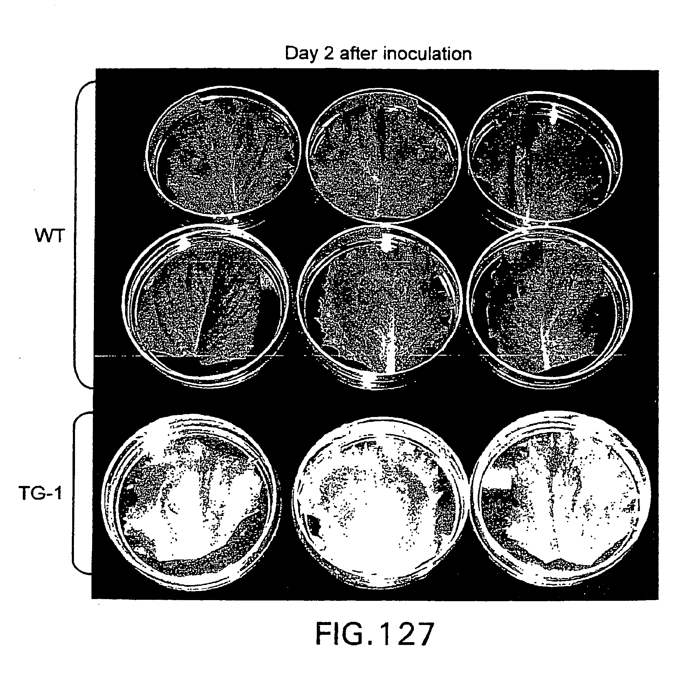

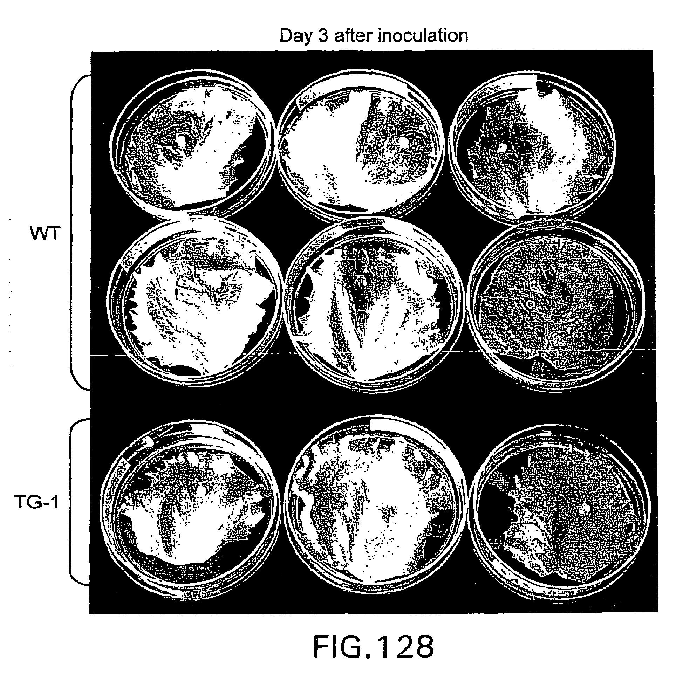

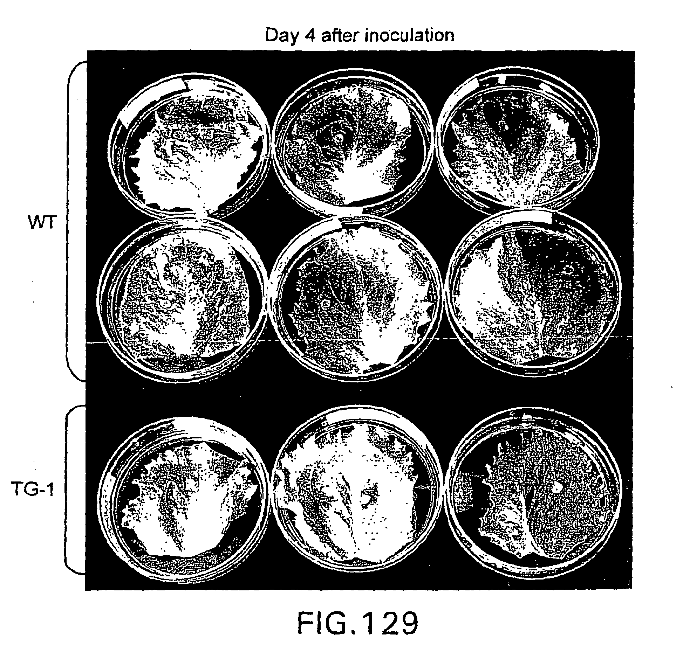

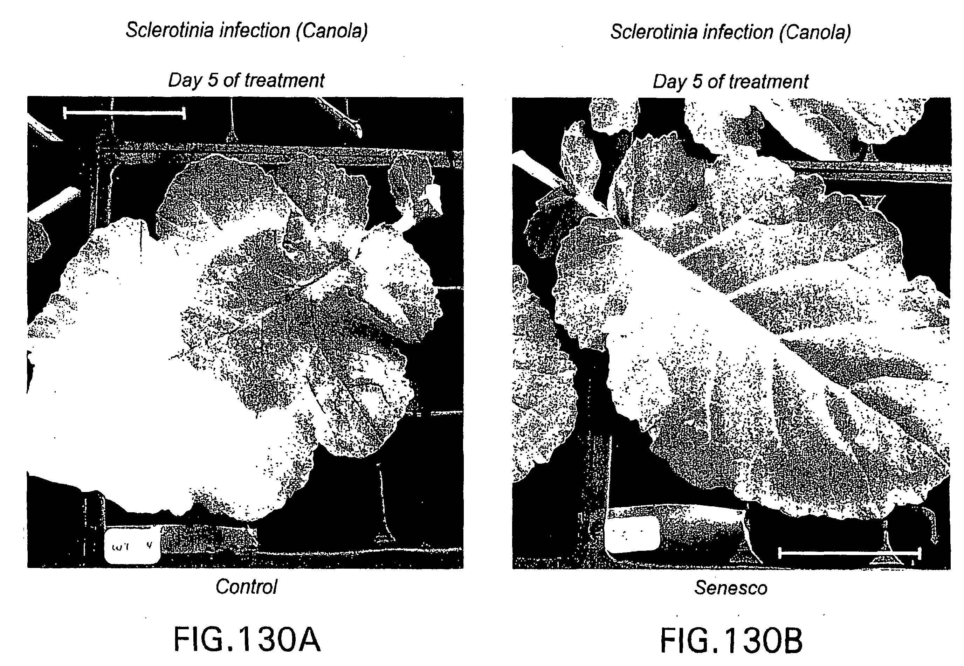

Down regulation of the wounding/pathogen-induced isoform of eIF-5A inhibits pathogen-induced cell death. Figure 126 shows that antisense suppression of wounding/pathogen-induced isoform of eIF-5A inhibits infection by virulent pathogens. Transgenic plants showed a greater than 99% increase in an inhibition of infection over control plants. Figures 127-130 show that transgenic canola (antisense /pathogen-induced isoform of eIF-5A) shows a resistance to infection by *Sclerotinia sclerotiorum.*

Wounding/pathogen induced eIF-5A regulates programmed cell death accompanying infection by necrotrophic bacterial and fungal pathogens. Within 72 h of infection with virulent Pseudomonas syringae pv Tomato DC 3000, wounding/pathogen induced eIF-5A protein is strongly up-regulated in the leaves of Arabidopsis wild-type plants. The up-regulation coincides with visible symptoms of disease progression. Moreover, wounding/pathogen induced eIF-5A is the only eIF5A isoform to show increased expression following infection. In addition, the up-regulation appears to be post-transcriptionally regulated. To further test the role of wounding/pathogen induced eIF-5A in disease development, transgenic Arabidopsis plants with constitutively suppressed wounding/pathogen induced eIF-5A expression were developed using antisense T-DNA insertions. The suppressed plants showed marked resistance to virulent *Pseudomonas syringae* pv Tomato DC 3000, with some lines exhibiting a 99 % decrease in bacterial load, relative to wild-type plants. This suppression of pathogen growth correlated with the absence of disease symptoms. The same lines also showed strong resistance to infection by the necrotrophic fungal pathogen, *Sclerotinia sclerotiorum.* The results have been interpreted as indicating that wounding/pathogen induced eIF-5A regulates programmed cell death induced by necrotrophic pathogens, and that suppression of wounding/pathogen induced eIF-5A inhibits host cell death upon infection and the ensuing release of nutrients that normally support growth of the pathogen.

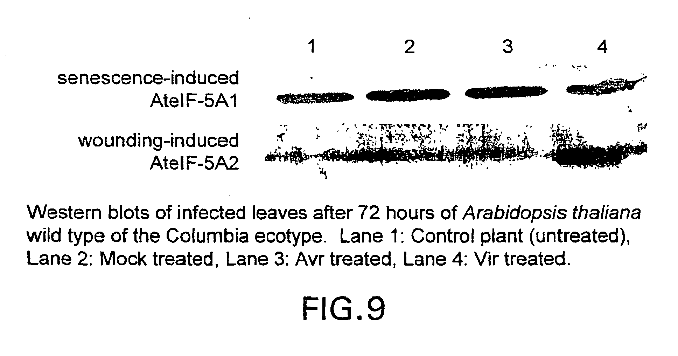

Figure 9 shows that senescence-induced eIF-5A remains constant in the control plant, the mock treated plant, the Avr treated plant and the Vir treated plant (it is detected as the plants were 4 weeks old). But wounding/pathogen induced eIF-5A is up-regulated in the Vir treated plant.

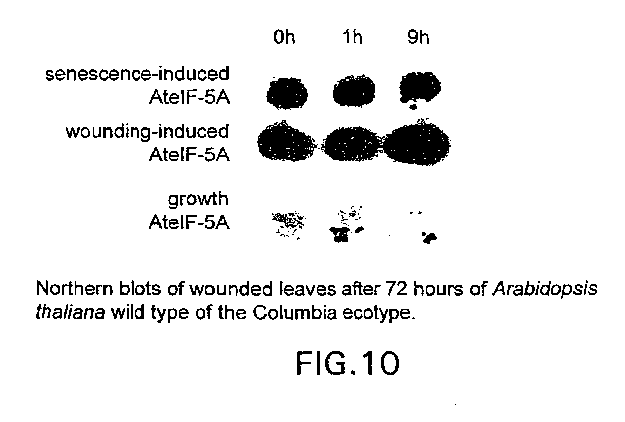

Figure 10 shows the results of an experiment where leaves of a plant were wounded with a hemostat. Levels of senescence-induced eIF-5A, wounding/pathogen induced eIF-5A and growth eIF-5A in *arabidopsis thaliana* ("At") were measured immediately after the wounding, 1 hour, and 9 hours after the wounding. The Northern Blots show that senescence-induced eIF-5A remained constant, but there was a noticeable increase in the levels expression of the wounding/pathogen induced eIF-5A. The levels of expression of the growth eIF-5A began to decrease in the event of wounding.

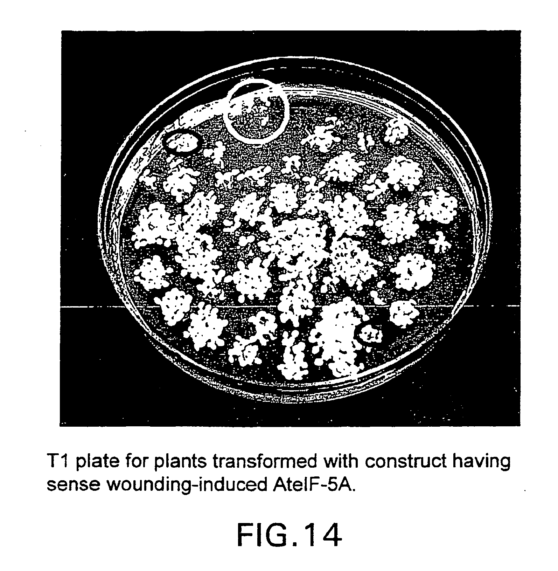

The present inventors have demonstrated that when wounding/pathogen induced eIF-5A is up-regulated and a wounding event is imposed upon the plants (such as occurs when the seedlings are transplanted), this wounding results in a very strong suppression of growth eIF-5A. See figures 14-17. The resulting plants have very stunted growth. But when the seeds are soaked in kanomycin and are planted directly into the soil (no need to transplant and thus no transplant wounding), the seeds develop into normal sized plants.

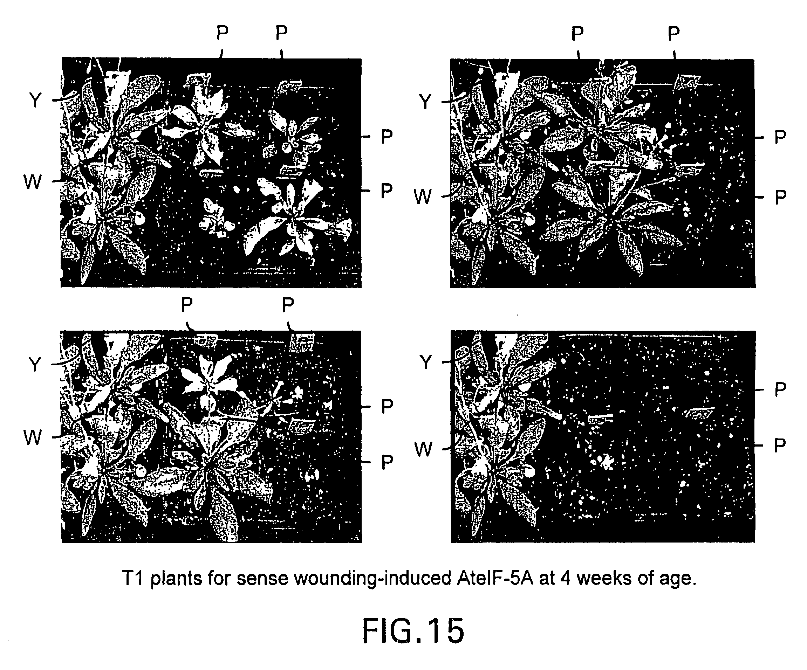

The differences seen between the various test plants all having a sense wounding/pathogen induced eIF-5A construct (figure 15) incorporated is due to varying degrees of expression of the wounding/pathogen induced eIF-5A. One skilled in the art will appreciate that when a gene is introduced (either sense or antisense) one gets varying degrees of either gene up-regulation or down-regulation. The degree of differences depends on where the gene gets incorporated and how many copies get incorporated. By having varying degrees of expression, one can correlate the various phenotypes to the gene expression. Once the desired phenotype is produced, that plant can be picked and used to create the desired progeny. Thus in figure 15, the plants that were strongly up-regulated for wounding/pathogen induced eIF-5A barely grew after the wounding event (plant tag 10), but the plants that grew a little better (but not as good as wild type) (plant tag 4) were not as strongly up-regulated.

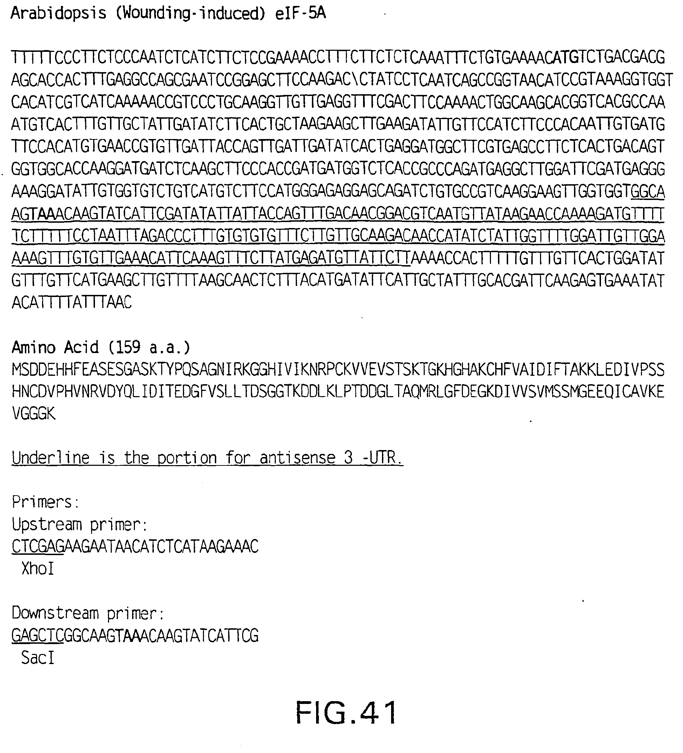

One embodiment of the present invention is isolated wounding/pathogen induced eIF-5A from *Arabidopsis thaliana.* The amino acid sequence is provided in Figure 41 and is SEQ ID NO: _. The polynucleotide encoding the amino acid is provided in Figure 41 and is SEQ ID NO: _.

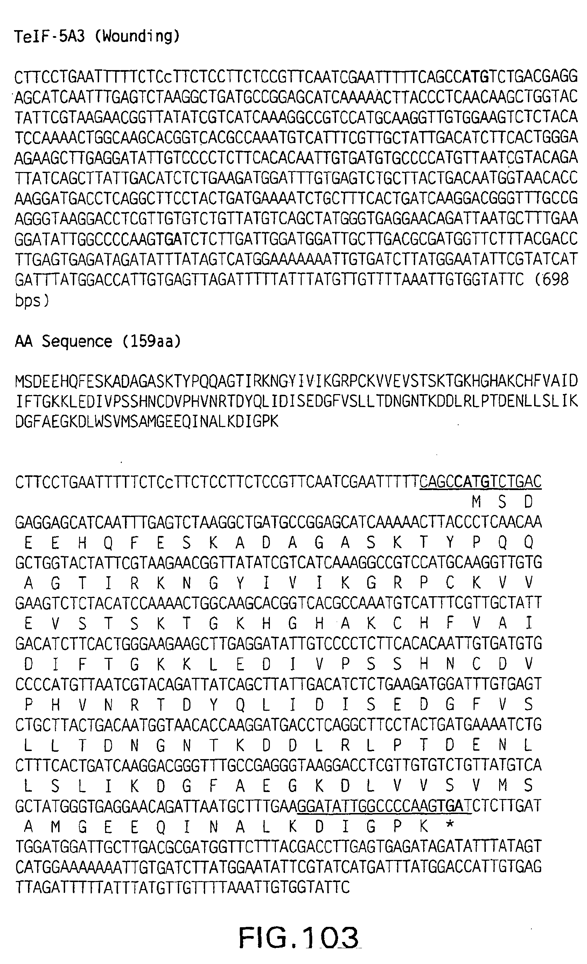

Another embodiment of the present invention is isolated wounding/pathogen induced eIF-5A from tomato. The amino acid sequence is provided in Figure 103 and is SEQ ID NO: _. The polynucleotide encoding the amino acid is provided in Figure 103 and is SEQ ID NO: _.

Another embodiment of the present invention is isolated wounding/pathogen induced eIF-5A from alfalfa. The amino acid sequence is provided in Figure 137 and is SEQ ID NO: _. The polynucleotide encoding the amino acid is provided in Figure 137 and is SEQ ID NO: __.

The present invention also provides isolated polynucleotides of wounding/pathogen induced eIF-5A that have 65-100% sequence homology to the above enumerated SEQ ID NOs, and hybridize under high stringency conditions to the complement of the enumerated SEQ ID NOs and which encode wounding/pathogen induced eIF-5A.

The present invention also provides wounding/pathogen induced eIF-5A isolated polypeptides of wounding/pathogen induced eIF-5A that have 65-100% amino acid sequence homology to the above enumerated SEQ ID NOs.

The present invention also provides antisense polynucleotides of the wounding/pathogen induced eIF-5As. The antisense polynucleotides may be of any length as long as they are able to inhibit expression. In some embodiments the antisense polynucleotides comprise the full length coding sequence and in other particularly preferred embodiments the antisense polynucleotides are directed at the 3'UTR since the different isoforms of eIF-5A have a higher degree of variation at the 3'UTR. In some embodiments the antisense polynucleotides are directed at the 5'- non-coding sequence. Antisense polynucleotides primarily complementary to 5'-non-coding sequences are known to be effective inhibitors of expression of genes encoding transcription factors. Branch, M.A., Molec. Cell Biol., 13:4284-4290 (1993).

The term "antisense polynucleotide of wounding/pathogen induced eIF5A" as used herein and in the claims encompasses not only those antisense polynucleotides that share 100 % homology of the complement of an enumerated SEQ ID NO but also includes those antisense polynucleotides that are a functional variants. Functional variants are those variants, either natural or man made, that have at least 80% sequence homology to and hybridizes under high stringency conditions with the corresponding portion of the wounding/pathogen induced eIF-5A. Further the variant must have the function as intended by the present invention, that is it is capable of modulating expression of endogenous wounding/pathogen induced eIF-5A when introduced into an expression vector and wherein such vector is incorporated into the genome of at least one plant cell. One skilled in the art can appreciate that insubstantial changes can be made in the sequence that would not detrimentally effect the ability of the antisense polynucleotide to bind to the transcript and reduce or inhibition expression of the gene. Thus, the term "antisense polynucleotide" encompasses those polynucleotides that are substantially complementary to the transcript and that still maintain the ability to specifically bind to the transcript and inhibit or reduce gene expression.

One embodiment of the present invention provides expression vectors comprising either the wounding/pathogen induced eIF-5A polynucleotides (of the present invention as described above) or antisense polynucleotides of wounding/pathogen induced eIF-5A (of the present invention as described above). Vectors, regulatory sequences and promoter regulatory elements are as discussed above.

The invention also provides a transgenic plant cell transformed with a vector or combination of vectors of the present invention comprising polynucleotides of wounding/pathogen induced eIF-5A in sense or antisense orientation, a transgenic plantlet or mature transgenic plant generated from such a cell, or a plant part, such as a flower, fruit, leaves, seeds, etc. of the transgenic plant.

The present invention also provides methods of inhibiting expression of endogenous wounding/pathogen induced eIF-5A. These methods comprise integrating into the genome of at least one cell of a plant, expression vectors of the present invention comprising antisense polynucleotides of wounding/pathogen induced eIF-5A. The antisense polynucleotides of wounding/pathogen induced eIF-5A are transcribed and inhibit expression of endogenous wounding/pathogen induced eIF-5A.

In another method of inhibiting expression of endogenous wounding/pathogen induced eIF-5A, an expression vector containing a wounding/pathogen induced eIF-5A polynucleotide of the present invention in a sense orientation is integrated into the genome of at least one cell of a plant. The polynucleotide of wounding/pathogen induced eIF-5A is transcribed and the resulting co-expression of exogenous wounding/pathogen induced eIF-5A causes a down-regulation or inhibition of expression of endogenous wounding/pathogen induced eIF-5A.

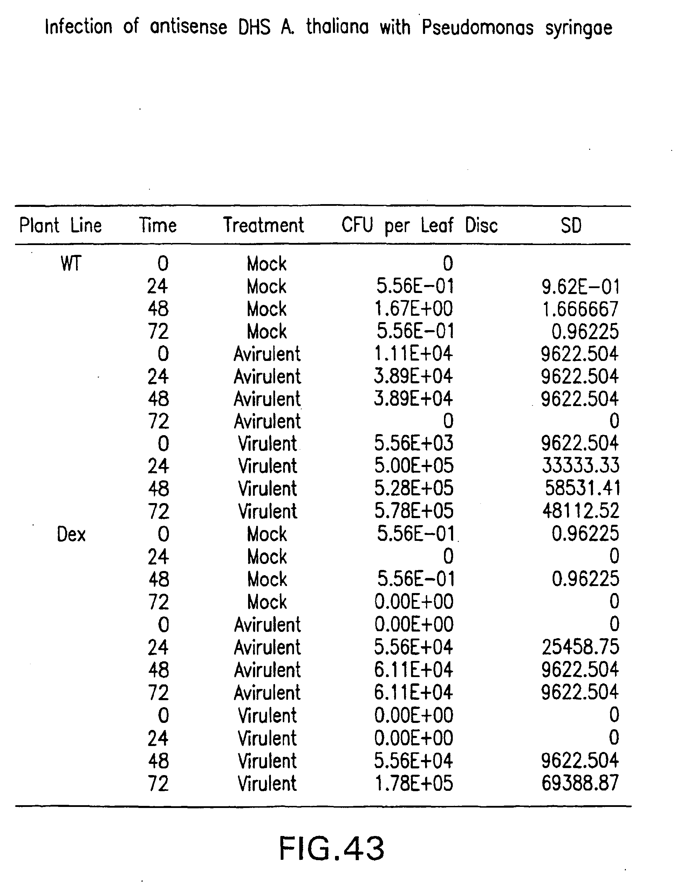

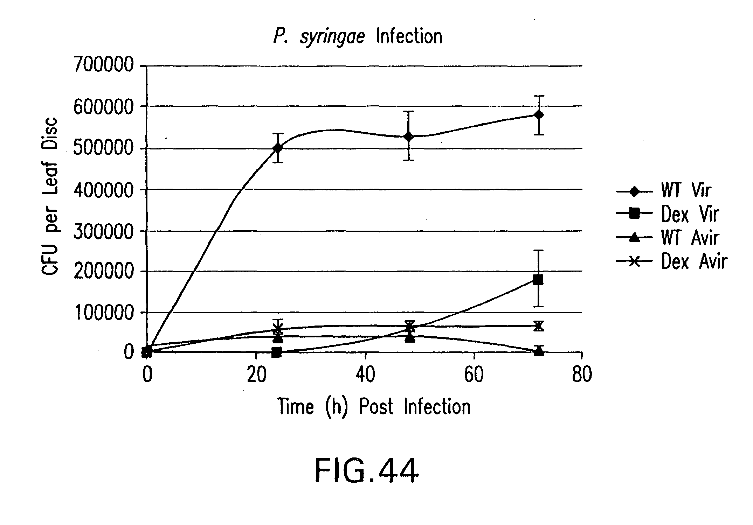

By inhibiting expression of endogenous wounding/pathogen induced eIF-5A, resulting transgenic plants have an increased resistance to virulent damage arising from pathogen ingression. Pathogens include, but are not limited to Black Sigatoka in bananas, *Sclerotinia sclerotiorum* (a major fungal disease of lettuce and turf grass) Pythium (a major fungal disease in green house vegetable crops) and *Pseudomonas syringae.* See example 16 and figures 43 and 44.

### Growth eIF-5A

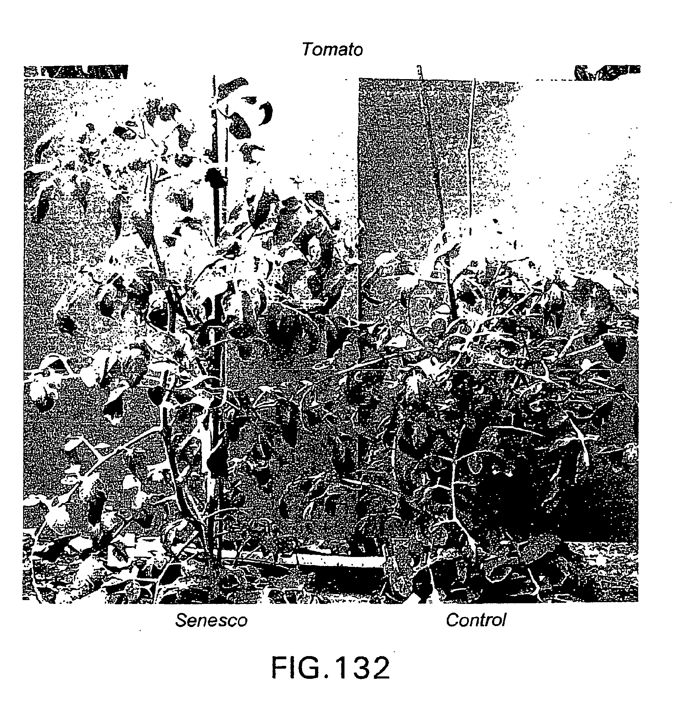

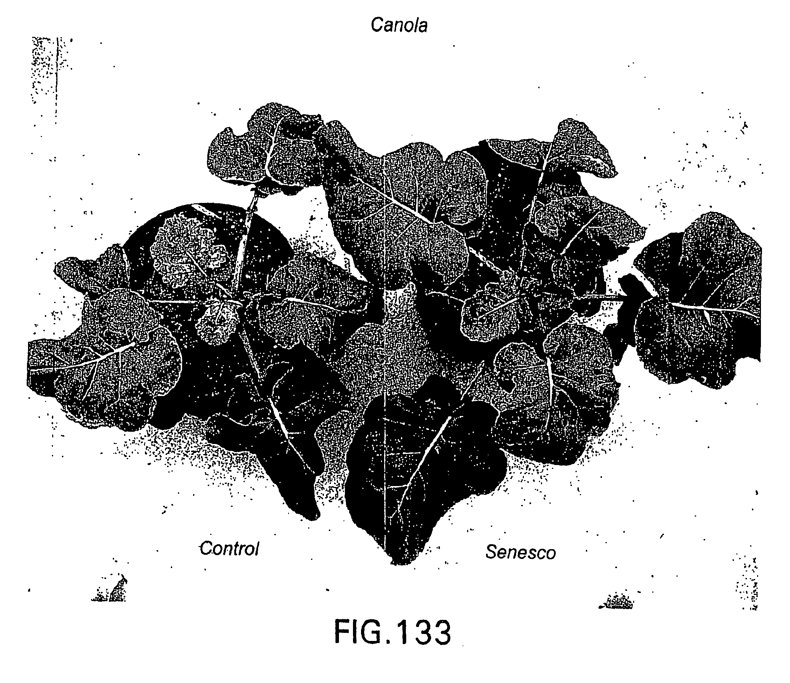

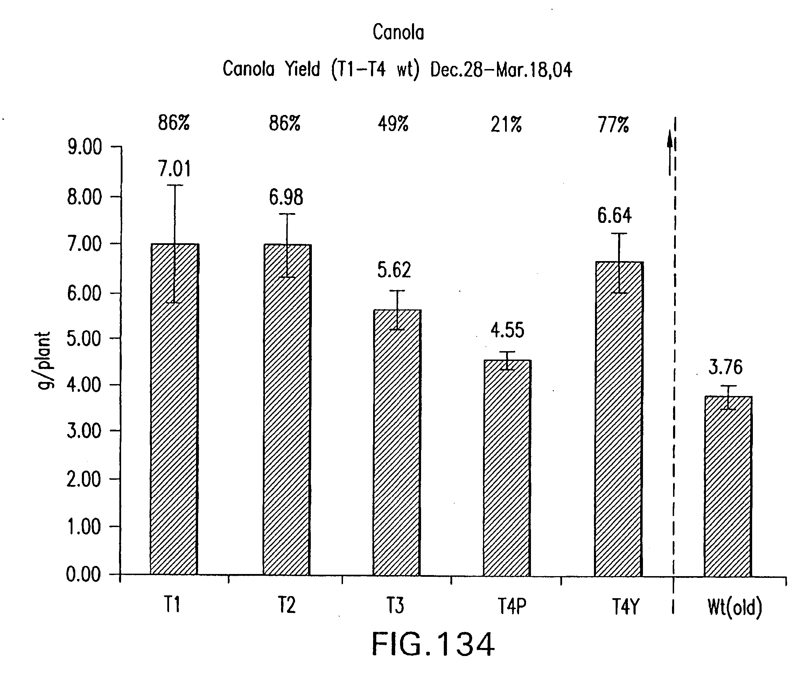

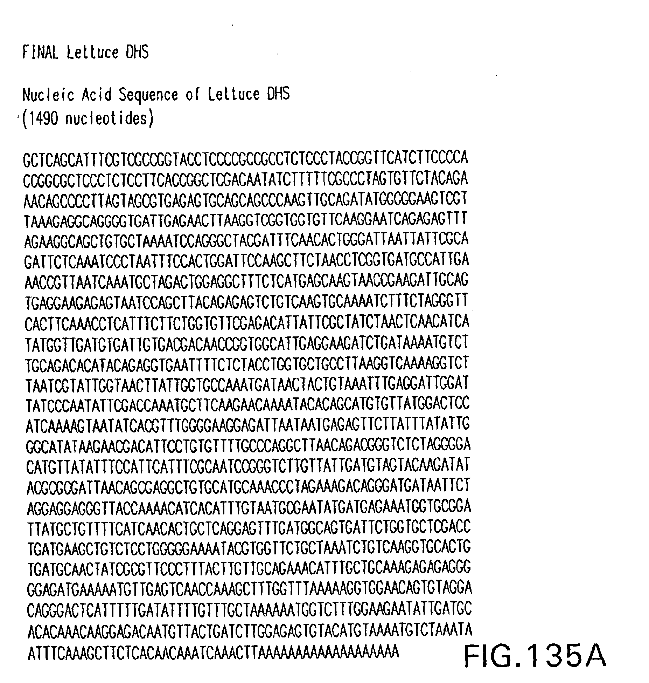

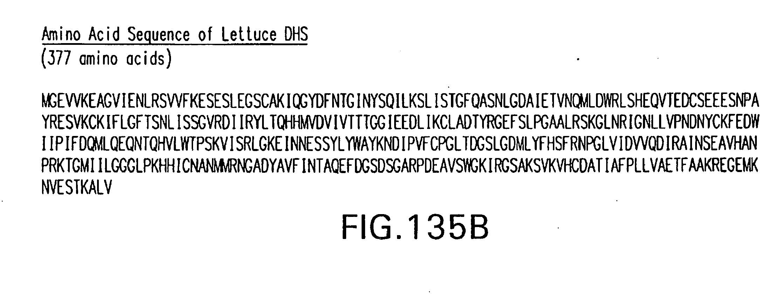

The present invention also relates to growth eIF-5A. Growth eIF-5A is expressed in growing tissues. When eIF-5A is up-regulated with polynucleotides of growth eIF-5A in sense orientation, three phenotypic changes are noticed: increased seed size, increased biomass, and increased seed yield. See Figures 132-134.

One embodiment of the present invention is isolated growth eIF-5A from *Arabidopsis thaliana.* The amino acid sequence is provided in Figure 1 and is SEQ ID NO: _. The polynucleotide encoding the amino acid is provided in Figure 2 and is SEQ ID NO:__.

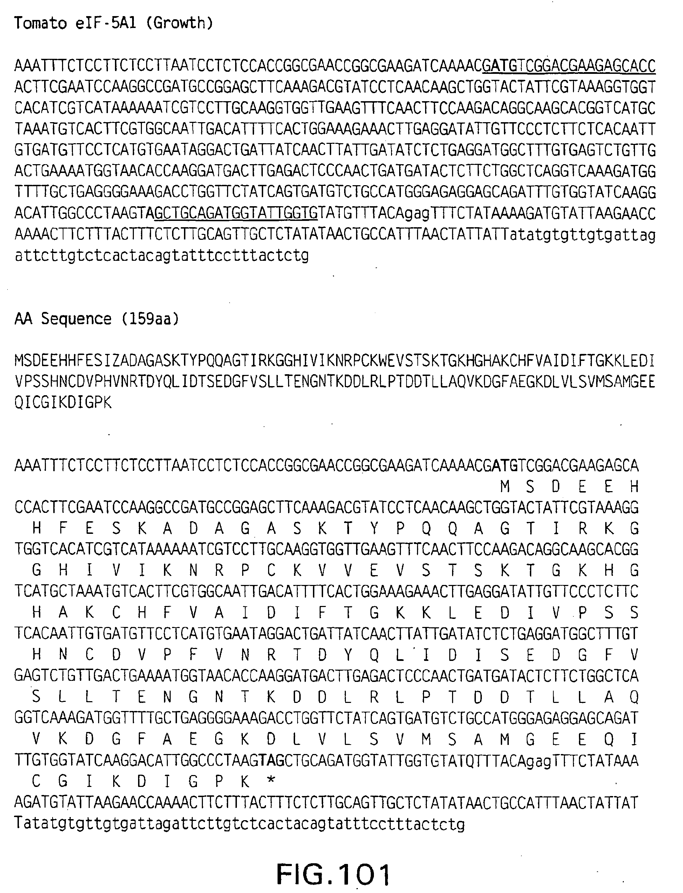

Another embodiment of the present invention is isolated growth eIF-5A from tomato. The amino acid sequence is provided in Figure 101 and is SEQ ID NO: _. The polynucleotide encoding the amino acid is provided in Figure 101 and is SEQ ID NO: _.

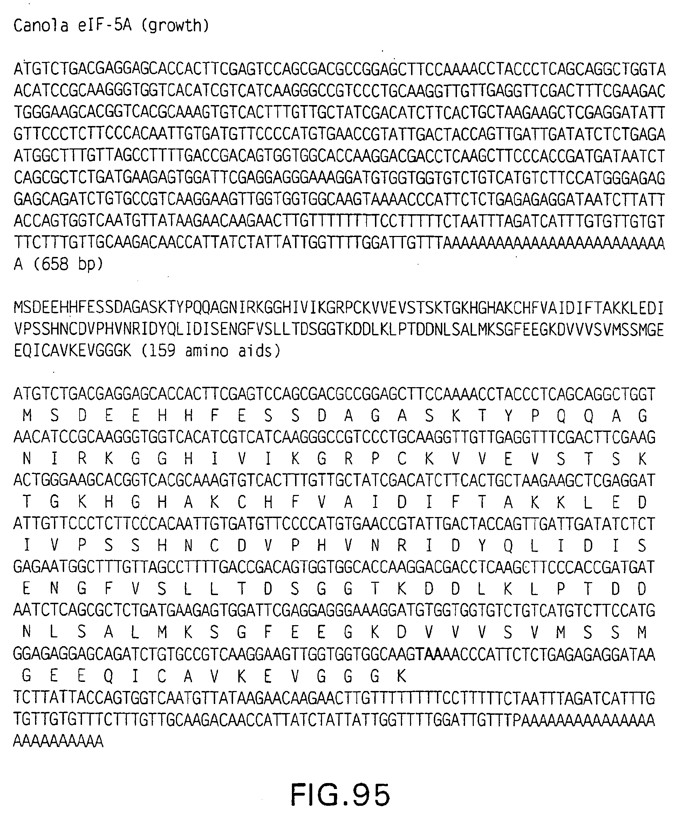

Another embodiment of the present invention is isolated growth eIF-5A from canola The amino acid sequence is provided in Figure 95 and is SEQ ID NO: _. The polynucleotide encoding the amino acid is provided in Figure 95 and is SEQ ID NO:_.

Another embodiment of the present invention is isolated growth eIF-5A from alfalfa. The amino acid sequence is provided in Figure 140 and is SEQ ID NO: _. The polynucleotide encoding the amino acid is provided in Figure 140 and is SEQ ID NO: _.

The present invention also provides isolated polynucleotides of growth eIF-5A that have 65-100% sequence homology to the above enumerated SEQ ID NOs, and hybridize under high stringency conditions to the complement of the enumerated SEQ ID NOs and which encode growth eIF-5A.

The present invention also provides isolated polypeptides of growth eIF-5A that have 65-100% amino acid sequence homology to the above enumerated SEQ ID NOs.

The present invention also provides antisense polynucleotides of the growth eIF-5As. The antisense polynucleotides may be of any length as long as they are able to inhibit expression. In some embodiments the antisense polynucleotides comprise the full length coding sequence and in other particularly preferred embodiments the antisense polynucleotides are directed at the 3'UTR since the different isoforms of eIF-5A have a higher degree of variation at the 3'UTR. In some embodiments the antisense polynucleotides are directed at the 5'- non-coding sequence. Antisense polynucleotides primarily complementary to 5'-non-coding sequences are known to be effective inhibitors of expression of genes encoding transcription factors. Branch, M.A., Molec. Cell Biol., 13:4284-4290 (1993).

The term "antisense polynucleotide of growth eIF5A" as used herein and in the claims encompasses not only those antisense polynucleotides that share 100 % homology of the complement of an enumerated SEQ ID NO but also includes those antisense polynucleotides that are a functional variants. Functional variants are those variants, either natural or man made, that have at least 80% sequence homology to and hybridizes under high stringency conditions with the corresponding portion of the growth eIF-5A. Further the variant must have the function as intended by the present invention, that is it is capable of modulating expression of endogenous growth eIF-5A when introduced into an expression vector and wherein such vector is incorporated into the genome of at least one plant cell. One skilled in the art can appreciate that insubstantial changes can be made in the sequence that would not detrimentally effect the ability of the antisense polynucleotide to bind to the transcript and reduce or inhibition expression of the gene. Thus, the term "antisense polynucleotide" encompasses those polynucleotides that are substantially complementary to the transcript and that still maintain the ability to specifically bind to the transcript and inhibit or reduce gene expression.

One embodiment of the present invention provides expression vectors comprising either the growth eIF-5A polynucleotides (of the present invention as described above) or antisense polynucleotides of growth eIF-5A (of the present invention as described above). Vectors, regulatory sequences and promoter regulatory elements are as discussed above.

The invention also provides a transgenic plant cell transformed with a vector or combination of vectors of the present invention comprising polynucleotides of growth eIF-5A in sense or antisense orientation, a transgenic plantlet or mature transgenic plant generated from such a cell, or a plant part, such as a flower, fruit, leaves, seeds, etc. of the transgenic plant.

The present invention also provides methods of inhibiting expression of endogenous growth eIF-5A. These methods comprise integrating into the genome of at least one cell of a plant, expression vectors of the present invention comprising antisense polynucleotides of growth eIF-5A. The antisense polynucleotides of growth eIF-5A are transcribed and inhibit expression of endogenous growth eIF-5A.

In another method of inhibiting expression of endogenous growth eIF-5A, an expression vector containing a growth eIF-5A polynucleotide of the present invention in a sense orientation is integrated into the genome of at least one cell of a plant. The polynucleotide of growth eIF-5A is transcribed and the resulting co-expression of exogenous growth eIF-5A causes a down-regulation or inhibition of expression of endogenous growth eIF-5A.

In another embodiment of the present invention there is provided a method of up-regulating expression of growth eIF-5A. An expression vector containing a growth eIF-5A polynucleotide of the present invention in a sense orientation is integrated into the genome of at least one cell of a plant. The polynucleotide of growth eIF-5A is transcribed and the resulting co-expression of exogenous growth eIF-5A causes the cells to express more growth eIF-5A than non-transgenic cells.

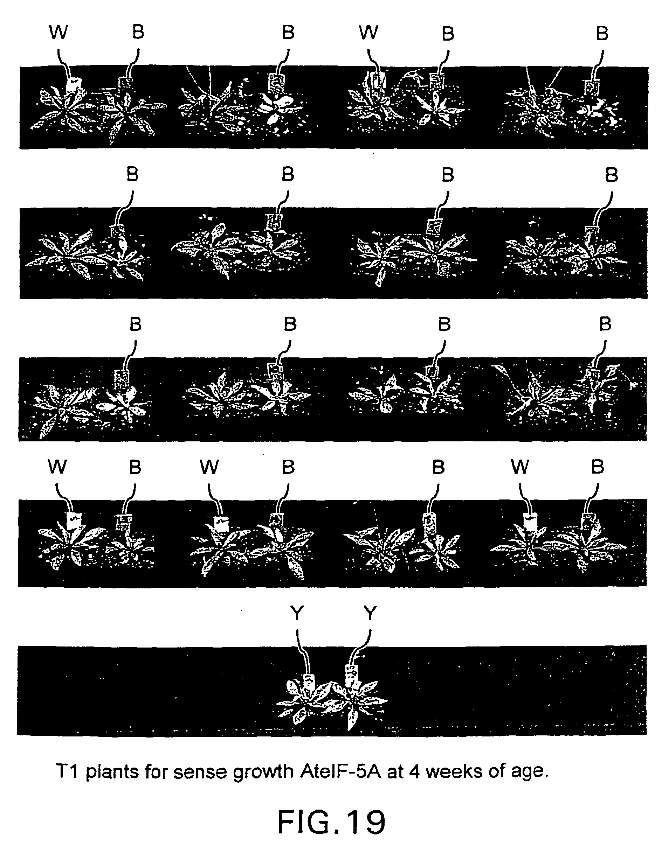

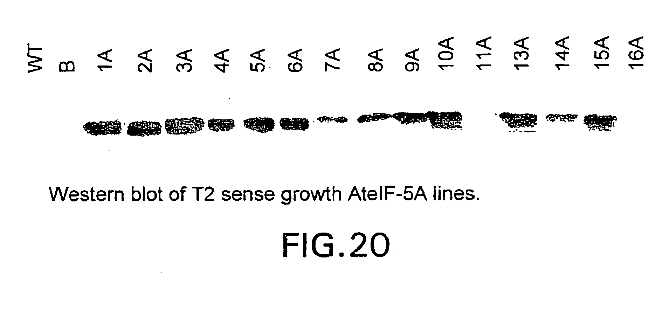

Figure 19 shows that plants that were up-regulated for growth eIF-5A had an increased biomass over that of the control plants. Growth eIF-5A was inserted into *Arabidopsis thaliana* plants in a sense orientation to up-regulate the expression of growth eIF-5A. Sixteen mother lines (1-16) were assayed to determine the general level of growth eIF-5A expression. From each mother line, 8 sister lines were produced (A-H). The level of expression of growth eIF-5A in each mother line was tested and the results shown in Figure 20. Various degrees of expression are noticed throughout the mother lines. For example, lines 2 and 10 have very high levels of expression whereas lines 11 and 16 have very low or no expression.

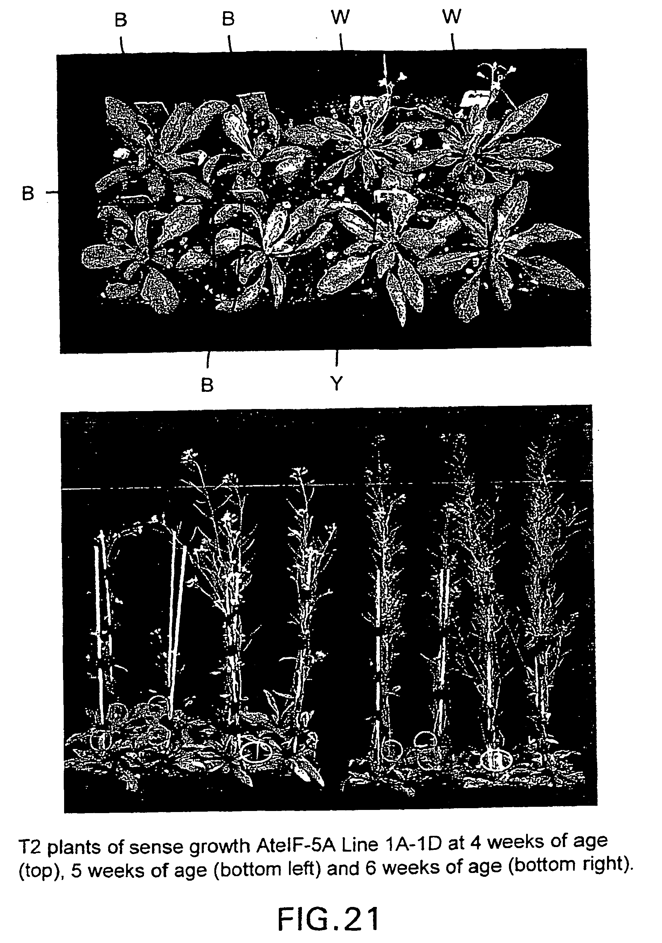

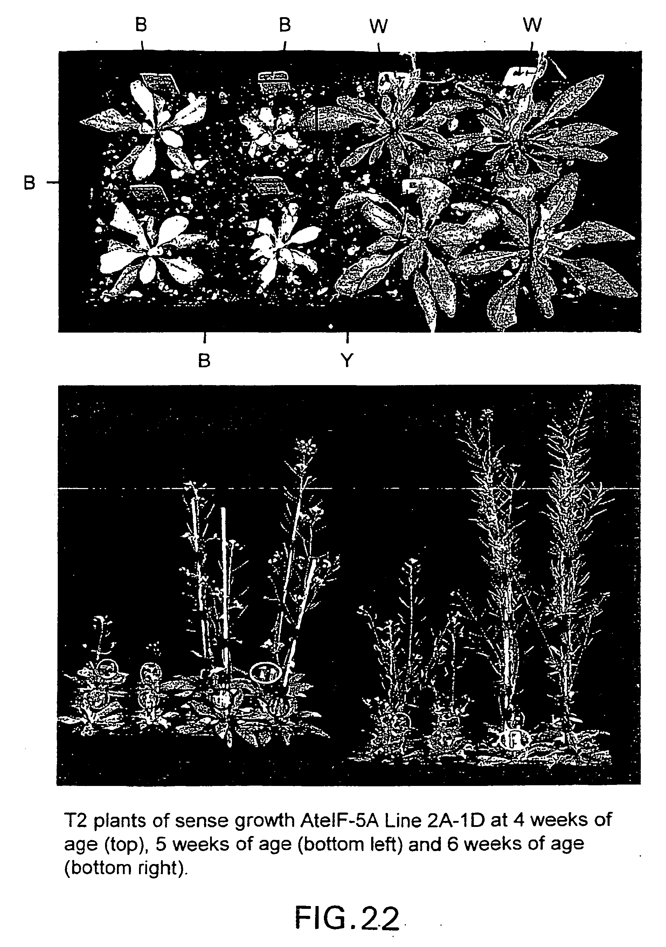

Figure 21 and 22 show the plants from lines 1 and 2. These plants are bigger than the control plants. Because the growth eIF-5A is a cell-division isoform and because it is constitutively expressed, there is increased cell division. A reduction in senescence occurs because the plant is locked into a growth mode and can not make the switch to the senescence pathway.

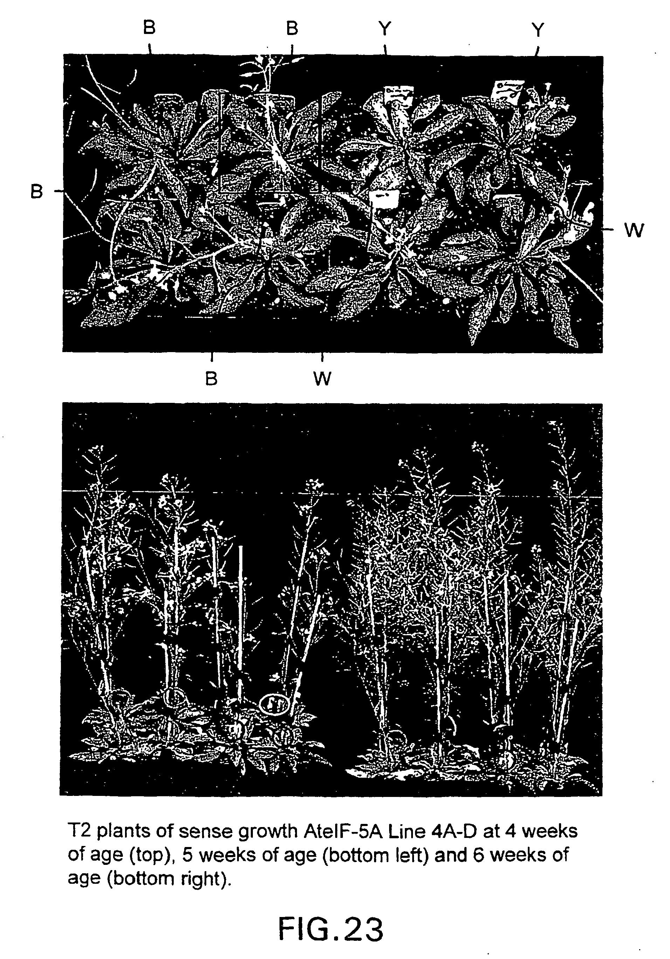

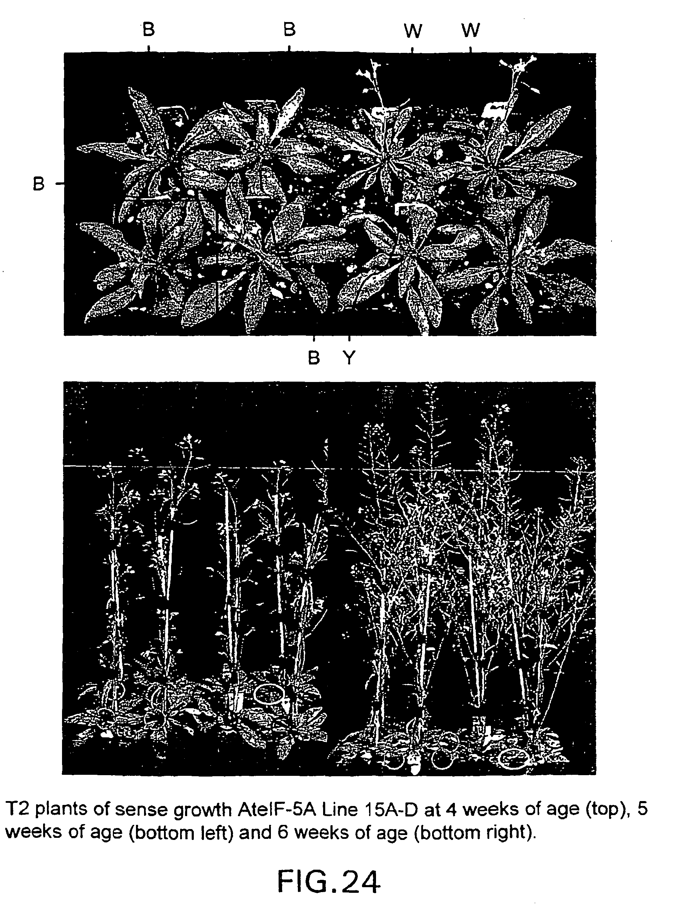

Figures 23 and 24 are from lines that had medium level of expression of growth eIF-5A. They appear to have bigger leaves and delayed senescence.

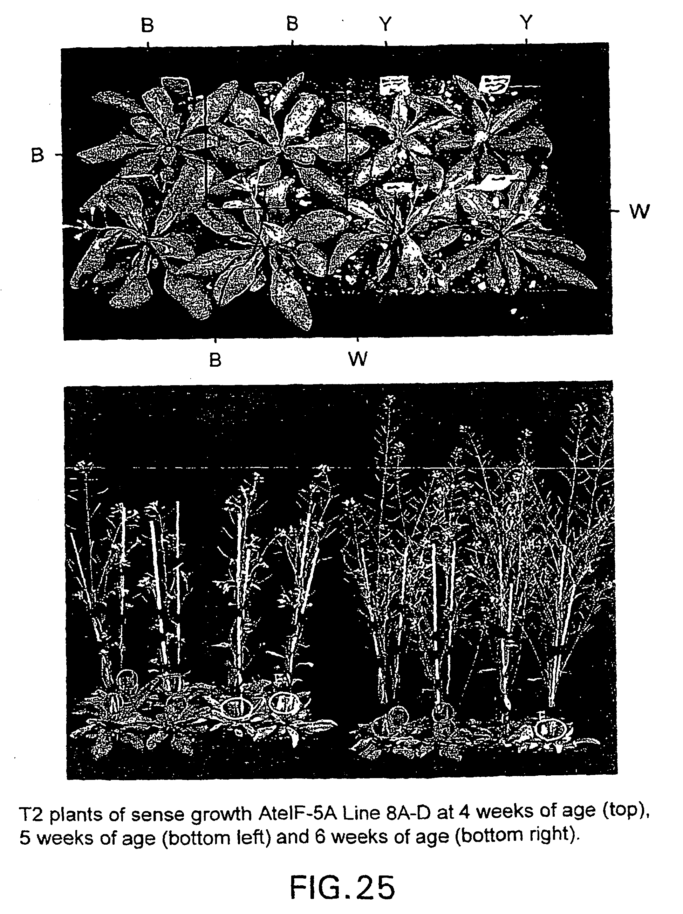

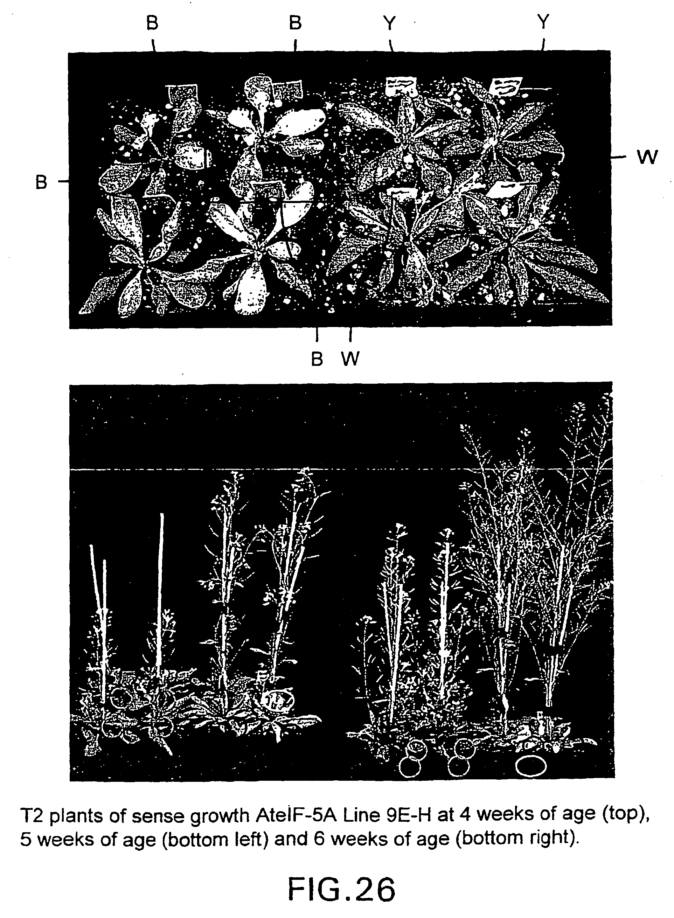

Figures 25 and 26 are from lines that had low levels of up-regulation. They have large leaves and large rosettes.

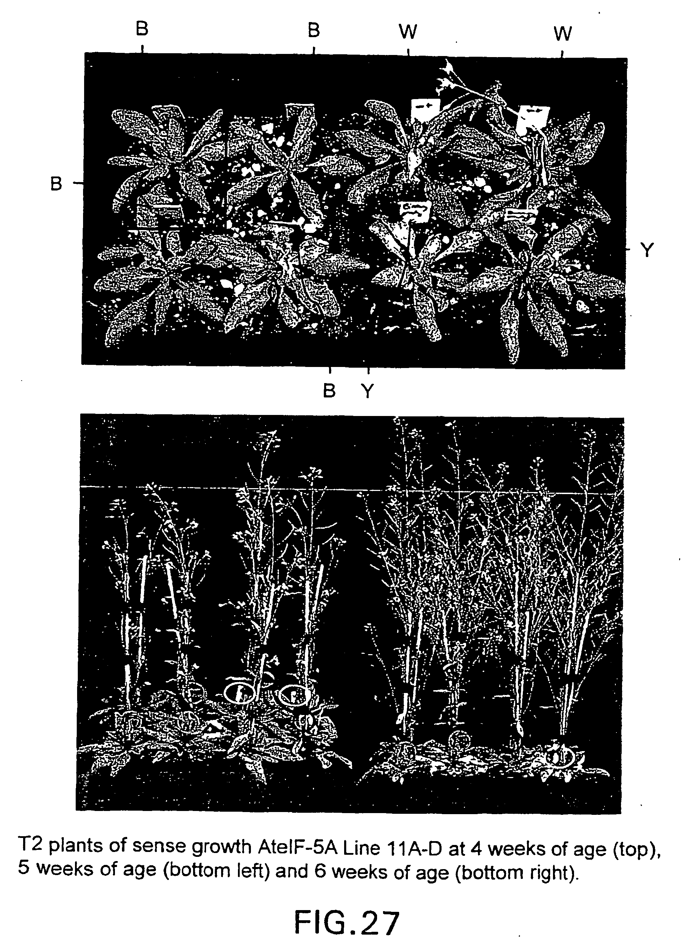

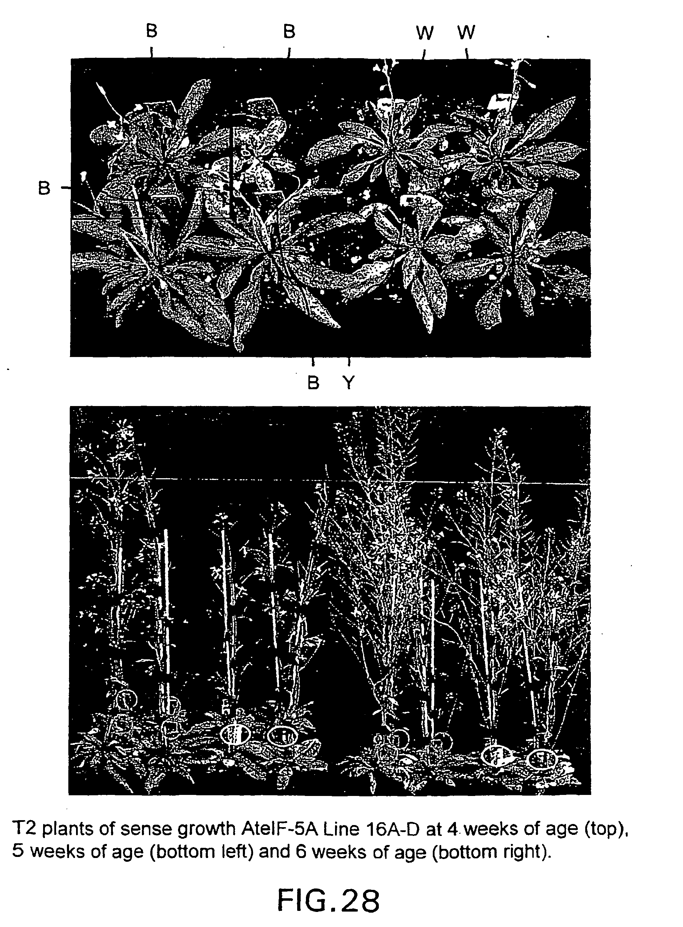

Figures 27 and 28 are from lines that have no up-regulation (which may be due to co-suppression of the gene). Since the plant is kanomycin resistant, the gene must be present in order for the plants to grow on the media. It appears that the senescence-induced eIF-5A is also co-suppressed as well thus giving rise to an increase in size.

In addition to increased biomass, there is also increased seed size in plants having growth eIF-5A up-regulated. The seed size of all of the lines was measured. In the lines having the highest levels of growth eIF-5A expression, a greater than 3x increase in seed size is seen. This occurs because up-regulation of growth eIF-5A, increases cell division and thus increases seed size.

The growth eIF-5A (from *Arabidopsis thaliana*) in the above examples was being constitutively expressed, i.e. is being expressed everywhere in the plant through the use of a universal promoter. In contrast, by using a tissue specific promoter, one may direct the up-regulation in particular tissues. For example, by using a seed specific promoter, the growth eIF-5A would only be up-regulated in the seed, allowing the leaves to grow normally, but produce an increase in the amount of seeds. Thus, using a specific promoter, the growth eIF-5A can be up-regulated in the desired plant part to get a desired phenotype.

By up-regulating growth eIF-5A, at least three phenotypes result - increased biomass, increased seed yield, or increased seed size, but not all three phenotypes are present at the same time (or in the same plant). For example, if a plant exhibits an increase in seed size, a smaller plant will be present. In the plant lines that had the highest up-regulation of growth eIF-5A, the biggest seeds were produced, but the plants were smaller because there was massive cell division going on throughout the whole plant, which was at the expense of cell enlargement (needed for bigger leaves). At lower levels of up-regulation of expression of growth AteIF-5A, one sees an impact on the leaves (bigger) without impacting the seed. Thus, one may use tissue specific expression and pick the phenotype desired. For example, one may place growth eIF-5A under a xylem specific promoter to achieve an increase in the amount of xylem produced. Thus, any desired promoter may be used to achieve the desired tissue-specific up-regulation.

Growth eIF-5A as well as the other isoforms of eIF-5A are highly conserved across plant species. Figure 85 shows an amino acid alignment of various eIF-5A isoforms from several plant species indicating that the hypusine region is very highly conserved. In fact, eIF-5A is so highly conserved that an isolated polynucleotide encoding one plant growth eIF-5A can be inserted into another plant species in sense orientation and achieve an up-regulation of growth eIF-5A. Figure 162 shows the effect of introducing cottonwood growth eIF-5A sense orientation into arabidopsis. Figure 162 shows T₁ plants at 7 weeks old. The larger plants are from two different transgenic lines. The smaller plants are the controls. Figure 163 shows T2 plants from three separate lines (labeled lines 2, 3 and 4) and the plants labeled "BIN" is the control empty vector. The plants are 28 days old. The transgenic arabidopsis have an increased growth rate as compared to the control plants. Figure 164 show T2 plants (Line 3) at 32 days old compared to control plants.

Further evidence of the interchangeability of growth eIF-5A, is shown in Figure 165. Growth factor eIF-5A was up-regulated in canola by with either canola growth factor eIF-5A or arabidopsis growth eIF-5A (full length) in the sense orientation under the control of a 35S promoter. The resulting transgenic plants had an increase in seed yield over the wild-type control plants. The increase in seed yield in the transgenic canola plant having an arabidopsis growth eIF-5A in the sense orientation and the transgenic canola plants having an exogenous canola growth eIF-5A in sense orientation is very similar.

### DHS

DHS is necessary for the activation of eIF-5A and is expressed in senescing tissues. The present invention provides isolated DHS from *Arabidopsis thaliana,* tomato, carnation, canola, lettuce, alfalfa, banana, cottonwood, yeast, petunia and mycosphaerella.

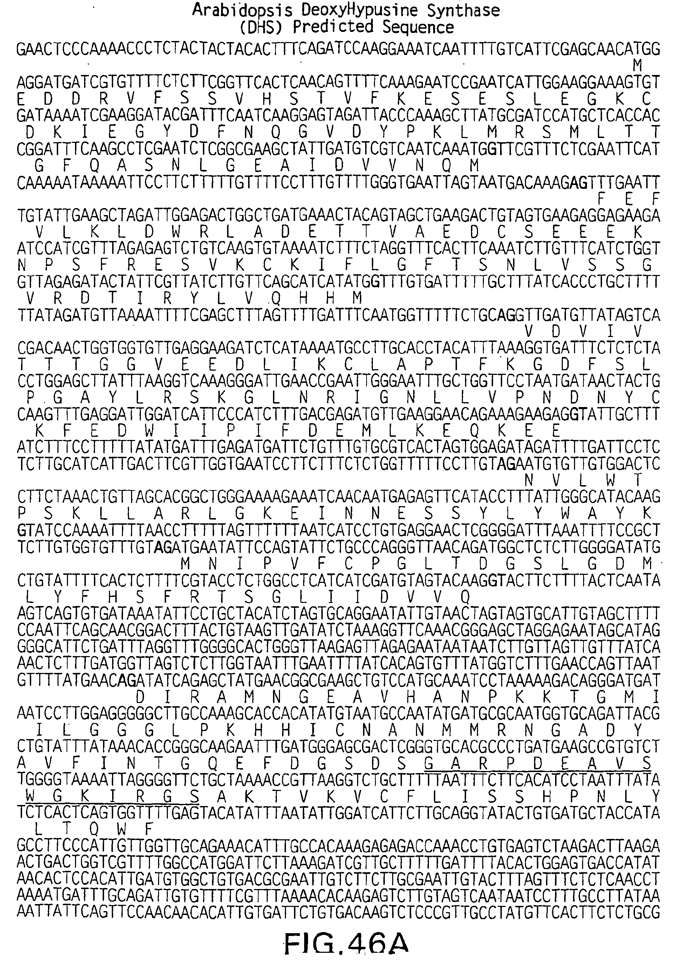

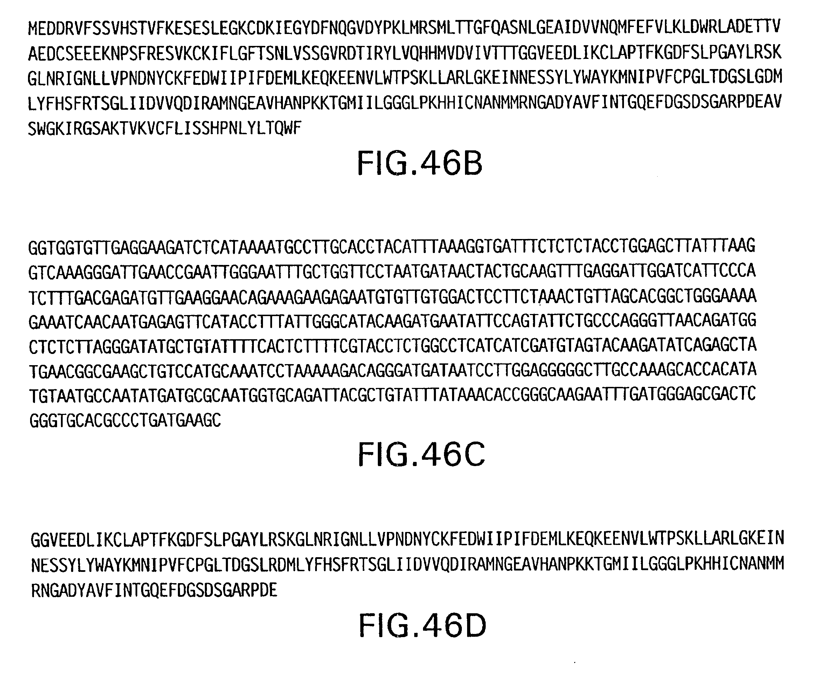

Thus one embodiment of the present invention is isolated DHS from *Arabidopsis thaliana.* The amino acid sequence is provided in Figure 46 and is SEQ ID NO:_. The polynucleotide encoding the amino acid is provided in Figure 46 and is SEQ ID NO:_.

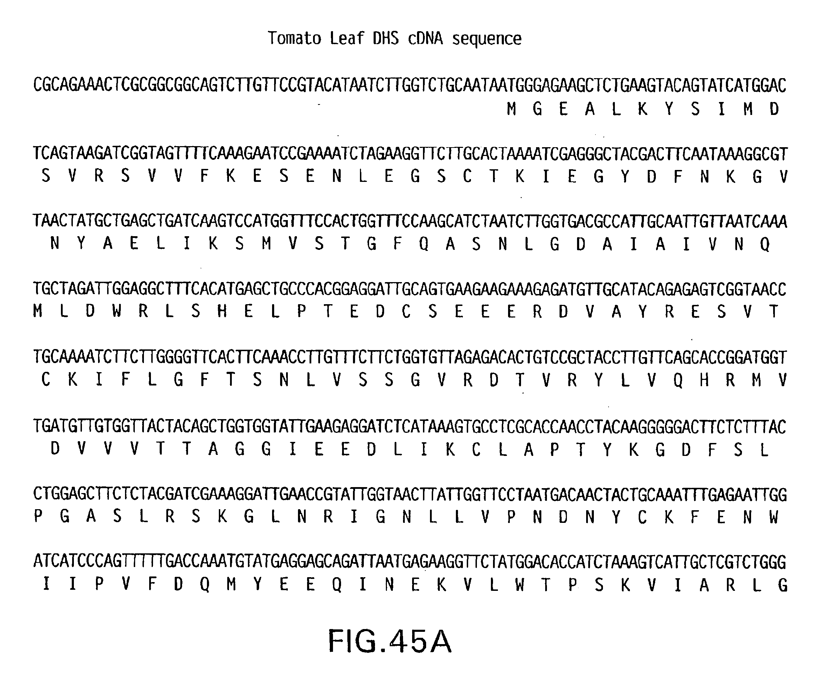

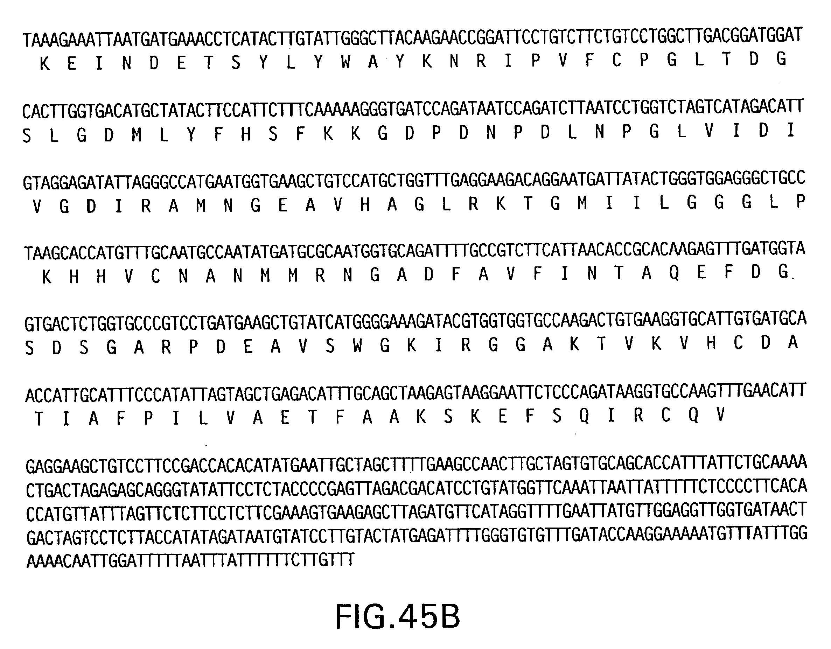

Another embodiment of the present invention is isolated DHS from tomato. The amino acid sequence is provided in Figure 45 A and B and is SEQ ID NO:_. The polynucleotide encoding the amino acid is provided in Figure 45 A and B and is SEQ ID NO: __.

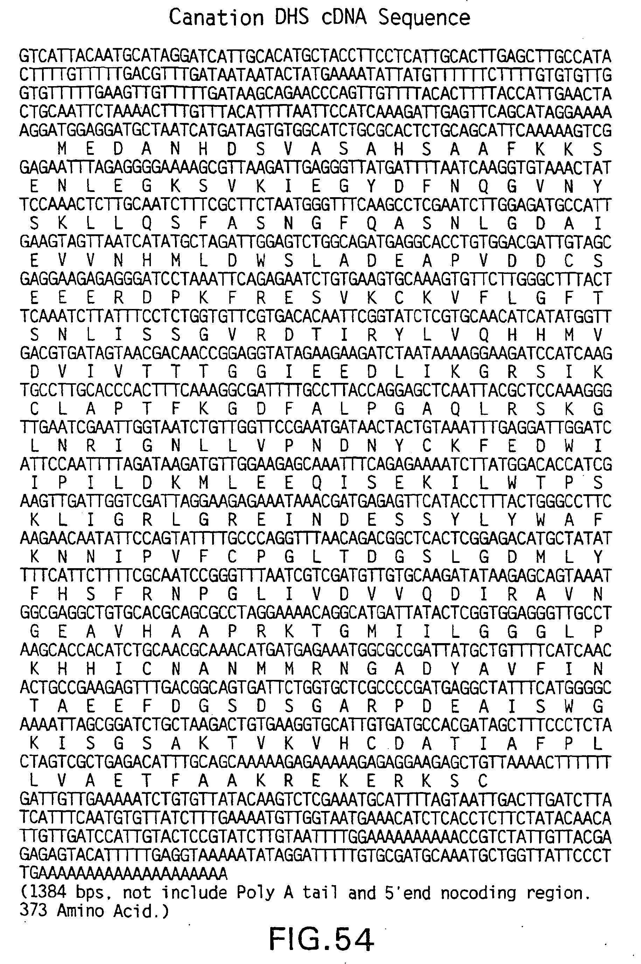

Another embodiment of the present invention is isolated DHS from carnation. The amino acid sequence is provided in Figure 54 and is SEQ ID NO: _. The polynucleotide encoding the amino acid is provided in Figure 54 and is SEQ ID NO: _,

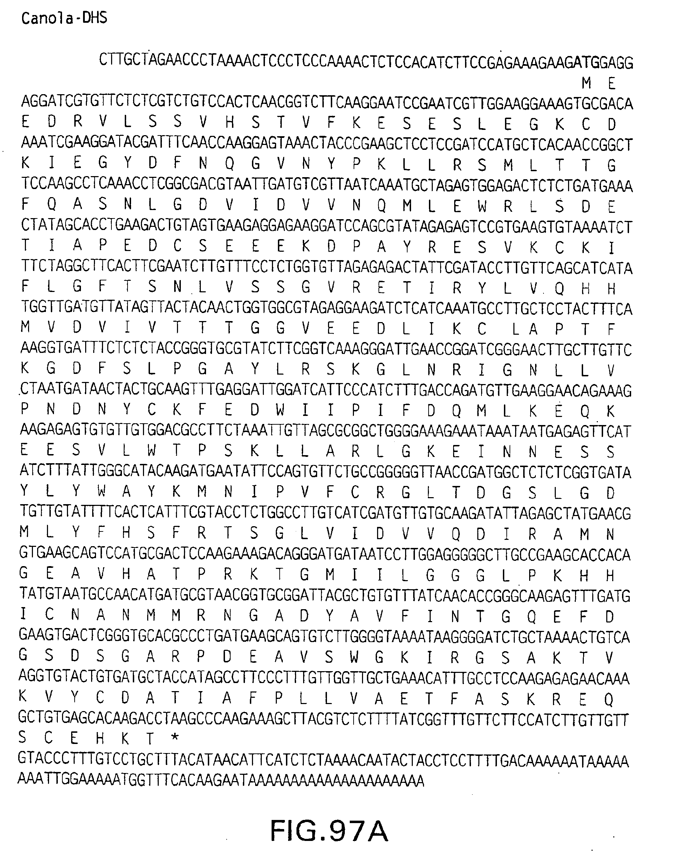

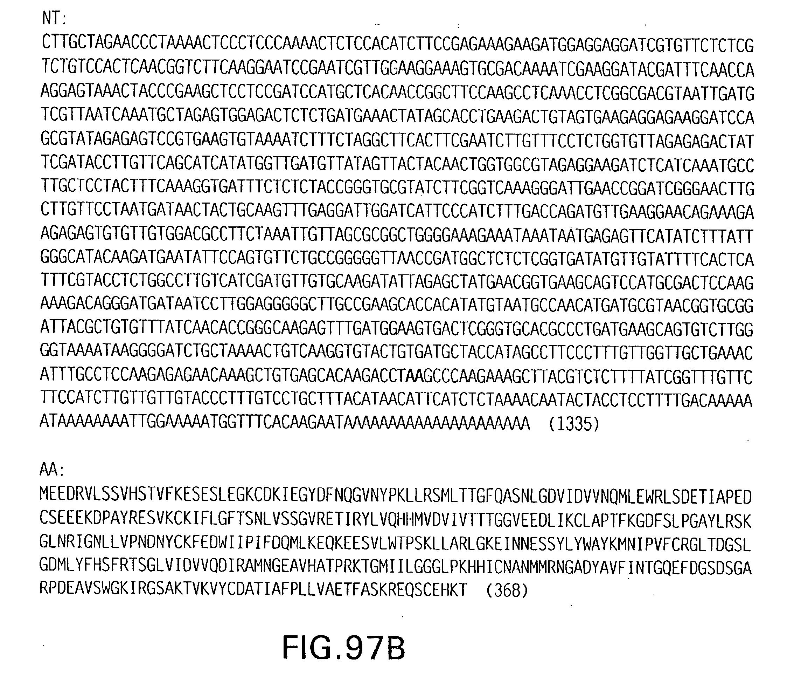

Another embodiment of the present invention is isolated DHS from canola. The amino acid sequence is provided in Figure 97 and is SEQ ID NO: _. The polynucleotide encoding the amino acid is provided in Figure 97 and is SEQ ID NO:__.

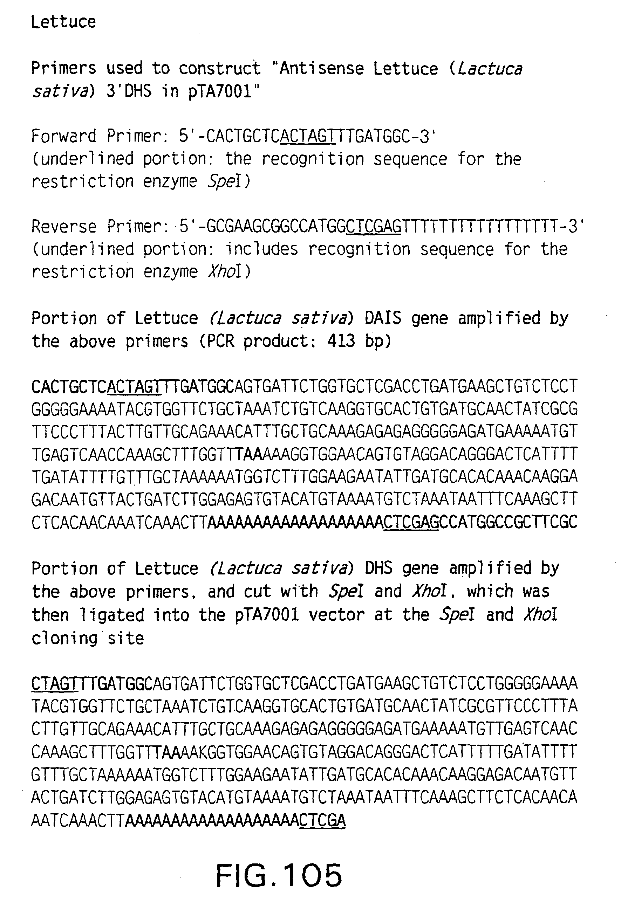

Another embodiment of the present invention is isolated DHS from lettuce. Figure 105 provides a portion of lettuce DHS polynucleotide sequence.

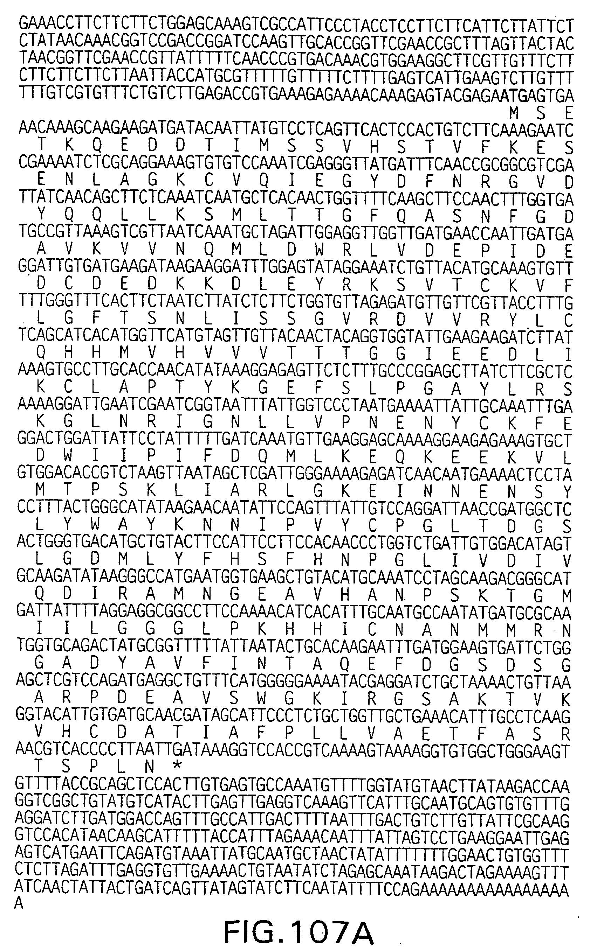

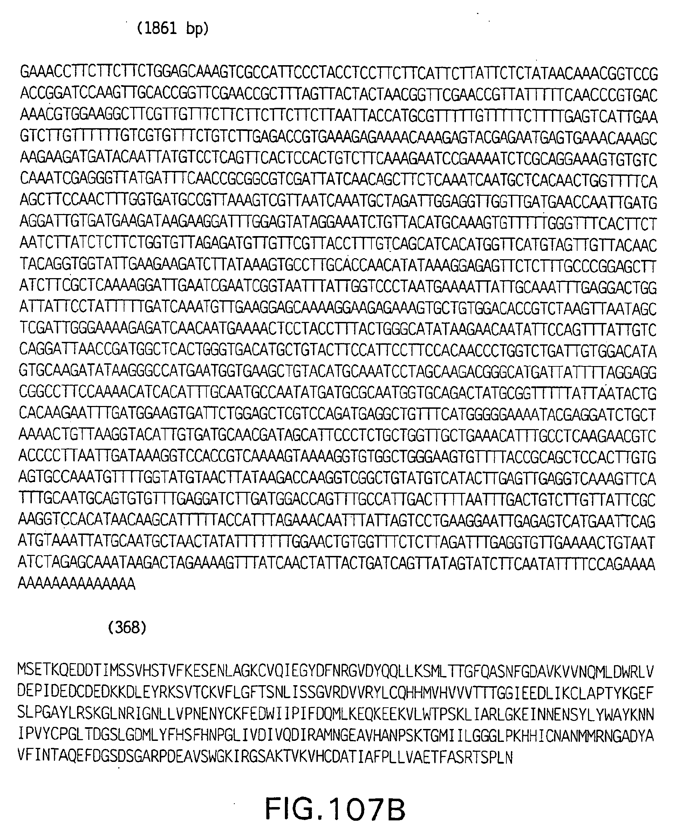

Another embodiment of the present invention is isolated DHS from alfalfa. The amino acid sequence is provided in Figure 107 A and B and is SEQ ID NO: _. The polynucleotide encoding the amino acid is provided in Figure 107 A and B and is SEQ ID NO:__.

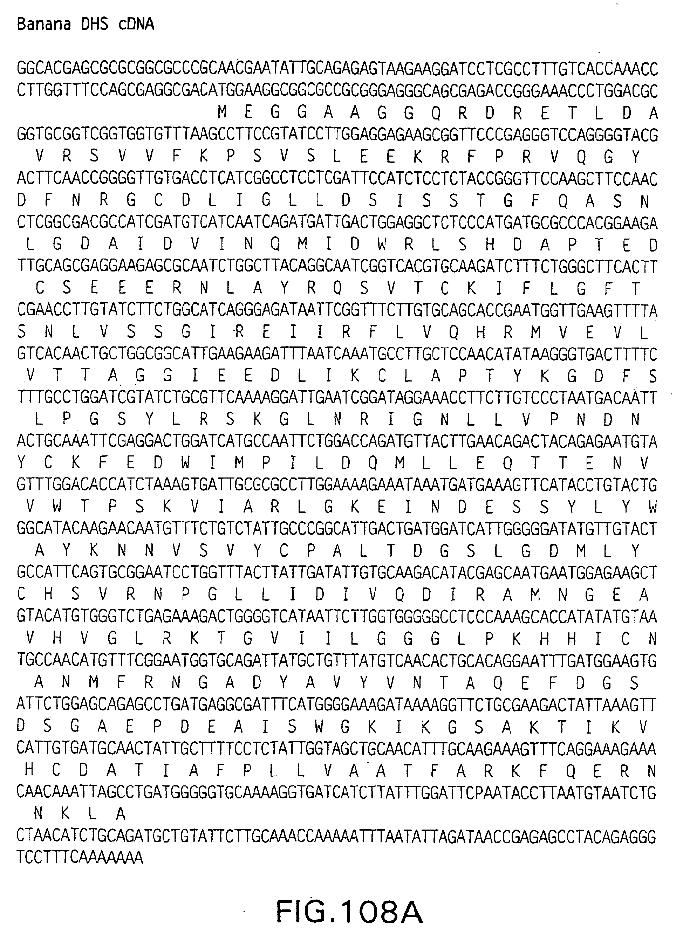

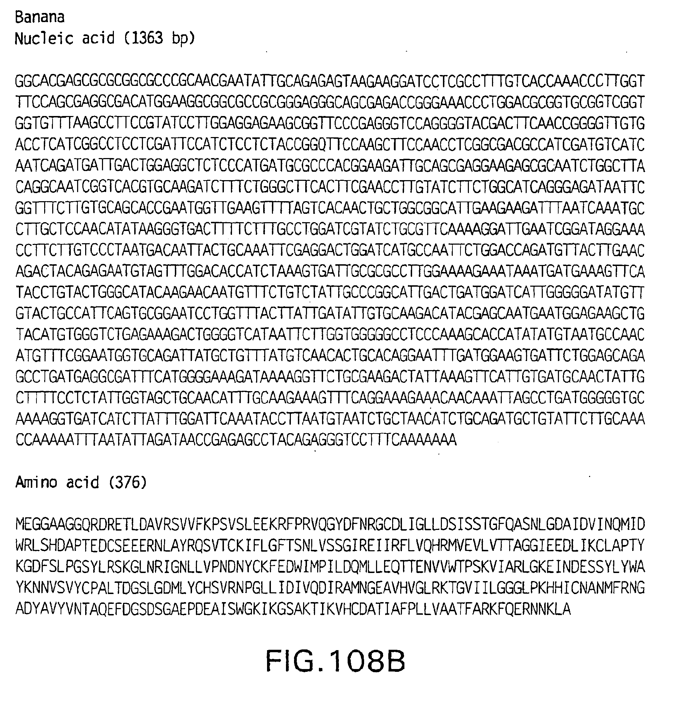

Another embodiment of the present invention is isolated DHS from banana. The amino acid sequence is provided in Figure 108 A and B and is SEQ ID NO: _. The polynucleotide encoding the amino acid is provided in Figure 108 A and B and is SEQ ID NO:_.

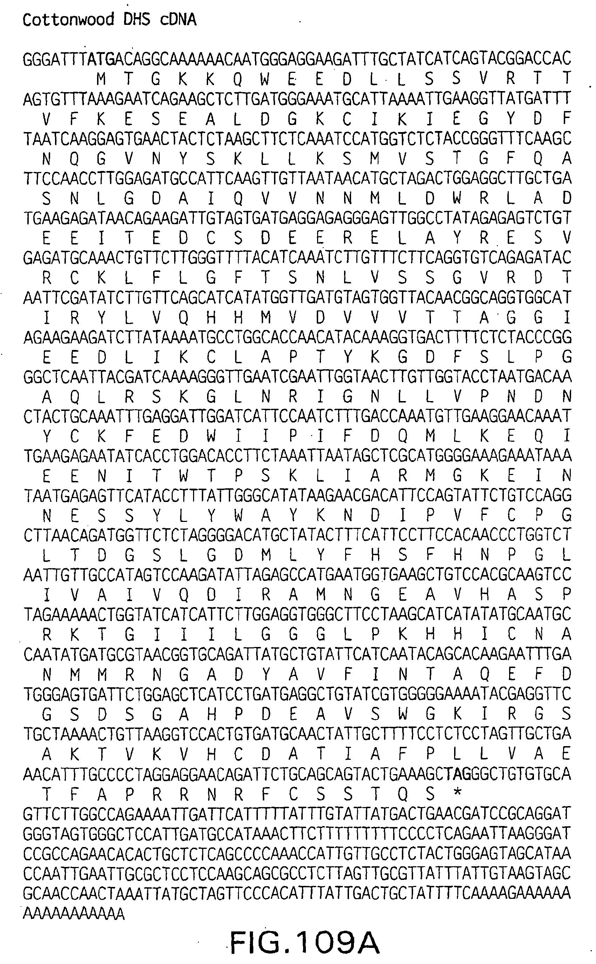

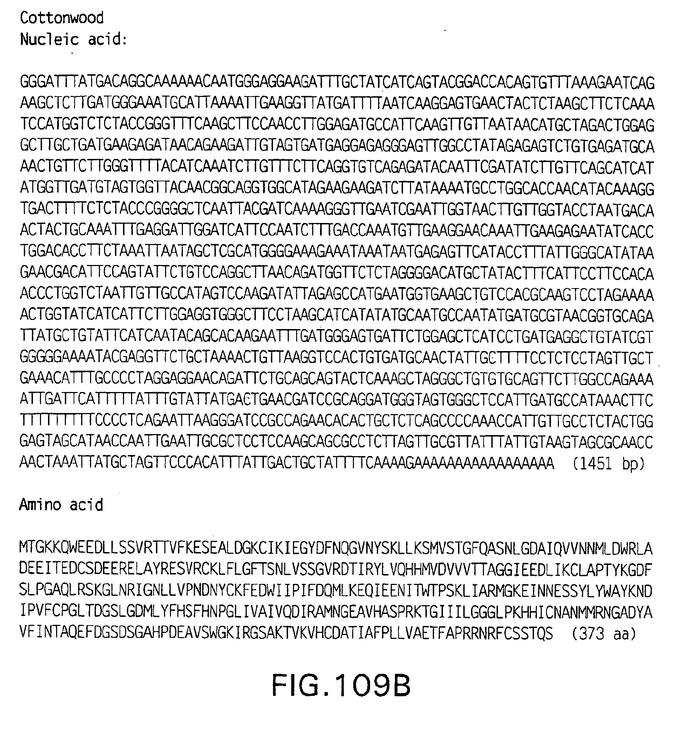

Another embodiment of the present invention is isolated DHS from cottonwood. The amino acid sequence is provided in Figure 109 A and B and is SEQ ID NO: _. The polynucleotide encoding the amino acid is provided in Figure 109 A and B and is SEQ ID NO:

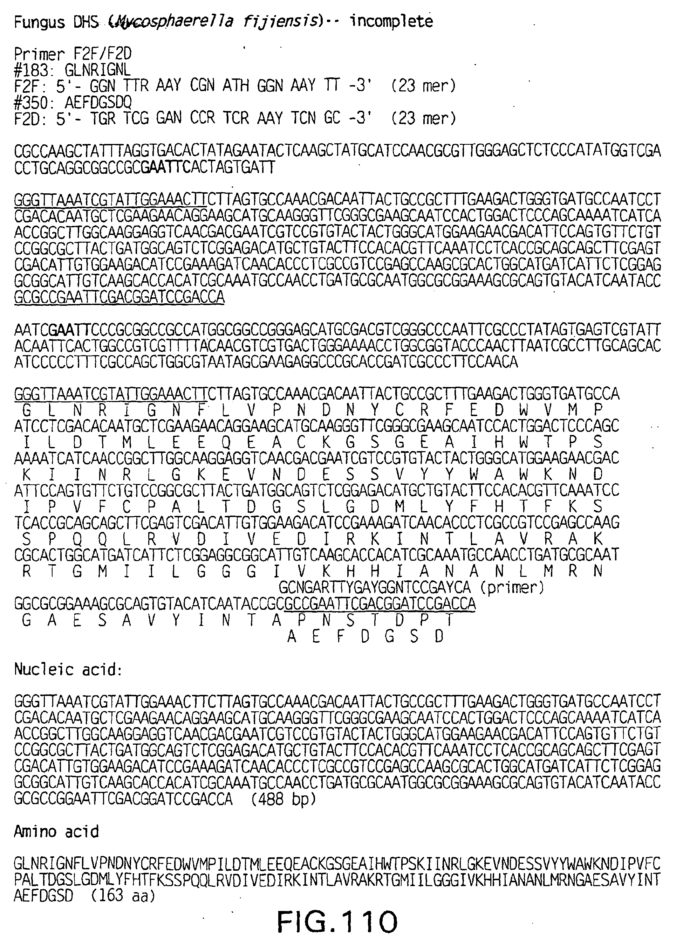

Another embodiment of the present invention is isolated DHS from mycosphaerella The sequence is provided at figure 110.

The present invention also provides isolated polynucleotides of DHS that have 65-100% sequence homology to the above enumerated SEQ ID NOs, and hybridize under high stringency conditions to the complement of the enumerated SEQ ID NOs and which encode DHS.

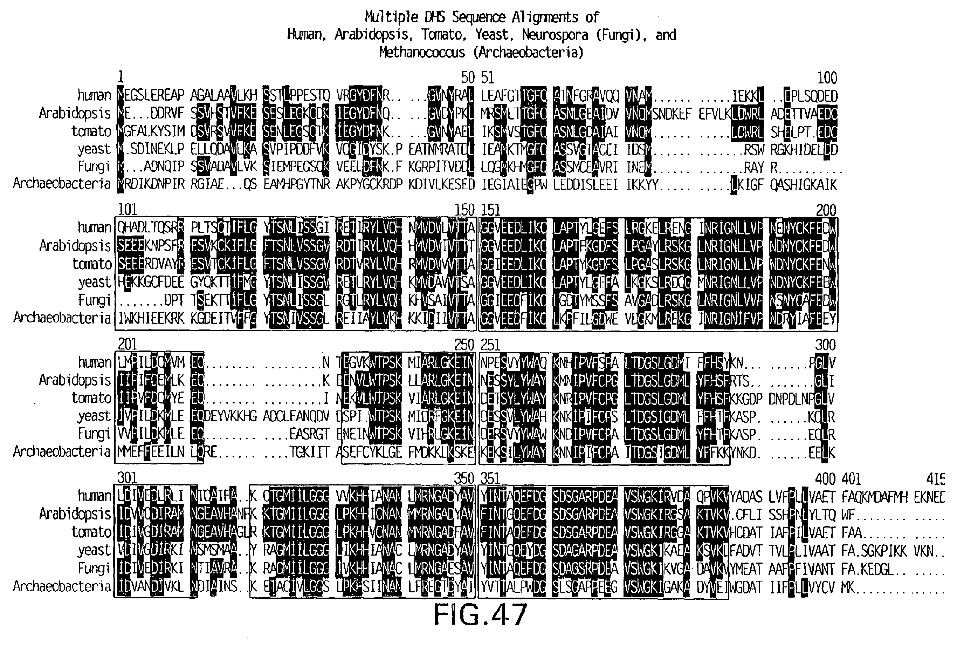

The present invention also provides isolated polypeptides of DHS that have 65-100% amino acid sequence homology to the above enumerated SEQ ID NOs. Figure 47 and Figure 148 (a) and (b) provide an amino acid alignment of the various DHS showing the highly conserved nature of DHS across species.

The present invention also provides antisense polynucleotides of DHS. The antisense polynucleotides may comprise the full length coding sequence, or may be directed at the 3'UTR, or may be directed at the 5'- non-coding sequence. Antisense polynucleotides primarily complementary to 5'-non-coding sequences are known to be effective inhibitors of expression of genes encoding transcription factors. Branch, M.A., Molec. Cell Biol., 13:4284-4290 (1993). The antisense polynucleotides may be of any length as long as they are able to inhibit expression.

The term "antisense polynucleotide of DHS" as used herein and in the claims encompasses not only those antisense polynucleotides that share 100 % homology of the complement of an enumerated SEQ ID NO but also includes those antisense polynucleotides that are a functional variants. Functional variants are as described above. The variant functions as intended by the present invention, are capable of modulating expression of endogenous DHS when introduced into an expression vector and wherein such vector is incorporated into the genome of at least one plant cell.

The present invention also provides sense polynucleotides of DHS. The sense polynucleotides may comprise the full length coding sequence, or may be directed at the 3'UTR, or may be directed at the 5'- non-coding sequence. The sense polynucleotides may be of any length as long as they are able to either down-regulate expression through co-suppression, or alternatively, up-regulate expression.

One embodiment of the present invention provides expression vectors comprising either DHS polynucleotides (of the present invention as described above) or antisense polynucleotides of DHS (of the present invention as described above) or sense polynucleotides of DHS (of the present invention as described above). Vectors are as described above.

The invention also provides a transgenic plant cell transformed with a vector or combination of vectors of the present invention comprising a polynucleotide of DHS either in the sense or antisense orientation, a transgenic plantlet or mature transgenic plant generated from such a cell, or a plant part, such as a flower, fruit, leaves, seeds, etc. of the transgenic plant.

The present invention also provides methods of inhibiting/decreasing expression of endogenous DHS. These methods comprise integrating into the genome of at least one cell of a plant, expression vectors of the present invention comprising antisense polynucleotides of DHS. The antisense polynucleotides of DHS are transcribed and inhibit/decrease expression of endogenous DHS. The resulting plants have an increased biomass, increased seed yield, and longer shelf life.

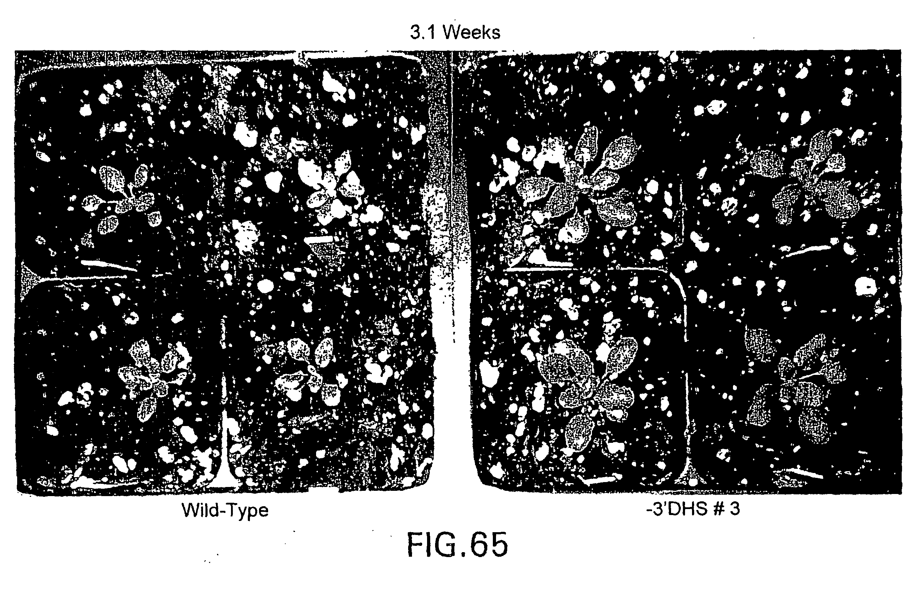

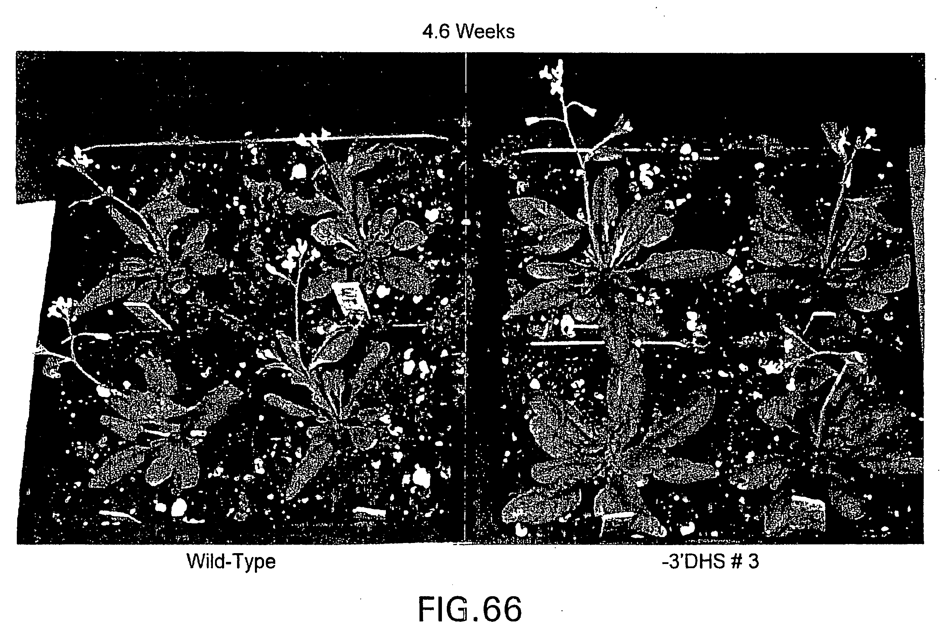

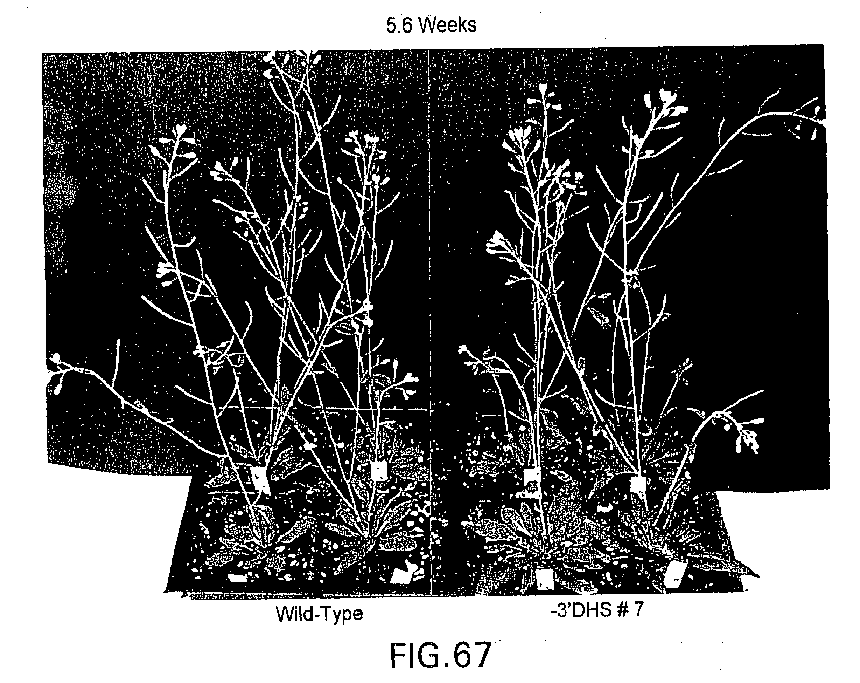

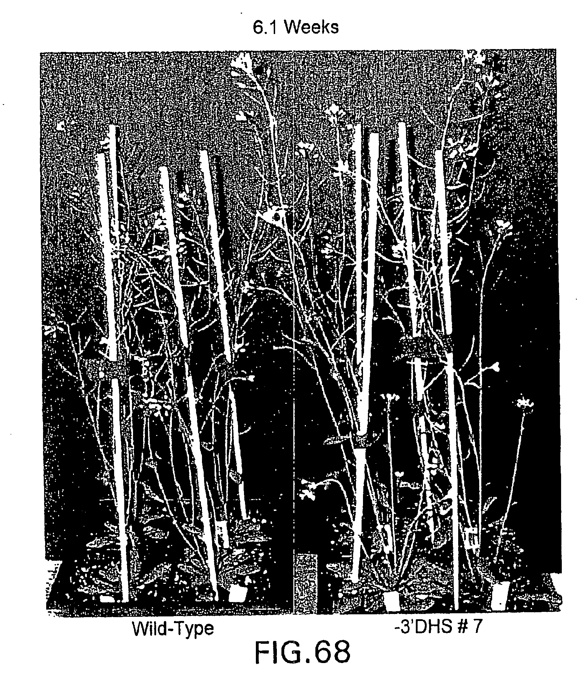

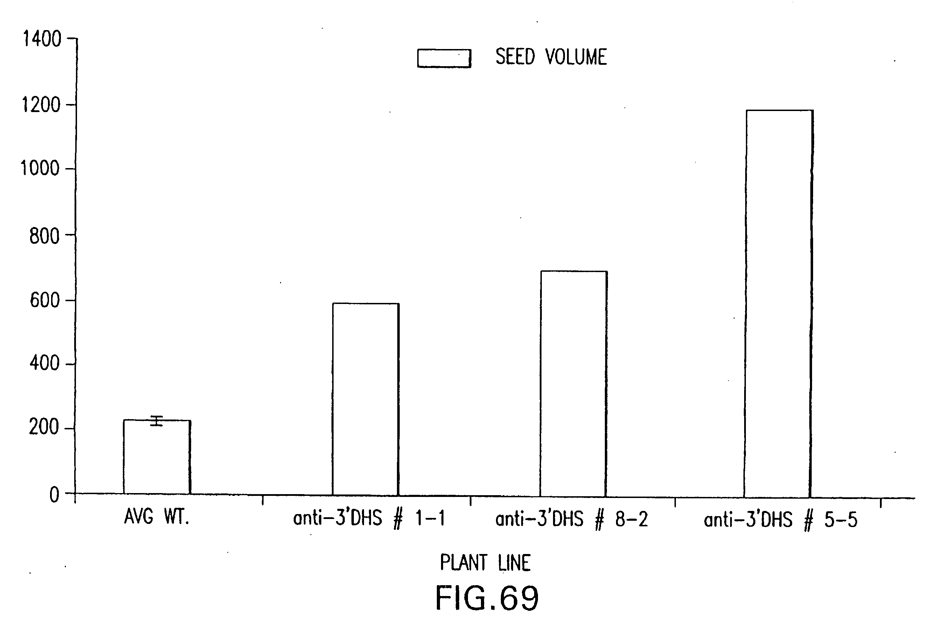

For example, figures 65-68 show pictures of wild type arabisdopsis plants compared to the arabidopsis plants having a down-regulation of DHS brought about by the use of antisense constructs. The arabidopsis plants having DHS down-regulated with antisense arabidopsis DHS constructs have an increased biomass as compared to wild type arabidopsis (not having a down-regulation of DHS). See Figures 153 and 154. Further, Figure 69 and 155 illustrates that the down-regulated DHS arabidopsis plants have an increased seed yield over the wild type plants. Figure 156 shows that down regulation of DHS causes an increase in root development. See Example 13 of a description of various constructs used to transform arabidopsis with either full length or the 3' region of arabiposis DHS (SEQ ID NO:30) in the antisense orientation.

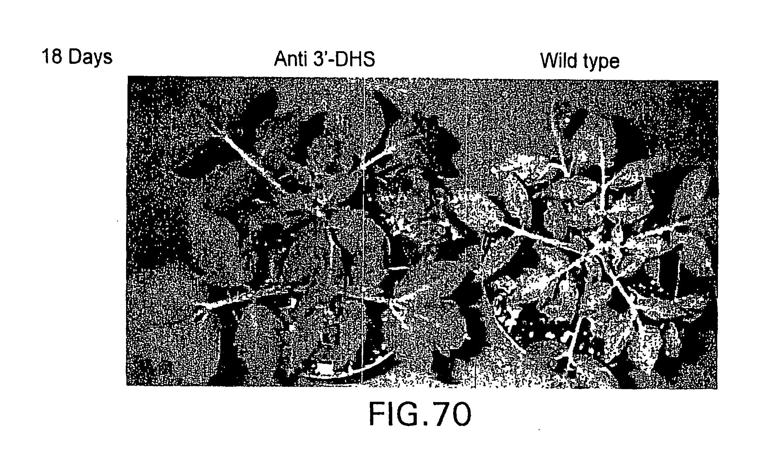

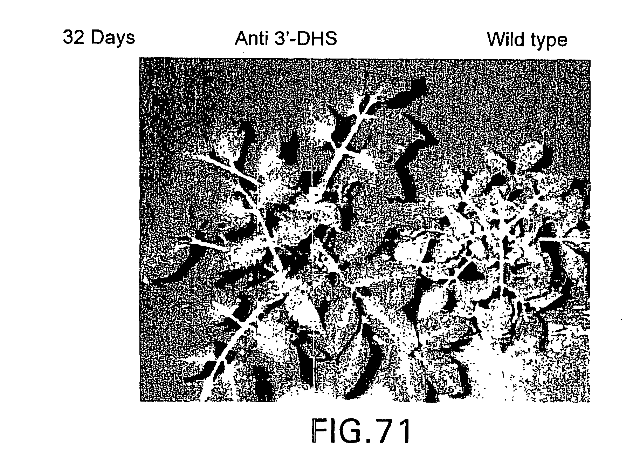

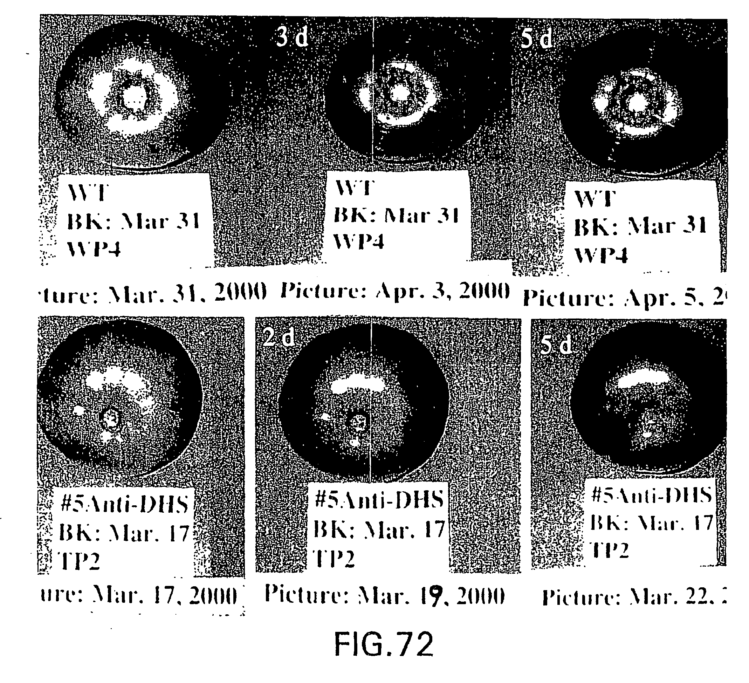

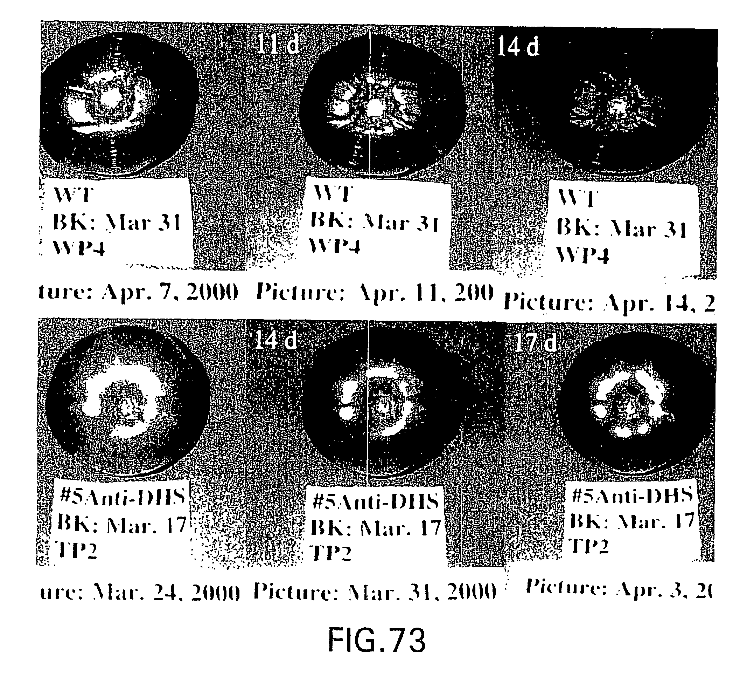

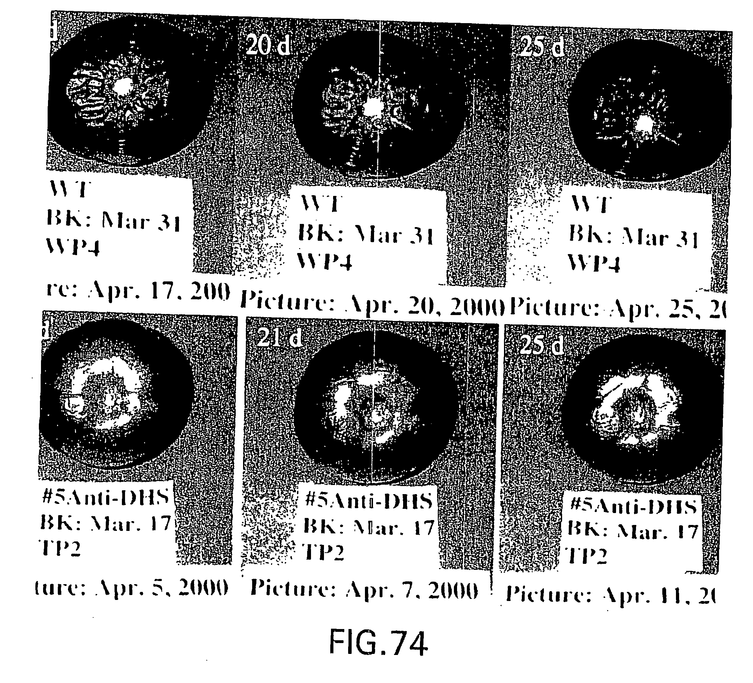

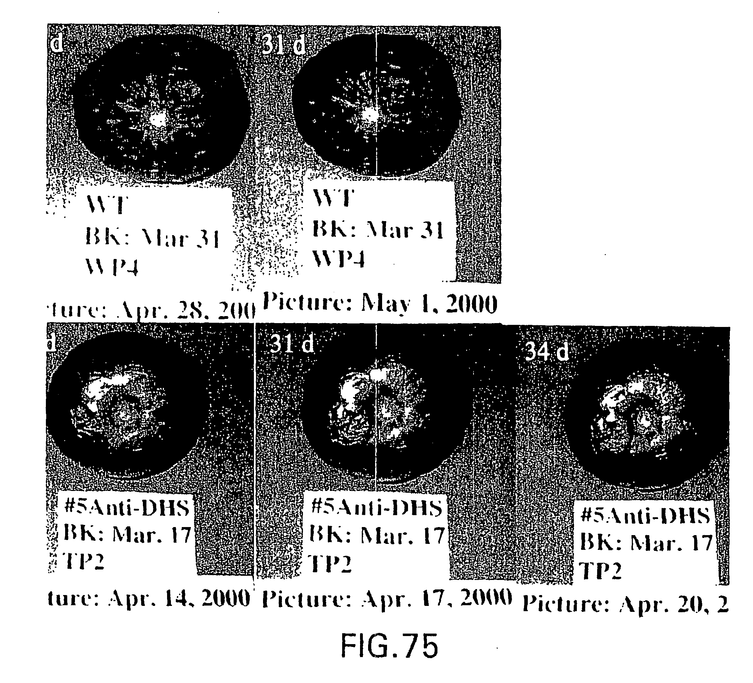

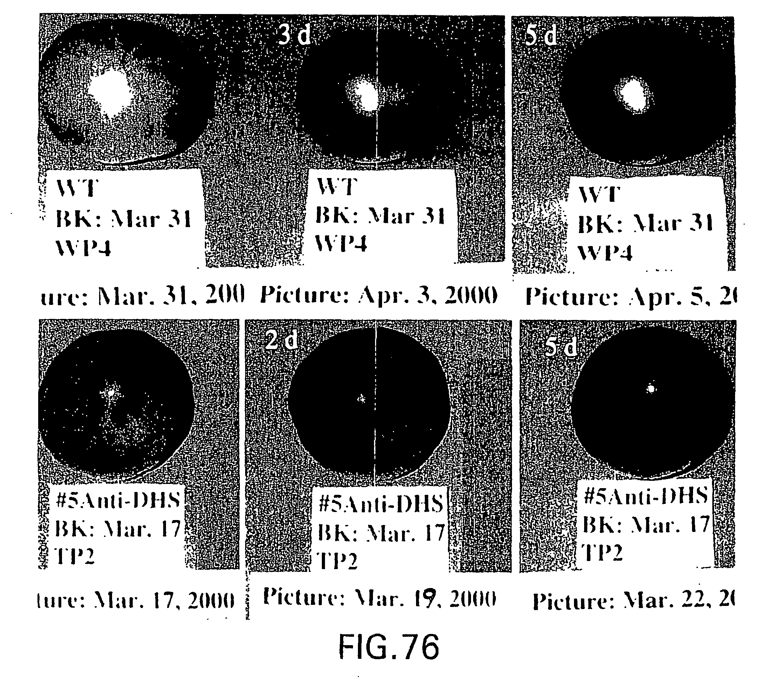

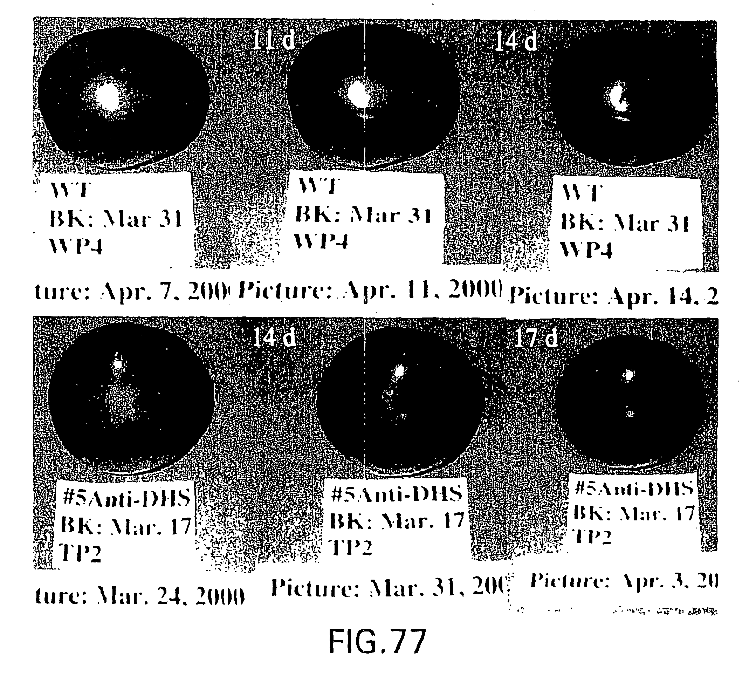

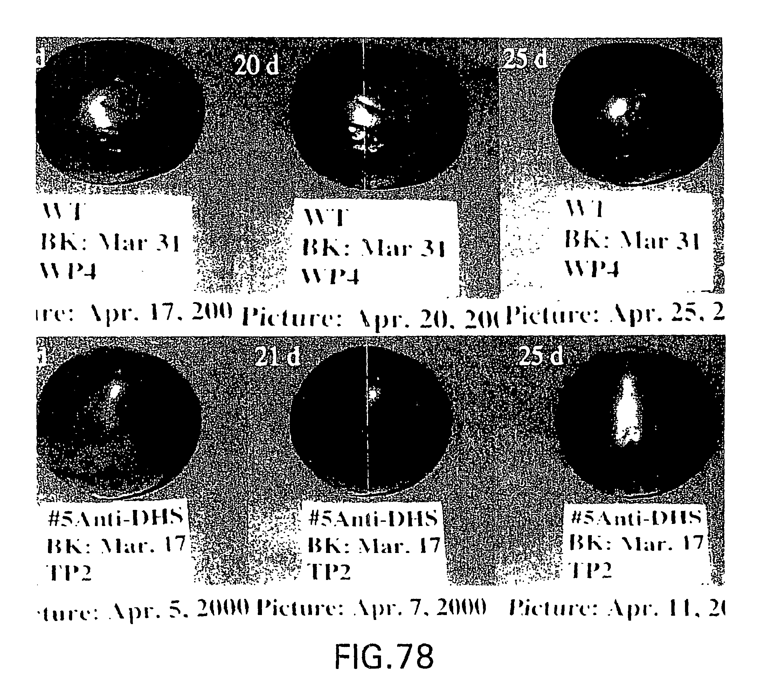

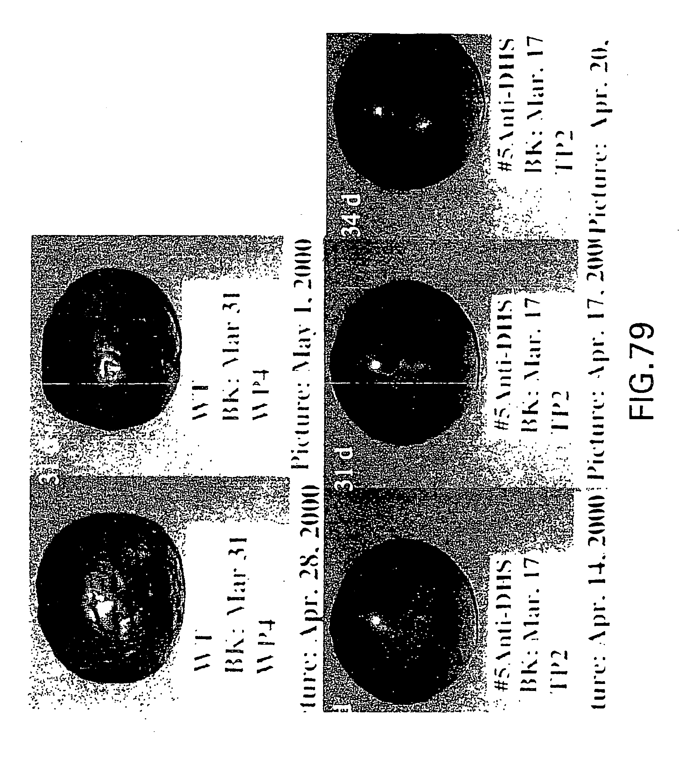

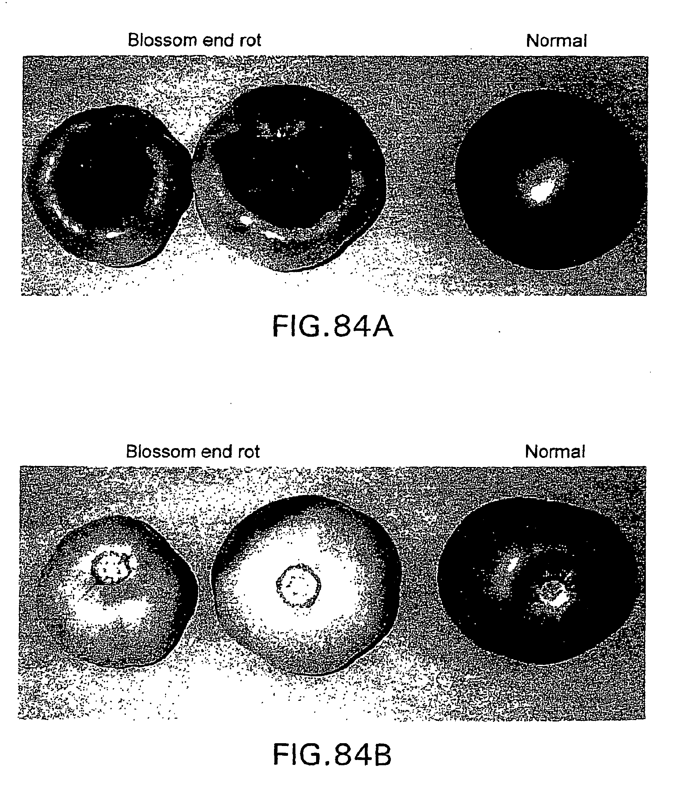

Example 14 and 15 describe the transformation of tomato plants with full-length or 3' region of tomato DHS in the antisense orientation. Figures 70-79 show that the resulting tomato plants have a delayed softening and spoilage of tomato as compared to wild type tomato plants. Figures 84(a) and 84(b) are photographs of tomato fruits showing that tomatoes having been transformed with tomato DHS in the antisense orientation have a reduced incidence of blossom end rot, thus indicating that reducing the expression of DHS prevents the onset of tissue and cell death arising from physiological disease.

Suppression of DHS expression in canola increases plant biomass and increases seed yield. See example 28 and Figures 157-161. Figure 157 shows the transgenic canola has increase biomass as demonstrated by increased leaf mass. Figure 158 shows the results of a Western blot showing partial down-regulation of DHS in one of the transgenic lines of canola. Figure 159 shows that down-regulation of DHS in canola results in larger siliques. Figure 160 shows that down-regulation of DHS in canola results in an increased seed yield. Figure 161 (a) illustrates that total yield of triacylglyercol expressed on a "per weight of seed" basis. This demonstrates that there is no change in the quantity of oil per seed. Thus, the ~65% increase in seed yield translates into and ~65% increase in seed oil. Figure 161 (b) illustrates that there is no change in the fatty acid composition of oil in the DHS down regulated canola plants.

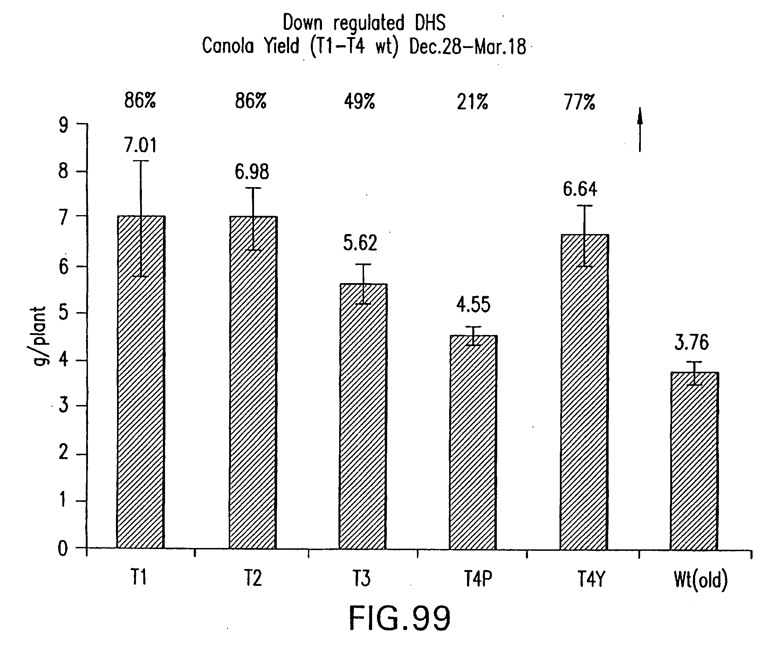

Figure 99 shows the result of an experiment where canola plants were grown in small pots, which puts a small amount of stress on the plants (sub-lethal). When DHS is down-regulated, an increase in seed yield can be observed. The 3'UTR of canola DHS was used as an antisense nucleotide. The antisense oligonucleotide was expressed constitutively using a 35S promoter. Using a constitutive promoter, the DHS antisense polynucleotide is expressed throughout the growth and development of the entire plant. When the plant is exposed to episodes of sub-lethal stress (such as being grown in too small of a pot, or periods of suboptimal growing conditions, etc.), the plant may enter the senescence pathway. However, when the plant is a transgenic plant having a reduced expression of DHS, the plants do not enter into the senescence mode when subjected to sub-lethal stress. Figure 99 shows an increase in canola yield in plants having DHS down-regulated. Figure 99 shows T₁ through T₄ generations, and two particular T₄ generation of a particular plant line (designated as the "P" and "T" line).

Down regulating DHS in carnations causes the flowers to have an extended vase life. Example 29 shows that down regulation brought about the use of antisense constructs, as well as the use of inhibitors of the DHS reaction extend the shelf life of flowers by up to 83%.

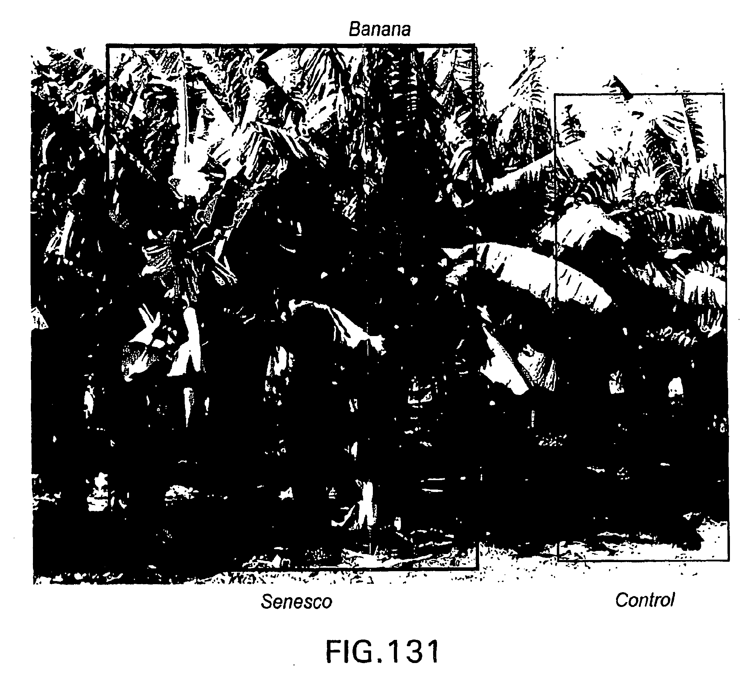

Transgenic bananas having antisense DHS exhibit increased biomass as evidenced by larger plants as compared to wild type. See figure 131. The fruit from the transgenic plant also show delayed spoilage as compared to the wild type. See Figure 114. A nucleotide encoding banana DHS was inserted into a vector in an antisense orientation either under the control of 35S promoter or a fruit specific promoter (pPDK). Either the full length banana DHS or the 3' UTR was used.

Commercially-available pre-packaged salad is commonly stored under conditions of controlled atmosphere, whereby the level of oxygen is greatly reduced below its atmospheric concentration in order to extend the shelf life of the product. The most common symptom of spoiled pre-packaged salad is browning on the cut surfaces of lettuce. Although controlled atmosphere packaging does achieve a delay in browning, it can also result in off-odor and off-flavor. Down-regulation of DHS is shown to have potential as an alternative strategy for delaying browning on the cut surfaces of lettuce. DHS catalyzes the activation of eukaryotic translation initiation factor 5A (eIF5A), which acts as a nucleocytoplasmic shuttle protein for select populations of mRNAs. DHS appears to play a role in browning of cut lettuce inasmuch as suppression of DHS expression (by antisense technology) resulted in a significant delay in the onset of browning under atmospheric conditions. Specifically, 80% of the cut segments of wild-type lettuce plants showed browning at 6 days after cutting, whereas only 27%, on average, of the cut segments of transgenic plants from 5 segregating lines turned brown over the same period, with some individual plants showing 0% browning. Suppression of DHS expression in lettuce through the use of antisense constructs results in a delay in the onset of browning of cut lettuce as compared to wild type lettuce. See Example 27 and figures 149-151.

Jatropha curcas L. is a plant known for its high oil content and may possible be a source of bio-fuel. The clear oil expressed from the seed has been used for illumination and lubricating, and more recently has been suggested for energetic purposes, one ton of nuts can yield 70 kg refined petroleum, 40 kg "gasoil leger" (light fuel oil), 40 kg regular fuel oil, 34 kg dry tar/pitch/rosin, 270 kg coke-like char, and 200 kg ammoniacal water, natural gas, creosote, etc. Thus, the ability to enhance the seed yield (and thus oil content) may prove valuable. Increasing the seed yield can be achieved through three strategies. The first strategy involves constitutively up-regulating growth eIF-5A. Preferably this is done with a heterologous isoform of growth eIF-5A (i.e. using a canola or arabidopsis growth eIF-5A) to minimize the prospect of co-suppression. This has a dual effect - there is up-regulation of hypusinated growth eIF-5A, and there is also an up-regulation of unhypusinated senescence eIF-5A because there is more eIF-5A being produced than there is DHS available to activate (hypusinate) it. Any plant growth eIF-5A may be used, for example, a tomato or cottonwood growth eIF-5A could be used in Jatropha. The second strategy involves constitutively up-regulating mutated senescence eIF-5A in which the conserved lysine that normally gets hypusinated is converted to alanine. This mutated eIF-5A can not be hypusinated (activated), and in its unhypusinated form promotes growth. See Figures 166 and 167 showing transgenic arabidopsis plants having a mutated senescence eIF-5A are larger than wild type arabidopsis palnts. Larger plants have mor bolts and more seeds. Also, unhypusinated senescence eIF-5A confer upon the plants enhanced nitrogen utilization efficiency, thus enabling them to out perform control plants under conditions of low nitrogen in the soil. The third strategy involves constitutively down-regulating DHS. This must be a partial down-regulation of DHS because the plant needs some DHS to function properly. But as seen above, partial down-regulation of DHS substantially promotes growth and increases seed yield, presumably because of an accumulation of unhypusinated senescence eIF-5A.

Figures 47 and 148 (a) and (b) show an amino acid alignment of various DHS, revealing that DHS is highly conserved. Figure 148(a) and (b) indicate the very highly conserved lysine of DHS as well as other regions. In fact, DHS is so highly conserved that an isolated polynucleotide encoding one plant DHS can be inserted into another plant species in antisense orientation and achieve down-regulation of DHS.

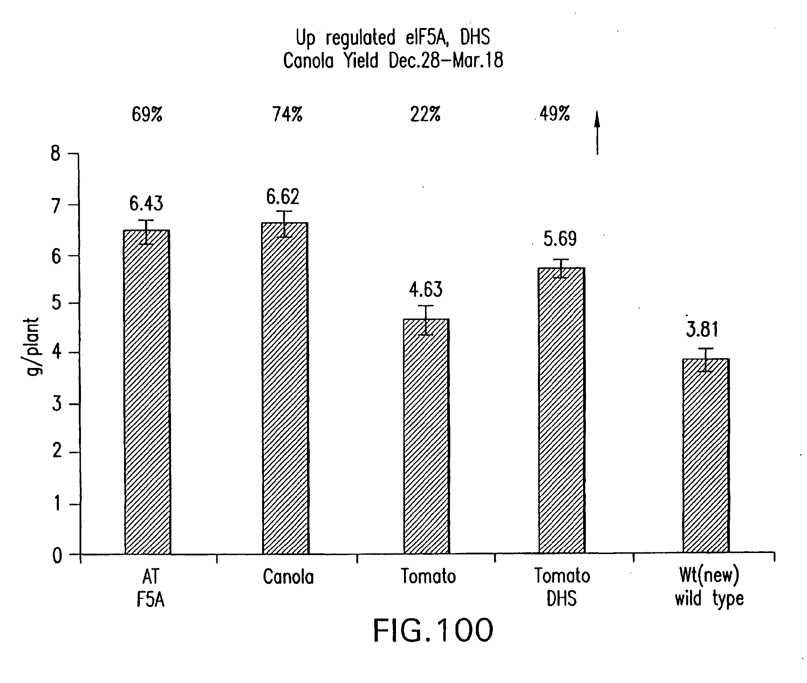

Figure 100 shows a bar graph measuring canola yield (made by enhancing the capacity for plant growth. This figure shows that can increase the yield both by increasing growth eIF-5A expression (use of sense constructs) or by increasing expression of DHS (use of sense constructs). The second bar labeled "canola" shows the increase achieved when upregulated canola DHS by using a canola DHS in the sense orientation. The fourth bar shows an increase in canola yield by using a tomato DHS in sense orientation in a canola plant.

In another method of inhibiting expression of endogenous DHS, an expression vector containing a DHS polynucleotide of the present invention in a sense orientation is integrated into the genome of at least one cell of a plant. The polynucleotide of DHS is transcribed and the resulting co-expression of exogenous DHS causes a down-regulation or inhibition of expression of endogenous DHS.

By inhibiting expression of endogenous DHS, resulting transgenic plants have no or substantially less DHS protein to activate eIF-5A. As discussed earlier, eIF-5A must be activated to render it biologically useful. Thus, by inhibiting or reducing the expression of DHS either by antisense polynucleotides or by co-suppression with sense polynucleotides, the resulting transgenic plants will either have no active eIF-5A or reduced active eIF-5A. These transgenic plants will exhibit an increase in biomass of the plant, increased seed yield and/or increased seed size. Transgenic plants having antisense polynucleotides of DHS show an increase in photosynthesis and also have an increased starch content. See Examples 24 and 25.

The present invention also provides for up-regulation of DHS. By providing a sense DHS nucleotide into a plant to cause up-regulation, the resulting plant has an increased amount of DHS. The resulting transgenic plants have an increased biomass and seed yield.

Further evidence to support the contention that DHS and eIF-5A play regulatory roles in senescence was provided by treating carnation flowers with inhibitors that are specific for DHS. Spermidine and eIF-5A are the substrates of DHS reaction (Park *et al.,* 1993; Park *et al.,* 1997). Several mono-, di-, and polyamines that have structural features similar to spermidine inhibit DHS activity *in vitro* (Jakus *et al.,* 1993). Some polyamines, such as spermidine, putrescine, and spermine, have been generally used to extend carnation vase life (Wang and Baker, 1980). Through treatment with different polyamines at different concentrations Wang et al (unpublished b) were able to extend the vase life of carnation flowers by 2 fold. Further studies employing a transient infection system to down-regulate DHS is in progress. Preliminary data indicates that the percent survival rate is almost 4 fold higher at day 8 in cut carnations that were vacuum infiltrated with a transient infection system expressing antisense DHS than untreated flowers (Wang *et al.,* unpublished b).

A further major loss in agriculture besides the loss of growth due to stress is post harvest stress-induced senescence (McCabe *et al.,* 2001). This is especially true for plants that are partially processed such as cut lettuce. A symptom of cutting lettuce is browning which is a result of phenolics production (Matile *et al.,* 1999). A field trial of lettuce with antisense polynucleotides of lettuce eIF-5A (LeIF-5A) or antisense full length DHS demonstrated that the transgenic lettuce was significantly more resistant to browning after cutting than the control lettuce. See figures 149-151. It appears that even though stress induced senescence due to harvesting has distinct circuitry (Page *et al.,* 2001), the translational control upstream of browning and likely other senescence symptoms is regulated at least in part by DHS and eIF-5A. Downstream of the regulation of senescence are the execution genes. These are the effectors of senescence and cause the metabolic changes that bring on the senescence syndrome. It appears that eIF-5A and DHS when down-regulated are capable of dampening down a whole range of symptoms caused by senescence.

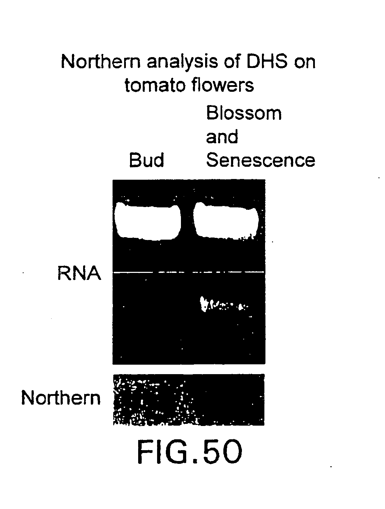

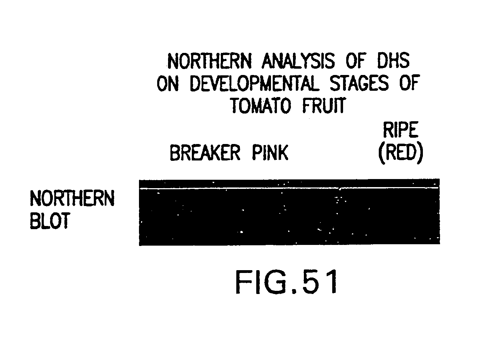

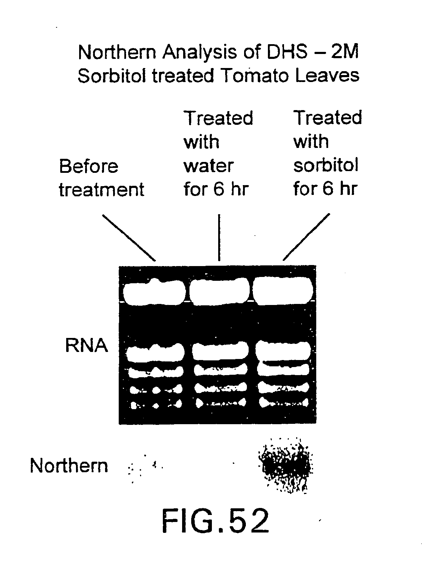

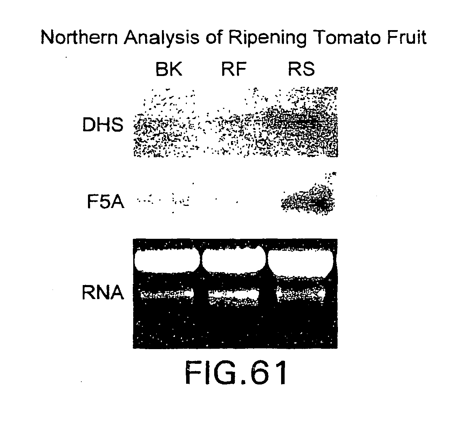

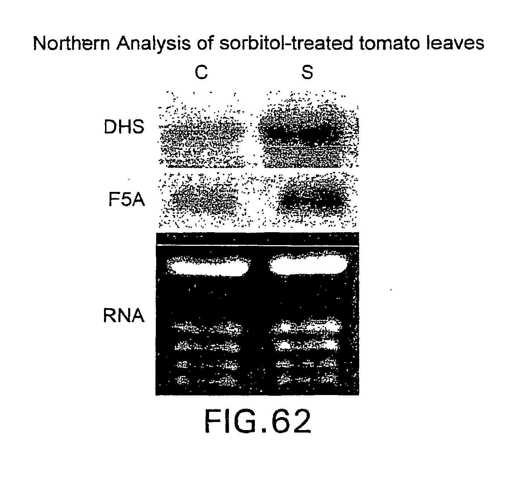

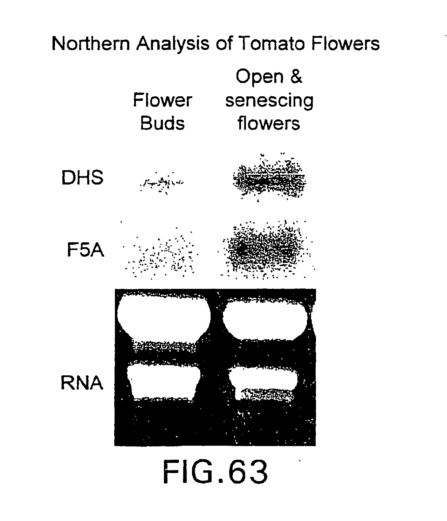

Example 1 describes the isolation and mRNA encoding DHS in various plants. Example 2 describes the induction of senescence-induced DHS in tomatoes by treating tomato leaves with sorbitol. Figures 52 and 62 show that DHS expression is increased when leaves are treated with sorbitol. Example 3 and figures 50 and 63 show that DHS is up-regulated in senescing flowers. Example 4 and figures 51 and 61 show that DHS is up-regulated in ripening fruit.

The present invention also relates to antibodies that recognize the three isoforms of eIF-5A(senescence-induced factor eIF-5A); (wounding factor eiF-5A) and (growth factor eIF-5A).

The present invention also provides a method of identifying senescence-induced eIF-5A, wounding/pathogen induced eIF-5A, growth eIF-5A and DHS in other plants and fungi. By using the methods described herein and the sequences provided, probes are designed to isolate/identify the desired isoforms or DHS. Since the isoforms of eIF-5A (senescence-induced eIF-5A, wounding/pathogen induced eIF-5A, and growth eIF-5A) are often highly homologous in the coding region (see Figure 2), to ensure identification and even alter amplification of the desired isoform, probes or primers are preferably designed from the beginning of the 5'UTR and at the end of the 3" UTR. (See Figures 3, 4 and 5). A preferred set of primers for amplification of wounding/pathogen induced eIF-5A or probes for identification of wounding/pathogen induced eIF-5A are as follows. The downstream primer is 5' GAG CTC AAG AAT AAC ATC TCA TAA GAAAC3' (SEQ ID NO:__) The upstream primer is 5' CTC GAG TGC TCA CTT CTC TCT CTT AGG 3' (SEQ ID NO:__).

Before isolating wounding/pathogen induced eIF5A from a plant or plant part, it is best to introduce a wounding event to allow the plant to begin expressing wounding/pathogen induced eIF-5A. Any wounding event is acceptable and one such exemplary wound events included crushing the leaves at the central vein. Similarly, before isolating senescence-induced eIF-5A, it best to stress the plant tissue to induce senescence.

Having now generally described the invention, the same will be more readily understood through reference to the following examples, which are provided by way of illustration, and are not intended to be limiting to the present invention.

### EXAMPLES

### Example 1

### Messenger RNA (mRNA) Isolation

Total RNA was isolated from tomato flowers and tomato fruit at various developmental stages and from leaves (untreated or after chilling or sorbitol treatment). The tissue (5 g) was briefly ground in liquid nitrogen. The ground powder was mixed with 30 ml guanidinium buffer (4 M guanidinium isothiocyanate, 2.5 mM NaOAc pH 8.5, 0.8%-mercaptoethanol). The mixture was filtered through four layers of cheesecloth and centrifuged at 10,000 Xg at 4°C for 30 minutes. The supernatant was then subjected to cesium chloride density gradient centrifugation at 26,000 Xg for 20 hours. The pelleted RNA was rinsed with 75% ethanol, resuspended in 600 µl DEPC-treated water and the RNA precipitated at -70°C with 0.75 ml 95% ethanol and 30 µl of 3M NaOAc. Ten µg of RNA were fractionated on a 1.2% denaturing formaldehyde agarose gel and transferred to a nylon membrane. Randomly primed ³²P-dCTP-labeled full length DHS cDNA (SEQ ID NO:1) was used to probe the membrane at 42°C overnight. The membrane was then washed once in 1X SSC containing 0.1% SDS at room temperature for 15 minutes and three times in 0.2X SSC containing 0.1% SDS at 65°C for 15 minutes each. The membrane was exposed to x-ray film overnight at -70°C.

PolyA⁺ mRNA was isolated from total RNA using the PolyA⁺ tract mRNA Isolation System available from Promega. PolyA⁺ mRNA was used as a template for cDNA synthesis using the ZAP Express® cDNA synthesis system available from Stratagene (La Jolla, Calif.)

### Tomato Leaf cDNA Library Screening

A cDNA library made using mRNA isolated from Match F1 hybrid tomato leaves that had been exposed to 2 M sorbitol for six hours was diluted to approximately 5 x 10⁶ PFU/ml. The cDNA library was screened using a ³²P-labeled 600 bp RT-PCR fragment. Three positive cDNA clones were excised and recircularized into a pBK-CMV® (Stratagene) phagemid using the method in the manufacturer's instructions. The full length cDNA was inserted into the pBK-CMV vector.

### Plasmid DNA Isolation, DNA Sequencing

The alkaline lysis method described by Sambrook *et al.,* (Supra) was used to isolate plasmid DNA. The full length positive cDNA clone was sequenced using the dideoxy sequencing method. Sanger, et al., Proc. Natl. Acad. Sci. USA, 74:5463-5467. The open reading frame was compiled and analyzed using BLAST search (GenBank, Bethesda, MD) and alignment of the five most homologous proteins with the derived amino acid sequence of the encoded gene was achieved using a BCM Search Launcher: Multiple Sequence Alignments Pattern-Induced Multiple Alignment Method (See F. Corpet, Nuc. Acids Res., 16:10881-10890, (1987)). Functional motifs present in the derived amino acid sequence were identified by MultiFinder.

### Northern Blot Hybridizations of Tomato RNA

Ten µg of total RNA isolated from tomato flowers at various stages (bud and blossom and senescing petals that are open widely or drying), tomato leaves, and tomato fruit at various stages of ripening (breaker, i.e., green fruit with less than 10% red color, pink, i.e., the entire fruit is orange or pink, and red, either soft or firm) were separated on 1% denatured formaldehyde agarose gels and immobilized on nylon membranes. The full length tomato cDNA labeled with ³²P-dCTP using a random primer kit (Boehringer Mannheim) was used to probe the filters (7 x 10⁷ cpm). The filters were washed once with 1x SSC, 0.1% SDS at room temperature and three times with 0.2x SSC, 0.1% SDS at 65°C. The filters were dried and exposed to X-ray film overnight at -70°C. The results are shown in Figures 50-52.

### Northern Blot Hybridization of Arabidopsis RNA

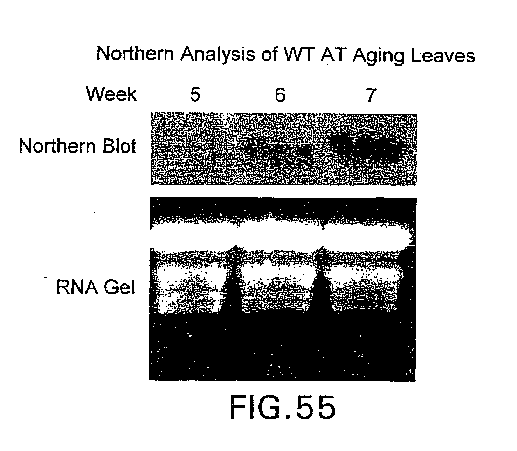

Total RNA from leaves of Arabidopsis plants at five weeks of age (lane 1), six weeks (lane 2) and seven weeks (lane 3) was isolated as above, separated on 1% denatured formaldehyde agarose gels and immobilized on nylon membranes. The full-length *Arabidopsis* senescence-induced DHS cDNA labeled with ³²P-dCTP using a random primer kit (Boehringer Mannheim) was used to probe the filters (7 x 10⁷ cpm). The filters were washed once with 1x SSC, 0.1% SDS at room temperature and three times with 0.2x SSC, 0.1% SDS at 65°C. The filters were dried and exposed to X-ray film overnight at -70°C. The results are shown in Figure 55.

### Northern Blot Hybridization of Carnation RNA

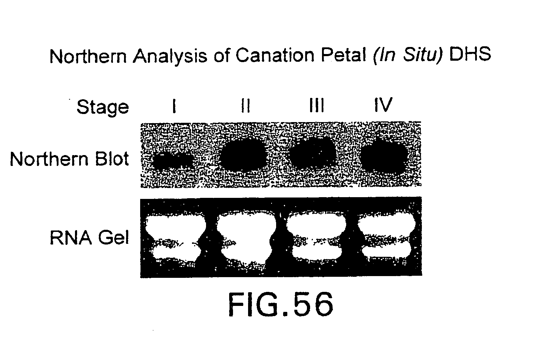

Total RNA from petals of carnation plants at various stages of flower development, i.e., tight-bud flowers (lane 1), beginning to open (lane 2), fully open flowers (lane 3), flowers with inrolling petals (lane 4), was isolated as above, separated on 1% denatured formaldehyde agarose gels and immobilized on nylon membranes. The full-length carnation senescence-induced DHS cDNA labeled with ³²P-dCTP using a random primer kit (Boehringer Mannheim) was used to probe the filters (7 x 10⁷ cpm). The filters were washed once with 1x SSC, 0.1% SDS at room temperature and three times with 0.2x SSC, 0.1 % SDS at 65°C. The filters were dried and exposed to X-ray film overnight at -70°C. The results are shown in Figure 56.

### Example 2

### Sorbitol Induction of Tomato Senescence-Induced DHS Gene

Tomato leaves were treated with 2 M sorbitol in a sealed chamber for six hours. RNA was extracted from the sorbitol treated leaves as follows.

Leaves (5 g) were ground in liquid nitrogen. The ground powder was mixed with 30 ml guanidinium buffer (4 M guanidinium isothiocyanate, 2.5 mM NaOAc pH 8.5, 0.8% -mercaptoethanol). The mixture was filtered through four layers of cheesecloth and centrifuged at 10,000 Xg at 4°C for 30 minutes. The supernatant was then subjected to cesium chloride density gradient centrifugation at 26,000 Xg for 20 hours. The pelleted RNA was rinsed with 75% ethanol, resuspended in 600 µl DEPC-treated water and the RNA precipitated at -70°C with 0.75 ml 95% ethanol and 30 µl of 3M NaOAc. Ten µg of RNA were fractionated on a 1.2% denaturing formaldehyde agarose gel and transferred to a nylon membrane. Randomly primed ³²P-dCTP-labeled full length DHS cDNA (SEQ ID NO:1) was used to probe the membrane at 42°C overnight. The membrane was then washed once in 1X SSC containing 0.1% SDS at room temperature for 15 minutes and three times in 0.2X SSC containing 0.1% SDS at 65°C for 15 minutes each. The membrane was exposed to x-ray film overnight at -70°C.

The results are shown in Figure 52. As can be seen, transcription of DHS is induced in leaves by sorbitol.

### Example 3

### Induction of the Tomato DHS gene in Senescing Flowers

Tight flower buds and open, senescing flowers of tomato plants were harvested, and RNA was isolated as in Example 2. Ten µg RNA were fractionated on a 1.2% denaturing formaldehyde agarose gel and transferred to a nylon membrane. Randomly primed ³²P-dCTP-labeled full length DHS cDNA (SEQ ID NO.1) was used to probe the membrane at 42°C overnight. The membrane then was washed once in 1X SSC containing 0.1 % SDS at room temperature for 15 minutes and then washed three times in 0.2X SSC containing 0.1% SDS at 65°C for fifteen minutes each. The membrane was exposed to x-ray film overnight at -70°C.

The results are shown in Figure 50. As can be seen, transcription of DHS is induced in senescing flowers.

### Example 4

### Induction of the Tomato DHS Gene in Ripening Fruit

RNA was isolated from breaker, pink and ripe fruit as in Example 2. Ten µg RNA were fractionated on a 1.2% denaturing formaldehyde agarose gel and transferred to a nylon membrane. Randomly primed ³²P-dCTP-labeled full length DHS cDNA (SEQ ID NO.1) (Figure 45) was used to probe the membrane at 42°C overnight. The membrane then was washed once in 1X SSC containing 0.1% SDS at room temperature for 15 minutes and then washed three times in 0.2X SSC containing 0.1% SDS at 65°C for fifteen minutes each. The membrane was exposed to x-ray film overnight at -70°C.

The results are shown in Figure 51. As can be seen, transcription of DHS is strongest in ripe, red fruit just prior to the onset of senescence leading to spoilage.

### Example 5

### Induction of Tomato Senescence-Induced DHS Gene by Chilling

Tomato plants in pots (7-8 weeks old) were exposed to 6°C for two days, three days or six days in a growth chamber. The light cycle was set for eight hours of dark and sixteen hours of light. Plants were rewarmed by moving them back into a greenhouse. Plants that were not rewarmed were harvested immediately after removal from the growth chamber. RNA was extracted from the leaves as follows.

Leaves (5 g) were ground in liquid nitrogen. The ground powder was mixed with 30 ml guanidinium buffer (4 M guanidinium isothiocyanate, 2.5 mM NaOAc pH 8.5, 0.8% -mercaptoethanol). The mixture was filtered through four layers of cheesecloth and centrifuged at 10,000g at 4°C for 30 minutes. The supernatant was then subjected to cesium chloride density gradient centrifugation at 26,000g for 20 hours. The pelleted RNA was rinsed with 75% ethanol, resuspended in 600 µl DEPC-treated water and the RNA precipitated at -70°C with 0.75 ml 95% ethanol and 30 µl of 3M NaOAc. Ten µg of RNA were fractionated on a 1.2% denaturing formaldehyde agarose gel and transferred to a nylon membrane. Randomly primed ³²P-dCTP-labeled full length DHS cDNA (SEQ ID NO:1) was used to probe the membrane at 42°C overnight. The membrane was then washed once in 1X SSC containing 0.1% SDS at room temperature for 15 minutes and three times in 0.2X SSC containing 0.1% SDS at 65°C for 15 minutes each. The membrane was exposed to x-ray film overnight at -70°C.

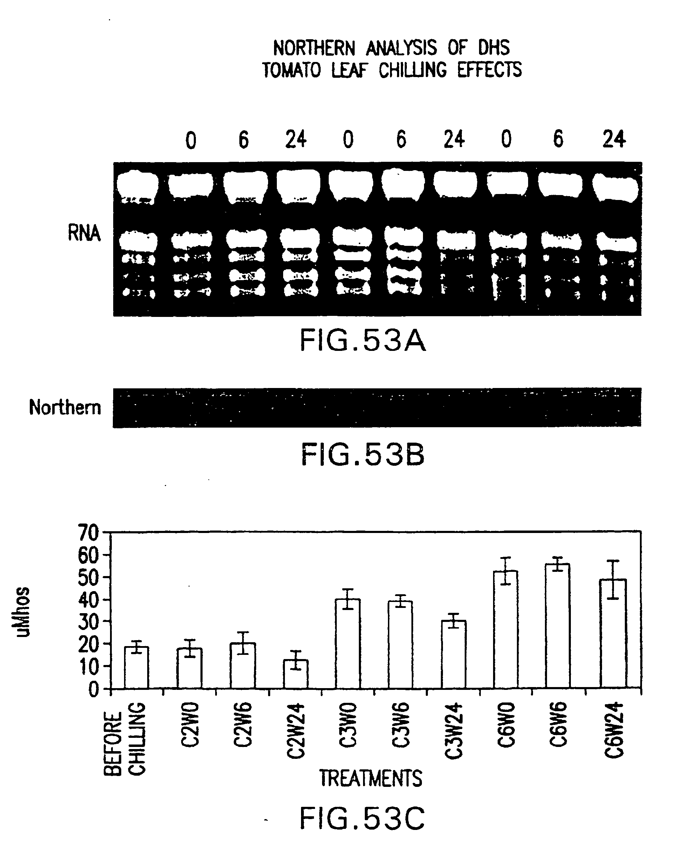

The results are shown in figure 53. As can be seen, transcription of DHS is induced in leaves by exposure to chilling temperature and subsequent rewarming, and the enhanced transcription correlates with chilling damage measured as membrane leakiness.

### Example 6

### Generation of an Arabidopsis PCR Product Using Primers Based on Unidentified Arabidopsis Genomic Sequence

A partial length senescence-induced DHS sequence from an *Arabidopsis* cDNA template was generated by PCR using a pair of oligonucleotide primers designed from *Arabidopsis* genomic sequence. The 5' primer is a 19-mer having the sequence, 5'-GGTGGTGT5TGAGGAAGATC (SEQ ID NO:7); the 3' primer is a 20 mer having the sequence, GGTGCACGCCCTGATGAAGC -3' (SEQ ID NO:8). A polymerase chain reaction using the Expand High Fidelity PCR System (Boehringer Mannheim) and an *Arabidopsis* senescing leaf cDNA library as template was carried out as follows.

### Reaction components:

| | |
|---|---|
| cDNA | 1 µl (5 x 10⁷ pfu) |
| dNTP (10 mM each) | 1 µl |
| MgCl₂ (5mM)+10x buffer | 5 µl |
| Primers 1 and 2 (100 µM each) | 2 µl |
| Expand High Fidelity DNA polymerase | 1.75 U |
| Reaction volume | 50 µl |

### Reaction parameters:

94°C for 3 min
94°C /1 min, 58°C /1 min, 72°C /2 min, for 45 cycles
72°C for 15 min.

### Example 7

### Isolation of Genomic DNA and Southern Analysis

Genomic DNA was extracted from tomato leaves by grinding 10 grams of tomato leaf tissue to a fine powder in liquid nitrogen. 37.5 ml of a mixture containing 25 ml homogenization buffer [100 mM Tris-HCl, pH 8.0, 100 mm EDTA, 250 mM NaCl, 1% sarkosyl, 1% 2-mercaptoethanol, 10 µg/ml RNase and 12.5 ml phenol] prewarmed to 60°C was added to the ground tissue. The mixture was shaken for fifteen minutes. An additional 12.5 ml of chloroform/isoamyl alcohol (24:1) was added to the mixture and shaken for another 15 minutes. The mixture was centrifuged and the aqueous phase reextracted with 25 ml phenol/chloroform/isoamylalcohol (25:24:1) and chloroform/isoamylalcohol (24:1). The nucleic acids were recovered by precipitation with 15 ml isopropanol at room temperature. The precipitate was resuspended in 1 ml of water.

Genomic DNA was subjected to restriction enzyme digestion as follows: 10 µg genomic DNA, 40 µl 10X reaction buffer and 100 U restriction enzyme (XbaI, EcoRI, EcoRV or HinDIII) were reacted for five to six hours in a total reaction volume of 400 µl. The mixture was then phenol-extracted and ethanol-precipitated. The digested DNA was subjected to agarose gel electrophoresis on a 0.8% agarose gel at 15 volts for approximately 15 hours. The gel was submerged in denaturation buffer [87.66 g NaCl and 20 g NaOH /Liter] for 30 minutes with gentle agitation, rinsed in distilled water and submerged in neutralization buffer [87.66 g NaCl and 60.55 g tris-HCl, pH 7.5/Liter] for 30 minutes with gentle agitation. The DNA was transferred to a Hybond-N⁺ nylon membrane by capillary blotting.

Hybridization was performed overnight at 42°C using 1 x 10⁶ cpm/ml of ³²P-dCTP-labeled full length DHS cDNA or 3'-non-coding region of the DHS cDNA clone. Prehybridization and hybridization were carried out in buffer containing 50% formamide, 6X SSC, 5X Denhardt's solution, 0.1% SDS and 100 mg/ml denatured salmon sperm DNA. The membrane was prehybridized for two to four hours; hybridization was carried out overnight.

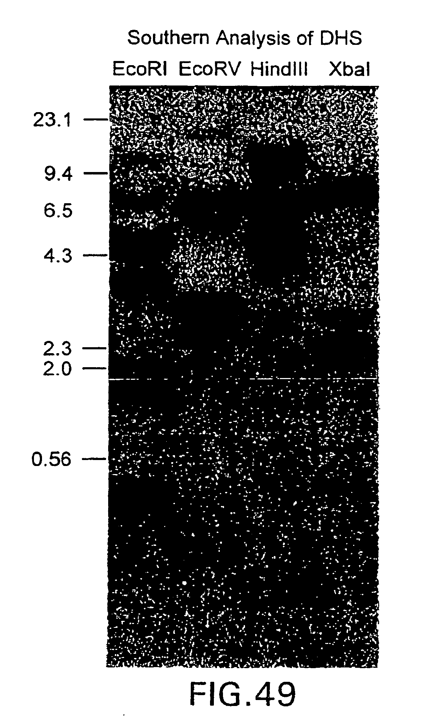

After hybridization was complete, membranes were rinsed at room temperature in 2X SSC and 0.1% SDS and then washed in 2X SSC and 0.1% SDS for 15 minutes and 0.2X SSC and 0.1% SDS for 15 minutes. The membrane was then exposed to x-ray film at -80°C overnight. The results are shown in Figure 49.

### Example 8

### Isolation OfA Senescence-Induced eIF-5A Gene From Arabidopsis

A full-length cDNA clone of the senescence-induced eIF-5A gene expressed in *Arabidopsis* leaves was obtained by PCR using an *Arabidopsis* senescing leaf cDNA library as template. Initially, PCR products corresponding to the 5'- and 3'- ends of the gene were made using a degenerate upstream primer <AAARRYCGMCCYTGCAAGGT>(SEQ ID NO:17) paired with vector T7 primer <AATACGACTCACTATAG> (SEQ ID NO:18), and a degenerate downstream primer <TCYTTNCCYTCMKCTAAHCC> (SEQ ID NO:19) paired with vector T3 primer <ATTAACCCTCACTAAAG> (SEQ ID NO: 20). The PCR products were subcloned into pBluescript for sequencing. The full-length cDNA was then obtained using a 5'-specific primer <CTGTTACCAAAAAATCTGTACC> (SEQ ID NO: 21) paired with a 3'-specific primer <AGAAGAAGTATAAAAACCATC> (SEQ ID NO: 22), and subcloned into pBluescript for sequencing.

### Example 9

### Isolation OfA Senescence-Induced eIF-5A Gene From Tomato Fruit

A full-length cDNA clone of the senescence-induced eIF-5A gene expressed in tomato fruit was obtained by PCR using a tomato fruit cDNA library as template. Initially, PCR products corresponding to the 5'- and 3'- ends of the gene were made using a degenerate upstream primer (SEQ ID NO:17) paired with vector T7 primer (SEQ ID NO: 18), and a degenerate downstream primer (SEQ ID NO:19) paired with vector T3 primer (SEQ ID NO: 20). The PCR products were subcloned into pBluescript for sequencing. The full-length cDNA was then obtained using a 5'-specific primer <AAAGAATCCTAGAGAGAGAAAGG> (SEQ ID NO: 23) paired with vector T7 primer (SEQ ID NO: 18), and subcloned into pBluescript for sequencing.

### Example 10

### Isolation OfA Senescence-Induced eIF-5A Gene From Carnation

A full-length cDNA clone of the senescence-induced eIF-5A gene expressed in carnation flowers was obtained by PCR using a carnation senescing flower cDNA library as template. Initially, PCR products corresponding to the 5'- and 3'- ends of the gene were made using a degenerate upstream primer (SEQ ID NO: 17) paired with vector T7 primer (SEQ ID NO:18), and a degenerate downstream primer (SEQ ID NO: 19) paired with vector T3 primer (SEQ ID NO: 20). The PCR products were subcloned into pBluescript for sequencing. The full-length cDNA was then obtained using a 5'-specific primer <TTTTACATCAATCGAAAA> (SEQ ID NO: 24) paired with a 3'-specific primer <ACCAAAACCTGTGTTATAACTCC> (SEQ ID NO: 25), and subcloned into pBluescript for sequencing.

### Example 11

### Isolation Of A Senescence-Induced DHS Gene From Arabidopsis

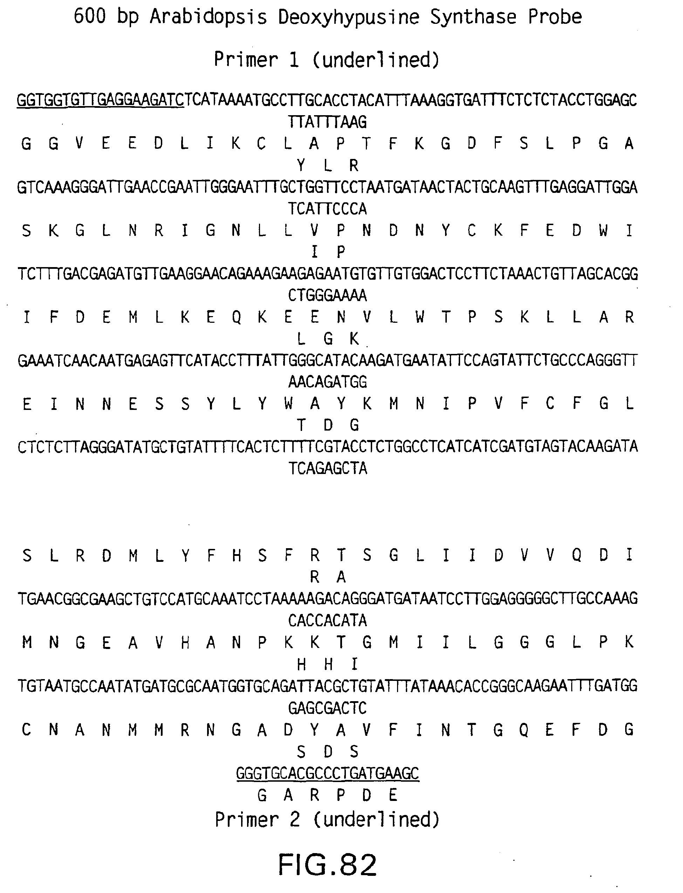

A full-length cDNA clone of the senescence-induced DHS gene expressed in *Arabidopsis* leaves was obtained by screening an *Arabidopsis* senescing leaf cDNA library. The sequence of the probe (SEQ ID NO: 26) that was used for screening is shown in Figure 82. The probe was obtained by PCR using the senescence leaf cDNA library as a template and primers designed from the unidentified genomic sequence (AB017060) in GenBank. The PCR product was subcloned into pBluescript for sequencing.

### Example 12

### Isolation Of A Senescence-Induced DHS Gene From Carnation

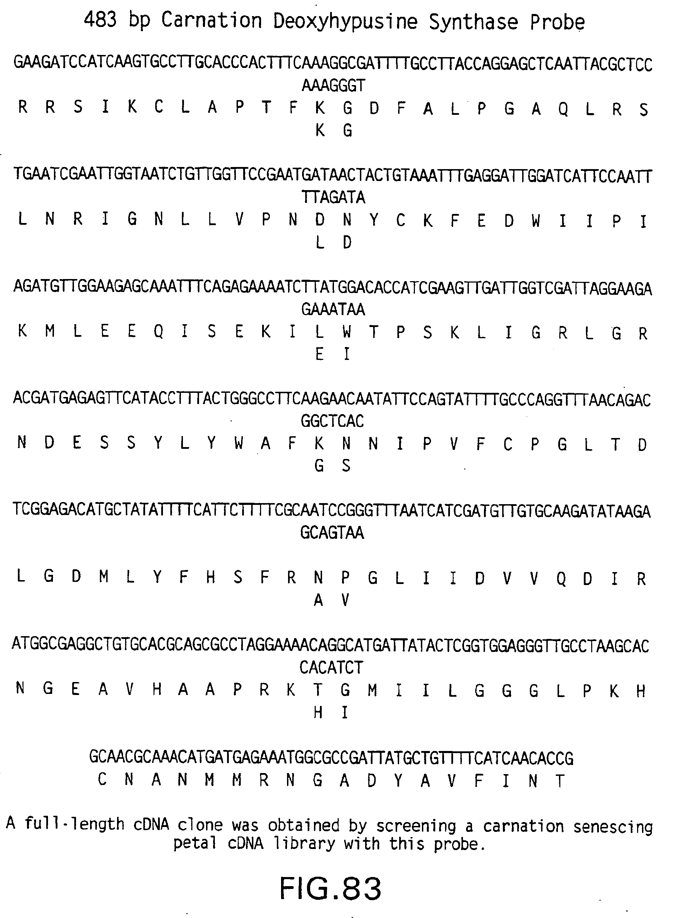

A full-length cDNA clone of the senescence-induced DHS gene expressed in carnation petals was obtained by screening a carnation senescing petal cDNA library. The sequence of the probe (SEQ ID NO: 27) that was used for screening is shown in Figure 83. The probe was obtained by PCR using the senescence petal cDNA library as a template and degenerate primers (upstream: 5' TTG ARG AAG ATY CAT MAA RTG CCT 3') (SEQ ID NO: 28); downstream: 5' CCA TCA AAY TCY TGK GCR GTG TT 3') (SEQ ID NO: 29). The PCR product was subcloned into pBluescript for sequencing.

### Example 13

### Transformation Of Arabidopsis With Full-Length Or 3' Region Of Arabidopsis DHS In Antisense Orientation

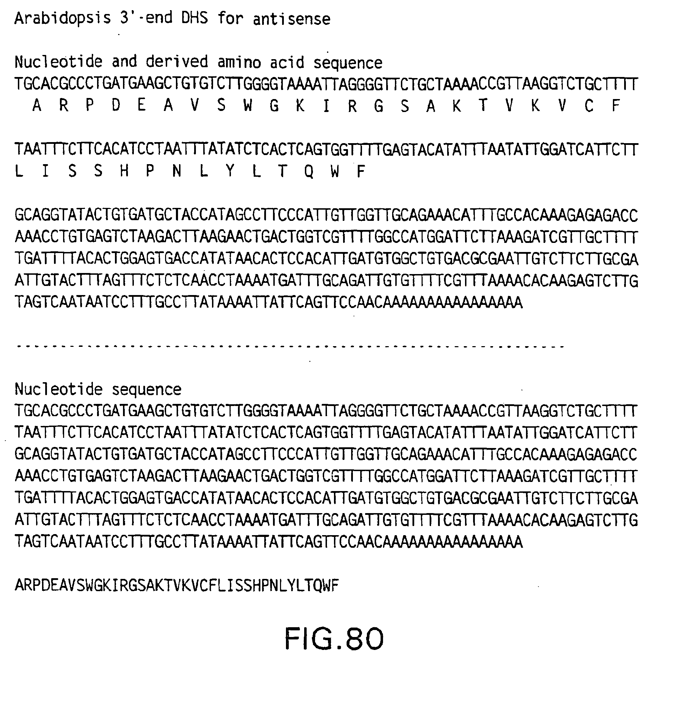

*Agrobacteria* were transformed with the binary vector, pKYLX71, containing the full-length senescence-induced *Arabidopsis* DHS cDNA sequence or the 3' end of the DHS gene (SEQ ID NO:30) (Figure 80), both expressed in the antisense configuration, under the regulation of double 35S promoter. *Arabidopsis* plants were transformed with the transformed *Agrobacteria* by vacuum infiltration, and transformed seeds from resultant T₀ plants were selected on ampicillin.

Figures 65-68 are photographs of the transformed *Arabidopsis* plants, showing that expression of the DHS gene or 3' end thereof in antisense orientation in the transformed plants results in increased biomass, e.g., larger leaves and increased plant size. Figure 69 illustrates that the transgenic *Arabidopsis* plants have increased seed yield.

In another experiment, a vector was constructed using the 3'end of the arabidopsis DHS gene (SEQ ID NO:30), in the antisense configuration under the regulation of an arabidopsis Rubisco promoter (At-rbcS promoter). The vector construct used plasmid pBI101. The GUS gene was removed but the NOS-ter remained. Rusbiso access number is X14564. The promoter sequence that was used is from nucleotide 2821 to 4780. See Figure 152 for the construct. This vector was used to transform Arabidopsis. Figures 153 and 154 show that resulting transgenic plants have increased biomass as compared to the wild type control and the empty binary vector control. Figure 155 shows that the transgenic lines had about a ~100% increase in seed yield. Figure 156 shows that the transgenic lines have increased root development.

### Example 14

### Transformation Of Tomato Plants With Full-Length Or 3' Region Of Tomato DHS In Antisense Orientation

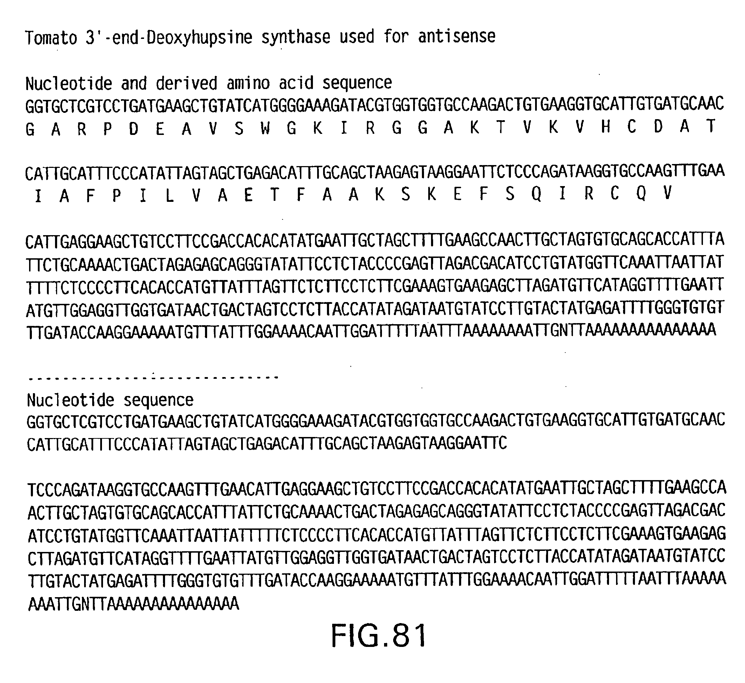

*Agrobacteria* were transformed with the binary vector, pKYLX71, containing the full-length senescence-induced tomato DHS cDNA sequence or the 3' end of the DHS gene (SEQ ID NO:31) (Figure 81), both expressed in the antisense configuration, under the regulation of double 35S promoter. Tomato leaf explants were formed with these *Agrobacteria,* and transformed callus and plantlets were generated and selected by standard tissue culture methods. Transformed plantlets were grown to mature fruit-producing T₁ plants under greenhouse conditions.

Figures 70-79 are photographs showing that reduced expression of the senescence-induced tomato DHS gene in the transformed plants results in increased biomass, e.g., larger leaf size and larger plants as seen in the transformed *Arabidopsis* plants, as well as delayed softening and spoilage of tomato fruit.

### Example 15

### Transformation of Tomato Plants With the 3' Region of Tomato DHS in antisense Orientation

Agrobacteria were transformed with the binary vector, pKYLX71, containing the 3'end of the DHS gene (Figure 81) expressed in the antisense configuration, under the regulation of double 35S promoter. Tomato leaf explants were formed with these Agrobacteria, and transformed callus and plantlets were generated and selected by standard tissue culture methods. Transformed plantlets were grown to mature fruit producing T₁ plants under green house conditions.

Fruit from these transgenic plants with reduced DHS expression were completely free of blossom end rot under conditions in which about 33% of fruit from control plants developed this disease. Blossom end rot is a physiological disease attributable to nutrient stress that causes the bottom (blossom) end of the fruit to senesce and rot. Figures 84A and 84B are photographs showing a control fruit exhibiting blossom end rot and a transgenic fruit that is free of blossom end rot.

The results indicate that reducing the expression of DHS prevents the onset of tissue and cell death arising from physiological disease.

### Example 16

### Expression of Arabidopsis thaliana translation initiation factor 5A (AteIF-5A) isoforms in wild type Columbia -- Plant material

Seeds of *Arabidopsis thaliana,* ecotype Columbia, were grown in Promix BX soil (Premier Brands, Brampton, ON, Canada) in 6-inch pots. Freshly seeded pots were maintained at 4°C for 2 days and then transferred to a growth chamber operating at 22°C with 16-h light/ 8-h dark cycles. Lighting at 150µmol radiation m^{-2.}s⁻¹ was provided by cool-white fluorescent bulbs. Whole rosettes were collected one week intervals at 2 weeks to 7 weeks of age, cauline leaves were collected at 5 weeks, stem, siliques, buds, and flowers were collected at 6 weeks and imbibed seeds (24 hours in water) were also collected, flash frozen in liquid nitrogen and stored at -80°C.

### Infection of Arabidopsis thaliana plants with Pseudomonas syringae

Seeds of *Arabidopsis thaliana* ecotype Columbia were sown onto Promix BX soil (Premier Brands, Brampton, ON, Canada) in flats containing 64 growth cells. The seeded flats were maintained at 4°C for 2 days and transferred to a growth chamber with photoperiod of 9-h light/15-h dark. All plants were treated at 4 weeks of age, though physiologically due to the shortened photoperiod these appear to be slower in development.

Rosette leaves of 4-week-old plants were infected with avirulent (avr) and virulent (vir) strains *Pseudomonas syringae* pv. *Tomato* DC 3000 obtained from Dr. Robin Cameron (university of Toronto, Toronto, Canada). The abaxial surface of the rosette leaves of each plant was inoculated using 1 ml syringe without a needle. Plants were treated using one of four treatments: no inoculation, mock-inoculation with 10mM MgCl₂, inoculation with avr *P. syringae* strain (10⁶ cfu/ml 10mM MgCl₂) or inoculation with vir *P. syringae* strain (10⁶ cfu/ml 10mM MgCl₂). Two bacterial counts were made, one immediately after inoculation and the second 3 days later, to ensure that a sufficient amount of bacteria was infiltrated to induce systemic acquired resistance in the avr treatment. The inoculated leaves were harvested at predetermined time points for subsequent analysis.

Plants with reduced DHS or wounding/pathogen induced eIF-5A expression were developed using antisense T-DNA insertions for either gene. These plant lines have shown marked resistance to Pseudomonas syringae pv Tomato DC 300, with transgenic lines exhibiting up to a 99% decrease in bacterial load, relative to the wild type plants. See figures 43 and 44. Data using crop plants have also indicated enhanced pathogen resistance.

### Wounding of Arabidopsis thaliana plants with hemostat

4-week-old plants grown under normal lighting conditions were wounded by crushing with hemostat along the midvein (approximately 10% of the leaf surface) according to Stotz et al (2000). Tissue was harvested at 0 minutes, 1 hour and 9 hours and immediately frozen in liquid nitrogen and stored at -80°C for further analysis.

### RNA Isolation and Northern Blotting

Total RNA for Northern blot analysis was isolated from Arabidopsis thaliana rosette leaves according to Davis *et al.* (1986). The RNA was fractionated on a 1% agarose gel and transferred to nylon membranes. (Davis et. al., 1986). Immobilized RNA was hybridized overnight at 42°C with radiolabeled 3'UTR portions of senescence-induced AteIF-5A, wounding/pathogen induced AteIF-5A or growth AteIF-5A. The 3'UTRs were labeled with [α-³²P]-dCTP using a random primer kit (Boehringer Mannheim). The hybridized membranes were washed twice in 2X SSC containing 0.1% SDS at 42°C for 15 minutes and twice in 1X SSC containing 0.1% SDS at 42°C for 30 minutes. Hybridization was visualized by autoradiography after an overnight exposure at - 80°C.

### Antibody Production and Purification

Eukaryotic translation initiation factor 5A (eIF-5A) isoforms of *Arabidopsis thaliana* (At) are highly homologous at the amino acid level, especially at the N-terminal region and the central region of the proteins (Figure 1). In order to obtain antibodies that will be isoform specific, peptides were designed against regions in the isoforms of AteIF-5A that appeared to be unique to each other. An additional cysteine residue was added to each peptide at the N-terminus for conjugation with KLH. The sequences used were: CNDDTLLQQIKS for senescence-induced AteIF-5A, CTDDGLTAQMRL for wounding/pathogen induced AteIF5A, and CTDEALLTQLKN for growth AteIF-5A. When these sequences were submitted to protein BLAST (short nearly exact sequences; limited by *Arabidopsis thaliana;* expected number 20000; word size 2; Matrix PAM90; Gap cost 91) the significant sequences that found in the database were only the matched AteIF-5A and no other. The peptides were synthesized at the University of Western Ontario Peptide Synthesis facility. The carrier protein, Keyhole Limpet Hemocyanin (Sigma), was conjugated to the N-terminal cysteine of the peptide using m-maleimidobenzoyl-N-hydroxysuccinimide ester according to Drenckhahn *et al.* (1993) and Collawn and Patterson (1999). The rabbits were injected four times at two-week intervals with the linked peptide. Two weeks after the final injection blood is collected by exsanguination of the rabbits and clotting of the collected blood in order to amass the antisera.

### Protein Fractionation and Western Blotting

Tissues list above were homogenized (~0.5g/ml) in buffer (50mM EPPS, pH 7.4, 0.25M sorbitol, 10mM EDTA, 2mM EGTA, 1mM DTT, 10mM amino-n-caproic acid, Protease Inhibitor Cocktail for Plant tissues (Sigma)) in an eppendorf tube with a small pestle, or in a large mortar and pestle. The homogenates were centrifuged briefly in the microcentrifuge at maximum speed and the pellet was discarded. The total protein was quantified according to Ghosh *et al.* (1988). SDS-PAGE was performed on Mini protein Dual Slab cells (BioRad, Mississauga, Ontario), and the gels (12% polyacrlyamide) were stained with Coomassie brilliant blue R250 (Fairbanks et. al. 1971) or transferred to polyvinyldiene difluoride (PVDF) membranes using the semi-dry transfer method (semi-dry transfer cell, Bio-Rad, Hercules, CA). The blots were blocked for 30 s in 1mg/ml polyvinyl alcohol (Miranda et. al., 1993) and for 1 hour in phosphate-buffered saline (PBS) containing 0.1% (v/v) Tween 20 and 5% (w/v) powdered milk. Primary antibody (from bleeds after second injection) was diluted 1:50 in PBS containing 0.1% (v/v) Tween 20 and 1% (w/v) powdered milk. Antigen was visualized using secondary antibody made in goat against rabbit antibody coupled to alkaline phosphatase (Bioshop, Burlington, Ontario) and the phosphatase substrates, NBT and BCIP (BioRad, Mississauga, ON).

### Example 17

### Production of Transformed Arabidopsis thaliana Plants over expressing three eIF-5A isoforms

### Primer design

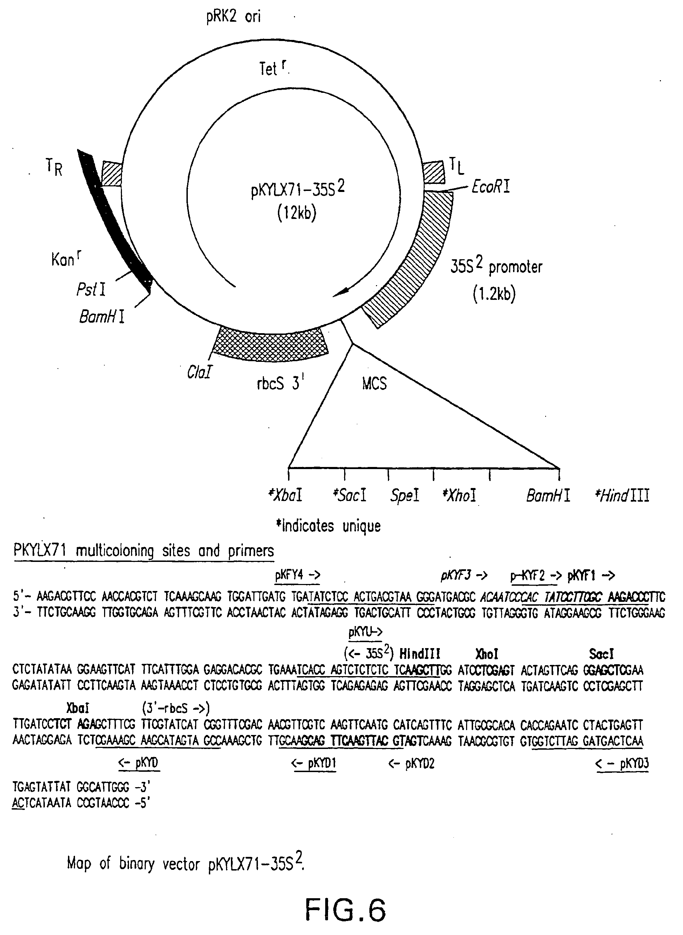

Eukaryotic translation initiation factor 5A (eIF-5A) isoforms of Arabidopsis thaliana (At) are highly homologous in the coding region (Figure 2). To avoid problems with amplification of the correct genes, primers for senescence-induced AteIF-5A, wounding/pathogen induced eIF-5A and growth eIF-5A were designed from the approximate beginning of the 5'UTR and at the end of the 3'UTR as shown in Figure 3, 4 and 5 respectively. The 5'UTR and 3'UTR were estimated based on EST information and other sequence information in the GenBank database. The appropriate restriction sites were added to the ends of the primers for ligation in the sense orientation in the pKYLX71 binary vector (Figure 6). For senescence-induced AteIF-5A the upstream primer is 5' AAGCTT GATCGTGGTCAACTTCCTCTGTTACC 3' and the downstream primer is 5' GAGCT CAGAAGAAGTATAAAAACCATC 3'. For wounding/pathogen induced AteIF-5A the upstream primer is 5' CTC GAGTGCTCACTTCTCTCTCTTAGG 3' and the downstream primer is 5' GAGCTCA AGAATAACATCTCATAAGAAAC 3'. The upstream primer for growth AteIF-5A is 5' CTC GAGCTAAACTCCATTCGCTGACTTCGC 3' and the downstream primer is 5' GAGC TCTAGTAAATATAAGAGTGTCTTGC 3'. The restriction sites that were added into the primers were *Hind*III and *Sac*I for senescence-induced AteIF-5A, *Xho*I and *Sac*I for wounding/pathogen induced AteIF-5A, and *Xho*I and *Sac*I for growthAteIF-5A as indicated by underlining in the primers listed above.

### Isolation of Genomic DNA from Arabidopsis thaliana

Genomic DNA was isolated from 3-week-old rosette leaf. The tissue was homogenized in extraction buffer (200mM Tris pH 7.5, 250mM NaCl, 25mM EDTA, 0.5% SDS) and the resulting homogenate was vortexed for 15 seconds. The remaining debris was removed by centrifugation in a microcentrifuge at maximum speed for 1 minute. The supernatant was collected and mixed in a 1:1 ratio with isopropanol, vortexed and left at room temperature for 2 minutes. A pellet was collected by centrifugation in a microcentrifuge at maximum speed for 5 minutes, washed with 70% ethanol and vacuum dried for 2 minutes. The dried pellet was resuspended in water and treated with 1:1 volume of chloroform and vortexed. After centrifugation in a microcentrifuge at maximum speed for 2 minutes the top layer was collected and treated with 20µl salt (3M sodium acetate) and 2 volumes of ethanol for precipitation at -20°C for 30 minutes. The purified genomic DNA was then centrifuged at maximum speed for 30 minutes in a microcentrifuge, dried and resuspended in water for PCR.

### PCR from Genomic DNA

PCR was performed with the primers described above. The PCR reaction mixture contained 1x *Tsg* or *Taq* polymerase reaction buffer, 1U of *Tsg* or *Taq* polymerase, 0.2mM dNTP, 2mM MgCl₂, and 15pmols of each specific primer accordingly. The reaction began with a hot start at 95°C for 10 minutes and first cycle consisted of 1 minute denaturing temperature of 95°C, 2 minutes annealing temperature of 55°C, and a 2 minute extension temperature of 72°C. The following 29 cycles proceeded a touchdown program where the annealing temperature was decreased by 0.5°C per cycle, and the final cycle had an annealing temperature of 40°C. The final extension of 72°C was held for 10 minutes. The PCR products were separated by 1% agarose gel electrophoresis, cut out and retrieved by Millipore Ultrafree-DA for DNA Extraction from Agarose spin columns (Millipore Corporation, Bedford, MA) according to directions.

### Ligation into pGEM®-T Easy

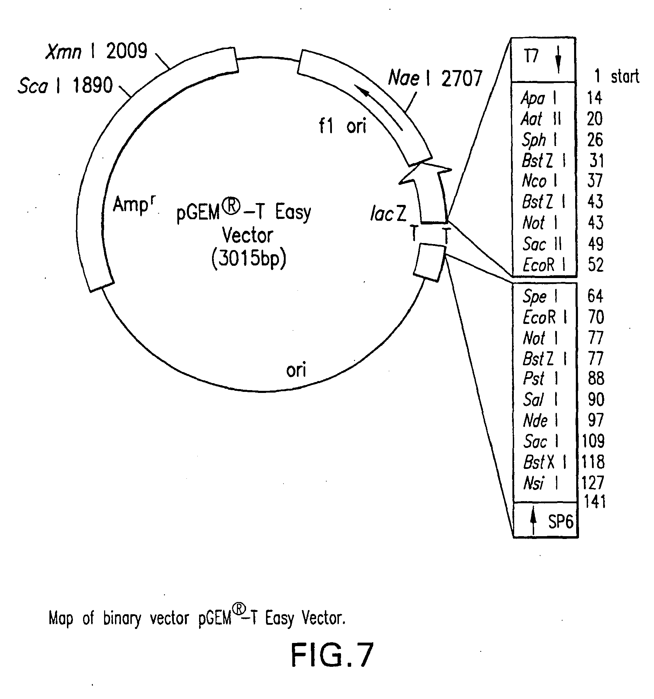

Purified PCR products were ligated into pGEM®-T Easy Vector (Figure 7) according to directions provided by Promega. Briefly, PCR products were mixed in a 3:1 ratio with pGEM T-Easy Vector, 3 Weiss Units T4 DNA ligase in Rapid Ligation Buffer (30mM Tris-HCl, 10mM MgCl₂, 10mM DTT, 1mM ATP, and 5% polyethylene glycol (MW8000, ACS Grade) pH 7.8) provided in the Promega pGEM®-T Easy Vector System (Promega Corporation, Madison WI). The ligation reaction was incubated overnight at 15°C and transformed into competent *E. coli* DH5-α cell suspension (made competent using RbCl/CaCl; Kushner, 1978). The transformation mixture was first incubated on ice for 30 minutes, heat shocked for 90 seconds at 42°C, and allowed to recover at 37°C for 1 hour after the addition of 1 ml 2xYT broth. The transformed cells were pelleted, resuspended in a small volume of 2xYT broth and plated on agar plates containing 50µg/ml ampicillin for selection. Only transformants are able to grow on the ampicillin-containing plates as the pGEM®-T Easy Vector provides ampicillin resistance to the cells. Transformants were selected and screened for the PCR product insert ligated into the pGEM®-T Easy Vector.

### Screening for PCR product inserts in pGEM®-T Easy Vector through Restriction Enzyme Digestions

Colonies that grew on selection media were grown in 5ml 2xYT broth containing 50µg/ml ampicillin overnight at 37°C. The recombinant plasmids from the selected colonies were purified using Wizard Prep DNA Purification Kit (Promega). The plasmid DNA was digested with *EcoR*I for 1 hour at 37°C and visualized on a 1% agarose gel for verification that the AteIF-5As insert sizes were present. The positive plasmids were then sequenced by the Core Molecular Biology Facility (University of Waterloo, Waterloo, ON) for confirmation that the sequence is suitable for over expression *in planta.*

### Ligation into pKYLX71

The constructs ofpGEM:wounding/pathogen induced AteIF-5A, and pGEM:growth AteIF-5A were double digested with *Xho*I and *Sac*I and sub-cloned into the binary vector, pKYLX71 that had also been digested with *Xho*I and *Sac*I*.* These enzyme digestions ensured that wounding/pathogen induced AteIF-5A and growth AteIF-5A would be inserted in the sense orientation in the binary vector pKYLX71 under the control of the cauliflower mosaic virus double 35S promoter. The ligation reactions used 1µg of binary vector and 3µg of either wounding/pathogen induced AteIF-5A or growth AteIF-5A. Ligation took place in ligation buffer (30mM Tris-HCl, 10mM MgCl₂, 10mM DTT, 1mM ATP, and 5% polyethylene glycol (MW8000, ACS Grade) pH 7.8) with 3 Weiss units of T4 DNA Ligase (Fermentas). The ligation reaction was incubated overnight at 15°C and transformed into competent *E. coli* DH5-α cell suspension (made competent using RbCl/CaCl; Kushner, 1978). The transformation mixture was first incubated on ice for 30 minutes; heat shocked for 90 seconds at 42°C and allowed to recover at 37°C for 1 hour after the addition of 1ml 2xYT broth. The transformed cells were pelleted, resuspended in a small volume of 2xYT broth and plated on agar plates containing 50µg/ml tetracycline for selection. Only transformants are able to grow on the tetracycline-containing plates as the binary vector pKYLX71 provides tetracycline resistance to bacterial cells. Transformants were selected and screened for wounding/pathogen induced AteIF-5A or growth AteIF5A insert by PCR and double digestion with *Xho*I and *Sac*I*.* Following PCR amplification (same as was done with genomic DNA explained above) and digestion, the products were separated using 1% agarose electrophoresis for conformation of the correct sized insert.

### Agrobacterium electroporation and selection

The constructs pKYLX71:wounding/pathogen induced AteIF-5A and pKYLX71:growth AteIF-5A was electroporated into competent *Agrobacterium tumefaciens* GV3010. The preparation of competent *Agrobacterium* cells a single colony was inoculated in 5ml of 2xYT broth containing 50µg/ml of rifampicin, and 50µg/ml gentamycin. This grew overnight at 28°C in a Forma Scientific Orbital Shaker (Fisher Scientific) at 280rpm and was used to inoculate 30ml cultures of 2xYT also with 50µg/ml of rifampicin, and 50µg/ml gentamycin at various dilutions (1:500, 1:1000, 1:2000). The newly inoculated cultures grew until OD₆₀₀ was between 0.5 and 0.8 before being cooled and centrifuged down in an SS-34 rotor (Sorvall) at 2000g for 15 minutes. The pellets were resuspended in 50ml of ice-cold water and centrifuged at 2000g for 15 minutes. This washing procedure was repeated for a total of four times to remove the salts and the dead cells from the culture. The final pellet was resuspended in 40 ml ice cold 10% (v/v) glycerol and centrifuged at 2000g for 15 minutes and repeated once. The pellet was then resuspended in 100 µl ice-cold 10% glycerol and mixed well. Cells were split up into aliquots of 100 µl and stored on ice.

For electroporation of the DNA constructs into the competent *Agrobacterium* cells the 100 µl aliquots were each mixed well with 500 ng of DNA construct. The bacteria:vector mixture was then transferred to a pre-cooled electroporation cuvette and placed in the Gene Pulser (Biorad) adjusted to the following settings: 2.5kV, 25µF, and 200Ω. After electroporation 1ml 2xYT broth was added and the whole suspension was transferred to a culture tube. The electroporated cultures were incubated at 28°C, 280rpm, for 3 hours to allow them to recover and then 2 ml 2x YT both was added as well as 50µg/ml of rifampicin, and 50µg/ml gentamycin. After 2 days of growing in culture the electroporated cells were plated on tetracycline, gentamycin and rifampicin (all at 50µg/ml) and colonies grew after an addition 2 days. The resulting colonies were screened for pKYLX71 :wounding/pathogen induced AteIF-5A or pKYLX71 :growth AteIF-5A by PCR and double digestion with *Sac*I and *Xho*I*,* and visualized by separation on a 1% agarose gel.

### Plant Transformation

A positive colony of *Agrobacterium tumefaciens* GV301 0 containing either pKYLX71:wounding/pathogen induced AteIF-5A or pKYLX71:growth AteIF-5A were used for the transformation of wild type *Arabidopsis thaliana* ecotype Columbia. In preparation of the bacterial slurry used for plant transformation a single colony positive for pKYLX71:wounding/pathogen induced AteIF-5A or pKYLX71:growth AteIF-5A construct was inoculated in 5ml of 2xYT broth containing 50µg/ml of tetracycline, 50µg/ml of rifampicin, and 50µg/ml gentamycin. This grew for 2 days at 28°C in a Forma Scientific Orbital Shaker (Fisher Scientific) at 280rpm and was used to inoculate 35ml (total) 2xYT also with 50µg/ml of rifampicin, and 50µg/ml gentamycin. The 35ml culture was grown overnight at 28°C, 280rpm, and used to inoculate 535ml (total) 2xYT with 50µg/ml of rifampicin, and 50µg/ml gentamycin. Again the culture was grown overnight at 28°C, 280rpm, to an OD₆₀₀ of about 2.0.

The cultures were transferred to two 250ml tubes before centrifugation for 15 minutes at 1945g at 4°C in a GSA rotor (Sorvall). The pellets were resuspended in 500ml of infiltration media (1.1 g MS salts, 25g sucrose, 0.25g MES, pH5.7 with KOH, 100ng/ml benzylaminopurine and 50µl Vac-In-Stuff (Silwet L-77; Lehle Seeds)) and placed in a large plastic dish in a vacuum desiccator with 4 large rubber stoppers. Five pots containing 8 plants each at the right stage of development were used sequentially for infiltration. Each pot was first inverted over a trash can to remove any loose soil, then was placed (still inverted) into plastic container in the glass desiccator so that the 4 large rubber stoppers acted as stand for the inverted pot thus allowing the bolts to be dipped into the *Agrobacterium* slurry, but not the rosettes. The plants were then subjected to a vacuum (400mm Hg) in this inverted state for 10 minutes. The vacuum infiltrated plants were then allowed to recover and grown as usual in the growth chamber conditions explained in the plant material section. After several weeks when the siliques were dry and seed matured, the seeds were collected with each pot pooled together.

### Selecting plant transformants and Segregation Analysis

To identify primary transformants, seeds from the vacuum-infiltrated plants were surface sterilized in a solution of 1% (v/v) sodium hypochlorite and 0.1 % (v/v) Tween 80 for 20 minutes on a rotator (Barnstead/Thermolyne), rinsed four times with sterile water, and resuspended in a sterile 0.8% agar. The resuspended seeds were then planted onto sterile, half-strength Murashige and Skoog (MS) medium (2.2g/L) supplemented with 1% (w/v) sucrose, 0.5g/L 2-[N-Morpholino] ethanesulfonic acid (MES), 0.7% (w/v) bacteriological agar and 40 to 50µg/ml kanamycin (Murashige and Shoog, 1962). Only transformants are able to grow on the kanamycin-containing plates since the binary vector provides the kanamycin resistance gene to the transformant seedlings (Figure 6). Seedlings that do not harbour the binary vector become yellow and die, as there is no kanamycin resistance gene. Wild-type seedlings were used as controls and plated onto MS medium without kanamycin added to the medium, as well seeds from a homozygous line containing empty pKYLX71 vectors were seeded as controls on kanamycin containing plates. The empty vector control is useful in demonstrating the effect kanamycin has on growth of the seedlings as well as the effect of random integration of the binary vector into the genome of *Arabidopsis thaliana.* A small amount of wild type seed was plated onto a small area of each plate containing MS medium and 40 to 50µg/ml kanamycin. This was done in order to make sure the medium was selective enough for the transformants and to test the strength of the kanamycin.

The seeded plates were kept at 4°C for 3 days to synchronize the germination. After 3 days the plates were transferred to growth chambers where they grew for an additional 7 days under 16-h light/ 8-h dark cycles at 20±2°C. Lighting was maintained at 150µmol radiation m^{-2.}s⁻¹ and was provided by cool-white fluorescent bulbs. The efficiency for transformation of *Arabidopsis thaliana* plants with the pKYLX71:wounding/pathogen induced AteIF-5A and pKYLX71:growth AteIF-5A vectors was determined.

After a total of 10 days since seeding, the 14 transformants or the 16 transformants for Sense wounding/pathogen induced AteIF-5A and Sense growth AteIF-5A respectively were transplanted to Promix BX soil (Premier Brands, Brampton, ON, Canada) in flats containing 32 cells. These transplanted T1 generation plants were then transferred into another growth chamber operating at 22°C with 16-h light/ 8-h dark cycles. Lighting at 150µmol radiation m^{-2.}s⁻¹ was provided by cool-white fluorescent bulbs. The T1 generation plants grew to maturity and produced T2 generation seeds. These were harvested and stored at-20°C until further screening was done. The T1 generation was named 1, 2, 3, etc. All 16 lines of Sense growth AteIF-5A plants survived and produced seeds, but only 9 out of 14 transformants of the Sense wounding/pathogen induced AteIF-5A plants survived and produced seeds.

The selection of T2 generation transformants was conducted in the same way as the T1 generation transformants. Line 12 of the Sense growth AteIF-5A plants produced no transformants on the selectable media and was not included in any further work. Lines 1 through to 16 (minus line 12) of the Sense growth AteIF-5A plants each had 8 sublines carried through. These were named A through H so that for example in the T1 line 1, the T2 generation plants were named 1A, 1B, 1C, etc. Lines 1, 2, 3, 4, 5, 7, 9, and 11 of the Sense wounding/pathogen induced AteIF-5A plants each had 8 sublines (A-H) carried through. Line 12 T1 plants had only produced about 30 T2 seeds and only 1 subline in the T2 generation will be carried through. T2 plants of Sense wounding/pathogen induced AteIF-5A are still growing and being characterized. The T2 plants for the Sense growth AteIF-5A have matured and produced seeds, which were harvested and stored at -20°C until further analysis.

The selection of the T3 generation transformants of Sense growth AteIF-5A was conducted in the same manner as the T2. Eight lines were chosen based on phenotype analysis as well as the degree of over expression of Sense growth AteIF-5A. The levels of expression were broken down into four categories: high-level expression, medium-level expression, low-level expression, and no expression (due to co-suppression). Two lines were chosen for each of the levels of expression and 12 plants from each line were transplanted. The corresponding lines for these four levels of expression are: 1A, 2D, 4D, 15A, 8D, 9H, 11C and 16C. The T3 generation for Sense growth AteIF-5A plants are still growing and being characterized.

### Example 18

### Phenotype Analysis of Sense wounding/pathogen induced AteIF5A and Sense growth AteIF5A:

### Photographic Record

Morphological phenotypes of the Sense wounding/pathogen induced AteIF-5A and Sense growth AteIF-5A lines were recorded photographically during segregation, as were the phenotypes of the corresponding control wild type plants (*Arabidopsis thaliana* ecotype Columbia) and plants transformed with an empty binary vector pKYLX71.

### Seed Measurements

T3 seeds collected from T2 plants of Sense growth AteIF-5A were measured for total seed yield (both weight and volume), seed size (length and width), and calculated individual weight and volume of produced seed. Total seed yield by weight was measured on a Sartorius analytical digitized scale, and the volume was determined by pouring and packing down the total seed yielded by each plant into a glass 1ml syringe that was graduated every 100µl. To determine the seed size by length, width and calculated volume, the seeds were placed on a slide containing a micrometer and viewed on an Olympus BX51 Microscope. Photographs of the seeds on the micrometer were taken with a Spot Insight Color Camera (Diagnostic Instruments Inc.) attached to a Compaq Evo D500 (Compaq Company Corporation; Intel® Pentium 4 CPU 1.7GHz, 262 MG RAM, running Windows 2000). Using Image-Pro Express Version 4.0 for Windows. Measurements of 10 seeds in each subline were made using the micrometer in the image for size calibration. The measurements were imported into Microsoft Excel, and calculations such as standard error and volume were performed.

### Example 19

### Biochemical Analysis of Sense wounding/pathogen inducedAteIF5A and Sense growth AteIF5A - Protein Fractionation and Western Blotting

The first cauline leaf from each subline of Sense growth AteIF-5A T2 plants were collected and proteins extracted as described above. Total protein from lines 1A, 2A, up to 16A were fractionated by 12% SDS-PAGE and transferred to a PVDF membrane. The blot was probed with growth αAteIF-5A at a 1:50 dilution. Control total protein was extracted from the first cauline leaf from wild type and empty binary vector control plants.

### Example 20

### Expression of Arabidopsis thaliana translation initiation factor 5A (AteIF-5A) isoforms in wild type Columbia

Several tissues were collected at different developmental stages and the extracted proteins from these tissues were used for Western blotting. The Western blot in Figure 8 demonstrates that senescence-induced AteIF-5A is not present in the 2 week old rosette leaves, but is upregulated in the 3 week old rosette leaves and increases in abundance until 5 weeks and declines in abundance, but is still present at 7 weeks. No senescence AteIF-5A was detected in the PEG treated plants or control, but was present in the flower lane (which included senescent flowers) and in the imbibed seed lane reflecting senescence of cotyledonary tissues. When the blot was probed with the wounding/pathogen induced αATeIF-5A antibody, faint bands appeared in the siliques, imbibed seed and stem lanes. The band seen in the siliques and stem lanes may be due to the wounding that occurred with collection of the tissue. Since it is difficult to collect the siliques and stem, they were not flash frozen immediately allowing for some up-regulation of the wounding/pathogen induced isoform of AteIF-5A. The only band that appeared when the blot was probed with growth αAT-eIF5A was imbibed seeds, keeping with the notion that this is the isoform involved in cell division.

Plants that were treated with either no treatment, mock inoculation with MgCl₂, avr *P. syringae* or with vir *P. syringae* were collected at several time points to analyze the expression of the AteIF-5As during pathogen ingress. The avr strain is recognizable by the plant and induces the hypersensitive response that leads to cell death or necrosis in the region of infection, thus disallowing the pathogen to cause disease. Furthermore the localized response eventually becomes a systemic response in order to protect the plant from further ingress. This is known as Systemic Acquired Resistance (SAR), which involves the expression of a suite of genes known as the Pathogenesis Response (PR) genes. On the other hand the vir strain will not be recognized by the plant, and will not induce a hypersensitive response and will lead to disease. The diseased state of *Arabidopsis thaliana* includes yellowing leaves and cell death after a few days post infection. After 72 hours post treatment control plants, mock treated plants, avr treated plants and vir treated plants were collected for western blotting with the three αAteIF-5A antibodies (Figure 9). At this point both SAR and disease were visible in the avr treated and the vir treated plants respectively. When probed with the senescence-induced αAteIF-5A antibody, a band that was relatively the same in all the samples was observed. Since all of the plants were 4 weeks old this came with no surprise, since the senescence isoform was seen starting at 3 weeks in Figure 8. When the blot was then probed with the wounding/pathogen induced αAteIF-5A antibody, a faint band was detectable in the untreated, mock treated and avr treated plants where there was a strong band detected in the vir treated plants. This upregulation of the wounding isoform may be due to cell death caused by disease (also a type of cellular wounding). The blot probed with growth αAteIF-5A did not show any bands and thus was not included in the figure. As the senescence-induced AteIF-5A did not change in expression during these treatments demonstrates its specificity for natural senescence. The increase in wounding/pathogen induced AteIF-5A expression also demonstrates its specificity for death due to wounding. To further investigate this possibility, an experiment was performed with wounding leaves of Arabidopsis thaliana.

The wounding experiment showed similar results as the pathogenesis experiment (Figure 10). Northern blots were used to show the transcriptional change in of senescence-induced AteIF-5A, wounding/pathogen induced AteIF-5A and growth AteIF-5A. The probes were specific to each of the AteIF-5As and consisted of the 3' UTR of each. It was observed that like the pathogenesis experiment senescence-induced AteIF-5A expression did not change, as these were 4-week-old plants and samples were only taken over a 9-hour interval. This again is consistent with the fact that senescence-induced AteIF-5A is natural senescence specific isoform. The expression of wounding/pathogen induced AteIF-5A however did increase after 9 hours. There is probably some translational control occurring, as the transcript appears fairly constitutive (Figure 10), but the protein does not appear as highly expressed when not induced (Figure 9). The transcript for growth AteIF-5A was barely detectable in all the samples, and shows a decline in expression post wounding.

### Example 21

### Production of Transformed Arabidopsis thaliana Plants over expressing three eIF-5A isoforms

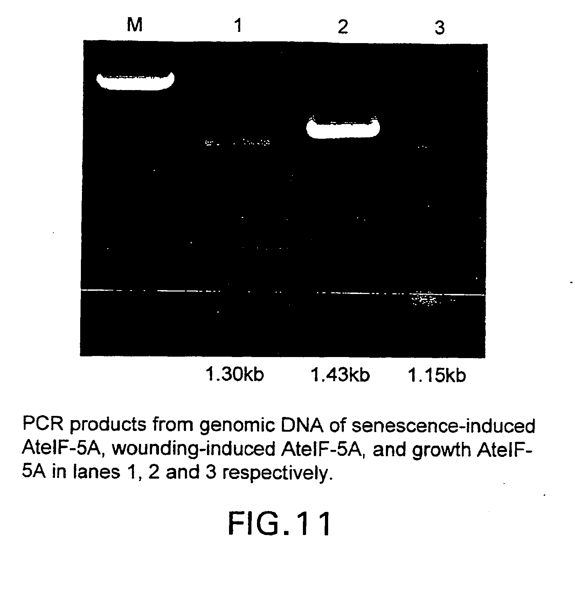

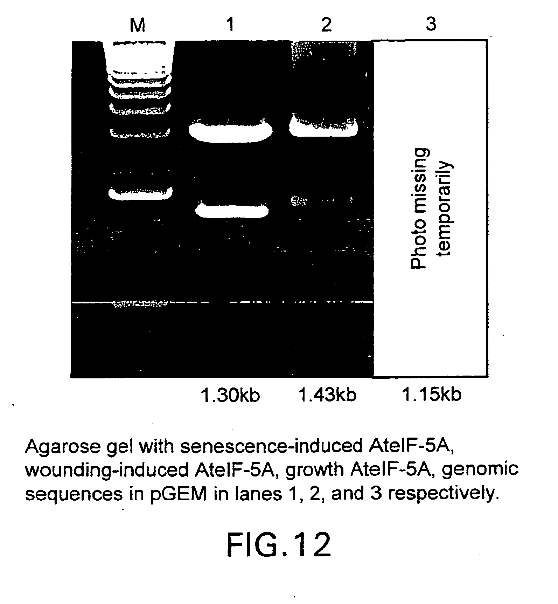

The AteIF-5As were isolated from genomic DNA by PCR (Figure 11). The products were ligated in pGEM (Figure 12) and the sequence was verified for suitability for over-expression *in planta.* Wounding/pathogen induced AteIF-5A and growth AteIF-5A were double digested out of pGEM with *Xho*I and *Sac*I and ligated in the sense orientation behind the cauliflower mosaic virus 35S² promoter in pKYLX71. Positive ligation was confirmed by digestion and PCR (Figure 13). The pKYLX71:senescence-induced AteIF-5A and the pKYLX71:growth AteIF-5A were then electroporated into *Agrobacterium tumefaciens* GV3010 for transformation via vacuum infiltration of *Arabidopsis thaliana* wild type of the ecotype Columbia. After plant transformation the seeds were collected and transformants selected for on Kanamycin containing MS plates.

### Arabidopsis thaliana plants over expressing wounding/pathogen induced AteIF-5A (Sense wounding/pathogen induced AteIF-5A)

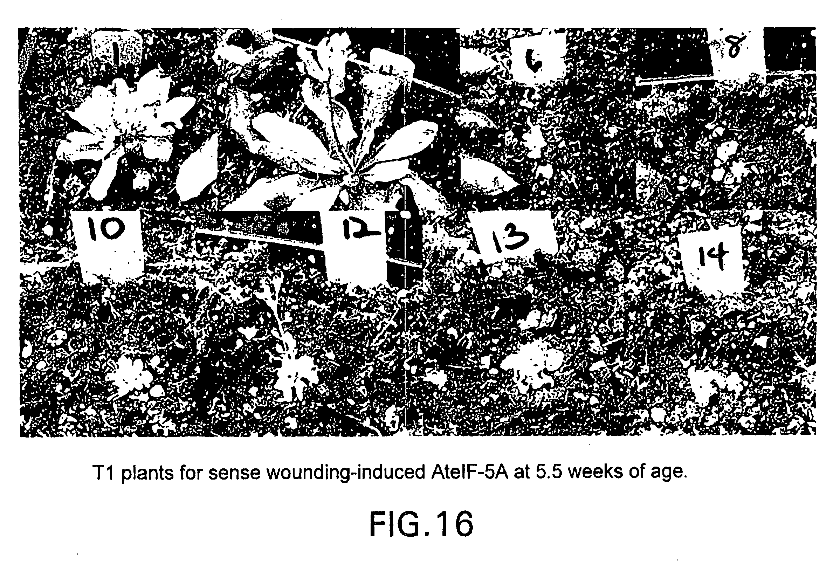

T1 generation plants were seeded on MS plates containing 50µg/ml Kanamycin and were stored at 4°C for 3 days and in the growth chamber for 7 days (Figure 14). There were 14 transformants that were transplanted to soil. A common phenotype in these 14 T1 generation plants was stunted growth. Lines 1, 4, 6, 8, 10, 11, 12, 13, and 14 were severely stunted in their growth and 6, 8, 10, 13 and 14 did not produce any seed. Lines 2 and 3 were moderately stunted whereas lines 5, 7 and 9 grew similarly to wild type plants (Figure 15 and Figure 16). Some other phenotypes observed in the T1 generation of Sense wounding/pathogen induced AteIF-5A plants included yellow leaves, purple cotyledons, curled up leaves and differences in flower shape. It is interesting to note that the appearance in the stunted growth was not observed until the plants were transplanted to soil. A possible explanation of this would be that during transplant the roots are damaged slightly (a consequence of transplanting that is unavoidable) and were unable to recover. In fact a preliminary experiment where seeds were soaked in a Kanamycin solution and seeded to soil directly no stunted plants were observed (whereas previously 70% of the plants had some degree of stunting), as no root damage would be invoked without transplantation.

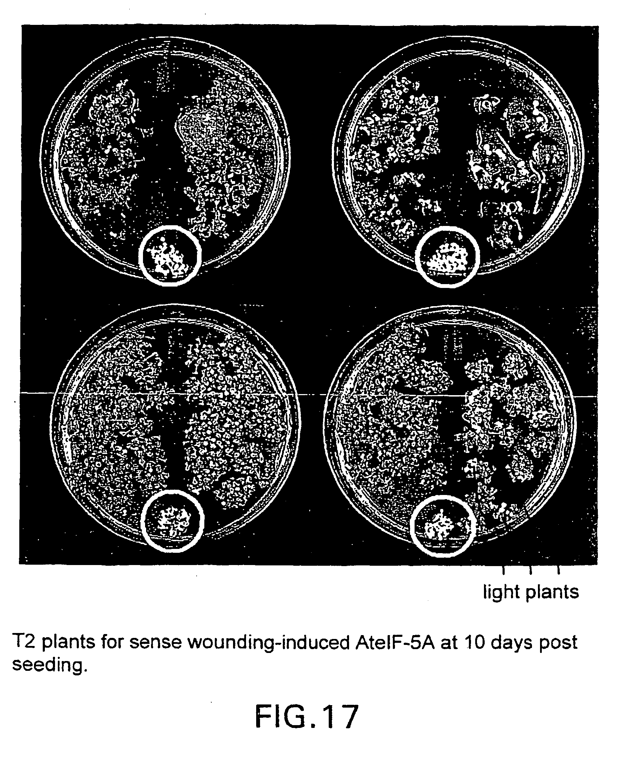

Lines 1, 2, 3, 4, 5, 7, 8, 11 and 12 produced T2 seeds and were carried through (Figure 17). Each T2 line has sublines A-H, except for 12, which only grew one transformant, and are currently being analyzed.

### Arabidopsis thaliana plants over expressing growth AteIF-5A (Sense growth AteIF-5A)

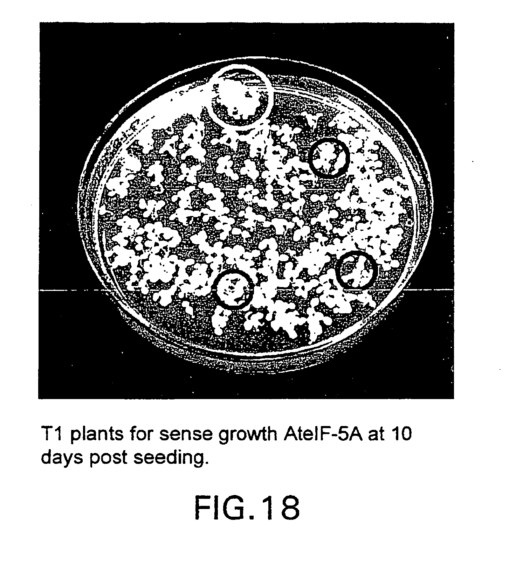

The T1 generation seeds of Sense growth AteIF-5A were grown on selective media and 16 transformants grew (Figure 18). The transformants were photographed over their lifetime. The phenotypes varied from similar to wild type (Lines 1, 2, 5, 6, 7, 8, 10, 11, 12, 13, 14, 15, and 16) to moderately stunted and yellow (Lines 2, 4 and 9; Figure 19). All the lines were carried through to T2 and each line had 8 sublines labeled A-H. Line 12 did not produce any transformants in T2 and was deemed to be wild type. The T2 generation plants had much more exaggerated phenotypes than that of T1 generation plants. The lines that were carried to T3 will be discussed in detail.

The Sense growth AteIF-5A T2 generation lines were characterized in groups according to the level of expression of the growth AteIF-5A transgene. A Western blot was performed on protein extracted from cauline leaves from each line (Figure 20). Since most of the sublines A-H demonstrated similar phenotypes within a line, the Western blot was only done with subline A of each line to get a general overview of level of expression of growth AteIF-5A. Protein from the cauline leaves of wild type plants and plants containing the empty binary vector were used as controls on the gels. The level of expression observed in these sublines can be categorized as high (Lines 1, 2, 3, 10, 13), medium (Lines 4, 5, 6, 15), low (Lines 7,8,9,14) or none (Lines 11, 16, wild type and binary control). The blots were also probed with antibodies against senescence-induced AteIF-5A and wounding/pathogen induced AteIF-5A. These westerns indicated that the increase in expression in the Sense growth AteIF-5A lines is due to growth AteIF-5A and not a general upregulation of other AteIF-5A isoforms, as no significant amount of either isoform was detected. This also demonstrated that the specificity of the isoform specific antibodies is acceptable.

The Sense growth AteIF-5A lines be carried through to the T3 generation were chosen based on phenotype as well as the level of expression of growth AteIF-5A (See Table 1 for a summary of phenotypes within each line). Two lines from each category of level of expression were chosen. The lines that will be carried through are 1A, 2D, 4D, 15A, 8D, 9H, 11C, and 16C.

Line 1 according to the western blot in Figure 20, has a high level of growth AteIF-5A expression. These plants had large, dark green rosettes with leaves that were quite round in comparison to wild type plants (Figure 21). The rosettes of line 1 also had a whorled phenotype, where the leaves all curl in the same direction. These Sense growth AteIF-5A plants bolted slightly later than wild type. Line 2 also demonstrated high level of growth AteIF-5A expression, but differed from line 1 in that these plants were small and yellowed (Figure 22). Line 2 plants also bolted later than the wild type and binary control plants, as well produced smaller bolts (about half the size) and fewer siliques.

Of the medium level of expression lines, line 4 appeared similar to wild type in leaf/rosette size and in bolt size, though appeared to bolt just a few days before the wild type and binary control plants. The second line with a medium level of expression of growth AteIF-5A is line 15. These plants are, like line 4, very similar to wild type, but the area that the rosette occupied was larger than the controls (fig. 23 and 24). The leaves of the rosette also appeared to be rounder at the tips than the controls. The bolts however did not appear to have any distinctive phenotype.

The low expressing Sense growth AteIF-5A lines that will be carried through to T3 are from lines 8 and 9. Line 8 had very large leaves and large rosettes compared to the control plants (Figure 25). The leaves also appeared to be wider and rounder than the control plants. The time of bolting, bolt size and number seemed to be consistent with the controls. The Sense growth AteIF-5A line 9 had similar leaf shape as in line 8, but was far more yellow and smaller (Figure 26). As in line 2 (one of the high expressing lines), these plants show stunted growth, shorter bolts, but unlike line 2, line 9 bolted about the same time as the control plants.

The two lines 11 and 16 of the Sense growth AteIF-5A plants according to the western blot (Figure 20) have no upregulated expression of growth AteIF-5A. This may be due to cosuppression of the transgene as well as the endogenous gene. Though these plants do look similar to the controls (Figure 27 and Figure 28), it is believed that the transgene is incorporated into the genome of lines 11 and 16 for several reasons. Firstly, they do have Kanamycin resistance as demonstrated by the selectivity on the Kanamycin containing MS plates. Secondly, the rosette size, leaf size, and bolt size of line 16 (Figure 28) are at least 50% larger than the controls. But the strongest evidence is in the size and composition of the T3 seeds that they produced.

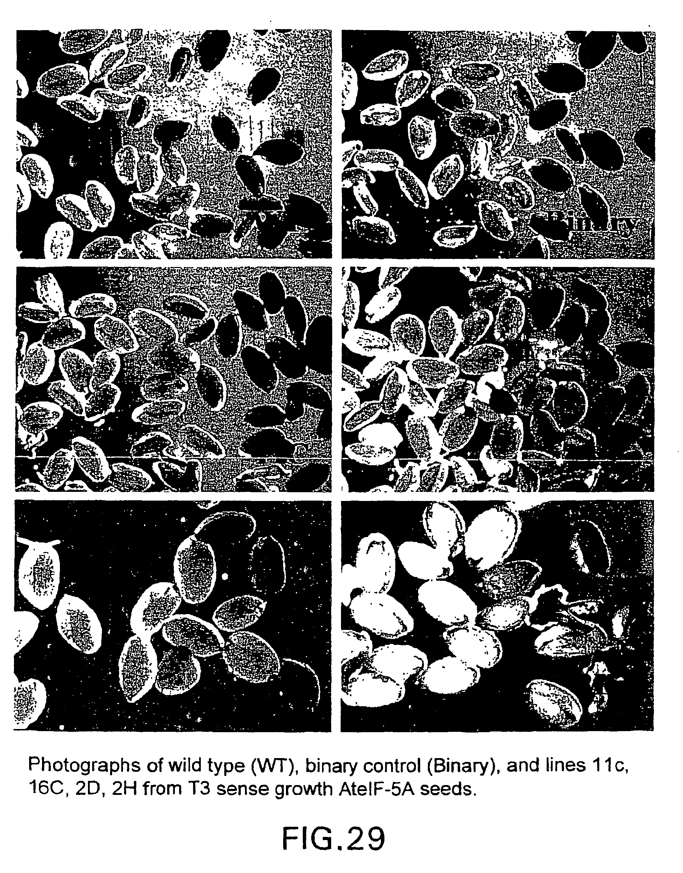

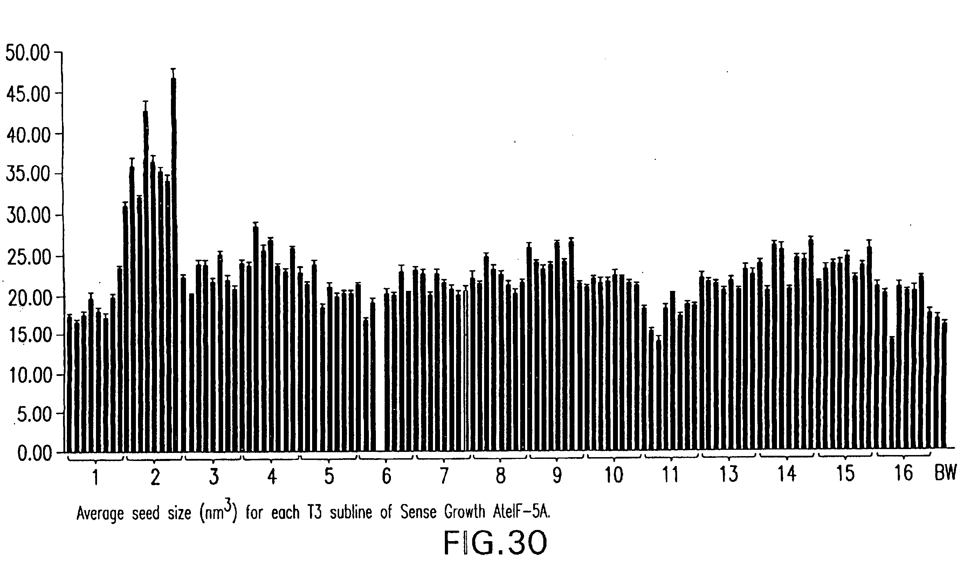

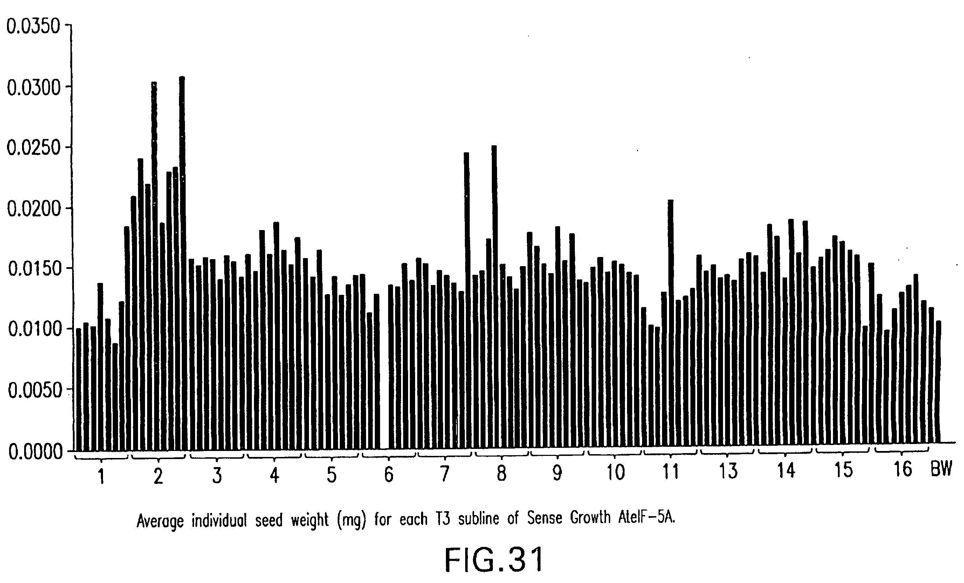

The T3 seeds were measured from all lines of T2 Sense growth AteIf-5A plants. Photographs were taken of each line (the largest and the smallest highlighted in Figure 29), and measurements were made *in silico* with a micrometer in the photographs used for calibration. For each line and for the controls, ten of the largest seeds in the field of view were measured and used for calculations. It was found that the high expression line 2 had seeds that were up to 3 times as large as the wild type and binary controls. Whereas the lines that demonstrated the lowest expression (Lines 11 and 16) had some of the smallest seeds that were only about 88% the size of wild type or binary control seeds. The average seed size for each line was expressed as nm³ (Figure 30) and was calculated using an equation for the volume of an ellipsoid as seeds from *Arabidopsis thaliana* are approximately ellipsoid. The measured size of the control seeds fell into published guidelines as determined by Boyes et al (2001). From the measured size of individual seeds and the total seed yield (both weight and volume), the average individual seed weight was calculated and plotted (Figure 31). It appeared that most of the lines that demonstrated a different size than that of the control seeds also had the same trend in individual seed weight. In fact when the seed weight was plotted against the seed size (volume) the relationship was mostly linear with an R²=0.7412. There were 5 lines that were outliers that had either an increased density (3 of them) or a decreased density (2 of them). One of the lines with the increased density is 8D and will be carried through T3 generation. The total seed yield from all the T2 generation plants were quite variable, with few trends. One notable line however is the medium expressing Sense growth AteIF-5A line 4D, which produced the most seeds (both weight and volume). In fact 4D produced 2.5 fold more than the control plants and will be carried through T3.

T3 seeds were plated on selection media as described previously. Lines 1A, 2D, 4D, 15A, 8D, 9H, 11C and 16C were transplanted to soil. Several other sublines of Sense growth AteIF-5A line 1 did not germinate, as well as line 2H, which had the largest seeds of all the sublines did not germinate. Plants from line 11 (one of the cosuppression lines) were not as healthy as typically found at this age. These seeds were also one of the smallest measured. It appears that these lines are still segregating, as there were still non-Kanamycin resistant plants as well as seeds that did not germinate from all the lines. This is probably a side effect of the transgene and not technique as the control seeds that were treated in the same manner, all germinated.

### Example 22

### Characterization of Arabidopsis senescence-induced eIF-5A

### Methodology for obtaining Full-length Arabidopsis senescence-induced eIF-5A

Degenerate primers based on several plant eIF-5A genes, in combination with vector primers T3 & T7 were used in order to PCR an eIF-5A gene from an *Arabidopsis* cDNA library. Specifically, the 5' region of the eIF-5A gene was obtained from a PCR reaction utilizing both the T3 primer (located upstream of the F5A gene in the library vector) and one of the downstream (reverse-orientation) degenerate primers. Likewise, the 3' region of the gene was obtained from a PCR reaction utilizing both the T7 primer (located downstream of the eIF-5A gene in the library vector) and one of the upstream (forward-orientation) degenerate primers. The full-length eIF-5A gene was derived from alignment analysis of the 5' region and 3' region of the gene.

There are 2-3 major products for each PCR reaction. These fragments were cloned to pBluescript plasmid and sequenced. The eIF-5A positive PCR fragments were identified based on the mapping analysis against the gene bank. There is only one upstream and downstream positive eIF-5A PCR fragments for Arabidopsis.

The specific 5'- and 3'-end primers for the *Arabidopsis* eIF-5A gene were designed according to the 5' and 3' PCR fragment sequencing results. The full-length *Arabidopsis* eIF-5A gene was obtained from a PCR reaction utilizing their specific 5'- and 3'-end primers and the corresponding cDNA library as a template. The full-length gene was further confirmed by sequencing. In the end, we cloned one *Arabidopsis* eIF-5A isoform gene, which was termed senescence-induced eIF-5A.
T3 and T7 Primers:
T3: 5' - ATT AAC CCT CAC TAA AG - 3'
T7: 5' - AAT ACG ACT CAC TAT AG - 3'
Degenerate Primers for Arabidopsis eIF5A:
Forward (upstream) primer: 5' - AAA RRY CGM CCY TGC AAG GT - 3'
Reverse (downstream) primer: 5' - TCY TTN CCY TCM KCT AAH CC - 3'

### Subclonng Arabidopsis antisense full-length senescence-induced eIF-5A into pKYLX71 vector (containing the SAG12 promoter)

Specific (Homologous) Primers for Arabidopsis senescence-induced eIF-5A, antisense full-length construct: Forward Full-length senescence-induced eIF-5A primer (30-mer): 5'-CCGAGCTCCTGTTACCAAAAAATCTGTACC -3' (note: underlined portion is the *Sac*I recognition sequence, used for ligating the 5'-end of the PCR fragment into the *Sac*I site in the Multiple Cloning Site (MCS) of pBluescript). Reverse full-length senescence-induced eIF-5A primer (36-mer): 5'-ACCTCGAGCGGCCGCAGAAGAAGTATAAAAACCATC -3' (note: underlined portion is the *Not*I recognition sequence, used for ligation into the MCS of pBluescript).

The orientation of the *Sac*I and *Not*I sites within the MCS of the pBluescript vector was such that the gene was subcloned in its antisense orientation (i.e. the *Not*I site is upstream of the *Sac*I site).

### Example 23

### SAG 12 promoter was used to express the antisense senescence-induced Arabidopsis Full-length eIF-5A

Experimental evidence shows that transcription of a set of "senescence-associated genes" or SAGs increases during the onset of senescence (Lohman *et al.,* 1994; Weaver *et al.,* 1998). In fact, senescence appears to begin with the synthesis of new mRNAs and probably down-regulation of other mRNAs, indicating that selective synthesis of proteins is necessary for senescence (Nooden, 1988). That the leaf senescence program is accompanied by changes in gene expression was first demonstrated by Watanabe and Imaseki (1982) using *in vitro* translation followed by gel electrophoresis to detect changes occurring in translatable mRNA populations. This initial work and subsequent analysis of the *in vitro* translated proteins revealed the abundance of most mRNAs diminished significantly during the progression of senescence while other translatable mRNAs increased (Watanabe and Imaseki, 1982; Davies and Grierson, 1989; Becker and Apel, 1993; Buchanan-Wollaston, 1994; Smart *et al.,* 1995). Differential screening of cDNA libraries made from mRNAs of senescent leaf tissues also demonstrated that the expression of many genes is down-regulated, whereas the expression of other genes is up-regulated during senescence. SAGs have been identified from a variety of plant species, including *Arabidopsis* (Hensel *et al.,* 1993; Taylor *et al.,* 1993; Lohman *et al.,* 1994; Oh *et al.,* 1996), asparagus (King *et al.,* 1995), barley (Becker and Apel, 1993), *Brassica napus* (Buchanan-Wollaston, 1994), maize (Smart *et al.,* 1995), radish (Azumi and Watanabe, 1991) and tomato (Davies and Grierson, 1989; Drake *et al.,* 1996). Senescence can be morphologically identified as a characteristically patterned leaf yellowing that begins at the edges of a leaf and reaches the veins last (Weaver *et al.,* 1998). Visible senescence in *Arabidopsis thaliana* rosette leaves appears approximately 21 days after germination with dramatic upregulation of SAG 12 at the time (Noh an Amasino, 1999). SAG 12 is a gene with the closest specificity for natural senescence and is thus termed a senescence marker. With no detectable expression in young leaves, SAG 12 is induced in older leaves after they are ~20% yellow but cannot be induced by treatment that does not induce yellowing of leaves (Weaver *et al.,* 1998). Its high degree of specificity for natural senescence can be explained by the fact that the gene product of SAG 12 shows similarity to cysteine proteases and may be involved in protein turnover during senescence (Lohman *et al.,* 1994; Weaver *et al.,* 1998).

### Description of transgenic plants

Transgenic *Arabidopsis* plants were generated expressing the full-length antisense senescence-induced eIF-5A transgene under the control of the SAG 12 (leaf senescence-specific) promoter, which is activated at the onset of natural leaf senescence, approximately 21 days after germination (Noh and Amasino, 1994), but not in the event of stress-induced senescence. At this point, the transgenic plants express phenotypes characteristic of suppressed full-length senescence-induced eIF-5A expression. Rosette leaves were harvested from 3 to 8-week-old transgenic *Arabidopsis* antisense full-length senescence-induced eIF-5A plants.

### Methodology for the production of homozygous transgenic antisense senescence-induced eIF-5A Arabidopsis thaliana plants under control of the SAG 12 promoter

### Inserting the SAG 12-antisense-full-length senescence-induced eIF-5A construct in pKYLX71

First, the plasmid pKYLX71 was cut with *EcoRI* and *HindIII* to remove its double 35S promoter, and resultant sticky ends were filled in with Klenow enzyme to create blunt ends. pKYLX71 without the promoter was then ligated to re-circularize the plasmid.

Secondly, the *Arabidopsis* SAG 12 promoter was amplified from genomic DNA by PCR using primers containing *SalI* and *XbaI,* as described below. This promoter sequence was then inserted into the Multiple Cloning Site (MCS) of pBlueScript using the restriction enzymes *SalI* and *XbaI* followed by ligation with T4 DNA ligase.

The forward SAG 12 Primer was 5'-GGC CGTCGACGATATCTCTTTTTATATTCAAAC-3' (underlined portion is *Sal*I recognition site, used for ligating the 5'-end of the PCR fragment into the *Sal*I site in the Multiple Cloning Site (MCS) ofpBluescript). The Reverse SAG 12 Primer was 5'-CGTCTAGACATTGTTTTAGGAAAGTTAAATGA-3' (underlined portion is the *Xba*I recognition site, used for ligating the 5'-end of the PCR fragment into the *Sac*I site in the Multiple Cloning Site (MCS) of pBluescript).

Thirdly, to create the pBlueScript-SAG 12:antisense-full length-senescence-induced eIF-5A construct, full length senescence-induced eIF-5A was amplified by PCR from the *Arabidopsis* cDNA library using primers with *SacI* and *NotI* restriction sites, as outlined below, and subcloned into the pBluescript-SAG 12 described in the previous paragraph. Note that the orientation of the *Sac*I and *Not*I sites within the MCS of the pBluescript-SAG 12 vector was such that the gene was subcloned in its antisense orientation (i.e. the *NotI* site is upstream of the *Sac*I site).

The forward full-length senescence-induced eIF-5A Primer was 5'-CCGAGCTCCTGTTACCAAAAAATCTGTACC -3' (note: underlined portion is the *Sac*I recognition sequence, used for ligating the 5'-end of the PCR fragment into the *Sac*I site in the Multiple Cloning Site (MCS) of pBluescript-SAG 12 vector). The reverse Full-length senescence-induced eIF-5A Primer was 5'-ACCTCGAGCGGCCGCAGAAGAAGTATAAAAACCATC -3' (note: underlined portion is the *Not*I recognition sequence, used for ligation into the Multiple Cloning Site (MCS) of pBluescript-SAG 12 vector).

Finally, the desired construct was created in the binary vector, pKYLX71, by digesting pKYLX71 was digested with *SacI* and *XhoI,* and also cutting out the SAG 12:full-length senescence-induced eIF-5A cassette from pBluescript with *SalI* and *SacI.*

The *Xho*I and *Sal*I sticky ends are partially complementary. Hence, these two sets of digested overhangs (specifically, *Sac*I with *Sac*I*,* and *XhoI* with *Sal*I*)* were able to be ligated together with T4 DNA ligase, creating the final construct (SAG 12:antisense-senescence-induced eIF-5A in pKYLX71).

### Transformation and T1 seed harvest

The pKYLX71-SAG 12:antisense-eIF-5A construct was proliferated in *E.coli* DHα cells, isolated and electroporated into a competent *Agrobacterium* strain. The bacteria were then used to infiltrate 4.5 week old wildtype *Arabidopsis* plants and the resulting infiltrated plants were designated as "T₀" plants, which were then grown to the end of their life-cycle. Seeds were harvested, collected and designated as T₁ seeds. 10 plates of T₁ seeds were plated and screened for kanamycin resistance (1/2 MS salt and 50µg kanamycin/mL) with wildtype as a control; only those seeds containing pKYLX71-SAG 12-antisense-eIF-5A construct survive and grow on kanamycin (K50) media. 24 T₁ seedlings were chosen from these plates and placed in soil. The seeds harvested from T₁ transgenic plants were labeled as T₂ seeds. Each seedling yielded one plant line (#1 = 1 line containing 1 plant, #2 = 1 line containing 1 plant, etc.).

### Screening and Identification of Phenotypes

Once kanamycin resistant T₁ seeds were identified, successive generations of T₂, T₃ and T₄ plants were grown. By screening seeds on K50 media, it was possible to distinguish between those plants which inherited the genetic construct and were homozygous for the construct. A phenotypic expression of stunted growth was observed in one T₃ plant line when grown in a pot. However, when the same set of seeds was regrown in identical conditions, the phenotype was not observed.

From the 24 T₁ plants, 4 lines were chosen on the basis of high seed yield (lines T2.14, T2.18, T2.19 and T2.23) and plated on K50 media with wildtype seeds as a control. Approximately 75% of the seeds from each line survived on K50 media and fell into size categories of Small, Medium and Large. From each line, small, medium and large seedlings were removed from plates and planted in soil. Under greenhouse conditions, the Small seedlings did not recover as quickly as their Medium and Large counterparts. At week 6, the Small plants were just beginning to show signs of bolting while the other plants had bolted and flowered. In total, six transgenic T₂ plants (from a total of 3 lines x 8 plants = 96 transgenic plants) demonstrated dramatic delay in bolting and were deemed "Late Bolt" plants. The seed yields of these plants were also dramatically lower than other transgenics.

From the 96 T₂ plants, 3 lines were selected to produce T₃ plants (T3.19.S8 and T3.14.L7 which were Late Bolts; and, T3.23.S3 which was not a Late Bolt). When planted on K50 media plates, these lines showed homozygous survival. 13 seedlings were transplanted into pots (10 seedlings per pot). From this set of plants, a dramatic dwarf phenotype was observed in T3.14.L7 plant line. T₄ seeds were collected, and lower seed yield was observed in that line. A dense growth (dense silique growth, more branches) phenotype was observed in line T3.19.S8, while a phenotype similar to wildtype was observed in line T3.23.S3. Seed sizes from the 3 transgenic lines were compared but no statistically significant differences were determined. Chlorophyll levels were also analyzed but no statistically significant differences from wildtype control were determined.

T₄ seeds of lines T3.19.S8, T3.14.L7 and T3.23.S3 were screened on K50 to obtain the next generation of plants and showed evidence of inherited gene construct (uniform green growth on plates) compared with wild-type seed that died. However, when planted in individual flats, the dwarf phenotype was not expressed suggesting that the eIF-F5A antisense transgene had been lost. Finally, seeds collected from all T₅ plants were screened on K50 plates and showed evidence of kanamycin resistance. Work is now underway to confirm that the antisense transgene has been lost, and these T4 plants are azygous.

Eight daughter lines were chosen from mother lines T2.14, T2.19 and T2.23 and screened on K50 media with wild-type seeds as a control. Three lines were chosen based on low seed yield: T3.14.L8, T3.14.58, and T3.23.S1. The other five lines chosen are: T3.18.S7, T3.18.S2, T3.19.S1, T3.19.S5, and T3.23.S6. All the lines screened on K50 media showed homozygous survival, while T3.14.L8, T3.14.S8 and T3.23.S6 showed heterozygous survival. Seedlings from lines T3.14.L8 and T3.14.S8 that survived were white in color with green vascular tissue, while seedlings from T3.23.S6 that survived were entirely dark green in color. These seedlings were selected for transplantation. In total, 28 seedlings from each line were transplanted into cells and grown in greenhouse conditions.

At week 3, all lines started bolting except for lines T3.14.L8 and T3.23.S1 and several plants within lines T3.18.S7, T3.18.S2, T3.19.S1, T3.19.S5, T3.23.S1 and T3.23.56. An irregular rosette leaf morphology (elongation of 2^{nd} pair leaves phenotype) was observed in T3.14.L8 and T3.14.S8 lines. At week 5, additional irregular leaf morphologies of increased number of rosette leaves and crinkle-edged rosette leaves phenotypes were also observed in lines T3.18.S7 and T3.23.S6. Rosettes smaller than wild-type were observed in lines T3.23.S1, T3.19.S1, and T3.19.S5. At week 7, spindly stem and no stem elongation phenotypes were observed in lines T3.18.S7, T3.18.S2, T3.19.S1, T3.19.S5, T3.23.S1 and T3.23.S6. The first and second cauline leaf of each plant was collected at week 5 and 6, respectively, for investigation of senescence eIF-5A protein expression.

### Example 24

### Determination of Oxygen output

The leaves were harvested and the areas were measured before they were weighed. The leaves were ground to a fine powder using 1mL of cold degassed grinding buffer with a mortar and pestle. Then the homogenate was transferred into an eppendorf tube and placed immediately on ice. For tomato leaves, the homogenate isolated required to be filtered through a piece of Miracloth.

50 µl of homogenate from all samples were added into 10ml test tubes containing 5ml grinding buffer and 25µl DCPIP (2,6-dichlorophenol indophenol). The samples were shaken well and then one set of samples were placed for 15 mins under illumination by a pair of lamps and the second set of samples were placed in the dark for 15 mins. After the 15minute incubation, 50 µL of DCMU(3-(3,4-dichlorophenyl)-1,1 dimethylurea) was added to both set of samples in order to stop the reaction and then centrifuged in a microcentrifuge for 2mins at 14,000g. The absorbencies of the supernatant collected were read at 590nm using grinding buffer as a blank.

The molar extinction coefficient for this assay is 16 x 10³, that is, a change in concentration of 1 mole per liter changes the absorbance of the solution by 16 x 10³ µmole of DCPIP reduced/h/ml = (difference in absorbance) x [1/ 16 x 10³ (moles/l)] x [reaction volume(ml)/10³ (ml/l)] x [10⁶ (µmole/mole)] x [60 (min/hr)/ reaction time (min)] x [1/sarnple volume(ml)].

For every 2 moles of DCPIP that are reduced, 1 mole of O₂ is generated. Reference: Allen J. F. and Holmes N.G., 1986 Electron Transport and Redox Titration s in Photosynthesis: Energy Transduction. Edited by M.F. Hipkins & N.R. Baker., IRL Press, Oxford Pp 107-108.

### Example 25

### Quantitative determination of Starch

Starch content in tomato stems was determined using a method adapted from Lustinec et al. Quantitative determination of starch, amylose, and amylopectin in plant tissues using glass fiber paper. Anal. Biochem. 132:265-271 (1983). Tomato stem tissue was homogenized in three volumes of water using an Omnimixer (12 reps of 5 sec each), followed by a Polytron homogenizer (30 sec). Homogenate was stored in 10 ml aliquots at -20°C prior to analysis. For analysis, 10 ml homogenate was thawed and mixed with an equal volume of concentrated perchloric acid (HClO₄, 70% w/w) and incubated for 20 min at room temperature to dissolve the starch. Simultaneously, several solutions of potato starch (in the range of 0.1 - 1.0 mg/ml) were processed alongside the tomato stem sample to generate a standard curve. The homogenate (or potato starch standard solution) was stirred and filtered through Whatman GF/A glass microfiber paper (9.0 cm diameter) using a vacuum flask attached to an aspirator. One ml of filtrate was mixed with 3 ml of iodine solution A (8 mM I₂, 17 mM KI, 514 mM NaCl) and incubated for 30 min at 4°C to form a starch-iodine precipitate. The precipitate was collected on Whatman GF/A glass microfiber paper (9.0 cm diameter) using a vacuum flask attached to an aspirator, and then wash the filtrate with the following solutions: once with 10 mL iodine solution B (83 mM I₂, 180 mM KI, 8% perchloric [HClO₄] acid); once with 5 mL ethanol-NaCl solution (67% ethanol, 342 mM NaCl); twice with 3 ml ethanol-NaOH solution (67% ethanol, 250 mM NaOH). Once ethanol had evaporated, the microfiber paper was removed from aspirator and inserted into screw-capped glass tube. Sulfuric [H₂SO₄] acid (9 mL of 0.75 M solution) was added to the tube and the tube was incubated in a boiling water bath for 30 min. Three 1 mL-aliquots of eluate were pipetted into glass test tubes and mixed with 1 mL of 5% phenol, quickly followed by 5 mL of concentrated H₂SO₄. The tubes were vortexed and incubated at room temperature for 30 min to allow the color to develop. Simultaneously, a blank for the spectrophotometer measurements was prepared by mixing 1 mL of 0.75 M H₂SO₄ with 1 mL of 5% phenol, and quickly adding 5 mL concentrated H₂SO₄; the blank was also incubated at room temperature for 30 min. A spectrophotometer was calibrated at 480 nm using the blank, and the O.D. of all samples and potato starch standards were measured and recorded. A standard curve was prepared using the potato starch solutions, and used to interpolate the quantity of starch in each sample.

### Example 26

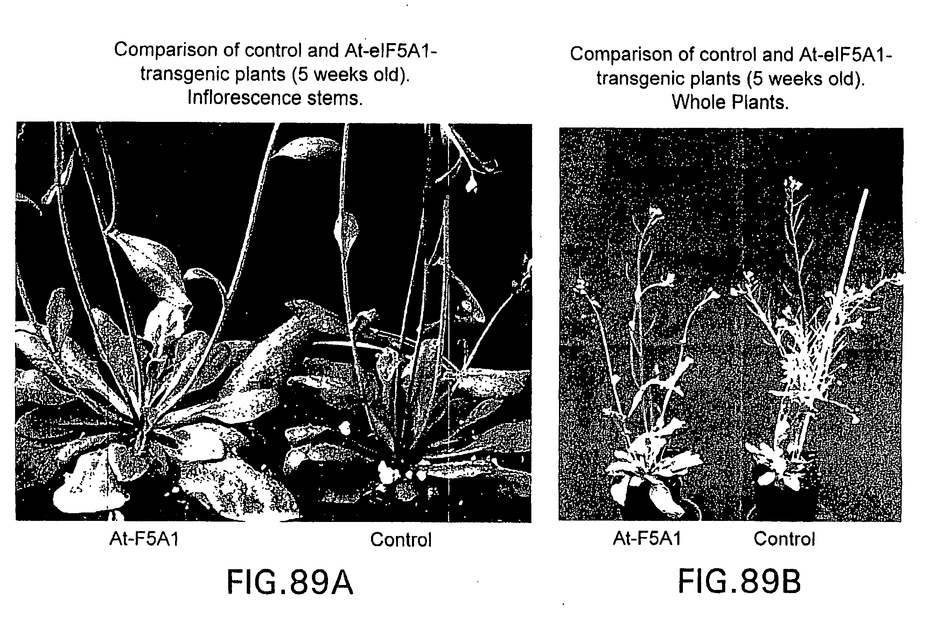

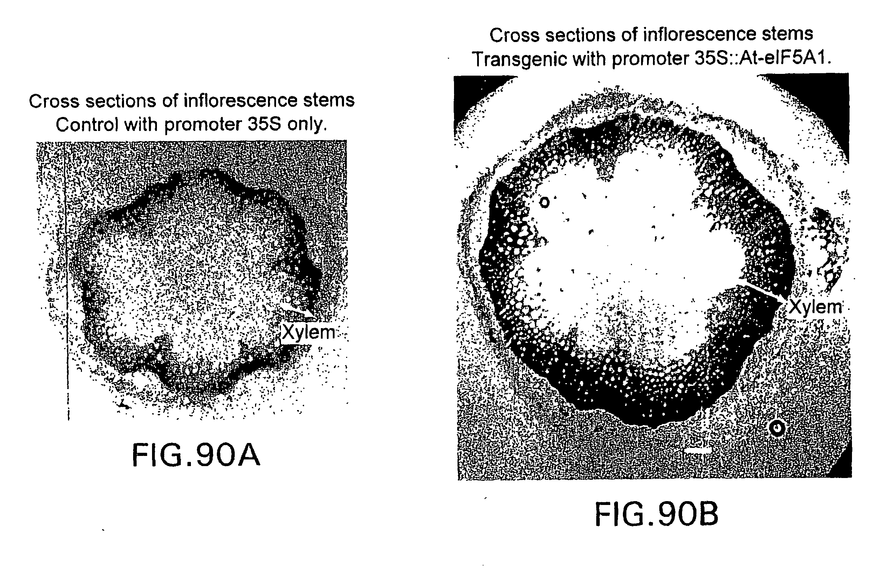

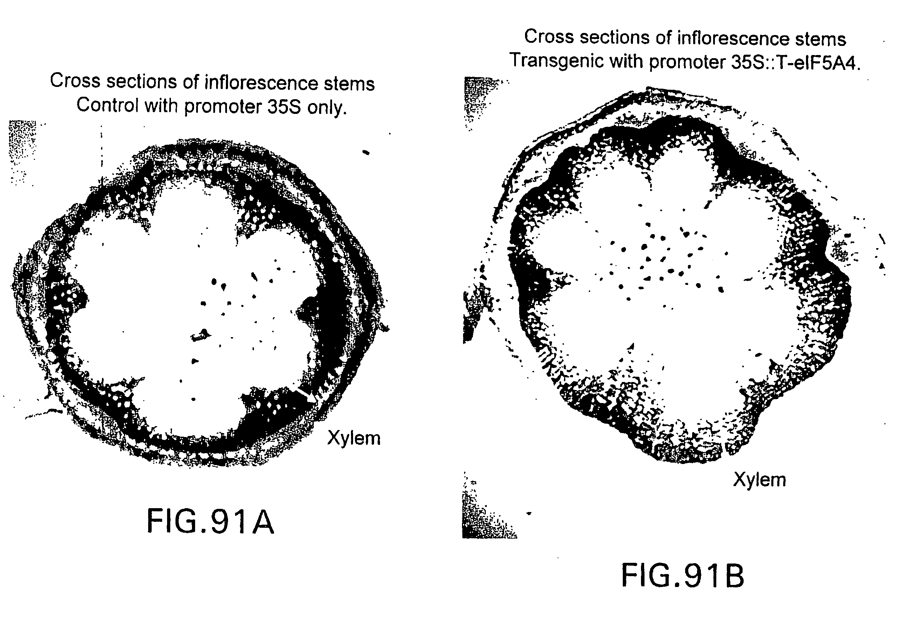

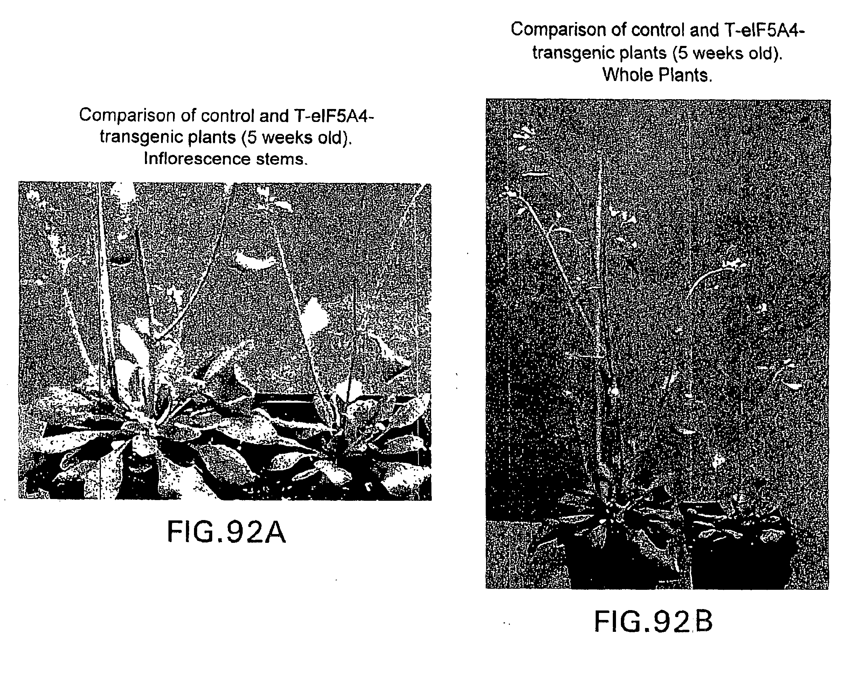

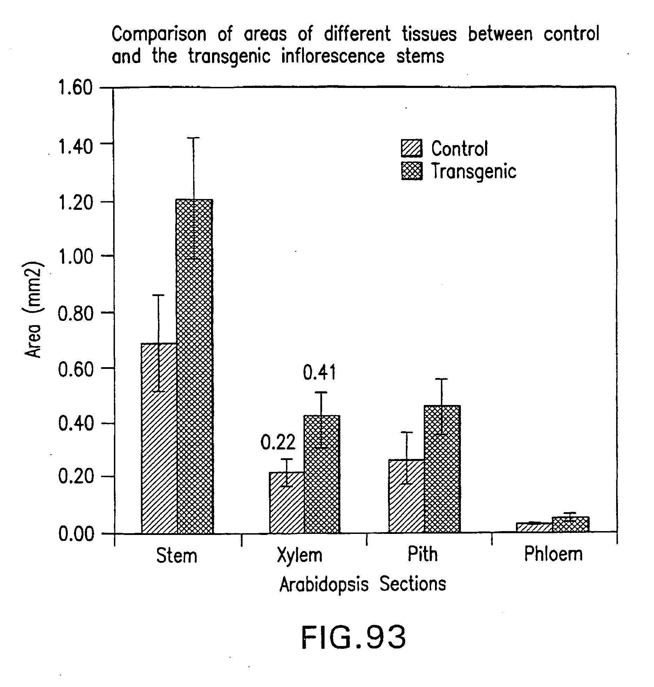

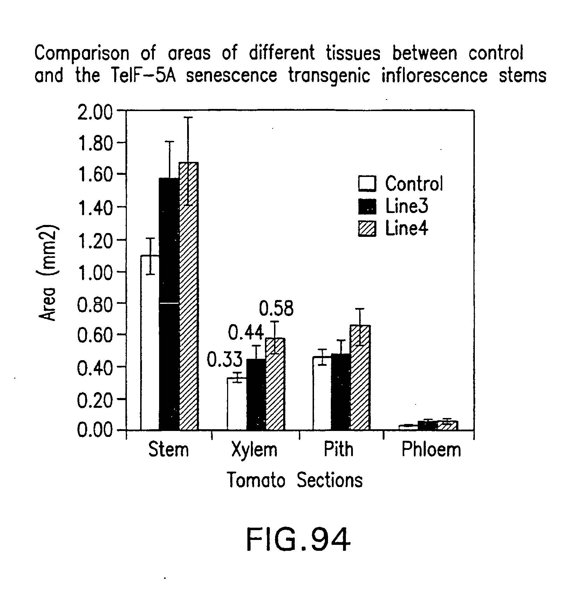

*Arabidopsis thaliana* (Columbia ecotype) was transformed by the *Arabidopsis thaliana* sense Senescence-induced eIF-5A (At-eIF) and Tomato sense senescence-induced eIF-5A genes independently. These genes were constitutively expressed in the whole life cycle of the transgenic plants. The inflorescence stems of these plants exhibited a significant increase of xylem development. See figures 89-94.

The seeds of transgenic and control plants were sown on ½ MS medium agar plates, and kept in a growth chamber at 22 °C, 80% rh, and 16 h light/day, for 9 days. Then, the seedlings were transferred to 32-well-flats with a commercial soil, and were maintained under the same conditions as above, for 48 days. The main inflorescence stems were selected for microscopic observation. Cross sections were hand-cut from the base of the stems within 2 mm above the rosette. The sections were stained with the phloroglucinol-HCl method. We found that the stem xylem at this age has achieved its maximum development. A comparison was made between transgenic and control plants in the sizes (sectional areas) of xylem. In addition, measurements were done for phloem and pith in both transgenic and control plants.

Measurement of tissue areas was as follows. Cross sections were photographed with a Zeiss microscope, and the micrographs were digitalized using Photoshop^{®}. These images were printed out on paper and different tissues were cut out, and their areas were measured by an area-measuring meter. To calculate the actual area of each tissue, the following formula was used: The actual area = (The area of an individual tissue on paper) / (Magnification)²

It thus appears that senescence-induced eIF-5A is also involved in programmed cell death associated with xylogenesis. Constitutive antisense suppression of senescence-induced AteIF-5A in *Arabidopsis* reduced the thickness of the inflorescence stem as well as the number of xylem cell layers. By contrast, the inflorescence stems of plants in which Arabidiposis or tomato senescence-induced eIF-5A was constitutively over-expressed were, on average, 1.7-fold thicker than those of corresponding wild-type plants, and the total xylem area per cross-section of inflorescence stem was 2 fold higher. The over-expressing transgenic plants also had greatly increased rosette leaf biomass and grew faster than wild-type plants, which may reflect enhanced nutrient uptake. The same phenotype was observed when the senescence-induced isoform of eIF-5A from tomato was over-expressed in *Arabidopsis* plants. These results collectively indicate that the senescence-induced isoform of eIF-5A not only regulates leaf and flower senescence, but is also involved in xylogenesis.

### Example 27

### Suppression of deoxyhypusine synthase delays browning of pre-packaged cut lettuce in ambient atmosphere

The lettuce was grown hydroponically, as this was an ideal for obtaining a controlled and continuous induction of the transgene (antisense DHS) by the glucocorticoid-inducible promoter. The inducer, dexamethasone, was applied directly to the hydroponic solution.

Figure 149(a) shows 6 week old plants immediately prior to harvest. Figure 149 (b) shows a view of air stone inside the pot. Figure 149(c) shows the lid with three openings to support plants, and an airline in the center. Figure 150 shows browning of cut lettuce in ambient atmosphere.

Figure 150(a) shows the onset of browning plotted as a factor of time for three transgenic lines: Line 13(n=4), Line 21 (n=5) and Line 34 (n=7), expressing the 3' UTR of lettuce DHS in the antisense orientation, as well as for wildtype romaine lettuce (Green Towers variety; n=12). The transgene was expressed under the control of a glucocorticoid-inducible promoter. The lettuce heads were grown hydroponically, and were harvested and chopped into small segments. The chopped lettuce was maintained in perforated zipped bags at 7.5 °C for 6 days. Browning of cut lettuce was evaluated on a scale of 0 to 4, where a rating of 1 was defined as the onset of browning. Standard error bars are shown. Figure 150(b) shows the results of two individual plants of Line 21 that showed 0% browning through the 6-day study. Figure 150(c) shows the result of one individual plant of line 34 that also did not turn brown over the same period, where as the wild type lettuce turned brown.

Figure 151 shows a Western blot of transgenic lettuce showing decreased expression of DHS protein. Total protein from four individual lettuce plants expressing the antisense 3'UTR of lettuce DHS, as well as one plant that had lost the transgene as the gene segregated in the T2 generation ("null"), and one wild type plant, was isolated and 20 µg of each sample was loaded on a 14% acrylamide gel stained with Coomassie blue. The gel was blotted onto a PVDF membrane and probed with a polyclonal antiboby raised against tomato DHS. The blot was developed colormetrically, an shows that the expression of DHS in the transgenic plants was reduced relative to the null and wild type plants.

### Example 28

### Suppression of deoxyhypusine synthase expression in Canola increases seed yield

Deoxyhypusine synthase (DHS) mediates the first of two enzymatic reactions that convert inactive eukaryotic translation initiation factor-5A (eIF-5A) to an activated form able to facilitate translation. A full-length cDNA clone encoding canola (Brassica napus cv Westar) DHS was isolated from a cDNA expression library prepared from senescing leaves. DHS was suppressed in transgenic canola plants by expressing the antisense 3'-UTR of canola DHS cDNA under the regulation of the constitutive cauliflower mosaic virus (CaMV-35S) promoter. See Figure 158 showing decreased expression of DHS protein. The transgenic canola plants were obtained by vacuum infiltration of canola inflorescences by modifying the protocol developed for *Arabidopsis.* The efficiency of transformation was enhanced by removal of new flowers that formed after the plants had been vacuum-infiltrated, and transformation rates of 50 to 60% were routinely obtained. Transgenic plants had reduced levels of leaf DHS protein and exhibited delayed natural leaf senescence. Suppression of DHS also increased leaf size by 1.5- to 2-fold (see Figure 157) and resulted in increases in seed yield of up to 65% (see Figure 160. This was attributable in part to an increase in the size of the siliques, which were on average 18% to 26% longer than wild-type siliques depending on the line (see Figure 159). When wild-type and transgenic plants were grown in 6-inch pots, the increase in seed yield accruing from suppression of DHS was ~4.5-fold greater than when the plants were grown in 12 inch pots. Thus suppression of DHS appears to ameliorate the effects of sub-lethal stress engendered by growth in small containers. The increase in seed yield for transgenic plants translates into a corresponding increase in seed oil content based on measurements of triacylglycerol, and there was no change in the fatty acid composition of the oil in transgenic seeds. See Figure 161 (a) and (b).

### Example 29

### Extending the vase life of carnation flowers by administering inhibitors of deoxyhypusine synthase and by antisense suppression of deoxyhypusine synthase

A full-length cDNA clone (AF296079) encoding deoxyhypusine synthase (DHS) was isolated from carnation petals. DHS mediates the first of two enzymatic reactions that convert inactive eukaryotic translation initiation factor-5A (eIF-5A) to an activated form able to facilitate translation. Northern analysis revealed that DHS expression is correlated with senescence of carnation flower petals. Treatment of cut carnation flowers with inhibitors of the DHS reaction, including diaminobutane (putrescine), diaminopropane, diaminohexane, diaminooctane and spermidine, extended the vase life of the flowers by up to 83%. In order to evaluate the role of DHS in carnation flower senescence more definitively, expression of the protein was suppressed in transgenic plants by introducing the antisense 3'-UTR of carnation DHS cDNA under regulation of the constitutive cauliflower mosaic virus promoter through Agrobacterium transformation. Three lines of transgenic flowers with reduced DHS expression were analyzed and found to have longer vase-life relative to wild-type flowers. Indeed, one of the lines exhibited an increase in vase life of >100%. These findings indicate that DHS plays a central role in flower senescence.

### Example 30

### The delayed bolting phenotype induced by suppression of deoxyhypusine synthase in Arabidopsis can be rescued by treatment with GA3

Deoxyhypusine synthase (DHS) is a ubiquitous enzyme required for post-translational activation of eukaryotic translation initiation factor 5A (eIF-5A) and appears to be essential for normal plant growth and development. DHS was suppressed in Arabidopsis by expressing full-length antisense Arabidopsis DHS cDNA in transgenic plants under the regulation of the senescence-specific SAG12 promoter. Plants expressing the transgene had reduced levels of leaf DHS protein, and exhibited delayed bolting and a pronounced delay (2 to 5 weeks) in the onset of leaf senescence. The bolts were also shorter, although this did not result in a reduction in biomass or seed yield. Treatment of the transgenic plants with GA3 reversed the delayed bolting phenotype. A similar phenotype was obtained by antisense suppression of DHS under the regulation of GCI, a glucacorticoid-inducible promoter that can be activated by administering dexamethasone (DEX). Again, administering GA3 rescued this phenotype; that is, the GA3-treated transgenic plants bolted normally, the bolts were of normal size and there was no delay in the onset of leaf senescence. These results collectively indicate that DHS, through activation of one or more of the three isoforms of eIF-5A in Arabidopsis, influences GA metabolism.

### Example 31: Transformation of Arabidopsis plants with sense growth eIF-5A from cottonwood to increase biomass

4 week-old-Arabidopsis plants were infiltrated with Agrobacterium tumefaciens strain GV3101 containing pKYLX71-sense-poplar Growth-eIF-5A3 (see figure 171).

Seeds of the first generation (T1) of transgenic as well as control plants (transformed with the empty pKYLX71 vector only, conferring kanamycin resistance) were sown on ½ MS medium agar plates supplemented with kanamycin. The plates were kept in a growth chamber at 22°C, 80% rh, and 16 h light/day, for 9 days. Then, the seedlings were transferred to 32-well-flats with a commercial soil mix, and were maintained under the same conditions as above, until T2 seeds were harvested. Different lines of T2 seeds as well as control plants were screened on ½ MS medium agar plates again. Seedlings were transferred into soil and kept under the same condition as for T1 plants.

Transgenic Line 3, which had the best phenotype, was chosen for this experiment. eIF5A protein was confirmed to be over-expressed by Western analysis. Transgenic Line 3 plants displayed a 1.5-fold increase in inflorescence stem height relative to vector-only control plants at the 6th week, and produced 4-fold more seed yield than the control plants did.

Seeds from transgenic line 3 were sown on plates with ½ MS medium (supplemented with kanamycin) as for screening transgenic seedlings harboring over-expressed poplar eIF5A3.

1-week old seedlings were transferred to soil, Sunshine LC1/LG3, 1:1 (basic soil, no added fertilizer). See Wang et al. (2003) Plant Mol Biol 53: 1223-1235 for a discussion on the soil analysis for this brand of soil, as compared with a richer soil brand that is routinely used (Promix BX)

When watering is needed (that is about 3 days later), a series of fertilizer (20:20:20) at 0 g/L, 0.5 g/L, 1 g/L, 3 g/L and 5 g/L was applied (to separate flats of plants) until the soil was saturated. The recommended amount of fertilizer for optimal growth is 1 g/L. Fertilizer was reapplied once per week.

Morphology and phenotype were observed for leaf size and color, bolting time and inflorescence stem height, silique size and number, etc. The resulting transgenic plants have increased biomass on optimal, suboptimal and supraoptimal concentrations of fertilizer. See figures 172 and 173.

### Example 32: Transformation of Arabidopsis plants with mutant senescence eIF-5A in sense orientation to increase biomass

Arabidpsosis plants were transformed with a mutant senescence eIF-5A as described above (not able to be hypusinated). The resulting transgenic plants have increased biomass on optimal, suboptimal and supraoptimal concentrations of fertilizer. See figures 174 and 175.

### REFERENCES

Azumi, Y and Watanabe, A (1991) Evidence for a senescence-associated gene induced by darkness. Plant Physiol 95: 577-583.
Becker W, Apel K (1993) Differences in gene expression between natural and artificially induced leaf senescence. Planta 189: 74-79
Bevec , D., Jaksche, H., Oft, M., Wohl, T., Himmelspach, M., Pacher, A., Schebesta, M., Koettnitz, K., Dobrovnik, M., Csonga, R., Lottspeich, F., and Hauber, J (1996) Inhibition of HIV-1 replication in lymphocytes by mutants of the Rev cofactor of eIF-5A. Science 271:1858-1860.
Bevec, D, and Hauber, J (1997) Eukaryotic initiation factor 5A activity and HIV-1 Rev function. Biol Signals 6:124-133.
Bleecker, A.B., and Patterson, SE (1997) Last exit: Senescence, abscission, and meristem arrest in Arabidopsis. Plant Cell. 9:1169-1179.
Buchanan-Wollaston, V (1997) The molecular biology of leaf senescence. J Exp Bot 48:181-191.
Buchanan-Wollaston V (1994) Isolation of cDNA clones for genes that are expressed during leaf senescence in Brassica napus. Plant Physiol 105: 839-846
Chen, KY, and Liu, AYC. (1997) Biochemistry and function of hypusine formation on eukaryotic initiation factor 5A. Biol Signals 6:105-109.
Davies KM, and Grierson D (1989) Identification of cDNA clones for tomato (Lycopersicon esculentum Mill.) mRNAs that accumulate during fruit ripening and leaf senescence in response to ethylene. Plant Cell 179: 73-80.
Drake R, John I, Farrell A, Cooper W, Schuch W, and Grierson D (1996) Isolation and analysis of cDNAs encoding tomato cysteine proteases expressed during leaf senescence. Plant Mol Biol 30: 755-767
Gan, S and Amasino, RM (1997). Molecular genetic regulation and manipulation of leaf senescence. Plant Physiol 113: 313-319.
Gan, S and Amasino, RM (1995) Inhibition of leaf senescence by autoregulated production of cytokinin. Science 270: 1986-1988.
Hanauske-Abel, H.M., Park, M.H., Hanauske, A.-R., Popowicz, A.M., Lalande, M., and Folk, J.E. Inhibition of G1-S transition by inhibitors of deoxyhypusine hydroxylation. Biochem Biophys Acta 1221: 115-124, 1994.
Henderson, BR, and Percipalle, P. (1997) Interactions between HIV Rev and nuclear import and export factors: the Rev nuclear localization signal mediate specific binding to human importin-beta. J Mol Biol 274: 693-707.
Hensel, L.L., V. Grbic, D.B. Baumgarten, and A.B. Bleecker. (1993). Developmental and age-related processes that influence the longevity and senescence of photosynthetic tissues in Arabidopsis. Plant Cell 5:553-564.
Jakus, J., Wolff, E.C., Park, M.H., and Folk, J.E. Features of the spermidine-binding site of deoxyhypusine synthase as derived from inhibition studies: effective inhibition by bis-and mono-guanylated diamines and polyamines. J Biol Chem 268:13151-13159, 1993.
Kang, HA, and Hershey, JWB (1994) Effect of initiation factor eIF-5A depletion on protein synthesis and proliferation of Saccharomyces cerevisiae. J Biol Chem 269: 3934-3940.
Katahira, J, Ishizaki, T, Sakai, H, Adachi, A, Yamamoto, K, and Shida, H. (1995) Effects of translation initiation factor eIF-5A on the functioning of human T-cell leukemia virus type I Rex and human immunodeficiency virus Rev inhibited trans dominantly by a Rex mutant deficient in RNA binding. J Virol 69: 3125-3133
Kemper, WM, Berry, KW, and Merrick, WC (1976) Purification and properties of rabbit reticulocyte protein synthesis initiation factors M2Bα and M2Bβ. J Biol Chem 251: 5551-5557.
Lohman, KN, Gan, S, John, MC and Amasino, R (1994) Molecular analysis of natural leaf senescence in Arabidopsis thaliana. Phys Plant 92: 322:328.
Lipowsky, G., Bischoff, F.R., Schwarzmaier, P, Kraft, R., Kostka, S., Hartmann, E., Kutay, U., and Gorlich, D. (2000) Exportin 4: a mediator of a novel nuclear export pathway in higher eukaryotes. EMBOJ. 19:4362-4371.
Liu, YP, Nemeroff, M, Yan, YP, and Chen, KY. (1997) Interaction of eukaryotic initiation facto 5A with the human immunodeficiency virus type 1 Rev response element RNA and U6 snRNA requires deoxyhypusine or hypusine modification. Biol Signals 6:166-174.
Martinez-Zapater, JM and Salinas, J (1994) Arabidopsis Protocols. Humana Press: p. 197. Mattaj, I.W., and Englmeier, L. (1998) Nucleocytoplasmic transport: The soluble phase. Annu Rev Biochem 67: 265-306
Mehta, AM, Saftner, RA, Mehta, RA, and Davies, PJ (1994) Identification of posttranslationally modified 18-kilodalton protein from rice as eukaryotic translation initiation factor 5A. Plant Physiol 106:1413-1419.
Noh, Y-S and Amasino, R (1999) Identification of a promoter region responsible for the senescence-specific expression of SAG12. Plant Mol Biol 41:181-194.
Noodén, LD, Guaimét, JJ and John, I (1997) Senescence mechanisms. Physiol Plant 101: 746-753.
Noodén, LD and Leopold, AC (eds) (1988) The phenomena of senescence and aging. Senescence and Aging in Plants. Academic Press: pp.1-50.
Ober, D and Hartmann, T (1999) Deoxyhypusine synthase from tobacco. J Biol Chem 274: 32040-32047.
Oh SA, Lee SY, Chung IK, Lee CH, and Nam HG (1996) A senescence-associated gene of Arabidopsis thaliana is distinctively regulated during natural and artificially induced leaf senescence. Plant Mol Biol 30: 739-754
Page, DR and Grossniklaus, U (2002) The art and design of genetic screens: Arabidopsis Thaliana. Nature Reviews Genetics 3:124-136
Park, MH, Joe, YA, and Kang KR (1998) Deoxyhypusine synthase activity is essential for cell viability in the yeast Saccharomyces cerevisiae. J Biol Chem 16:1677-1683.
Park, MH, Wolff, EC, Lee, YB and Folk, JE (1994) Antiproliferative effects of inhibitors of deoxyhypusine synthase: inhibition of growth of Chinese hamster ovary cells by guanyl diamines. J Biol Chem 269:27827-27832.
Park, M.H., Wolff E.C., and Folk J.E. (1993) Hypusine: its post-translational formation in eukaryotic initiation factor 5A and its potential role in cellular regulation. BioFactors 4:95-104.
Park, MH, Wolff, EC and Folk, JE (1993) Is hypusine essential for eukaryotic cell proliferation? Trends Biochem Sci 18:475-479.
Park, MH, Wolff, EC, Smit-McBride, Z, Hershey, JWB and Folk, JE (1991) Comparison of the activities of variant forms of eIF-4D: the requirement for hypusine or deoxyhypusine. J Biol Chem 266:7988-7994, 1991.
Park, MH and Wolff, EC (1988) Cell-free synthesis of deoxyhypusine. J Biol Chem 263:15264-15269.
Quirino, BF, Noh, Y-S, Himelblau, E and Amasino, R (2000) Molecular aspects of leaf senescence. Trends Plant Sci 5:278-282.
Rosorius, O., Reichart, B., Kratzer, F., Heger, P., Dabauvalle, M.C. & Hauber, J. (1999) Nuclear pore localization & nucleocytoplasmic transport of eIF-5A: evidence for direct interaction with the export receptor CRM1. J.Cell Sci. 112, 2369-2380.
Ruhl, M., Himmelspach, M., Bahr, G.M., Hammerschmid, F., Jaschke, H., Wolff, B., Aschauer, H., Farrington, G.K., Probst, H., Bevec, D., and Hauber, J. (1993) Eukaryotic initiation factor 5A is a cellular target of the human immunodeficiency virus type 1 Rev activation domain mediating transactivation. J Cell Biol 123:1309-1320.
Schardl, CL, Byrd, AD, Bension, G, Altschuler, MS, Hildebrand, DF and Hunt, AG (1987) Design and construction of a versatile system for the expression of foreign genes in plants. Genes 61: 1-11.
Schnier J, Schwelberger HG, Smit-McBride Z, Kang HA and Hershey JWB (1991) Translation initiation factor 5A and its hypusine modification are essential for cell viability in the yeast Saccharomyces cerevisiae. Mol Cell Bioll 1:3105-3114
Smart CM, Hosken SE, Thomas H, Greaves JA, Blair BG, Schuch W (1995) The timing of maize leaf senescence and characterization of senescence-related cDNAs. Physiol Plant 93: 673-682
Smart, CM (1994) Gene expression during leaf senescence. New Phytologist 126:419-448.
Taylor CB, Bariola PA, Delcardayre SB, Raines RT, Green RT (1993) RNS2: A senescence-associated RNase of Arabidopsis that diverged from the S-RNases before speciation. Proc Natl Acad Sci USA 90: 5118-5122
Tome, M., Fiser, S.M., Payne, C.M., and Gerner, EW. (1997) Excess putrescine accumulation inhibits the formation of modified eukaryotic initiation factor 5A (eIF-5A) and induces apoptosis. Biochem. J. 328: 847-854.
Tome, M., and Gerner, EW. (1997) Cellular eukaryotic initation factor 5A content as a mediator of polyamine effects on growth and apoptosis. Biol Signals 6:150-156.
Walbot V (2000) A green chapter in the book of life. Nature 408: 794-795.
Wang, T-W, Lu, L, Wang, D, and Thompson, JE (2001) Isolation and characterization of senescence-induced cDNAs encoding deoxyhypusine synthase and eucaryotic translation initiation factor 5A from tomato. J Biol Chem 276:17541-17549.
Watanabe, A and Imaseki, H (1982) Changes in translatable mRNA in senescing wheat leaves. Plant Cell Physiol 23:489-497.
Weaver, L.M., Gan, S, Quirino, B and Amasino, R (1998) A comparison of the expression patterns of several senescence-associated genes in response to stress and hormone treatment. Plant Mol Biol 37:455-469.
Xu, A. and Chen, K.Y. (2001) Hypusine is required for a sequence-specific interaction of eukaryotic initiation factor 5A with post-SELEX RNA. J. Biol Chem. 276:2555-2561.
Zuk, D., and Jacobson, A. (1998) A single amino acid substitution in yeast eIF-5A results in mRNA stabilization. EMBO J. 17:2914 -2925.
Boyes, D.C., A.M. Zayed, R. Ascenzi, A.J. McCaskill, N.E. Hoffman, K.R. Davis, and J. Goerlach. 2001. Growth stage-based phenotypic analysis of Arabidopsis: A model for high throughput functional genomics in plants. Plant Cell, 13: 1499-1510.
Collawn, J.F., and Y. Patterson. 1999. Production of antipeptide antibodies. In FM Ausubel, R Brent, RE Kingston, DD Moore, JA Smith, JG Seidman, K Struhl, eds, Current Protocols in Molecular Biology on CD. John Wiley & Sons, New York.
Chamot, D. and C. Kuhlemeier. 1992. Differential expression of genes encoding the hypusine-containing translation initiation factor, eIF-5A, in tobacco. Nucleic Acids Res 20: 665-669.
Clemens, M. J., and U.-A. Bommer. 1999. Translational control: The cancer connection. Int. J. of Biochem. Cell Biol. 31: 1-23.
Davis, L.G., M.D. Dibner, and J. B. Battey. 1986. Basic methods in molecular biology. Elsevier Science Publishing Co., Inc, New York, pp. 130-5.
Drenckhahn, D., T. Jons, and F. Schmitz. 1993. Production of polyclonal antibodies against proteins and peptides. In DJ Asai, ed, Methods in Cell Biology, Vol 37. Academic Press, New York, pp 7-56.
Dresselhaus, T., C. Simone, and H. Lörz. 1999. A transcript encoding translation factor eIF-5A is stored in unfertilized egg cells of maize. Plant Mol. Biol. 39: 1063-1071. Fagard, M. and H. Vaucheret. 2000. (Trans)Gene silencing in plants: How many mechanisms? Annu. Rev. Plant Physiol. Mol. Biol. 51: 167-94.
Fairbanks, G., T.L. Steck, and D.F.H. Wallach. 1971. Coomassie blue R250 used in isopropanol-acetic acid. Biochem 10: 2606-2618.
Ghosh, S., S. Gepstein, J.J. Heikkila, and E.B. Dumbroff. 1988. Use of a scanning densitometer or an ELISA plate reader for measurement of nanogram amounts of protein in crude extracts from biological tissues. Anal. Biochem. 169: 227-233.
Jao, D.L.-E., and K.Y. Chen. 2002. Subcellular localization of the hypusin-containing eukaryotic initiation factor 5A by immunofluorescent staining and green fluorescent protein tagging. J. Cell. Biochem. 86: 590-600.
Jakus, J., E.C. Wolff, M.H. Park, and J.E. Folk. 1993. Features of the spermidine-binding site of deoxyhypusine synthase as derived from inhibition studies: effective inhibition by bis- and mono-guanylated diamines and polyamines. J. Biol. Chem. 268, 13151-13159.
Kushner, S. R. 1978. An improved method for transformation of Escherichia coli with ColE1 derived plasmids. In: Genetic Engineering: Proceedings of the International Symposium on Genetic Engineering (H.W. Boyer and S. Nicosia, eds.), Elsevier/North-Holland Press, New York. Pp. 17-23.
Liu, Y.P., M. Nemeroff, Y.P. Yan, and K.Y. Chen. 1997. Interaction of eukaryotic initiation factor 5A with the human immunodeficiency virus type 1 Rev response element RNA and U6 snRNA requires deoxyhypusine or hypusine modification. Biol. Signals 6(3), 166-74.
Matile, P., S. Hoertensteiner, and H. Thomas. 1999. Chlorophyll degradation. Annu. Rev. Plant Physiol. Plant Mol. Biol. 50: 67-95.
McCabe, M.S, L.C. Garratt, F. Schepers, W.J.R.M. Jordi, G.M. Stoopen, E. Davelaar, J.H.A. van Rhijn, J.B. Power, and M.R. Davey. 2001. Effects of PSAG12-IPT gene expression on development and senescence in transgenic lettuce. Plant Physiol. 127: 505-516.
Miranda, P.V., A. Brandelli, and J. G. Tezon. 1993. Instantaneous blocking for immunoblots. Anal. Biochem. 209: 376-377.
Murashige, T. and F. Skoog. 1962. A revised medium for rapid growth and bioassays with tobacco tissue cultures. Physiol. Plant. 15:473-497.
Nowack, L.N. 2002 personal communications. Ober, D., and T. Hartmann. 1999. Deoxyhypusine synthase from tobacco. J. Biol. Chem. 274(45): 32040-32047.
Page, T., G. Griffiths, and V. Buchanan-Wollaston. 2001. Molecular and biochemical characterization of postharvest senescence in broccoli. Plant Physiol. 125: 718-727.
Park, J.-H., S.A. Oh, Y.H. Kim, H.R. Woo, and H.G. Nam. 1998. Differential expression of senescence-associated mRNAs during leaf senescence induced by different senescence-inducing factors in Arabidopsis. Plant Mol. Biol. 37: 445-454.
Park, M.H., Y.B. Lee, and Y.A . Joe. 1997. Hypusine is essential for eukaryotic cell proliferation. Biol. Signals 6, 115-123.
Park, M.H., E.C. Wolff, and J.E. Folk. 1993. Hypusine: its post-translational formation in eukaryotic initiation factor 5A and its potential role in cellular regulation. BioFactors 4(2), 95-104.
Park, M.H., and E.C. Wolff. 1988. Cell-free synthesis of deoxyhypusine. J. Biol. Chem. 263(30): 15264-15269.
Rosorius, O., B. Reicher, F. Kraetzer, P. Heger, M.-C. Dabauvalle, and J. Hauber. 1999. Nuclear pore localization and nucleocytoplasmic transport of eIF-5A: evidence for direct interaction with the export receptor CRM1. J. Cell Sci. 112: 2369-2380. Stotz 2000.
Tome, M.E., and E.W. Gerner. 1997. Cellular eukaryotic initiation factor 5A content as a mediator of polyamine effects of growth and apoptosis. Biol. Signals 6, 150-156.
Wang, C.Y. and J.E. Baker. 1980. Extending vase life of carnations with minooxyacetic acid, polyamines, EDU, and CCCP. HortSci. 15, 805-806.
Wang, T.-W., L. Lu, C.-G. Zhang, C.A. Taylor, and J.E. Thompson. Unpublished a. Suppression of deoxyhypusine synthase expression in Arabidopsis thaliana delays leaf senescence.
Wang, T.-W., W. Wu, C. Taylor, and J.E. Thompson. Unpublished b. Characterization of eukaryotic translation initiation factor-5A cDNA isoforms from tomato.
Wang, T.-W., C.-G. Zhang, L. Lu, L.N. Nowack, and J. E. Thompson. Unpublished c. Extending vase life of carnation by deoxyhypusine synthase inhibitors and transient transfection with antisense deoxyhypusine synthase.
Wang, T.-W., L. Lu, D. Wang, and J.E. Thompson. 2001. Isolation and characterization of senescence-induced cDNAs ecoding deoxyhypusine synthase and eucaryotic translation initiation factor 5A from tomato. J. Biol. Chem. 276(20): 17541-17549.
Zuk, D., and A. Jacobson. 1998. A single amino acid substitution in yeast eIF-5A results in mRNA stabilization. EMBO J. 17(10): 2914-2925.

## Claims

1. A method of increasing seed yield in a plant comprising incorporating into the genome of at least one cell of a plant a vector comprising a sense polynucleotide of growth eIF-5A and regulatory sequences operatively linked to the sense polynucleotide to provide transcription of said sense polynucleotide, and whereby said transcription of said sense polynucleotide increases expression of growth eIF-5A in a plant, and wherein the increased expression of growth eIF-5A increases seed yield in the plant of the plant as compared to wild type plants.

2. The method of claim 1, wherein the plant is a canola.

3. The method of claim 1, wherein the growth eIF-5A polynucleotide is selected from the group consisting of a polynucleotide encoding a cottonwood growth eIF-5A, a polynucleotide encoding a canola growth eIF-5A, a polynucleotide encoding an alfalfa growth eIF-5A, a polynucleotide encoding a tomato growth eIF-5A and a polynucleotide encoding an Arabidopsis growth elF-5A.

4. The method of claim 1, wherein the plant is canola and the growth elF-5A polynucleotide is a canola growth elF-5A.

5. The method of claim 4, wherein the canola growth elF-5A polynucleotide comprises SEQ ID NO: 66.

6. The method of claim 4, wherein the growth eIF-5A polynucleotide has 70% homology with SEQ ID NO: 66.

7. The method of claim 3, wherein the cottonwood growth eIF-5A comprises the nucleotide sequence shown in Figure 170.

8. The method of claim 3, wherein the alfalfa growth eIF-5A comprises the nucleotide sequence shown in Figure 140.

9. The method of claim 3, wherein the tomato growth eIF-5A comprises SEQ ID NO: 64 or the nucleotide sequence shown in Figure 145.

10. The method of claim 3, wherein the Arabidopsis growth eIF-5A comprises SEQ ID NO: 63 or SEQ ID NO: 52.

11. An alfalfa growth eIF-5A protein comprising the amino acid sequence shown in Figure 140.

12. A polynucleotide encoding the alfalfa growth eIF-5A protein of claim 11.

13. A polynucleotide encoding alfalfa growth elF5A comprising the amino acid sequence shown in Figure 140.

14. A cottonwood growth eIF-5A protein comprising the amino acid sequence of 159 amino acids shown in Figure 170.

15. A polynucleotide encoding the cottonwood growth eIF-5A protein of claim 14.

16. A polynucleotide encoding cottonwood growth eIF5A comprising the nucleotide sequence shown in Figure 170.

17. A polynucleotide encoding tomato growth eIF-5A comprising the nucleotide sequence shown in Figure 145.

18. A method of producing a transgenic plant that exhibits an increased seed yield, the method comprising incorporating into the genome of at least one cell of a plant a vector comprising a sense polynucleotide of growth eIF-5A and regulatory sequences operatively linked to the sense polynucleotide to provide transcription of said sense polynucleotide, and whereby said transcription of said sense polynucleotide increases expression of growth eIF-5A in the plant, and wherein the increased expression of growth eIF-5A increases seed yield in the transgenic plant as compared to wild type plants.

19. A plant produced by the method of claim 18.

20. The plant of claim 19, wherein the plant is canola.

21. Progeny, plant parts or seeds of the plant of claim 19 or claim 20.


SEQUENCE LISTING

<110> THOMPSON, JOHN E.

<120> POLYNUCLEOTIDES OF DHA AND ISOFORMS OF eIF-5A AND METHODS OF
      USING SAME

<130> 10799.207

<140>
<141> 2005-12-05

<150> 60/632,567
<151> 2004-12-03

<150> 60/690,160
<151> 2005-06-14

<150> 60/710,999
<151> 2005-08-25

<150> 60/728,737
<151> 2005-10-21

<160> 165

<170> PatentIn Ver. 3.2

<210> 1
<211> 1584
<212> DNA
<213> Lycopersicon esculentum

<220>
<221> CDS
<222> (54)..(1196)

<400> 1
cgcagaaact cgcggcggca gtcttgttcc gtacataatc ttggtctgca ata atg     56
                                                            Met
                                                              1

gga gaa gct ctg aag tac agt atc atg gac tca gta aga tcg gta gtt    104
Gly Glu Ala Leu Lys Tyr Ser Ile Met Asp Ser Val Arg Ser Val Val
            5                  10                  15

ttc aaa gaa tcc gaa aat cta gaa ggt tct tgc act aaa atc gag ggc    152
Phe Lys Glu Ser Glu Asn Leu Glu Gly Ser Cys Thr Lys Ile Glu Gly
        20                  25                  30

tac gac ttc aat aaa ggc gtt aac tat gct gag ctg atc aag tcc atg    200
Tyr Asp Phe Asn Lys Gly Val Asn Tyr Ala Glu Leu Ile Lys Ser Met
    35                  40                  45

gtt tcc act ggt ttc caa gca tct aat ctt ggt gac gcc att gca att    248
Val Ser Thr Gly Phe Gln Ala Ser Asn Leu Gly Asp Ala Ile Ala Ile
 50                  55                  60                  65

gtt aat caa atg cta gat tgg agg ctt tca cat gag ctg ccc acg gag    296
Val Asn Gln Met Leu Asp Trp Arg Leu Ser His Glu Leu Pro Thr Glu
                70                  75                  80

gat tgc agt gaa gaa gaa aga gat gtt gca tac aga gag tcg gta acc 344
Asp Cys Ser Glu Glu Glu Arg Asp Val Ala Tyr Arg Glu Ser Val Thr
            85                  90                  95

tgc aaa atc ttc ttg ggg ttc act tca aac ctt gtt tct tct ggt gtt 392
Cys Lys Ile Phe Leu Gly Phe Thr Ser Asn Leu Val Ser Ser Gly Val
        100                 105                 110

aga gac act gtc cgc tac ctt gtt cag cac cgg atg gtt gat gtt gtg 440
Arg Asp Thr Val Arg Tyr Leu Val Gln His Arg Met Val Asp Val Val
    115                 120                 125

gtt act aca gct ggt ggt att gaa gag gat ctc ata aag tgc ctc gca 488
Val Thr Thr Ala Gly Gly Ile Glu Glu Asp Leu Ile Lys Cys Leu Ala
130                 135                 140                 145

cca acc tac aag ggg gac ttc tct tta cct gga gct tct cta cga tcg 536
Pro Thr Tyr Lys Gly Asp Phe Ser Leu Pro Gly Ala Ser Leu Arg Ser
                150                 155                 160

aaa gga ttg aac cgt att ggt aac tta ttg gtt cct aat gac aac tac 584
Lys Gly Leu Asn Arg Ile Gly Asn Leu Leu Val Pro Asn Asp Asn Tyr
            165                 170                 175

tgc aaa ttt gag aat tgg atc atc cca gtt ttt gac caa atg tat gag 632
Cys Lys Phe Glu Asn Trp Ile Ile Pro Val Phe Asp Gln Met Tyr Glu
        180                 185                 190

gag cag att aat gag aag gtt cta tgg aca cca tct aaa gtc att gct 680
Glu Gln Ile Asn Glu Lys Val Leu Trp Thr Pro Ser Lys Val Ile Ala
    195                 200                 205

cgt ctg ggt aaa gaa att aat gat gaa acc tca tac ttg tat tgg gct 728
Arg Leu Gly Lys Glu Ile Asn Asp Glu Thr Ser Tyr Leu Tyr Trp Ala
210                 215                 220                 225

tac aag aac cgg att cct gtc ttc tgt cct ggc ttg acg gat gga tca 776
Tyr Lys Asn Arg Ile Pro Val Phe Cys Pro Gly Leu Thr Asp Gly Ser
                230                 235                 240

ctt ggt gac atg cta tac ttc cat tct ttc aaa aag ggt gat cca gat 824
Leu Gly Asp Met Leu Tyr Phe His Ser Phe Lys Lys Gly Asp Pro Asp
            245                 250                 255

aat cca gat ctt aat cct ggt cta gtc ata gac att gta gga gat att 872
Asn Pro Asp Leu Asn Pro Gly Leu Val Ile Asp Ile Val Gly Asp Ile
        260                 265                 270

agg gcc atg aat ggt gaa gct gtc cat gct ggt ttg agg aag aca gga 920
Arg Ala Met Asn Gly Glu Ala Val His Ala Gly Leu Arg Lys Thr Gly
    275                 280                 285

atg att ata ctg ggt gga ggg ctg cct aag cac cat gtt tgc aat gcc 968
Met Ile Ile Leu Gly Gly Gly Leu Pro Lys His His Val Cys Asn Ala
290                 295                 300                 305

aat atg atg cgc aat ggt gca gat ttt gcc gtc ttc att aac acc gca 1016
Asn Met Met Arg Asn Gly Ala Asp Phe Ala Val Phe Ile Asn Thr Ala
                310                 315                 320

caa gag ttt gat ggt agt gac tct ggt gcc cgt cct gat gaa gct gta    1064
Gln Glu Phe Asp Gly Ser Asp Ser Gly Ala Arg Pro Asp Glu Ala Val
            325                 330                 335

tca tgg gga aag ata cgt ggt ggt gcc aag act gtg aag gtg cat tgt    1112
Ser Trp Gly Lys Ile Arg Gly Gly Ala Lys Thr Val Lys Val His Cys
        340                 345                 350

gat gca acc att gca ttt ccc ata tta gta gct gag aca ttt gca gct    1160
Asp Ala Thr Ile Ala Phe Pro Ile Leu Val Ala Glu Thr Phe Ala Ala
    355                 360                 365

aag agt aag gaa ttc tcc cag ata agg tgc caa gtt tgaacattga         1206
Lys Ser Lys Glu Phe Ser Gln Ile Arg Cys Gln Val
370                 375                 380

ggaagctgtc cttccgacca cacatatgaa ttgctagctt ttgaagccaa cttgctagtg 1266

tgcagcacca tttattctgc aaaactgact agagagcagg gtatattcct ctaccccgag 1326

ttagacgaca tcctgtatgg ttcaaattaa ttatttttct ccccttcaca ccatgttatt 1386

tagttctctt cctcttcgaa agtgaagagc ttagatgttc ataggttttg aattatgttg 1446

gaggttggtg ataactgact agtcctctta ccatatagat aatgtatcct tgtactatga 1506

gattttgggt gtgtttgata ccaaggaaaa atgtttattt ggaaaacaat tggattttta 1566

atttattttt tcttgttt                                                1584

<210> 2
<211> 381
<212> PRT
<213> Lycopersicon esculentum

<400> 2
Met Gly Glu Ala Leu Lys Tyr Ser Ile Met Asp Ser Val Arg Ser Val
1               5                   10                  15

Val Phe Lys Glu Ser Glu Asn Leu Glu Gly Ser Cys Thr Lys Ile Glu
            20                  25                  30

Gly Tyr Asp Phe Asn Lys Gly Val Asn Tyr Ala Glu Leu Ile Lys Ser
        35                  40                  45

Met Val Ser Thr Gly Phe Gln Ala Ser Asn Leu Gly Asp Ala Ile Ala
    50                  55                  60

Ile Val Asn Gln Met Leu Asp Trp Arg Leu Ser His Glu Leu Pro Thr
65                  70                  75                  80

Glu Asp Cys Ser Glu Glu Glu Arg Asp Val Ala Tyr Arg Glu Ser Val
                85                  90                  95

Thr Cys Lys Ile Phe Leu Gly Phe Thr Ser Asn Leu Val Ser Ser Gly
            100                 105                 110

Val Arg Asp Thr Val Arg Tyr Leu Val Gln His Arg Met Val Asp Val
        115                 120                 125

Val Val Thr Thr Ala Gly Gly Ile Glu Glu Asp Leu Ile Lys Cys Leu
    130                 135                 140

Ala Pro Thr Tyr Lys Gly Asp Phe Ser Leu Pro Gly Ala Ser Leu Arg
145                 150                 155                 160

Ser Lys Gly Leu Asn Arg Ile Gly Asn Leu Leu Val Pro Asn Asp Asn
                165                 170                 175

Tyr Cys Lys Phe Glu Asn Trp Ile Ile Pro Val Phe Asp Gln Met Tyr
            180                 185                 190

Glu Glu Gln Ile Asn Glu Lys Val Leu Trp Thr Pro Ser Lys Val Ile
        195                 200                 205

Ala Arg Leu Gly Lys Glu Ile Asn Asp Glu Thr Ser Tyr Leu Tyr Trp
    210                 215                 220

Ala Tyr Lys Asn Arg Ile Pro Val Phe Cys Pro Gly Leu Thr Asp Gly
225                 230                 235                 240

Ser Leu Gly Asp Met Leu Tyr Phe His Ser Phe Lys Lys Gly Asp Pro
                245                 250                 255

Asp Asn Pro Asp Leu Asn Pro Gly Leu Val Ile Asp Ile Val Gly Asp
            260                 265                 270

Ile Arg Ala Met Asn Gly Glu Ala Val His Ala Gly Leu Arg Lys Thr
        275                 280                 285

Gly Met Ile Ile Leu Gly Gly Gly Leu Pro Lys His His Val Cys Asn
    290                 295                 300

Ala Asn Met Met Arg Asn Gly Ala Asp Phe Ala Val Phe Ile Asn Thr
305                 310                 315                 320

Ala Gln Glu Phe Asp Gly Ser Asp Ser Gly Ala Arg Pro Asp Glu Ala
                325                 330                 335

Val Ser Trp Gly Lys Ile Arg Gly Gly Ala Lys Thr Val Lys Val His
            340                 345                 350

Cys Asp Ala Thr Ile Ala Phe Pro Ile Leu Val Ala Glu Thr Phe Ala
        355                 360                 365

Ala Lys Ser Lys Glu Phe Ser Gln Ile Arg Cys Gln Val
    370                 375                 380

<210> 3
<211> 369
<212> PRT
<213> Homo sapiens

<400> 3
Met Glu Gly Ser Leu Glu Arg Glu Ala Pro Ala Gly Ala Leu Ala Ala
1               5                   10                  15

Val Leu Lys His Ser Ser Thr Leu Pro Pro Glu Ser Thr Gln Val Arg
            20                  25                  30

Gly Tyr Asp Phe Asn Arg Gly Val Asn Tyr Arg Ala Leu Leu Glu Ala
            35                  40                  45

Phe Gly Thr Thr Gly Phe Gln Ala Thr Asn Phe Gly Arg Ala Val Gln
    50                  55                  60

Gln Val Asn Ala Met Ile Glu Lys Lys Leu Glu Pro Leu Ser Gln Asp
65                  70                  75                  80

Glu Asp Gln His Ala Asp Leu Thr Gln Ser Arg Arg Pro Leu Thr Ser
                85                  90                  95

Cys Thr Ile Phe Leu Gly Tyr Thr Ser Asn Leu Ile Ser Ser Gly Ile
            100                 105                 110

Arg Glu Thr Ile Arg Tyr Leu Val Gln His Asn Met Val Asp Val Leu
        115                 120                 125

Val Thr Thr Ala Gly Gly Val Glu Glu Asp Leu Ile Lys Cys Leu Ala
    130                 135                 140

Pro Thr Tyr Leu Gly Glu Phe Ser Leu Arg Gly Lys Glu Leu Arg Glu
145                 150                 155                 160

Asn Gly Ile Asn Arg Ile Gly Asn Leu Leu Val Pro Asn Glu Asn Tyr
                165                 170                 175

Cys Lys Phe Glu Asp Trp Leu Met Pro Ile Leu Asp Gln Met Val Met
            180                 185                 190

Glu Gln Asn Thr Glu Gly Val Lys Trp Thr Pro Ser Lys Met Ile Ala
        195                 200                 205

Arg Leu Gly Lys Glu Ile Asn Asn Pro Glu Ser Val Tyr Tyr Trp Ala
    210                 215                 220

Gln Lys Asn His Ile Pro Val Phe Ser Pro Ala Leu Thr Asp Gly Ser
225                 230                 235                 240

Leu Gly Asp Met Ile Phe Phe His Ser Tyr Lys Asn Pro Gly Leu Val
                245                 250                 255

Leu Asp Ile Val Glu Asp Leu Arg Leu Ile Asn Thr Gln Ala Ile Phe
            260                 265                 270

Ala Lys Cys Thr Gly Met Ile Ile Leu Gly Gly Gly Val Val Lys His
        275                 280                 285

His Ile Ala Asn Ala Asn Leu Met Arg Asn Gly Ala Asp Tyr Ala Val
    290                 295                 300

Tyr Ile Asn Thr Ala Gln Glu Phe Asp Gly Ser Asp Ser Gly Ala Arg
305                 310                 315                 320

Pro Asp Glu Ala Val Ser Trp Gly Lys Ile Arg Val Asp Ala Gln Pro
                325                 330                 335

Val Lys Val Tyr Ala Asp Ala Ser Leu Val Phe Pro Leu Leu Val Ala
            340                 345                 350

Glu Thr Phe Ala Gln Lys Met Asp Ala Phe Met His Glu Lys Asn Glu
        355                 360                 365

Asp

<210> 4
<211> 6
<212> PRT
<213> Artificial Sequence

<220>
<223> Description of Artificial Sequence: Conserved
      peptide fragment

<400> 4
Thr Gly Lys His Gly His
  1               5

<210> 5
<211> 2272
<212> DNA
<213> Arabidopsis thaliana

<220>
<221> CDS
<222> (68)..(265)

<220>
<221> CDS
<222> (348)..(536)

<220>
<221> CDS
<222> (624)..(842)

<220>
<221> CDS
<222> (979)..(1065)

<220>
<221> CDS
<222> (1154)..(1258)

<220>
<221> CDS
<222> (1575)..(1862)

<400> 5
gaactcccaa aaccctctac tactacactt tcagatccaa ggaaatcaat tttgtcattc 60

gagcaac atg gag gat gat cgt gtt ttc tct tcg gtt cac tca aca gtt    109
        Met Glu Asp Asp Arg Val Phe Ser Ser Val His Ser Thr Val
          1               5                  10

ttc aaa gaa tcc gaa tca ttg gaa gga aag tgt gat aaa atc gaa gga    157
Phe Lys Glu Ser Glu Ser Leu Glu Gly Lys Cys Asp Lys Ile Glu Gly
 15                  20                  25                  30

tac gat ttc aat caa gga gta gat tac cca aag ctt atg cga tcc atg 205
Tyr Asp Phe Asn Gln Gly Val Asp Tyr Pro Lys Leu Met Arg Ser Met
                35                  40                  45

ctc acc acc gga ttt caa gcc tcg aat ctc ggc gaa gct att gat gtc 253
Leu Thr Thr Gly Phe Gln Ala Ser Asn Leu Gly Glu Ala Ile Asp Val
            50                  55                  60

gtc aat caa atg gttcgtttct cgaattcatc aaaaataaaa attccttctt 305
Val Asn Gln Met
        65

tttgttttcc tttgttttgg gtgaattagt aatgacaaag ag ttt gaa ttt gta 359
Phe Glu Phe Val
            70

ttg aag cta gat tgg aga ctg gct gat gaa act aca gta gct gaa gac 407
Leu Lys Leu Asp Trp Arg Leu Ala Asp Glu Thr Thr Val Ala Glu Asp
                75                  80                  85

tgt agt gaa gag gag aag aat cca tcg ttt aga gag tct gtc aag tgt 455
Cys Ser Glu Glu Glu Lys Asn Pro Ser Phe Arg Glu Ser Val Lys Cys
            90                  95                  100

aaa atc ttt cta ggt ttc act tca aat ctt gtt tca tct ggt gtt aga 503
Lys Ile Phe Leu Gly Phe Thr Ser Asn Leu Val Ser Ser Gly Val Arg
        105                 110                 115

gat act att cgt tat ctt gtt cag cat cat atg gtttgtgatt tttgctttat 556
Asp Thr Ile Arg Tyr Leu Val Gln His His Met
    120                 125

caccctgctt ttttatagat gttaaaattt tcgagcttta gttttgattt caatggtttt 616

tctgcag gtt gat gtt ata gtc acg aca act ggt ggt gtt gag gaa gat 665
Val Asp Val Ile Val Thr Thr Thr Gly Gly Val Glu Glu Asp
130                 135                 140

ctc ata aaa tgc ctt gca cct aca ttt aaa ggt gat ttc tct cta cct 713
Leu Ile Lys Cys Leu Ala Pro Thr Phe Lys Gly Asp Phe Ser Leu Pro
    145                 150                 155

gga gct tat tta agg tca aag gga ttg aac cga att ggg aat ttg ctg 761
Gly Ala Tyr Leu Arg Ser Lys Gly Leu Asn Arg Ile Gly Asn Leu Leu
160                 165                 170                 175

gtt cct aat gat aac tac tgc aag ttt gag gat tgg atc att ccc atc 809
Val Pro Asn Asp Asn Tyr Cys Lys Phe Glu Asp Trp Ile Ile Pro Ile
                180                 185                 190

ttt gac gag atg ttg aag gaa cag aaa gaa gag gtattgcttt atctttcctt 862
Phe Asp Glu Met Leu Lys Glu Gln Lys Glu Glu
            195                 200

tttatatgat ttgagatgat tctgtttgtg cgtcactagt ggagatagat tttgattcct 922

ctcttgcatc attgacttcg ttggtgaatc cttctttctc tggtttttcc ttgtag aat 981
Asn

gtg ttg tgg act cct tct aaa ctg tta gca cgg ctg gga aaa gaa atc 1029
Val Leu Trp Thr Pro Ser Lys Leu Leu Ala Arg Leu Gly Lys Glu Ile

205                 210                 215

aac aat gag agt tca tac ctt tat tgg gca tac aag gtatccaaaa        1075
Asn Asn Glu Ser Ser Tyr Leu Tyr Trp Ala Tyr Lys
220                 225                 230

ttttaacctt tttagttttt taatcatcct gtgaggaact cggggattta aattttccgc 1135

ttcttgtggt gtttgtag atg aat att cca gta ttc tgc cca ggg tta aca   1186
                    Met Asn Ile Pro Val Phe Cys Pro Gly Leu Thr
                                    235                 240

gat ggc tct ctt ggg gat atg ctg tat ttt cac tct ttt cgt acc tct   1234
Asp Gly Ser Leu Gly Asp Met Leu Tyr Phe His Ser Phe Arg Thr Ser
        245                 250                 255

ggc ctc atc atc gat gta gta caa ggtacttctt ttactcaata agtcagtgtg  1288
Gly Leu Ile Ile Asp Val Val Gln
    260                 265

ataaatattc ctgctacatc tagtgcagga atattgtaac tagtagtgca ttgtagcttt 1348

tccaattcag caacggactt tactgtaagt tgatatctaa aggttcaaac gggagctagg 1408

agaatagcat aggggcattc tgatttaggt ttggggcact gggttaagag ttagagaata 1468

ataatcttgt tagttgttta tcaaactctt tgatggttag tctcttggta atttgaattt 1528

tatcacagtg tttatggtct ttgaaccagt taatgtttta tgaaca gat atc aga    1583
                                                   Asp Ile Arg

gct atg aac ggc gaa gct gtc cat gca aat cct aaa aag aca ggg atg   1631
Ala Met Asn Gly Glu Ala Val His Ala Asn Pro Lys Lys Thr Gly Met
270                 275                 280                 285

ata atc ctt gga ggg ggc ttg cca aag cac cac ata tgt aat gcc aat   1679
Ile Ile Leu Gly Gly Gly Leu Pro Lys His His Ile Cys Asn Ala Asn
                290                 295                 300

atg atg cgc aat ggt gca gat tac gct gta ttt ata aac acc ggg caa   1727
Met Met Arg Asn Gly Ala Asp Tyr Ala Val Phe Ile Asn Thr Gly Gln
            305                 310                 315

gaa ttt gat ggg agc gac tcg ggt gca cgc cct gat gaa gcc gtg tct   1775
Glu Phe Asp Gly Ser Asp Ser Gly Ala Arg Pro Asp Glu Ala Val Ser
        320                 325                 330

tgg ggt aaa att agg ggt tct gct aaa acc gtt aag gtc tgc ttt tta   1823
Trp Gly Lys Ile Arg Gly Ser Ala Lys Thr Val Lys Val Cys Phe Leu
    335                 340                 345

att tct tca cat cct aat tta tat ctc act cag tgg ttt tgagtacata    1872
Ile Ser Ser His Pro Asn Leu Tyr Leu Thr Gln Trp Phe
350                 355                 360

tttaatattg gatcattctt gcaggtatac tgtgatgcta ccatagcctt cccattgttg 1932

gttgcagaaa catttgccac aaagagagac caaacctgtg agtctaagac ttaagaactg 1992

actggtcgtt ttggccatgg attcttaaag atcgttgctt tttgatttta cactggagtg 2052

accatataac actccacatt gatgtggctg tgacgcgaat tgtcttcttg cgaattgtac 2112

tttagtttct ctcaacctaa aatgatttgc agattgtgtt ttcgtttaaa acacaagagt 2172

cttgtagtca ataatccttt gccttataaa attattcagt tccaacaaca cattgtgatt 2232

ctgtgacaag tctcccgttg cctatgttca cttctctgcg 2272

<210> 6
<211> 362
<212> PRT
<213> Arabidopsis thaliana

<400> 6
Met Glu Asp Asp Arg Val Phe Ser Ser Val His Ser Thr Val Phe Lys
1               5                   10                  15

Glu Ser Glu Ser Leu Glu Gly Lys Cys Asp Lys Ile Glu Gly Tyr Asp
            20                  25                  30

Phe Asn Gln Gly Val Asp Tyr Pro Lys Leu Met Arg Ser Met Leu Thr
        35                  40                  45

Thr Gly Phe Gln Ala Ser Asn Leu Gly Glu Ala Ile Asp Val Val Asn
    50                  55                  60

Gln Met Phe Glu Phe Val Leu Lys Leu Asp Trp Arg Leu Ala Asp Glu
65                  70                  75                  80

Thr Thr Val Ala Glu Asp Cys Ser Glu Glu Glu Lys Asn Pro Ser Phe
                85                  90                  95

Arg Glu Ser Val Lys Cys Lys Ile Phe Leu Gly Phe Thr Ser Asn Leu
            100                 105                 110

Val Ser Ser Gly Val Arg Asp Thr Ile Arg Tyr Leu Val Gln His His
        115                 120                 125

Met Val Asp Val Ile Val Thr Thr Thr Gly Gly Val Glu Glu Asp Leu
    130                 135                 140

Ile Lys Cys Leu Ala Pro Thr Phe Lys Gly Asp Phe Ser Leu Pro Gly
145                 150                 155                 160

Ala Tyr Leu Arg Ser Lys Gly Leu Asn Arg Ile Gly Asn Leu Leu Val
                165                 170                 175

Pro Asn Asp Asn Tyr Cys Lys Phe Glu Asp Trp Ile Ile Pro Ile Phe
            180                 185                 190

Asp Glu Met Leu Lys Glu Gln Lys Glu Glu Asn Val Leu Trp Thr Pro
        195                 200                 205

Ser Lys Leu Leu Ala Arg Leu Gly Lys Glu Ile Asn Asn Glu Ser Ser
    210                 215                 220

Tyr Leu Tyr Trp Ala Tyr Lys Met Asn Ile Pro Val Phe Cys Pro Gly
225                 230                 235                 240

Leu Thr Asp Gly Ser Leu Gly Asp Met Leu Tyr Phe His Ser Phe Arg

245                 250                 255

Thr Ser Gly Leu Ile Ile Asp Val Val Gln Asp Ile Arg Ala Met Asn
            260                 265                 270

Gly Glu Ala Val His Ala Asn Pro Lys Lys Thr Gly Met Ile Ile Leu
        275                 280                 285

Gly Gly Gly Leu Pro Lys His His Ile Cys Asn Ala Asn Met Met Arg
    290                 295                 300

Asn Gly Ala Asp Tyr Ala Val Phe Ile Asn Thr Gly Gln Glu Phe Asp
305                 310                 315                 320

Gly Ser Asp Ser Gly Ala Arg Pro Asp Glu Ala Val Ser Trp Gly Lys
                325                 330                 335

Ile Arg Gly Ser Ala Lys Thr Val Lys Val Cys Phe Leu Ile Ser Ser
            340                 345                 350

His Pro Asn Leu Tyr Leu Thr Gln Trp Phe
        355                 360

<210> 7
<211> 19
<212> DNA
<213> Artificial Sequence

<220>
<223> Description of Artificial Sequence: Primer

<400> 7
ggtggtgttg aggaagatc                                                19

<210> 8
<211> 20
<212> DNA
<213> Artificial Sequence

<220>
<223> Description of Artificial Sequence: Primer

<400> 8
ggtgcacgcc ctgatgaagc                                               20

<210> 9
<211> 1660
<212> DNA
<213> Dianthus caryophyllus

<220>
<221> CDS
<222> (256)..(1374)

<400> 9
gtcattacaa tgcataggat cattgcacat gctaccttcc tcattgcact tgagcttgcc 60

atacttttgt ttttgacgtt tgataataat actatgaaaa tattatgttt tttcttttgt 120

gtgttggtgt ttttgaagtt gtttttgata agcagaaccc agttgtttta cacttttacc 180

attgaactac tgcaattcta aaactttgtt tacattttaa ttccatcaaa gattgagttc 240

agcataggaa aaagg atg gag gat gct aat cat gat agt gtg gca tct gcg 291
Met Glu Asp Ala Asn His Asp Ser Val Ala Ser Ala
1               5                   10

cac tct gca gca ttc aaa aag tcg gag aat tta gag ggg aaa agc gtt 339
His Ser Ala Ala Phe Lys Lys Ser Glu Asn Leu Glu Gly Lys Ser Val
        15                  20                  25

aag att gag ggt tat gat ttt aat caa ggt gta aac tat tcc aaa ctc 387
Lys Ile Glu Gly Tyr Asp Phe Asn Gln Gly Val Asn Tyr Ser Lys Leu
    30                  35                  40

ttg caa tct ttc gct tct aat ggg ttt caa gcc tcg aat ctt gga gat 435
Leu Gln Ser Phe Ala Ser Asn Gly Phe Gln Ala Ser Asn Leu Gly Asp
45                  50                  55                  60

gcc att gaa gta gtt aat cat atg cta gat tgg agt ctg gca gat gag 483
Ala Ile Glu Val Val Asn His Met Leu Asp Trp Ser Leu Ala Asp Glu
                65                  70                  75

gca cct gtg gac gat tgt agc gag gaa gag agg gat cct aaa ttc aga 531
Ala Pro Val Asp Asp Cys Ser Glu Glu Glu Arg Asp Pro Lys Phe Arg
            80                  85                  90

gaa tct gtg aag tgc aaa gtg ttc ttg ggc ttt act tca aat ctt att 579
Glu Ser Val Lys Cys Lys Val Phe Leu Gly Phe Thr Ser Asn Leu Ile
        95                  100                 105

tcc tct ggt gtt cgt gac aca att cgg tat ctc gtg caa cat cat atg 627
Ser Ser Gly Val Arg Asp Thr Ile Arg Tyr Leu Val Gln His His Met
    110                 115                 120

gtt gac gtg ata gta acg aca acc gga ggt ata gaa gaa gat cta ata 675
Val Asp Val Ile Val Thr Thr Thr Gly Gly Ile Glu Glu Asp Leu Ile
125                 130                 135                 140

aaa gga aga tcc atc aag tgc ctt gca ccc act ttc aaa ggc gat ttt 723
Lys Gly Arg Ser Ile Lys Cys Leu Ala Pro Thr Phe Lys Gly Asp Phe
                145                 150                 155

gcc tta cca gga gct caa tta cgc tcc aaa ggg ttg aat cga att ggt 771
Ala Leu Pro Gly Ala Gln Leu Arg Ser Lys Gly Leu Asn Arg Ile Gly
            160                 165                 170

aat ctg ttg gtt ccg aat gat aac tac tgt aaa ttt gag gat tgg atc 819
Asn Leu Leu Val Pro Asn Asp Asn Tyr Cys Lys Phe Glu Asp Trp Ile
        175                 180                 185

att cca att tta gat aag atg ttg gaa gag caa att tca gag aaa atc 867
Ile Pro Ile Leu Asp Lys Met Leu Glu Glu Gln Ile Ser Glu Lys Ile
    190                 195                 200

tta tgg aca cca tcg aag ttg att ggt cga tta gga aga gaa ata aac 915
Leu Trp Thr Pro Ser Lys Leu Ile Gly Arg Leu Gly Arg Glu Ile Asn
205                 210                 215                 220

gat gag agt tca tac ctt tac tgg gcc ttc aag aac aat att cca gta 963
Asp Glu Ser Ser Tyr Leu Tyr Trp Ala Phe Lys Asn Asn Ile Pro Val
                225                 230                 235

ttt tgc cca ggt tta aca gac ggc tca ctc gga gac atg cta tat ttt 1011
Phe Cys Pro Gly Leu Thr Asp Gly Ser Leu Gly Asp Met Leu Tyr Phe
            240                 245                 250

cat tct ttt cgc aat ccg ggt tta atc gtc gat gtt gtg caa gat ata 1059
His Ser Phe Arg Asn Pro Gly Leu Ile Val Asp Val Val Gln Asp Ile
        255                 260                 265

aga gca gta aat ggc gag gct gtg cac gca gcg cct agg aaa aca ggc 1107
Arg Ala Val Asn Gly Glu Ala Val His Ala Ala Pro Arg Lys Thr Gly
    270                 275                 280

atg att ata ctc ggt gga ggg ttg cct aag cac cac atc tgc aac gca 1155
Met Ile Ile Leu Gly Gly Gly Leu Pro Lys His His Ile Cys Asn Ala
285                 290                 295                 300

aac atg atg aga aat ggc gcc gat tat gct gtt ttc atc aac act gcc 1203
Asn Met Met Arg Asn Gly Ala Asp Tyr Ala Val Phe Ile Asn Thr Ala
                305                 310                 315

gaa gag ttt gac ggc agt gat tct ggt gct cgc ccc gat gag gct att 1251
Glu Glu Phe Asp Gly Ser Asp Ser Gly Ala Arg Pro Asp Glu Ala Ile
            320                 325                 330

tca tgg ggc aaa att agc gga tct gct aag act gtg aag gtg cat tgt 1299
Ser Trp Gly Lys Ile Ser Gly Ser Ala Lys Thr Val Lys Val His Cys
        335                 340                 345

gat gcc acg ata gct ttc cct cta cta gtc gct gag aca ttt gca gca 1347
Asp Ala Thr Ile Ala Phe Pro Leu Leu Val Ala Glu Thr Phe Ala Ala
    350                 355                 360

aaa aga gaa aaa gag agg aag agc tgt taaaactttt ttgattgttg 1394
Lys Arg Glu Lys Glu Arg Lys Ser Cys
365                 370

aaaaatctgt gttatacaag tctcgaaatg cattttagta attgacttga tcttatcatt 1454

tcaatgtgtt atctttgaaa atgttggtaa tgaaacatct cacctcttct atacaacatt 1514

gttgatccat tgtactccgt atcttgtaat tttggaaaaa aaaaaccgtc tattgttacg 1574

agagagtaca tttttgaggt aaaaatatag gatttttgtg cgatgcaaat gctggttatt 1634

cccttgaaaa aaaaaaaaaa aaaaaa 1660

<210> 10
<211> 373
<212> PRT
<213> Dianthus caryophyllus

<400> 10
Met Glu Asp Ala Asn His Asp Ser Val Ala Ser Ala His Ser Ala Ala
1               5                   10                  15

Phe Lys Lys Ser Glu Asn Leu Glu Gly Lys Ser Val Lys Ile Glu Gly
            20                  25                  30

Tyr Asp Phe Asn Gln Gly Val Asn Tyr Ser Lys Leu Leu Gln Ser Phe
        35                  40                  45

Ala Ser Asn Gly Phe Gln Ala Ser Asn Leu Gly Asp Ala Ile Glu Val
    50                  55                  60

Val Asn His Met Leu Asp Trp Ser Leu Ala Asp Glu Ala Pro Val Asp
65                  70                  75                  80

Asp Cys Ser Glu Glu Glu Arg Asp Pro Lys Phe Arg Glu Ser Val Lys
                85                  90                  95

Cys Lys Val Phe Leu Gly Phe Thr Ser Asn Leu Ile Ser Ser Gly Val
            100                 105                 110

Arg Asp Thr Ile Arg Tyr Leu Val Gln His His Met Val Asp Val Ile
        115                 120                 125

Val Thr Thr Thr Gly Gly Ile Glu Glu Asp Leu Ile Lys Gly Arg Ser
    130                 135                 140

Ile Lys Cys Leu Ala Pro Thr Phe Lys Gly Asp Phe Ala Leu Pro Gly
145                 150                 155                 160

Ala Gln Leu Arg Ser Lys Gly Leu Asn Arg Ile Gly Asn Leu Leu Val
                165                 170                 175

Pro Asn Asp Asn Tyr Cys Lys Phe Glu Asp Trp Ile Ile Pro Ile Leu
            180                 185                 190

Asp Lys Met Leu Glu Glu Gln Ile Ser Glu Lys Ile Leu Trp Thr Pro
        195                 200                 205

Ser Lys Leu Ile Gly Arg Leu Gly Arg Glu Ile Asn Asp Glu Ser Ser
    210                 215                 220

Tyr Leu Tyr Trp Ala Phe Lys Asn Asn Ile Pro Val Phe Cys Pro Gly
225                 230                 235                 240

Leu Thr Asp Gly Ser Leu Gly Asp Met Leu Tyr Phe His Ser Phe Arg
                245                 250                 255

Asn Pro Gly Leu Ile Val Asp Val Val Gln Asp Ile Arg Ala Val Asn
            260                 265                 270

Gly Glu Ala Val His Ala Ala Pro Arg Lys Thr Gly Met Ile Ile Leu
        275                 280                 285

Gly Gly Gly Leu Pro Lys His His Ile Cys Asn Ala Asn Met Met Arg
    290                 295                 300

Asn Gly Ala Asp Tyr Ala Val Phe Ile Asn Thr Ala Glu Glu Phe Asp
305                 310                 315                 320

Gly Ser Asp Ser Gly Ala Arg Pro Asp Glu Ala Ile Ser Trp Gly Lys
                325                 330                 335

Ile Ser Gly Ser Ala Lys Thr Val Lys Val His Cys Asp Ala Thr Ile

340                 345                 350

Ala Phe Pro Leu Leu Val Ala Glu Thr Phe Ala Ala Lys Arg Glu Lys
        355                 360                 365

Glu Arg Lys Ser Cys
    370

<210> 11
<211> 780
<212> DNA
<213> Lycopersicon esculentum

<220>
<221> CDS
<222> (43)..(522)

<400> 11
aaagaatcct agagagagaa agggaatcct agagagagaa gc atg tcg gac gaa      54
                                               Met Ser Asp Glu
                                               1

gaa cac cat ttt gag tca aag gca gat gct ggt gcc tca aaa act ttc     102
Glu His His Phe Glu Ser Lys Ala Asp Ala Gly Ala Ser Lys Thr Phe
5                   10                  15                  20

cca cag caa gct gga acc atc cgt aag aat ggt tac atc gtt atc aaa     150
Pro Gln Gln Ala Gly Thr Ile Arg Lys Asn Gly Tyr Ile Val Ile Lys
                25                  30                  35

ggc cgt ccc tgc aag gtt gtt gag gtc tcc act tca aaa act gga aaa     198
Gly Arg Pro Cys Lys Val Val Glu Val Ser Thr Ser Lys Thr Gly Lys
            40                  45                  50

cac gga cat gct aaa tgt cac ttt gtg gca att gac att ttc aat gga     246
His Gly His Ala Lys Cys His Phe Val Ala Ile Asp Ile Phe Asn Gly
        55                  60                  65

aag aaa ctg gaa gat atc gtt ccg tcc tcc cac aat tgt gat gtg cca     294
Lys Lys Leu Glu Asp Ile Val Pro Ser Ser His Asn Cys Asp Val Pro
    70                  75                  80

cat gtt aac cgt acc gac tat cag ctg att gat atc tct gaa gat ggt     342
His Val Asn Arg Thr Asp Tyr Gln Leu Ile Asp Ile Ser Glu Asp Gly
85                  90                  95                  100

ttt gtc tca ctt ctt act gaa agt gga aac acc aag gat gac ctc agg     390
Phe Val Ser Leu Leu Thr Glu Ser Gly Asn Thr Lys Asp Asp Leu Arg
                105                 110                 115

ctt ccc acc gat gaa aat ctg ctg aag cag gtt aaa gat ggg ttc cag     438
Leu Pro Thr Asp Glu Asn Leu Leu Lys Gln Val Lys Asp Gly Phe Gln
            120                 125                 130

gaa gga aag gat ctt gtg gtg tct gtt atg tct gcg atg ggc gaa gag     486
Glu Gly Lys Asp Leu Val Val Ser Val Met Ser Ala Met Gly Glu Glu
        135                 140                 145

cag att aac gcc gtt aag gat gtt ggt acc aag aat tagttatgtc          532

Gln Ile Asn Ala Val Lys Asp Val Gly Thr Lys Asn
    150                 155                 160

atggcagcat aatcactgcc aaagctttaa gacattatca tatcctaatg tggtactttg 592

atatcactag attataaact gtgttatttg cactgttcaa aacaaaagaa agaaaactgc 652

tgttatggct agagaaagta ttggctttga gcttttgaca gcacagttga actatgtgaa 712

aattctactt tttttttttt gggtaaaata ctgctcgttt aatgttttgc aaaaaaaaaa 772

aaaaaaaa                                                          780

<210> 12
<211> 160
<212> PRT
<213> Lycopersicon esculentum

<400> 12
Met Ser Asp Glu Glu His His Phe Glu Ser Lys Ala Asp Ala Gly Ala
1               5                   10                  15

Ser Lys Thr Phe Pro Gln Gln Ala Gly Thr Ile Arg Lys Asn Gly Tyr
            20                  25                  30

Ile Val Ile Lys Gly Arg Pro Cys Lys Val Val Glu Val Ser Thr Ser
        35                  40                  45

Lys Thr Gly Lys His Gly His Ala Lys Cys His Phe Val Ala Ile Asp
    50                  55                  60

Ile Phe Asn Gly Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Glu Ser Gly Asn Thr Lys
            100                 105                 110

Asp Asp Leu Arg Leu Pro Thr Asp Glu Asn Leu Leu Lys Gln Val Lys
        115                 120                 125

Asp Gly Phe Gln Glu Gly Lys Asp Leu Val Val Ser Val Met Ser Ala
    130                 135                 140

Met Gly Glu Glu Gln Ile Asn Ala Val Lys Asp Val Gly Thr Lys Asn
145                 150                 155                 160

<210> 13
<211> 812
<212> DNA
<213> Dianthus caryophyllus

<220>
<221> CDS
<222> (67)..(546)

<400> 13
ctcttttaca tcaatcgaaa aaaaattagg gttcttattt tagagtgaga ggcgaaaaat 60

cgaacg atg tcg gac gac gat cac cat ttc gag tca tcg gcc gac gcc 108
Met Ser Asp Asp Asp His His Phe Glu Ser Ser Ala Asp Ala
1               5                   10

gga gca tcc aag act tac cct caa caa gct ggt aca atc cgc aag agc 156
Gly Ala Ser Lys Thr Tyr Pro Gln Gln Ala Gly Thr Ile Arg Lys Ser
15                  20                  25                  30

ggt cac atc gtc atc aaa aat cgc cct tgc aag gtg gtt gag gtt tct 204
Gly His Ile Val Ile Lys Asn Arg Pro Cys Lys Val Val Glu Val Ser
                35                  40                  45

acc tcc aag act ggc aag cac ggt cat gcc aaa tgt cac ttt gtt gcc 252
Thr Ser Lys Thr Gly Lys His Gly His Ala Lys Cys His Phe Val Ala
            50                  55                  60

att gac att ttc aac ggc aag aag ctg gaa gat att gtc ccc tca tcc 300
Ile Asp Ile Phe Asn Gly Lys Lys Leu Glu Asp Ile Val Pro Ser Ser
        65                  70                  75

cac aat tgt gat gtt cca cat gtc aac cgt gtc gac tac cag ctg ctt 348
His Asn Cys Asp Val Pro His Val Asn Arg Val Asp Tyr Gln Leu Leu
    80                  85                  90

gat atc act gaa gat ggc ttt gtt agt ctg ctg act gac agt ggt gac 396
Asp Ile Thr Glu Asp Gly Phe Val Ser Leu Leu Thr Asp Ser Gly Asp
95                  100                 105                 110

acc aag gat gat ctg aag ctt cct gct gat gag gcc ctt gtg aag cag 444
Thr Lys Asp Asp Leu Lys Leu Pro Ala Asp Glu Ala Leu Val Lys Gln
                115                 120                 125

atg aag gag gga ttt gag gcg ggg aaa gac ttg att ctg tca gtc atg 492
Met Lys Glu Gly Phe Glu Ala Gly Lys Asp Leu Ile Leu Ser Val Met
            130                 135                 140

tgt gca atg gga gaa gag cag atc tgc gcc gtc aag gac gtt agt ggt 540
Cys Ala Met Gly Glu Glu Gln Ile Cys Ala Val Lys Asp Val Ser Gly
        145                 150                 155

ggc aag tagaagcttt tgatgaatcc aatactacgc ggtgcagttg aagcaatagt 596
Gly Lys
    160

aatctcgaga acattctgaa ccttatatgt tgaattgatg gtgcttagtt tgttttggaa 656

atctctttgc aattaagttg taccaaatca atggatgtaa tgtcttgaat ttgttttatt 716

tttgttttga tgtttgctgt gattgcatta tgcattgtta tgagttatga cctgttataa 776

cacaaggttt tggtaaaaaa aaaaaaaaaa aaaaaa 812

<210> 14
<211> 160
<212> PRT
<213> Dianthus caryophyllus

<400> 14
Met Ser Asp Asp Asp His His Phe Glu Ser Ser Ala Asp Ala Gly Ala
1               5                   10                  15

Ser Lys Thr Tyr Pro Gln Gln Ala Gly Thr Ile Arg Lys Ser Gly His
            20                  25                  30

Ile Val Ile Lys Asn Arg Pro Cys Lys Val Val Glu Val Ser Thr Ser
        35                  40                  45

Lys Thr Gly Lys His Gly His Ala Lys Cys His Phe Val Ala Ile Asp
    50                  55                  60

Ile Phe Asn Gly Lys Lys Leu Glu Asp Ile Val Pro Ser Ser His Asn
65                  70                  75                  80

Cys Asp Val Pro His Val Asn Arg Val Asp Tyr Gln Leu Leu Asp Ile
                85                  90                  95

Thr Glu Asp Gly Phe Val Ser Leu Leu Thr Asp Ser Gly Asp Thr Lys
            100                 105                 110

Asp Asp Leu Lys Leu Pro Ala Asp Glu Ala Leu Val Lys Gln Met Lys
        115                 120                 125

Glu Gly Phe Glu Ala Gly Lys Asp Leu Ile Leu Ser Val Met Cys Ala
    130                 135                 140

Met Gly Glu Glu Gln Ile Cys Ala Val Lys Asp Val Ser Gly Gly Lys
145                 150                 155                 160

<210> 15
<211> 702
<212> DNA
<213> Arabidopsis thaliana

<220>
<221> CDS
<222> (56)..(529)

<400> 15
ctgttaccaa aaaatctgta ccgcaaaatc ctcgtcgaag ctcgctgctg caacc atg   58
                                                             Met
                                                             1

tcc gac gag gag cat cac ttt gag tcc agt gac gcc gga gcg tcc aaa   106
Ser Asp Glu Glu His His Phe Glu Ser Ser Asp Ala Gly Ala Ser Lys
            5                   10                  15

acc tac cct caa caa gct gga acc atc cgt aag aat ggt tac atc gtc   154
Thr Tyr Pro Gln Gln Ala Gly Thr Ile Arg Lys Asn Gly Tyr Ile Val
        20                  25                  30

atc aaa aat cgt ccc tgc aag gtt gtt gag gtt tca acc tcg aag act   202
Ile Lys Asn Arg Pro Cys Lys Val Val Glu Val Ser Thr Ser Lys Thr
    35                  40                  45

ggc aag cat ggt cat gct aaa tgt cat ttt gta gct att gat atc ttc   250
Gly Lys His Gly His Ala Lys Cys His Phe Val Ala Ile Asp Ile Phe

50                  55                  60                  65

acc agc aag aaa ctc gaa gat att gtt cct tct tcc cac aat tgt gat 298
Thr Ser Lys Lys Leu Glu Asp Ile Val Pro Ser Ser His Asn Cys Asp
                70                  75                  80

gtt cct cat gtc aac cgt act gat tat cag ctg att gac att tct gaa 346
Val Pro His Val Asn Arg Thr Asp Tyr Gln Leu Ile Asp Ile Ser Glu
            85                  90                  95

gat gga tat gtc agt ttg ttg act gat aac ggt agt acc aag gat gac 394
Asp Gly Tyr Val Ser Leu Leu Thr Asp Asn Gly Ser Thr Lys Asp Asp
        100                 105                 110

ctt aag ctc cct aat gat gac act ctg ctc caa cag atc aag agt ggg 442
Leu Lys Leu Pro Asn Asp Asp Thr Leu Leu Gln Gln Ile Lys Ser Gly
    115                 120                 125

ttt gat gat gga aaa gat cta gtg gtg agt gta atg tca gct atg gga 490
Phe Asp Asp Gly Lys Asp Leu Val Val Ser Val Met Ser Ala Met Gly
130                 135                 140                 145

gag gaa cag atc aat gct ctt aag gac atc ggt ccc aag tgagactaac 539
Glu Glu Gln Ile Asn Ala Leu Lys Asp Ile Gly Pro Lys
                150                 155

aaagcctccc ctttgttatg agattcttct tcttctgtag gcttccatta ctcgtcggag 599

attatcttgt ttttgggtta ctcctatttt ggatatttaa acttttgtta ataatgccat 659

cttcttcaac cttttccttc tagatggttt ttatacttct tct 702

<210> 16
<211> 158
<212> PRT
<213> Arabidopsis thaliana

<400> 16
Met Ser Asp Glu Glu His His Phe Glu Ser Ser Asp Ala Gly Ala Ser
1               5                   10                  15

Lys Thr Tyr Pro Gln Gln Ala Gly Thr Ile Arg Lys Asn Gly Tyr Ile
            20                  25                  30

Val Ile Lys Asn Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ser Lys Lys Leu Glu Asp Ile Val Pro Ser Ser His Asn Cys
65                  70                  75                  80

Asp Val Pro His Val Asn Arg Thr Asp Tyr Gln Leu Ile Asp Ile Ser
                85                  90                  95

Glu Asp Gly Tyr Val Ser Leu Leu Thr Asp Asn Gly Ser Thr Lys Asp
            100                 105                 110

Asp Leu Lys Leu Pro Asn Asp Asp Thr Leu Leu Gln Gln Ile Lys Ser

115                 120                 125

Gly Phe Asp Asp Gly Lys Asp Leu Val Val Ser Val Met Ser Ala Met
    130                 135                 140

Gly Glu Glu Gln Ile Asn Ala Leu Lys Asp Ile Gly Pro Lys
145                 150                 155

<210> 17
<211> 20
<212> DNA
<213> Artificial Sequence

<220>
<223> Description of Artificial Sequence: Primer

<400> 17
aaarrycgmc cytgcaaggt                                              20

<210> 18
<211> 17
<212> DNA
<213> Artificial Sequence

<220>
<223> Description of Artificial Sequence: Primer

<400> 18
aatacgactc actatag                                                 17

<210> 19
<211> 20
<212> DNA
<213> Artificial Sequence

<220>
<223> Description of Artificial Sequence: Primer

<220>
<221> modified_base
<222> (6)
<223> a, t, c or g

<400> 19
tcyttnccyt cmkctaahcc                                              20

<210> 20
<211> 17
<212> DNA
<213> Artificial Sequence

<220>
<223> Description of Artificial Sequence: Primer

<400> 20
attaaccctc actaaag                                                 17

<210> 21
<211> 22
<212> DNA
<213> Artificial Sequence

<220>
<223> Description of Artificial Sequence: Primer

<400> 21
ctgttaccaa aaaatctgta cc                                                22

<210> 22
<211> 21
<212> DNA
<213> Artificial Sequence

<220>
<223> Description of Artificial Sequence: Primer

<400> 22
agaagaagta taaaaaccat c                                                 21

<210> 23
<211> 23
<212> DNA
<213> Artificial Sequence

<220>
<223> Description of Artificial Sequence: Primer

<400> 23
aaagaatcct agagagagaa agg                                               23

<210> 24
<211> 18
<212> DNA
<213> Artificial Sequence

<220>
<223> Description of Artificial Sequence: Primer

<400> 24
ttttacatca atcgaaaa                                                     18

<210> 25
<211> 23
<212> DNA
<213> Artificial Sequence

<220>
<223> Description of Artificial Sequence: Primer

<400> 25
accaaaacct gtgttataac tcc                                               23

<210> 26
<211> 581
<212> DNA
<213> Arabidopsis thaliana

<220>
<221> CDS
<222> (1)..(579)

<400> 26
ggt ggt gtt gag gaa gat ctc ata aaa tgc ctt gca cct aca ttt aaa 48
Gly Gly Val Glu Glu Asp Leu Ile Lys Cys Leu Ala Pro Thr Phe Lys
1               5                   10                  15

ggt gat ttc tct cta cct gga gct tat tta agg tca aag gga ttg aac 96
Gly Asp Phe Ser Leu Pro Gly Ala Tyr Leu Arg Ser Lys Gly Leu Asn
            20                  25                  30

cga att ggg aat ttg ctg gtt cct aat gat aac tac tgc aag ttt gag 144
Arg Ile Gly Asn Leu Leu Val Pro Asn Asp Asn Tyr Cys Lys Phe Glu
        35                  40                  45

gat tgg atc att ccc atc ttt gac gag atg ttg aag gaa cag aaa gaa 192
Asp Trp Ile Ile Pro Ile Phe Asp Glu Met Leu Lys Glu Gln Lys Glu
    50                  55                  60

gag aat gtg ttg tgg act cct tct aaa ctg tta gca cgg ctg gga aaa 240
Glu Asn Val Leu Trp Thr Pro Ser Lys Leu Leu Ala Arg Leu Gly Lys
65                  70                  75                  80

gaa atc aac aat gag agt tca tac ctt tat tgg gca tac aag atg aat 288
Glu Ile Asn Asn Glu Ser Ser Tyr Leu Tyr Trp Ala Tyr Lys Met Asn
                85                  90                  95

att cca gta ttc tgc cca ggg tta aca gat ggc tct ctt agg gat atg 336
Ile Pro Val Phe Cys Pro Gly Leu Thr Asp Gly Ser Leu Arg Asp Met
            100                 105                 110

ctg tat ttt cac tct ttt cgt acc tct ggc ctc atc atc gat gta gta 384
Leu Tyr Phe His Ser Phe Arg Thr Ser Gly Leu Ile Ile Asp Val Val
        115                 120                 125

caa gat atc aga gct atg aac ggc gaa gct gtc cat gca aat cct aaa 432
Gln Asp Ile Arg Ala Met Asn Gly Glu Ala Val His Ala Asn Pro Lys
    130                 135                 140

aag aca ggg atg ata atc ctt gga ggg ggc ttg cca aag cac cac ata 480
Lys Thr Gly Met Ile Ile Leu Gly Gly Gly Leu Pro Lys His His Ile
145                 150                 155                 160

tgt aat gcc aat atg atg cgc aat ggt gca gat tac gct gta ttt ata 528
Cys Asn Ala Asn Met Met Arg Asn Gly Ala Asp Tyr Ala Val Phe Ile
                165                 170                 175

aac acc ggg caa gaa ttt gat ggg agc gac tcg ggt gca cgc cct gat 576
Asn Thr Gly Gln Glu Phe Asp Gly Ser Asp Ser Gly Ala Arg Pro Asp
            180                 185                 190

gaa gc 581
Glu

<210> 27
<211> 523
<212> DNA
<213> Dianthus caryophyllus

<220>
<221> CDS
<222> (1)..(522)

<400> 27
mga aga tcc atc aag tgc ctt gca ccc act ttc aaa ggc gat ttt gcc     48
Arg Arg Ser Ile Lys Cys Leu Ala Pro Thr Phe Lys Gly Asp Phe Ala
1               5                   10                  15

tta cca gga gct caa tta cgc tcc aaa ggg ttg aat cga att ggt aat     96
Leu Pro Gly Ala Gln Leu Arg Ser Lys Gly Leu Asn Arg Ile Gly Asn
            20                  25                  30

ctg ttg gtt ccg aat gat aac tac tgt aaa ttt gag gat tgg atc att     144
Leu Leu Val Pro Asn Asp Asn Tyr Cys Lys Phe Glu Asp Trp Ile Ile
        35                  40                  45

cca att tta gat aag atg ttg gaa gag caa att tca gag aaa atc tta     192
Pro Ile Leu Asp Lys Met Leu Glu Glu Gln Ile Ser Glu Lys Ile Leu
    50                  55                  60

tgg aca cca tcg aag ttg att ggt cga tta gga aga gaa ata aac gat     240
Trp Thr Pro Ser Lys Leu Ile Gly Arg Leu Gly Arg Glu Ile Asn Asp
65                  70                  75                  80

gag agt tca tac ctt tac tgg gcc ttc aag aac aat att cca gta ttt     288
Glu Ser Ser Tyr Leu Tyr Trp Ala Phe Lys Asn Asn Ile Pro Val Phe
                85                  90                  95

tgc cca ggt tta aca gac ggc tca ctc gga gac atg cta tat ttt cat     336
Cys Pro Gly Leu Thr Asp Gly Ser Leu Gly Asp Met Leu Tyr Phe His
            100                 105                 110

tct ttt cgc aat ccg ggt tta atc atc gat gtt gtg caa gat ata aga     384
Ser Phe Arg Asn Pro Gly Leu Ile Ile Asp Val Val Gln Asp Ile Arg
        115                 120                 125

gca gta aat ggc gag gct gtg cac gca gcg cct agg aaa aca ggc atg     432
Ala Val Asn Gly Glu Ala Val His Ala Ala Pro Arg Lys Thr Gly Met
    130                 135                 140

att ata ctc ggt gga ggg ttg cct aag cac cac atc tgc aac gca aac     480
Ile Ile Leu Gly Gly Gly Leu Pro Lys His His Ile Cys Asn Ala Asn
145                 150                 155                 160

atg atg aga aat ggc gcc gat tat gct gtt ttc atc aac acc g           523
Met Met Arg Asn Gly Ala Asp Tyr Ala Val Phe Ile Asn Thr
                165                 170

<210> 28
<211> 24

<212> DNA
<213> Artificial Sequence

<220>
<223> Description of Artificial Sequence: Primer

<400> 28
ttgargaaga tycatmaart gcct                                          24


<210> 29
<211> 23
<212> DNA
<213> Artificial Sequence

<220>
<223> Description of Artificial Sequence: Primer

<400> 29
ccatcaaayt cytgkgcrgt gtt                                           23


<210> 30
<211> 484
<212> DNA
<213> Arabidopsis thaliana

<220>
<221> CDS
<222> (2)..(112)

<400> 30
t gca cgc cct gat gaa gct gtg tct tgg ggt aaa att agg ggt tct gct 49
  Ala Arg Pro Asp Glu Ala Val Ser Trp Gly Lys Ile Arg Gly Ser Ala
    1               5                   10                  15

aaa acc gtt aag gtc tgc ttt tta att tct tca cat cct aat tta tat   97
Lys Thr Val Lys Val Cys Phe Leu Ile Ser Ser His Pro Asn Leu Tyr
            20                  25                  30

ctc act cag tgg ttt tgagtacata tttaatattg gatcattctt gcaggtatac   152
Leu Thr Gln Trp Phe
        35

tgtgatgcta ccatagcctt cccattgttg gttgcagaaa catttgccac aaagagagac 212

caaacctgtg agtctaagac ttaagaactg actggtcgtt ttggccatgg attcttaaag 272

atcgttgctt tttgatttta cactggagtg accatataac actccacatt gatgtggctg 332

tgacgcgaat tgtcttcttg cgaattgtac tttagtttct ctcaacctaa aatgatttgc 392

agattgtgtt ttcgtttaaa acacaagagt cttgtagtca ataatccttt gccttataaa 452

attattcagt tccaacaaaa aaaaaaaaaa aa                                484




<210> 31
<211> 559

<212> DNA
<213> Lycopersicon esculentum

<220>
<221> CDS
<222> (1)..(156)

<220>
<221> modified_base
<222> (542)
<223> a, t, c or g

<400> 31
ggt gct cgt cct gat gaa gct gta tca tgg gga aag ata cgt ggt ggt    48
Gly Ala Arg Pro Asp Glu Ala Val Ser Trp Gly Lys Ile Arg Gly Gly
  1               5                  10                  15

gcc aag act gtg aag gtg cat tgt gat gca acc att gca ttt ccc ata    96
Ala Lys Thr Val Lys Val His Cys Asp Ala Thr Ile Ala Phe Pro Ile
             20                  25                  30

tta gta gct gag aca ttt gca gct aag agt aag gaa ttc tcc cag ata    144
Leu Val Ala Glu Thr Phe Ala Ala Lys Ser Lys Glu Phe Ser Gln Ile
         35                  40                  45

agg tgc caa gtt tgaacattga ggaagctgtc cttccgacca cacatatgaa        196
Arg Cys Gln Val
     50

ttgctagctt ttgaagccaa cttgctagtg tgcagcacca tttattctgc aaaactgact 256

agagagcagg gtatattcct ctaccccgag ttagacgaca tcctgtatgg ttcaaattaa 316

ttatttttct ccccttcaca ccatgttatt tagttctctt cctcttcgaa agtgaagagc 376

ttagatgttc ataggttttg aattatgttg gaggttggtg ataactgact agtcctctta 436

ccatatagat aatgtatcct tgtactatga gattttgggt gtgtttgata ccaaggaaaa 496

atgtttattt ggaaaacaat tggattttta atttaaaaaa aattgnttaa aaaaaaaaaa 556

aaa                                                               559

<210> 32
<211> 22
<212> PRT
<213> Artificial Sequence

<220>
<223> Description of Artificial Sequence: Conserved
      peptide fragment

<400> 32
Cys Lys Val Val Glu Val Ser Thr Ser Lys Thr Gly Lys His Gly His
  1               5                  10                  15

Ala Lys Cys His Phe Val
             20

<210> 33
<211> 29
<212> DNA
<213> Artificial Sequence

<220>
<223> Description of Artificial Sequence: Primer

<400> 33
gagctcaaga ataacatctc ataagaaac                                    29


<210> 34
<211> 27
<212> DNA
<213> Artificial Sequence

<220>
<223> Description of Artificial Sequence: Primer

<400> 34
ctcgagtgct cacttctctc tcttagg                                      27


<210> 35
<211> 12
<212> PRT
<213> Artificial Sequence

<220>
<223> Description of Artificial Sequence: Synthetic
      peptide

<400> 35
Cys Asn Asp Asp Thr Leu Leu Gln Gln Ile Lys Ser
  1               5                  10


<210> 36
<211> 12
<212> PRT
<213> Artificial Sequence

<220>
<223> Description of Artificial Sequence: Synthetic
      peptide

<400> 36
Cys Thr Asp Asp Gly Leu Thr Ala Gln Met Arg Leu
  1               5                  10


<210> 37
<211> 12
<212> PRT
<213> Artificial Sequence

<220>
<223> Description of Artificial Sequence: Synthetic

peptide

<400> 37
Cys Thr Asp Glu Ala Leu Leu Thr Gln Leu Lys Asn
1               5                   10


<210> 38
<211> 32
<212> DNA
<213> Artificial Sequence

<220>
<223> Description of Artificial Sequence: Primer

<400> 38
aagcttgatc gtggtcaact tcctctgtta cc                                32


<210> 39
<211> 27
<212> DNA
<213> Artificial Sequence

<220>
<223> Description of Artificial Sequence: Primer

<400> 39
gagctcagaa gaagtataaa aaccatc                                      27


<210> 40
<211> 27
<212> DNA
<213> Artificial Sequence

<220>
<223> Description of Artificial Sequence: Primer

<400> 40
ctcgagtgct cacttctctc tcttagg                                      27


<210> 41
<211> 29
<212> DNA
<213> Artificial Sequence

<220>
<223> Description of Artificial Sequence: Primer

<400> 41
gagctcaaga ataacatctc ataagaaac                                    29


<210> 42
<211> 30
<212> DNA
<213> Artificial Sequence

<220>

<223> Description of Artificial Sequence: Primer

<400> 42
ctcgagctaa actccattcg ctgacttcgc                                        30


<210> 43
<211> 29
<212> DNA
<213> Artificial Sequence

<220>
<223> Description of Artificial Sequence: Primer

<400> 43
gagctctagt aaatataaga gtgtcttgc                                         29



<210> 44
<211> 353
<212> PRT
<213> Unknown Organism

<220>
<223> Description of Unknown Organism: Fungi sequence

<400> 44
Met Ala Asp Asn Gln Ile Pro Ser Ser Val Ala Asp Ala Val Leu Val
  1               5                  10                  15

Lys Ser Ile Glu Met Pro Glu Gly Ser Gln Lys Val Glu Glu Leu Asp
             20                  25                  30

Phe Asn Lys Phe Lys Gly Arg Pro Ile Thr Val Asp Asp Leu Leu Gln
         35                  40                  45

Gly Met Lys His Met Gly Phe Gln Ala Ser Ser Met Cys Glu Ala Val
     50                  55                  60

Arg Ile Ile Asn Glu Met Arg Ala Tyr Arg Asp Pro Thr Thr Ser Glu
 65                  70                  75                  80

Lys Thr Thr Ile Phe Leu Gly Tyr Thr Ser Asn Leu Ile Ser Ser Gly
                 85                  90                  95

Leu Arg Gly Thr Leu Arg Tyr Leu Val Gln His Lys His Val Ser Ala
            100                 105                 110

Ile Val Thr Thr Ala Gly Gly Ile Glu Glu Asp Phe Ile Lys Cys Leu
        115                 120                 125

Gly Asp Thr Tyr Met Ser Ser Phe Ser Ala Val Gly Ala Asp Leu Arg
    130                 135                 140

Ser Lys Gly Leu Asn Arg Ile Gly Asn Leu Val Val Pro Asn Ser Asn
145                 150                 155                 160

Tyr Cys Ala Phe Glu Asp Trp Val Val Pro Ile Leu Asp Lys Met Leu
                165                 170                 175

Glu Glu Gln Glu Ala Ser Arg Gly Thr Glu Asn Glu Ile Asn Trp Thr
            180                 185                 190

Pro Ser Lys Val Ile His Arg Leu Gly Lys Glu Ile Asn Asp Glu Arg
        195                 200                 205

Ser Val Tyr Tyr Trp Ala Trp Lys Asn Asp Ile Pro Val Phe Cys Pro
    210                 215                 220

Ala Leu Thr Asp Gly Ser Leu Gly Asp Met Leu Tyr Phe His Thr Phe
225                 230                 235                 240

Lys Ala Ser Pro Glu Gln Leu Arg Ile Asp Ile Val Glu Asp Ile Arg
                245                 250                 255

Lys Ile Asn Thr Ile Ala Val Arg Ala Lys Arg Ala Gly Met Ile Ile
            260                 265                 270

Leu Gly Gly Gly Ile Val Lys His His Ile Ala Asn Ala Cys Leu Met
        275                 280                 285

Arg Asn Gly Ala Glu Ser Ala Val Tyr Ile Asn Thr Ala Gln Glu Phe
    290                 295                 300

Asp Gly Ser Asp Ala Gly Ala Arg Pro Asp Glu Ala Val Ser Trp Gly
305                 310                 315                 320

Lys Ile Lys Val Gly Ala Asp Ala Val Lys Val Tyr Met Glu Ala Thr
                325                 330                 335

Ala Ala Phe Pro Phe Ile Val Ala Asn Thr Phe Ala Lys Glu Asp Gly
            340                 345                 350

Leu

<210> 45
<211> 387
<212> PRT
<213> Unknown Organism

<220>
<223> Description of Unknown Organism: Yeast sequence

<400> 45
Met Ser Asp Ile Asn Glu Lys Leu Pro Glu Leu Leu Gln Asp Ala Val
  1               5                  10                  15

Leu Lys Ala Ser Val Pro Ile Pro Asp Asp Phe Val Lys Val Gln Gly
             20                  25                  30

Ile Asp Tyr Ser Lys Pro Glu Ala Thr Asn Met Arg Ala Thr Asp Leu
         35                  40                  45

Ile Glu Ala Met Lys Thr Met Gly Phe Gln Ala Ser Ser Val Gly Thr
     50                  55                  60

Ala Cys Glu Ile Ile Asp Ser Met Arg Ser Trp Arg Gly Lys His Ile
 65                  70                  75                  80

Asp Glu Leu Asp Asp His Glu Lys Lys Gly Cys Phe Asp Glu Glu Gly
                85                  90                  95

Tyr Gln Lys Thr Thr Ile Phe Met Gly Tyr Thr Ser Asn Leu Ile Ser
            100                 105                 110

Ser Gly Val Arg Glu Thr Leu Arg Tyr Leu Val Gln His Lys Met Val
        115                 120                 125

Asp Ala Val Val Thr Ser Ala Gly Gly Val Glu Glu Asp Leu Ile Lys
    130                 135                 140

Cys Leu Ala Pro Thr Tyr Leu Gly Glu Phe Ala Leu Lys Gly Lys Ser
145                 150                 155                 160

Leu Arg Asp Gln Gly Met Asn Arg Ile Gly Asn Leu Leu Val Pro Asn
                165                 170                 175

Asp Asn Tyr Cys Lys Phe Glu Glu Trp Ile Val Pro Ile Leu Asp Lys
            180                 185                 190

Met Leu Glu Glu Gln Asp Glu Tyr Val Lys Lys His Gly Ala Asp Cys
        195                 200                 205

Leu Glu Ala Asn Gln Asp Val Asp Ser Pro Ile Trp Thr Pro Ser Lys
    210                 215                 220

Met Ile Asp Arg Phe Gly Lys Glu Ile Asn Asp Glu Ser Ser Val Leu
225                 230                 235                 240

Tyr Trp Ala His Lys Asn Lys Ile Pro Ile Phe Cys Pro Ser Leu Thr
                245                 250                 255

Asp Gly Ser Ile Gly Asp Met Leu Phe Phe His Thr Phe Lys Ala Ser
            260                 265                 270

Pro Lys Gln Leu Arg Val Asp Ile Val Gly Asp Ile Arg Lys Ile Asn
        275                 280                 285

Ser Met Ser Met Ala Ala Tyr Arg Ala Gly Met Ile Ile Leu Gly Gly
    290                 295                 300

Gly Leu Ile Lys His His Ile Ala Asn Ala Cys Leu Met Arg Asn Gly
305                 310                 315                 320

Ala Asp Tyr Ala Val Tyr Ile Asn Thr Gly Gln Glu Tyr Asp Gly Ser
                325                 330                 335

Asp Ala Gly Ala Arg Pro Asp Glu Ala Val Ser Trp Gly Lys Ile Lys
            340                 345                 350

Ala Glu Ala Lys Ser Val Lys Leu Phe Ala Asp Val Thr Thr Val Leu
        355                 360                 365

Pro Leu Ile Val Ala Ala Thr Phe Ala Ser Gly Lys Pro Ile Lys Lys
    370                 375                 380

Val Lys Asn
385

<210> 46
<211> 370
<212> PRT
<213> Unknown Organism

<220>
<223> Description of Unknown Organism: Archaebacterial
      sequence

<400> 46
Met Arg Asp Ile Lys Asp Asn Pro Ile Arg Arg Gly Ile Ala Glu Gln
  1               5                  10                  15

Ser Glu Ala Met His Pro Gly Tyr Thr Asn Arg Ala Lys Pro Tyr Gly
             20                  25                  30

Cys Lys Arg Asp Pro Lys Asp Ile Val Leu Lys Glu Ser Glu Asp Ile
         35                  40                  45

Glu Gly Ile Ala Ile Glu Gly Pro Trp Leu Glu Asp Asp Ile Ser Leu
     50                  55                  60

Glu Glu Ile Ile Lys Lys Tyr Tyr Leu Lys Ile Gly Phe Gln Ala Ser
 65                  70                  75                  80

His Ile Gly Lys Ala Ile Lys Ile Trp Lys His Ile Glu Glu Lys Arg
                 85                  90                  95

Lys Lys Gly Asp Glu Ile Thr Val Phe Phe Gly Tyr Thr Ser Asn Ile
            100                 105                 110

Val Ser Ser Gly Leu Arg Glu Ile Ile Ala Tyr Leu Val Lys His Lys
        115                 120                 125

Lys Ile Asp Ile Ile Val Thr Thr Ala Gly Gly Val Glu Glu Asp Phe
    130                 135                 140

Ile Lys Cys Leu Lys Pro Phe Ile Leu Gly Asp Trp Glu Val Asp Gly
145                 150                 155                 160

Lys Met Leu Arg Glu Lys Gly Ile Asn Arg Ile Gly Asn Ile Phe Val
                165                 170                 175

Pro Asn Asp Arg Tyr Ile Ala Phe Glu Glu Tyr Met Met Glu Phe Phe
            180                 185                 190

Glu Glu Ile Leu Asn Leu Gln Arg Glu Thr Gly Lys Ile Ile Thr Ala
        195                 200                 205

Ser Glu Phe Cys Tyr Lys Leu Gly Glu Phe Met Asp Lys Lys Leu Lys
    210                 215                 220

Ser Lys Glu Lys Glu Lys Ser Ile Leu Tyr Trp Ala Tyr Lys Asn Asn
225                 230                 235                 240

Ile Pro Ile Phe Cys Pro Ala Ile Thr Asp Gly Ser Ile Gly Asp Met
                245                 250                 255

Leu Tyr Phe Phe Lys Lys Tyr Asn Lys Asp Glu Glu Leu Lys Ile Asp
            260                 265                 270

Val Ala Asn Asp Ile Val Lys Leu Asn Asp Ile Ala Ile Asn Ser Lys
        275                 280                 285

Glu Thr Ala Cys Ile Val Leu Gly Gly Ser Leu Pro Lys His Ser Ile
    290                 295                 300

Ile Asn Ala Asn Leu Phe Arg Glu Gly Thr Asp Tyr Ala Ile Tyr Val
305                 310                 315                 320

Thr Thr Ala Leu Pro Trp Asp Gly Ser Leu Ser Gly Ala Pro Pro Glu
                325                 330                 335

Glu Gly Val Ser Trp Gly Lys Ile Gly Ala Lys Ala Asp Tyr Val Glu
            340                 345                 350

Ile Trp Gly Asp Ala Thr Ile Ile Phe Pro Leu Leu Val Tyr Cys Val
        355                 360                 365

Met Lys
    370

<210> 47
<211> 23
<212> DNA
<213> Artificial Sequence

<220>
<223> Description of Artificial Sequence: Primer

<220>
<221> modified_base
<222> (3)
<223> a, t, c or g

<220>
<221> modified_base
<222> (15)
<223> a, t, c or g

<400> 47
gcngarttyg ayggntccga yca                                          23

<210> 48
<211> 30
<212> DNA
<213> Artificial Sequence

<220>
<223> Description of Artificial Sequence: Primer

<400> 48
ccgagctcct gttaccaaaa aatctgtacc                                   30

<210> 49

<211> 36
<212> DNA
<213> Artificial Sequence

<220>
<223> Description of Artificial Sequence: Primer

<400> 49
acctcgagcg gccgcagaag aagtataaaa accatc 36


<210> 50
<211> 30
<212> DNA
<213> Artificial Sequence

<220>
<223> Description of Artificial Sequence: Primer

<400> 50
cgtcgacgat atctcttttt atattcaaac 30


<210> 51
<211> 32
<212> DNA
<213> Artificial Sequence

<220>
<223> Description of Artificial Sequence: Primer

<400> 51
cgtctagaca ttgttttagg aaagttaaat ga 32


<210> 52
<211> 1235
<212> DNA
<213> Arabidopsis thaliana

<400> 52
accctagatc gctttcttca gtgttctata aaaactaaac tccattcgct gacttcgcaa 60
agaagaacac tttctctctg aaatctcaaa ttcatctctt ctcttccgat ttcgctgaat 120
catgtcagac gacgagcatc acttcgaatc cagcgacgcc ggagcttcta agacttatcc 180
tcaacaagcc ggtaacattc gtaaaggtgg tcacatcgtc atcaagggac gtccctgcaa 240
ggttttgtct ctgatttgat tattattgat tttagaggaa tcatcttcat ggattgtatt 300
aaagcagtgt tccgttacct gatcgttgtg aatttttgag gtttagtgat tctggattgt 360
gatctggtgt ttagtgttga gaaaaacctc tgtttttgaa gtttatggat ttatagggtt 420
tttaaatcta taatagggtt taattcaatt ggtgatatgt ggggtttatg atataggtgg 480
ttgaggtatc gacttcgaag actgggaagc atggtcacgc caagtgtcac tttgttgcca 540
ttgatatctt tacttctaag aagcttgaag atatcgttcc ttcttcccac aattgtgatg 600
tgagtcttgt gaatggatta gaaacgttat acaaagtcta taatttttga ctcacaacac 660
aaaactgttt cctttttatt ggcacaggtt ccacatgtga atcgtgttga ttatcagttg 720
attgatatct ctgaagatgg ctttgtatgt catcttcttt ttcactagtt cagctttgtg 780
ttttgtcttt gcccatatgg ttgaattaga gggttttgtt ctttgattac atttacaggt 840
tagtcttctt actgataatg gtagcactaa ggatgatctg aagctgccaa cagatgaagc 900
tttactcaca cagctcaaga atggatttga ggagggtaag gatattgttg tgtctgtcat 960
gtctgcaatg ggagaggagc agatgtgtgc tctcaaggaa gttggtccca agtaataata 1020
ataagtaagc attctctctt ttacagaggc tatgtattat caagtttgac agagtcaaat 1080
gttataagaa caaagtttgg tccttttttt tggtcttctt agtataattt aagcccacat 1140
gtgtttccca tgcaagacac tcttatattt actagtatat cttactattg gttttggttg 1200

tggagaagtt actgttgaca gttccaaacc tctac 1235

<210> 53
<211> 161
<212> PRT
<213> Mycosphaerella fijiensis

<400> 53
Gly Leu Asn Arg Ile Gly Asn Phe Leu Val Pro Asn Asp Asn Tyr Cys
1               5                   10                  15

Arg Phe Glu Asp Trp Val Met Pro Ile Leu Asp Thr Met Leu Glu Glu
            20                  25                  30

Gln Glu Ala Cys Lys Gly Ser Gly Glu Ala Ile His Trp Thr Pro Ser
        35                  40                  45

Lys Ile Ile Asn Arg Leu Gly Lys Glu Val Asn Asp Glu Ser Ser Val
    50                  55                  60

Tyr Tyr Trp Ala Trp Lys Asn Asp Ile Pro Val Phe Cys Pro Ala Leu
65                  70                  75                  80

Thr Asp Gly Ser Leu Gly Asp Met Leu Tyr Phe His Thr Phe Lys Ser
                85                  90                  95

Ser Pro Gln Gln Leu Arg Val Asp Ile Val Glu Asp Ile Arg Lys Ile
            100                 105                 110

Asn Thr Leu Ala Val Arg Ala Lys Arg Thr Gly Met Ile Ile Leu Gly
        115                 120                 125

Gly Gly Ile Val Lys His His Ile Ala Asn Ala Asn Leu Met Arg Asn
    130                 135                 140

Gly Ala Glu Ser Ala Val Tyr Ile Asn Thr Ala Glu Phe Asp Gly Ser
145                 150                 155                 160

Asp

<210> 54
<211> 861
<212> DNA
<213> Arabidopsis sp.

<400> 54
tttttccctt ctcccaatct catcttctcc gaaaaccttt cttctctcaa atttctgtga 60
aaacatgtct gacgacgagc accactttga ggccagcgaa tccggagctt ccaagaccta 120
tcctcaatca gccggtaaca tccgtaaagg tggtcacatc gtcatcaaaa accgtccctg 180
caaggttgtt gaggtttcga cttccaaaac tggcaagcac ggtcacgcca aatgtcactt 240
tgttgctatt gatatcttca ctgctaagaa gcttgaagat attgttccat cttcccacaa 300
ttgtgatgtt ccacatgtga accgtgttga ttaccagttg attgatatca ctgaggatgg 360
cttcgtgagc cttctcactg acagtggtgg caccaaggat gatctcaagc ttcccaccga 420
tgatggtctc accgcccaga tgaggcttgg attcgatgag ggaaaggata ttgtggtgtc 480
tgtcatgtct tccatgggag aggagcagat ctgtgccgtc aaggaagttg gtggtggcaa 540

gtaaacaagt atcattcgat atattattac cagtttgaca acggacgtca atgttataag 600
aaccaaaaga tgtttttctt tttcctaatt tagacccttt gtgtgtgttt cttgttgcaa 660
gacaaccata tctattggtt ttggattgtt ggaaaagttt gtgttgaaac attcaagttt 720
tcttatgaga tgttattctt aaaaccactt tttgtttgtt cactggatat gtttgttcat 780
gaagcttgtt ttaagcaact ctttacatga tattcattgc tatttgcacg attcaagagt 840
gaaatataca ttttatttaa c 861

<210> 55
<211> 159
<212> PRT
<213> Arabidopsis thaliana

<400> 55
Met Ser Asp Asp Glu His His Phe Glu Ala Ser Glu Ser Gly Ala Ser
1               5                   10                  15

Lys Thr Tyr Pro Gln Ser Ala Gly Asn Ile Arg Lys Gly Gly His Ile
            20                  25                  30

Val Ile Lys Asn Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ala Lys Lys Leu Glu Asp Ile Val Pro Ser Ser His Asn Cys
65                  70                  75                  80

Asp Val Pro His Val Asn Arg Val Asp Tyr Gln Leu Ile Asp Ile Thr
                85                  90                  95

Glu Asp Gly Phe Val Ser Leu Leu Thr Asp Ser Gly Gly Thr Lys Asp
            100                 105                 110

Asp Leu Lys Leu Pro Thr Asp Asp Gly Leu Thr Ala Gln Met Arg Leu
        115                 120                 125

Gly Phe Asp Glu Gly Lys Asp Ile Val Val Ser Val Met Ser Ser Met
    130                 135                 140

Gly Glu Glu Gln Ile Cys Ala Val Lys Glu Val Gly Gly Gly Lys
145                 150                 155

<210> 56
<211> 698
<212> DNA
<213> Lycopersicon esculentum

<220>
<221> CDS
<222> (52)..(528)

<400> 56
cttcctgaat ttttctcctt ctccttctcc gttcaatcga atttttcagc c atg tct 57
Met Ser
1

gac gag gag cat caa ttt gag tct aag gct gat gcc gga gca tca aaa 105
Asp Glu Glu His Gln Phe Glu Ser Lys Ala Asp Ala Gly Ala Ser Lys

5                   10                  15

act tac cct caa caa gct ggt act att cgt aag aac ggt tat atc gtc    153
Thr Tyr Pro Gln Gln Ala Gly Thr Ile Arg Lys Asn Gly Tyr Ile Val
     20                  25                  30

atc aaa ggc cgt cca tgc aag gtt gtg gaa gtc tct aca tcc aaa act    201
Ile Lys Gly Arg Pro Cys Lys Val Val Glu Val Ser Thr Ser Lys Thr
 35                  40                  45                  50

ggc aag cac ggt cac gcc aaa tgt cat ttc gtt gct att gac atc ttc    249
Gly Lys His Gly His Ala Lys Cys His Phe Val Ala Ile Asp Ile Phe
                 55                  60                  65

act ggg aag aag ctt gag gat att gtc ccc tct tca cac aat tgt gat    297
Thr Gly Lys Lys Leu Glu Asp Ile Val Pro Ser Ser His Asn Cys Asp
             70                  75                  80

gtg ccc cat gtt aat cgt aca gat tat cag ctt att gac atc tct gaa    345
Val Pro His Val Asn Arg Thr Asp Tyr Gln Leu Ile Asp Ile Ser Glu
         85                  90                  95

gat gga ttt gtg agt ctg ctt act gac aat ggt aac acc aag gat gac    393
Asp Gly Phe Val Ser Leu Leu Thr Asp Asn Gly Asn Thr Lys Asp Asp
    100                 105                 110

ctc agg ctt cct act gat gaa aat ctg ctt tca ctg atc aag gac ggg    441
Leu Arg Leu Pro Thr Asp Glu Asn Leu Leu Ser Leu Ile Lys Asp Gly
115                 120                 125                 130

ttt gcc gag ggt aag gac ctc gtt gtg tct gtt atg tca gct atg ggt    489
Phe Ala Glu Gly Lys Asp Leu Val Val Ser Val Met Ser Ala Met Gly
                135                 140                 145

gag gaa cag att aat gct ttg aag gat att ggc ccc aag tgatctcttg    538
Glu Glu Gln Ile Asn Ala Leu Lys Asp Ile Gly Pro Lys
            150                 155

attggatgga ttgcttgacg cgatggttct ttacgacctt gagtgagata gatatttata 598

gtcatggaaa aaaattgtga tcttatggaa tattcgtatc atgatttatg gaccattgtg 658

agttagattt ttatttatgt tgttttaaat tgtggtattc                       698

<210> 57
<211> 159
<212> PRT
<213> Lycopersicon esculentum

<400> 57
Met Ser Asp Glu Glu His Gln Phe Glu Ser Lys Ala Asp Ala Gly Ala
  1               5                  10                  15

Ser Lys Thr Tyr Pro Gln Gln Ala Gly Thr Ile Arg Lys Asn Gly Tyr
             20                  25                  30

Ile Val Ile Lys Gly Arg Pro Cys Lys Val Val Glu Val Ser Thr Ser
         35                  40                  45

Lys Thr Gly Lys His Gly His Ala Lys Cys His Phe Val Ala Ile Asp

50                  55                  60

Ile Phe Thr Gly Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Asp Asn Gly Asn Thr Lys
            100                 105                 110

Asp Asp Leu Arg Leu Pro Thr Asp Glu Asn Leu Leu Ser Leu Ile Lys
        115                 120                 125

Asp Gly Phe Ala Glu Gly Lys Asp Leu Val Val Ser Val Met Ser Ala
    130                 135                 140

Met Gly Glu Glu Gln Ile Asn Ala Leu Lys Asp Ile Gly Pro Lys
145                 150                 155

<210> 58
<211> 158
<212> PRT
<213> Arabidopsis thaliana

<400> 58
Met Ser Asp Glu Glu His His Phe Glu Ser Ser Asp Ala Gly Ala Ser
1               5                   10                  15

Lys Thr Tyr Pro Gln Gln Ala Gly Thr Ile Arg Lys Asn Gly Tyr Ile
            20                  25                  30

Val Ile Lys Asn Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ser Lys Lys Leu Glu Asp Ile Val Pro Ser Ser His Asn Cys
65                  70                  75                  80

Asp Val Pro His Val Asn Arg Thr Asp Tyr Gln Leu Ile Asp Ile Ser
                85                  90                  95

Glu Asp Gly Tyr Val Ser Leu Leu Thr Asp Asn Gly Ser Thr Lys Asp
            100                 105                 110

Asp Leu Lys Leu Pro Asn Asp Asp Thr Leu Leu Gln Gln Ile Lys Ser
        115                 120                 125

Gly Phe Asp Asp Gly Lys Asp Leu Val Val Ser Val Met Ser Ala Met
    130                 135                 140

Gly Glu Glu Gln Ile Asn Ala Leu Lys Asp Ile Gly Pro Lys
145                 150                 155

<210> 59
<211> 159

<212> PRT
<213> Arabidopsis thaliana

<400> 59
Met Ser Asp Asp Glu His His Phe Glu Ala Ser Glu Ser Gly Ala Ser
1               5                   10                  15

Lys Thr Tyr Pro Gln Ser Ala Gly Asn Ile Arg Lys Gly Gly His Ile
            20                  25                  30

Val Ile Lys Asn Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ala Lys Lys Leu Glu Asp Ile Val Pro Ser Ser His Asn Cys
65                  70                  75                  80

Asp Val Pro His Val Asn Arg Val Asp Tyr Gln Leu Ile Asp Ile Thr
                85                  90                  95

Glu Asp Gly Phe Val Ser Leu Leu Thr Asp Ser Gly Gly Thr Lys Asp
            100                 105                 110

Asp Leu Lys Leu Pro Thr Asp Asp Gly Leu Thr Ala Gln Met Arg Leu
        115                 120                 125

Gly Phe Asp Glu Gly Lys Asp Ile Val Val Ser Val Met Ser Ser Met
    130                 135                 140

Gly Glu Glu Gln Ile Cys Ala Val Lys Glu Val Gly Gly Gly Lys
145                 150                 155


<210> 60
<211> 158
<212> PRT
<213> Arabidopsis thaliana

<400> 60
Met Ser Asp Asp Glu His His Phe Glu Ser Ser Asp Ala Gly Ala Ser
1               5                   10                  15

Lys Thr Tyr Pro Gln Gln Ala Gly Asn Ile Arg Lys Gly Gly His Ile
            20                  25                  30

Val Ile Lys Gly Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ser Lys Lys Leu Glu Asp Ile Val Pro Ser Ser His Asn Cys
65                  70                  75                  80

Asp Val Pro His Val Asn Arg Val Asp Tyr Gln Leu Ile Asp Ile Ser
                85                  90                  95

Glu Asp Gly Phe Val Ser Leu Leu Thr Asp Asn Gly Ser Thr Lys Asp
            100                 105                 110

Asp Leu Lys Leu Pro Thr Asp Glu Ala Leu Leu Thr Gln Leu Lys Asn
        115                 120                 125

Gly Phe Glu Glu Gly Lys Asp Ile Val Val Ser Val Met Ser Ala Met
    130                 135                 140

Gly Glu Glu Gln Met Cys Ala Leu Lys Glu Val Gly Pro Lys
145                 150                 155


<210> 61
<211> 477
<212> DNA
<213> Arabidopsis thaliana

<400> 61
atgtccgacg aggagcatca ctttgagtcc agtgacgccg gagcgtccaa aacctaccct 60
caacaagctg gaaccatccg taagaatggt tacatcgtca tcaaaaatcg tccctgcaag 120
gttgttgagg tttcaacctc gaagactggc aagcatggtc atgctaaatg tcattttgta 180
gctattgata tcttcaccag caagaaactc gaagatattg ttccttcttc ccacaattgt 240
gatgttcctc atgtcaaccg tactgattat cagctgattg acatttctga agatggatat 300
gtcagtttgt tgactgataa cggtagtacc aaggatgacc ttaagctccc taatgatgac 360
actctgctcc aacagatcaa gagtgggttt gatgatggaa aagatctagt ggtgagtgtg 420
atgtcagcta tgggagagga acagatcaat gctcttaagg acatcggtcc caagtga    477


<210> 62
<211> 480
<212> DNA
<213> Arabidopsis thaliana

<400> 62
atgtctgacg acgagcacca ctttgaggcc agcgaatccg gagcttccaa gacctatcct 60
caatcagccg gtaacatccg taaaggtggt cacatcgtca tcaaaaaccg tccctgcaag 120
gttgttgagg tttcgacttc caaaactggc aagcacggtc acgccaaatg tcactttgtt 180
gctattgata tcttcactgc taagaagctt gaagatattg ttccatcttc ccacaattgt 240
gatgttccac atgtgaaccg tgttgattac cagttgattg atatcactga ggatggcttc 300
gtgagccttc tcactgacag tggtggcacc aaggatgatc tcaagcttcc caccgatgat 360
ggtctcaccg cccagatgag gcttggattc gatgagggaa aggatattgt ggtgtctgtc 420
atgtcttcca tgggagagga gcagatctgt gccgtcaagg aagttggtgg tggcaagtaa 480


<210> 63
<211> 477
<212> DNA
<213> Arabidopsis thaliana

<400> 63
atgtcagacg acgagcatca cttcgaatcc agcgacgccg gagcttctaa gacttatcct 60
caacaagccg gtaacattcg taaaggtggt cacatcgtca tcaagggacg tccctgcaag 120
gtggttgagg tatcgacttc gaagactggg aagcatggtc acgccaagtg tcactttgtt 180
gccattgata tctttacttc taagaagctt gaagatatcg ttccttcttc ccacaattgt 240
gatgttccac atgtgaatcg tgttgattat cagttgattg atatctctga agatggcttt 300
gttagtcttc ttactgataa tggtagcact aaggatgatc tgaagctgcc aacagatgaa 360
gctttactca cacagctcaa gaatggattt gaggagggta aggatattgt tgtgtctgtc 420
atgtctgcaa tgggagagga gcagatgtgt gctctcaagg aagttggtcc caagtaa    477


<210> 64
<211> 700

<212> DNA
<213> Lycopersicon esculentum

<220>
<221> CDS
<222> (56)..(532)

<400> 64
aaatttctcc ttctccttaa tcctctccac cggcgaaccg gcgaagatca aaacg atg 58
Met
1

tcg gac gaa gag cac cac ttc gaa tcc aag gcc gat gcc gga gct tca 106
Ser Asp Glu Glu His His Phe Glu Ser Lys Ala Asp Ala Gly Ala Ser
            5                   10                  15

aag acg tat cct caa caa gct ggt act att cgt aaa ggt ggt cac atc 154
Lys Thr Tyr Pro Gln Gln Ala Gly Thr Ile Arg Lys Gly Gly His Ile
        20                  25                  30

gtc ata aaa aat cgt cct tgc aag gtg gtt gaa gtt tca act tcc aag 202
Val Ile Lys Asn Arg Pro Cys Lys Val Val Glu Val Ser Thr Ser Lys
    35                  40                  45

aca ggc aag cac ggt cat gct aaa tgt cac ttc gtg gca att gac att 250
Thr Gly Lys His Gly His Ala Lys Cys His Phe Val Ala Ile Asp Ile
50                  55                  60                  65

ttc act gga aag aaa ctt gag gat att gtt ccc tct tct cac aat tgt 298
Phe Thr Gly Lys Lys Leu Glu Asp Ile Val Pro Ser Ser His Asn Cys
                70                  75                  80

gat gtt cct cat gtg aat agg act gat tat caa ctt att gat atc tct 346
Asp Val Pro His Val Asn Arg Thr Asp Tyr Gln Leu Ile Asp Ile Ser
            85                  90                  95

gag gat ggc ttt gtg agt ctg ttg act gaa aat ggt aac acc aag gat 394
Glu Asp Gly Phe Val Ser Leu Leu Thr Glu Asn Gly Asn Thr Lys Asp
        100                 105                 110

gac ttg aga ctc cca act gat gat act ctt ctg gct cag gtc aaa gat 442
Asp Leu Arg Leu Pro Thr Asp Asp Thr Leu Leu Ala Gln Val Lys Asp
    115                 120                 125

ggt ttt gct gag ggg aaa gac ctg gtt cta tca gtg atg tct gcc atg 490
Gly Phe Ala Glu Gly Lys Asp Leu Val Leu Ser Val Met Ser Ala Met
130                 135                 140                 145

gga gag gag cag att tgt ggt atc aag gac att ggc cct aag 532
Gly Glu Glu Gln Ile Cys Gly Ile Lys Asp Ile Gly Pro Lys
                150                 155

tagctgcaga tggtattggt gtatgtttac agagtttcta taaaagatgt attaagaacc 592

aaaacttctt tactttctct tgcagttgct ctatataact gccatttaac tattattata 652

tgtgttgtga ttagattctt gtctcactac agtatttcct ttactctg 700

<210> 65
<211> 159

<212> PRT
<213> Lycopersicon esculentum

<400> 65
Met Ser Asp Glu Glu His His Phe Glu Ser Lys Ala Asp Ala Gly Ala
1               5                   10                  15

Ser Lys Thr Tyr Pro Gln Gln Ala Gly Thr Ile Arg Lys Gly Gly His
            20                  25                  30

Ile Val Ile Lys Asn Arg Pro Cys Lys Val Val Glu Val Ser Thr Ser
        35                  40                  45

Lys Thr Gly Lys His Gly His Ala Lys Cys His Phe Val Ala Ile Asp
    50                  55                  60

Ile Phe Thr Gly Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Glu Asn Gly Asn Thr Lys
            100                 105                 110

Asp Asp Leu Arg Leu Pro Thr Asp Asp Thr Leu Leu Ala Gln Val Lys
        115                 120                 125

Asp Gly Phe Ala Glu Gly Lys Asp Leu Val Leu Ser Val Met Ser Ala
    130                 135                 140

Met Gly Glu Glu Gln Ile Cys Gly Ile Lys Asp Ile Gly Pro Lys
145                 150                 155

<210> 66
<211> 658
<212> DNA
<213> Brassica napus

<220>
<221> CDS
<222> (1)..(477)

<400> 66
atg tct gac gag gag cac cac ttc gag tcc agc gac gcc gga gct tcc      48
Met Ser Asp Glu Glu His His Phe Glu Ser Ser Asp Ala Gly Ala Ser
1               5                   10                  15

aaa acc tac cct cag cag gct ggt aac atc cgc aag ggt ggt cac atc      96
Lys Thr Tyr Pro Gln Gln Ala Gly Asn Ile Arg Lys Gly Gly His Ile
            20                  25                  30

gtc atc aag ggc cgt ccc tgc aag gtt gtt gag gtt tcg act tcg aag      144
Val Ile Lys Gly Arg Pro Cys Lys Val Val Glu Val Ser Thr Ser Lys
        35                  40                  45

act ggg aag cac ggt cac gca aag tgt cac ttt gtt gct atc gac atc      192
Thr Gly Lys His Gly His Ala Lys Cys His Phe Val Ala Ile Asp Ile
    50                  55                  60

ttc act gct aag aag ctc gag gat att gtt ccc tct tcc cac aat tgt 240
Phe Thr Ala Lys Lys Leu Glu Asp Ile Val Pro Ser Ser His Asn Cys
65                  70                  75                  80

gat gtt ccc cat gtg aac cgt att gac tac cag ttg att gat atc tct 288
Asp Val Pro His Val Asn Arg Ile Asp Tyr Gln Leu Ile Asp Ile Ser
                85                  90                  95

gag aat ggc ttt gtt agc ctt ttg acc gac agt ggt ggc acc aag gac 336
Glu Asn Gly Phe Val Ser Leu Leu Thr Asp Ser Gly Gly Thr Lys Asp
            100                 105                 110

gac ctc aag ctt ccc acc gat gat aat ctc agc gct ctg atg aag agt 384
Asp Leu Lys Leu Pro Thr Asp Asp Asn Leu Ser Ala Leu Met Lys Ser
        115                 120                 125

gga ttc gag gag gga aag gat gtg gtg gtg tct gtc atg tct tcc atg 432
Gly Phe Glu Glu Gly Lys Asp Val Val Val Ser Val Met Ser Ser Met
    130                 135                 140

gga gag gag cag atc tgt gcc gtc aag gaa gtt ggt ggt ggc aag 477
Gly Glu Glu Gln Ile Cys Ala Val Lys Glu Val Gly Gly Gly Lys
145                 150                 155

taaaacccat tctctgagag aggataatct tattaccagt ggtcaatgtt ataagaacaa 537

gaacttgttt tttttccttt ttctaattta gatcatttgt gttgtgtttc tttgttgcaa 597

gacaaccatt atctattatt ggttttggat tgtttaaaaa aaaaaaaaaa aaaaaaaaaa 657

a 658

<210> 67
<211> 159
<212> PRT
<213> Brassica napus

<400> 67
Met Ser Asp Glu Glu His His Phe Glu Ser Ser Asp Ala Gly Ala Ser
1               5                   10                  15

Lys Thr Tyr Pro Gln Gln Ala Gly Asn Ile Arg Lys Gly Gly His Ile
            20                  25                  30

Val Ile Lys Gly Arg Pro Cys Lys Val Val Glu Val Ser Thr Ser Lys
        35                  40                  45

Thr Gly Lys His Gly His Ala Lys Cys His Phe Val Ala Ile Asp Ile
    50                  55                  60

Phe Thr Ala Lys Lys Leu Glu Asp Ile Val Pro Ser Ser His Asn Cys
65                  70                  75                  80

Asp Val Pro His Val Asn Arg Ile Asp Tyr Gln Leu Ile Asp Ile Ser
                85                  90                  95

Glu Asn Gly Phe Val Ser Leu Leu Thr Asp Ser Gly Gly Thr Lys Asp
            100                 105                 110

Asp Leu Lys Leu Pro Thr Asp Asp Asn Leu Ser Ala Leu Met Lys Ser
        115                 120                 125

Gly Phe Glu Glu Gly Lys Asp Val Val Val Ser Val Met Ser Ser Met
    130                 135                 140

Gly Glu Glu Gln Ile Cys Ala Val Lys Glu Val Gly Gly Gly Lys
145                 150                 155

<210> 68
<211> 8
<212> PRT
<213> Mycosphaerella fijiensis

<400> 68
Gly Leu Asn Arg Ile Gly Asn Leu
  1               5

<210> 69
<211> 8
<212> PRT
<213> Mycosphaerella fijiensis

<400> 69
Ala Glu Phe Asp Gly Ser Asp Gln
  1               5

<210> 70
<211> 1335
<212> DNA
<213> Brassica napus

<220>
<221> CDS
<222> (57)..(1160)

<400> 70
cttgctagaa ccctaaaact ccctcccaaa actctccaca tcttccgaga aagaag atg 59
                                                              Met
                                                                1

gag gag gat cgt gtt ctc tcg tct gtc cac tca acg gtc ttc aag gaa    107
Glu Glu Asp Arg Val Leu Ser Ser Val His Ser Thr Val Phe Lys Glu
            5                   10                  15

tcc gaa tcg ttg gaa gga aag tgc gac aaa atc gaa gga tac gat ttc    155
Ser Glu Ser Leu Glu Gly Lys Cys Asp Lys Ile Glu Gly Tyr Asp Phe
        20                  25                  30

aac caa gga gta aac tac ccg aag ctc ctc cga tcc atg ctc aca acc    203
Asn Gln Gly Val Asn Tyr Pro Lys Leu Leu Arg Ser Met Leu Thr Thr
    35                  40                  45

ggc ttc caa gcc tca aac ctc ggc gac gta att gat gtc gtt aat caa    251
Gly Phe Gln Ala Ser Asn Leu Gly Asp Val Ile Asp Val Val Asn Gln
50                  55                  60                  65

atg cta gag tgg aga ctc tct gat gaa act ata gca cct gaa gac tgt 299
Met Leu Glu Trp Arg Leu Ser Asp Glu Thr Ile Ala Pro Glu Asp Cys
                70                  75                  80

agt gaa gag gag aag gat cca gcg tat aga gag tcc gtg aag tgt aaa 347
Ser Glu Glu Glu Lys Asp Pro Ala Tyr Arg Glu Ser Val Lys Cys Lys
            85                  90                  95

atc ttt cta ggc ttc act tcg aat ctt gtt tcc tct ggt gtt aga gag 395
Ile Phe Leu Gly Phe Thr Ser Asn Leu Val Ser Ser Gly Val Arg Glu
        100                 105                 110

act att cga tac ctt gtt cag cat cat atg gtt gat gtt ata gtt act 443
Thr Ile Arg Tyr Leu Val Gln His His Met Val Asp Val Ile Val Thr
    115                 120                 125

aca act ggt ggc gta gag gaa gat ctc atc aaa tgc ctt gct cct act 491
Thr Thr Gly Gly Val Glu Glu Asp Leu Ile Lys Cys Leu Ala Pro Thr
130                 135                 140                 145

ttc aaa ggt gat ttc tct cta ccg ggt gcg tat ctt cgg tca aag gga 539
Phe Lys Gly Asp Phe Ser Leu Pro Gly Ala Tyr Leu Arg Ser Lys Gly
                150                 155                 160

ttg aac cgg atc ggg aac ttg ctt gtt cct aat gat aac tac tgc aag 587
Leu Asn Arg Ile Gly Asn Leu Leu Val Pro Asn Asp Asn Tyr Cys Lys
            165                 170                 175

ttt gag gat tgg atc att ccc atc ttt gac cag atg ttg aag gaa cag 635
Phe Glu Asp Trp Ile Ile Pro Ile Phe Asp Gln Met Leu Lys Glu Gln
        180                 185                 190

aaa gaa gag agt gtg ttg tgg acg cct tct aaa ttg tta gcg cgg ctg 683
Lys Glu Glu Ser Val Leu Trp Thr Pro Ser Lys Leu Leu Ala Arg Leu
    195                 200                 205

ggg aaa gaa ata aat aat gag agt tca tat ctt tat tgg gca tac aag 731
Gly Lys Glu Ile Asn Asn Glu Ser Ser Tyr Leu Tyr Trp Ala Tyr Lys
210                 215                 220                 225

atg aat att cca gtg ttc tgc cgg ggg tta acc gat ggc tct ctc ggt 779
Met Asn Ile Pro Val Phe Cys Arg Gly Leu Thr Asp Gly Ser Leu Gly
                230                 235                 240

gat atg ttg tat ttt cac tca ttt cgt acc tct ggc ctt gtc atc gat 827
Asp Met Leu Tyr Phe His Ser Phe Arg Thr Ser Gly Leu Val Ile Asp
            245                 250                 255

gtt gtg caa gat att aga gct atg aac ggt gaa gca gtc cat gcg act 875
Val Val Gln Asp Ile Arg Ala Met Asn Gly Glu Ala Val His Ala Thr
        260                 265                 270

cca aga aag aca ggg atg ata atc ctt gga ggg ggc ttg ccg aag cac 923
Pro Arg Lys Thr Gly Met Ile Ile Leu Gly Gly Gly Leu Pro Lys His
    275                 280                 285

cac ata tgt aat gcc aac atg atg cgt aac ggt gcg gat tac gct gtg 971
His Ile Cys Asn Ala Asn Met Met Arg Asn Gly Ala Asp Tyr Ala Val
290                 295                 300                 305

ttt atc aac acc ggg caa gag ttt gat gga agt gac tcg ggt gca cgc 1019

Phe Ile Asn Thr Gly Gln Glu Phe Asp Gly Ser Asp Ser Gly Ala Arg
                310                 315                 320

cct gat gaa gca gtg tct tgg ggt aaa ata agg gga tct gct aaa act    1067
Pro Asp Glu Ala Val Ser Trp Gly Lys Ile Arg Gly Ser Ala Lys Thr
            325                 330                 335

gtc aag gtg tac tgt gat gct acc ata gcc ttc cct ttg ttg gtt gct    1115
Val Lys Val Tyr Cys Asp Ala Thr Ile Ala Phe Pro Leu Leu Val Ala
        340                 345                 350

gaa aca ttt gcc tcc aag aga gaa caa agc tgt gag cac aag acc        1160
Glu Thr Phe Ala Ser Lys Arg Glu Gln Ser Cys Glu His Lys Thr
    355                 360                 365

taagcccaag aaagcttacg tctcttttat cggtttgttc ttccatcttg ttgttgtacc 1220

ctttgtcctg ctttacataa cattcatctc taaaacaata ctacctcctt ttgacaaaaa 1280

ataaaaaaaa ttggaaaaat ggtttcacaa gaataaaaaa aaaaaaaaaa aaaaa      1335

<210> 71
<211> 368
<212> PRT
<213> Brassica napus

<400> 71
Met Glu Glu Asp Arg Val Leu Ser Ser Val His Ser Thr Val Phe Lys
1               5                   10                  15

Glu Ser Glu Ser Leu Glu Gly Lys Cys Asp Lys Ile Glu Gly Tyr Asp
            20                  25                  30

Phe Asn Gln Gly Val Asn Tyr Pro Lys Leu Leu Arg Ser Met Leu Thr
        35                  40                  45

Thr Gly Phe Gln Ala Ser Asn Leu Gly Asp Val Ile Asp Val Val Asn
    50                  55                  60

Gln Met Leu Glu Trp Arg Leu Ser Asp Glu Thr Ile Ala Pro Glu Asp
65                  70                  75                  80

Cys Ser Glu Glu Glu Lys Asp Pro Ala Tyr Arg Glu Ser Val Lys Cys
                85                  90                  95

Lys Ile Phe Leu Gly Phe Thr Ser Asn Leu Val Ser Ser Gly Val Arg
            100                 105                 110

Glu Thr Ile Arg Tyr Leu Val Gln His His Met Val Asp Val Ile Val
        115                 120                 125

Thr Thr Thr Gly Gly Val Glu Glu Asp Leu Ile Lys Cys Leu Ala Pro
    130                 135                 140

Thr Phe Lys Gly Asp Phe Ser Leu Pro Gly Ala Tyr Leu Arg Ser Lys
145                 150                 155                 160

Gly Leu Asn Arg Ile Gly Asn Leu Leu Val Pro Asn Asp Asn Tyr Cys
                165                 170                 175

Lys Phe Glu Asp Trp Ile Ile Pro Ile Phe Asp Gln Met Leu Lys Glu
            180                 185                 190

Gln Lys Glu Glu Ser Val Leu Trp Thr Pro Ser Lys Leu Leu Ala Arg
        195                 200                 205

Leu Gly Lys Glu Ile Asn Asn Glu Ser Ser Tyr Leu Tyr Trp Ala Tyr
    210                 215                 220

Lys Met Asn Ile Pro Val Phe Cys Arg Gly Leu Thr Asp Gly Ser Leu
225                 230                 235                 240

Gly Asp Met Leu Tyr Phe His Ser Phe Arg Thr Ser Gly Leu Val Ile
                245                 250                 255

Asp Val Val Gln Asp Ile Arg Ala Met Asn Gly Glu Ala Val His Ala
            260                 265                 270

Thr Pro Arg Lys Thr Gly Met Ile Ile Leu Gly Gly Gly Leu Pro Lys
        275                 280                 285

His His Ile Cys Asn Ala Asn Met Met Arg Asn Gly Ala Asp Tyr Ala
    290                 295                 300

Val Phe Ile Asn Thr Gly Gln Glu Phe Asp Gly Ser Asp Ser Gly Ala
305                 310                 315                 320

Arg Pro Asp Glu Ala Val Ser Trp Gly Lys Ile Arg Gly Ser Ala Lys
                325                 330                 335

Thr Val Lys Val Tyr Cys Asp Ala Thr Ile Ala Phe Pro Leu Leu Val
            340                 345                 350

Ala Glu Thr Phe Ala Ser Lys Arg Glu Gln Ser Cys Glu His Lys Thr
        355                 360                 365

<210> 72
<211> 1862
<212> DNA
<213> Medicago sativa

<220>
<221> CDS
<222> (293)..(1396)

<400> 72
gaaaccttct tcttctggag caaagtcgcc attccctacc tccttcttca ttcttattct 60

ctataacaaa cggtccgacc ggatccaagt tgcaccggtt cgaaccgctt tagttactac 120

taacggttcg aaccgttatt tttcaacccg tgacaaacgt ggaaggcttc gttgtttctt 180

cttcttcttc ttaattacca tgcgtttttg tttttctttt gagtcattga agtcttgttt 240

tttgtcgtgt ttctgtcttg agaccgtgaa agagaaaaca aagagtacga ga atg agt 298
                                                          Met Ser
                                                          1

gaa aca aag caa gaa gat gat aca att atg tcc tca gtt cac tcc act   346

Glu Thr Lys Gln Glu Asp Asp Thr Ile Met Ser Ser Val His Ser Thr
            5                   10                  15

gtc ttc aaa gaa tcc gaa aat ctc gca gga aag tgt gtc caa atc gag     394
Val Phe Lys Glu Ser Glu Asn Leu Ala Gly Lys Cys Val Gln Ile Glu
        20                  25                  30

ggt tat gat ttc aac cgc ggc gtc gat tat caa cag ctt ctc aaa tca     442
Gly Tyr Asp Phe Asn Arg Gly Val Asp Tyr Gln Gln Leu Leu Lys Ser
 35                  40                  45                  50

atg ctc aca act ggt ttt caa gct tcc aac ttt ggt gat gcc gtt aaa     490
Met Leu Thr Thr Gly Phe Gln Ala Ser Asn Phe Gly Asp Ala Val Lys
                 55                  60                  65

gtc gtt aat caa atg cta gat tgg agg ttg gtt gat gaa cca att gat     538
Val Val Asn Gln Met Leu Asp Trp Arg Leu Val Asp Glu Pro Ile Asp
             70                  75                  80

gag gat tgt gat gaa gat aag aag gat ttg gag tat agg aaa tct gtt     586
Glu Asp Cys Asp Glu Asp Lys Lys Asp Leu Glu Tyr Arg Lys Ser Val
         85                  90                  95

aca tgc aaa gtg ttt ttg ggt ttc act tct aat ctt atc tct tct ggt     634
Thr Cys Lys Val Phe Leu Gly Phe Thr Ser Asn Leu Ile Ser Ser Gly
    100                 105                 110

gtt aga gat gtt gtt cgt tac ctt tgt cag cat cac atg gtt cat gta     682
Val Arg Asp Val Val Arg Tyr Leu Cys Gln His His Met Val His Val
115                 120                 125                 130

gtt gtt aca act aca ggt ggt att gaa gaa gat ctt ata aag tgc ctt     730
Val Val Thr Thr Thr Gly Gly Ile Glu Glu Asp Leu Ile Lys Cys Leu
                135                 140                 145

gca cca aca tat aaa gga gag ttc tct ttg ccc gga gct tat ctt cgc     778
Ala Pro Thr Tyr Lys Gly Glu Phe Ser Leu Pro Gly Ala Tyr Leu Arg
            150                 155                 160

tca aaa gga ttg aat cga atc ggt aat tta ttg gtc cct aat gaa aat     826
Ser Lys Gly Leu Asn Arg Ile Gly Asn Leu Leu Val Pro Asn Glu Asn
        165                 170                 175

tat tgc aaa ttt gag gac tgg att att cct att ttt gat caa atg ttg     874
Tyr Cys Lys Phe Glu Asp Trp Ile Ile Pro Ile Phe Asp Gln Met Leu
    180                 185                 190

aag gag caa aag gaa gag aaa gtg ctg tgg aca ccg tct aag tta ata     922
Lys Glu Gln Lys Glu Glu Lys Val Leu Trp Thr Pro Ser Lys Leu Ile
195                 200                 205                 210

gct cga ttg gga aaa gag atc aac aat gaa aac tcc tac ctt tac tgg     970
Ala Arg Leu Gly Lys Glu Ile Asn Asn Glu Asn Ser Tyr Leu Tyr Trp
                215                 220                 225

gca tat aag aac aat att cca gtt tat tgt cca gga tta acc gat ggc     1018
Ala Tyr Lys Asn Asn Ile Pro Val Tyr Cys Pro Gly Leu Thr Asp Gly
            230                 235                 240

tca ctg ggt gac atg ctg tac ttc cat tcc ttc cac aac cct ggt ctg     1066
Ser Leu Gly Asp Met Leu Tyr Phe His Ser Phe His Asn Pro Gly Leu

        245                 250                 255

att gtg gac ata gtg caa gat ata agg gcc atg aat ggt gaa gct gta 1114
Ile Val Asp Ile Val Gln Asp Ile Arg Ala Met Asn Gly Glu Ala Val
    260                 265                 270

cat gca aat cct agc aag acg ggc atg att att tta gga ggc ggc ctt 1162
His Ala Asn Pro Ser Lys Thr Gly Met Ile Ile Leu Gly Gly Gly Leu
275                 280                 285                 290

cca aaa cat cac att tgc aat gcc aat atg atg cgc aat ggt gca gac 1210
Pro Lys His His Ile Cys Asn Ala Asn Met Met Arg Asn Gly Ala Asp
                295                 300                 305

tat gcg gtt ttt att aat act gca caa gaa ttt gat gga agt gat tct 1258
Tyr Ala Val Phe Ile Asn Thr Ala Gln Glu Phe Asp Gly Ser Asp Ser
            310                 315                 320

gga gct cgt cca gat gag gct gtt tca tgg ggg aaa ata cga gga tct 1306
Gly Ala Arg Pro Asp Glu Ala Val Ser Trp Gly Lys Ile Arg Gly Ser
        325                 330                 335

gct aaa act gtt aag gta cat tgt gat gca acg ata gca ttc cct ctg 1354
Ala Lys Thr Val Lys Val His Cys Asp Ala Thr Ile Ala Phe Pro Leu
    340                 345                 350

ctg gtt gct gaa aca ttt gcc tca aga acg tca ccc ctt aat 1396
Leu Val Ala Glu Thr Phe Ala Ser Arg Thr Ser Pro Leu Asn
355                 360                 365

tgataaaggt ccaccgtcaa aagtaaaagg tgtggctggg aagtgtttta ccgcagctcc 1456

acttgtgagt gccaaatgtt ttggtatgta acttataaga ccaaggtcgg ctgtatgtca 1516

tacttgagtt gaggtcaaag ttcatttgca atgcagtgtg tttgaggatc ttgatggacc 1576

agtttgccat tgacttttaa tttgactgtc ttgttattcg caaggtccac ataacaagca 1636

tttttaccat ttagaaacaa tttattagtc ctgaaggaat tgagagtcat gaattcagat 1696

gtaaattatg caatgctaac tatatttttt tggaactgtg gtttctctta gatttgaggt 1756

gttgaaaact gtaatatcta gagcaaataa gactagaaaa gtttatcaac tattactgat 1816

cagttatagt atcttcaata ttttccagaa aaaaaaaaaa aaaaaa 1862

<210> 73
<211> 368
<212> PRT
<213> Medicago sativa

<400> 73
Met Ser Glu Thr Lys Gln Glu Asp Asp Thr Ile Met Ser Ser Val His
1               5                   10                  15

Ser Thr Val Phe Lys Glu Ser Glu Asn Leu Ala Gly Lys Cys Val Gln
            20                  25                  30

Ile Glu Gly Tyr Asp Phe Asn Arg Gly Val Asp Tyr Gln Gln Leu Leu
        35                  40                  45

Lys Ser Met Leu Thr Thr Gly Phe Gln Ala Ser Asn Phe Gly Asp Ala
        50                  55                  60

Val Lys Val Val Asn Gln Met Leu Asp Trp Arg Leu Val Asp Glu Pro
65                  70                  75                  80

Ile Asp Glu Asp Cys Asp Glu Asp Lys Lys Asp Leu Glu Tyr Arg Lys
                85                  90                  95

Ser Val Thr Cys Lys Val Phe Leu Gly Phe Thr Ser Asn Leu Ile Ser
            100                 105                 110

Ser Gly Val Arg Asp Val Val Arg Tyr Leu Cys Gln His His Met Val
        115                 120                 125

His Val Val Val Thr Thr Thr Gly Gly Ile Glu Glu Asp Leu Ile Lys
    130                 135                 140

Cys Leu Ala Pro Thr Tyr Lys Gly Glu Phe Ser Leu Pro Gly Ala Tyr
145                 150                 155                 160

Leu Arg Ser Lys Gly Leu Asn Arg Ile Gly Asn Leu Leu Val Pro Asn
                165                 170                 175

Glu Asn Tyr Cys Lys Phe Glu Asp Trp Ile Ile Pro Ile Phe Asp Gln
            180                 185                 190

Met Leu Lys Glu Gln Lys Glu Glu Lys Val Leu Trp Thr Pro Ser Lys
        195                 200                 205

Leu Ile Ala Arg Leu Gly Lys Glu Ile Asn Asn Glu Asn Ser Tyr Leu
    210                 215                 220

Tyr Trp Ala Tyr Lys Asn Asn Ile Pro Val Tyr Cys Pro Gly Leu Thr
225                 230                 235                 240

Asp Gly Ser Leu Gly Asp Met Leu Tyr Phe His Ser Phe His Asn Pro
                245                 250                 255

Gly Leu Ile Val Asp Ile Val Gln Asp Ile Arg Ala Met Asn Gly Glu
            260                 265                 270

Ala Val His Ala Asn Pro Ser Lys Thr Gly Met Ile Ile Leu Gly Gly
        275                 280                 285

Gly Leu Pro Lys His His Ile Cys Asn Ala Asn Met Met Arg Asn Gly
    290                 295                 300

Ala Asp Tyr Ala Val Phe Ile Asn Thr Ala Gln Glu Phe Asp Gly Ser
305                 310                 315                 320

Asp Ser Gly Ala Arg Pro Asp Glu Ala Val Ser Trp Gly Lys Ile Arg
                325                 330                 335

Gly Ser Ala Lys Thr Val Lys Val His Cys Asp Ala Thr Ile Ala Phe
            340                 345                 350

Pro Leu Leu Val Ala Glu Thr Phe Ala Ser Arg Thr Ser Pro Leu Asn
        355                 360                 365

<210> 74
<211> 1363
<212> DNA
<213> Musa acuminata

<220>
<221> CDS
<222> (93)..(1220)

<400> 74
ggcacgagcg cgcggcgccc gcaacgaata ttgcagagag taagaaggat cctcgccttt 60

gtcaccaaac ccttggtttc cagcgaggcg ac atg gaa ggc ggc gcc gcg gga 113
Met Glu Gly Gly Ala Ala Gly
1               5

ggg cag cga gac cgg gaa acc ctg gac gcg gtg cgg tcg gtg gtg ttt 161
Gly Gln Arg Asp Arg Glu Thr Leu Asp Ala Val Arg Ser Val Val Phe
        10                  15                  20

aag cct tcc gta tcc ttg gag gag aag cgg ttc ccg agg gtc cag ggg 209
Lys Pro Ser Val Ser Leu Glu Glu Lys Arg Phe Pro Arg Val Gln Gly
    25                  30                  35

tac gac ttc aac cgg ggt tgt gac ctc atc ggc ctc ctc gat tcc atc 257
Tyr Asp Phe Asn Arg Gly Cys Asp Leu Ile Gly Leu Leu Asp Ser Ile
40                  45                  50                  55

tcc tct acc ggg ttc caa gct tcc aac ctc ggc gac gcc atc gat gtc 305
Ser Ser Thr Gly Phe Gln Ala Ser Asn Leu Gly Asp Ala Ile Asp Val
                60                  65                  70

atc aat cag atg att gac tgg agg ctc tcc cat gat gcg ccc acg gaa 353
Ile Asn Gln Met Ile Asp Trp Arg Leu Ser His Asp Ala Pro Thr Glu
            75                  80                  85

gat tgc agc gag gaa gag cgc aat ctg gct tac agg caa tcg gtc acg 401
Asp Cys Ser Glu Glu Glu Arg Asn Leu Ala Tyr Arg Gln Ser Val Thr
        90                  95                  100

tgc aag atc ttt ctg ggc ttc act tcg aac ctt gta tct tct ggc atc 449
Cys Lys Ile Phe Leu Gly Phe Thr Ser Asn Leu Val Ser Ser Gly Ile
    105                 110                 115

agg gag ata att cgg ttt ctt gtg cag cac cga atg gtt gaa gtt tta 497
Arg Glu Ile Ile Arg Phe Leu Val Gln His Arg Met Val Glu Val Leu
120                 125                 130                 135

gtc aca act gct ggc ggc att gaa gaa gat tta atc aaa tgc ctt gct 545
Val Thr Thr Ala Gly Gly Ile Glu Glu Asp Leu Ile Lys Cys Leu Ala
                140                 145                 150

cca aca tat aag ggt gac ttt tct ttg cct gga tcg tat ctg cgt tca 593
Pro Thr Tyr Lys Gly Asp Phe Ser Leu Pro Gly Ser Tyr Leu Arg Ser
            155                 160                 165

aaa gga ttg aat cgg ata gga aac ctt ctt gtc cct aat gac aat tac 641
Lys Gly Leu Asn Arg Ile Gly Asn Leu Leu Val Pro Asn Asp Asn Tyr
        170                 175                 180

tgc aaa ttc gag gac tgg atc atg cca att ctg gac cag atg tta ctt 689
Cys Lys Phe Glu Asp Trp Ile Met Pro Ile Leu Asp Gln Met Leu Leu
    185                 190                 195

gaa cag act aca gag aat gta gtt tgg aca cca tct aaa gtg att gcg 737
Glu Gln Thr Thr Glu Asn Val Val Trp Thr Pro Ser Lys Val Ile Ala
200                 205                 210                 215

cgc ctt gga aaa gaa ata aat gat gaa agt tca tac ctg tac tgg gca 785
Arg Leu Gly Lys Glu Ile Asn Asp Glu Ser Ser Tyr Leu Tyr Trp Ala
                220                 225                 230

tac aag aac aat gtt tct gtc tat tgc ccg gca ttg act gat gga tca 833
Tyr Lys Asn Asn Val Ser Val Tyr Cys Pro Ala Leu Thr Asp Gly Ser
            235                 240                 245

ttg ggg gat atg ttg tac tgc cat tca gtg cgg aat cct ggt tta ctt 881
Leu Gly Asp Met Leu Tyr Cys His Ser Val Arg Asn Pro Gly Leu Leu
        250                 255                 260

att gat att gtg caa gac ata cga gca atg aat gga gaa gct gta cat 929
Ile Asp Ile Val Gln Asp Ile Arg Ala Met Asn Gly Glu Ala Val His
    265                 270                 275

gtg ggt ctg aga aag act ggg gtc ata att ctt ggt ggg ggc ctc cca 977
Val Gly Leu Arg Lys Thr Gly Val Ile Ile Leu Gly Gly Gly Leu Pro
280                 285                 290                 295

aag cac cat ata tgt aat gcc aac atg ttt cgg aat ggt gca gat tat 1025
Lys His His Ile Cys Asn Ala Asn Met Phe Arg Asn Gly Ala Asp Tyr
                300                 305                 310

gct gtt tat gtc aac act gca cag gaa ttt gat gga agt gat tct gga 1073
Ala Val Tyr Val Asn Thr Ala Gln Glu Phe Asp Gly Ser Asp Ser Gly
            315                 320                 325

gca gag cct gat gag gcg att tca tgg gga aag ata aaa ggt tct gcg 1121
Ala Glu Pro Asp Glu Ala Ile Ser Trp Gly Lys Ile Lys Gly Ser Ala
        330                 335                 340

aag act att aaa gtt cat tgt gat gca act att gct ttt cct cta ttg 1169
Lys Thr Ile Lys Val His Cys Asp Ala Thr Ile Ala Phe Pro Leu Leu
    345                 350                 355

gta gct gca aca ttt gca aga aag ttt cag gaa aga aac aac aaa tta 1217
Val Ala Ala Thr Phe Ala Arg Lys Phe Gln Glu Arg Asn Asn Lys Leu
360                 365                 370                 375

gcc tgatgggggt gcaaaaggtg atcatcttat ttggattcaa ataccttaat 1270
Ala

gtaatctgct aacatctgca gatgctgtat tcttgcaaac caaaaattta atattagata 1330

accgagagcc tacagagggt cctttcaaaa aaa 1363

<210> 75
<211> 376
<212> PRT
<213> Musa acuminata

<400> 75
Met Glu Gly Gly Ala Ala Gly Gly Gln Arg Asp Arg Glu Thr Leu Asp
1               5                   10                  15

Ala Val Arg Ser Val Val Phe Lys Pro Ser Val Ser Leu Glu Glu Lys
            20                  25                  30

Arg Phe Pro Arg Val Gln Gly Tyr Asp Phe Asn Arg Gly Cys Asp Leu
        35                  40                  45

Ile Gly Leu Leu Asp Ser Ile Ser Ser Thr Gly Phe Gln Ala Ser Asn
    50                  55                  60

Leu Gly Asp Ala Ile Asp Val Ile Asn Gln Met Ile Asp Trp Arg Leu
65                  70                  75                  80

Ser His Asp Ala Pro Thr Glu Asp Cys Ser Glu Glu Glu Arg Asn Leu
                85                  90                  95

Ala Tyr Arg Gln Ser Val Thr Cys Lys Ile Phe Leu Gly Phe Thr Ser
            100                 105                 110

Asn Leu Val Ser Ser Gly Ile Arg Glu Ile Ile Arg Phe Leu Val Gln
        115                 120                 125

His Arg Met Val Glu Val Leu Val Thr Thr Ala Gly Gly Ile Glu Glu
    130                 135                 140

Asp Leu Ile Lys Cys Leu Ala Pro Thr Tyr Lys Gly Asp Phe Ser Leu
145                 150                 155                 160

Pro Gly Ser Tyr Leu Arg Ser Lys Gly Leu Asn Arg Ile Gly Asn Leu
                165                 170                 175

Leu Val Pro Asn Asp Asn Tyr Cys Lys Phe Glu Asp Trp Ile Met Pro
            180                 185                 190

Ile Leu Asp Gln Met Leu Leu Glu Gln Thr Thr Glu Asn Val Val Trp
        195                 200                 205

Thr Pro Ser Lys Val Ile Ala Arg Leu Gly Lys Glu Ile Asn Asp Glu
    210                 215                 220

Ser Ser Tyr Leu Tyr Trp Ala Tyr Lys Asn Asn Val Ser Val Tyr Cys
225                 230                 235                 240

Pro Ala Leu Thr Asp Gly Ser Leu Gly Asp Met Leu Tyr Cys His Ser
                245                 250                 255

Val Arg Asn Pro Gly Leu Leu Ile Asp Ile Val Gln Asp Ile Arg Ala
            260                 265                 270

Met Asn Gly Glu Ala Val His Val Gly Leu Arg Lys Thr Gly Val Ile
        275                 280                 285

Ile Leu Gly Gly Gly Leu Pro Lys His His Ile Cys Asn Ala Asn Met
    290                 295                 300

Phe Arg Asn Gly Ala Asp Tyr Ala Val Tyr Val Asn Thr Ala Gln Glu
305                 310                 315                 320

Phe Asp Gly Ser Asp Ser Gly Ala Glu Pro Asp Glu Ala Ile Ser Trp
                325                 330                 335

Gly Lys Ile Lys Gly Ser Ala Lys Thr Ile Lys Val His Cys Asp Ala
            340                 345                 350

Thr Ile Ala Phe Pro Leu Leu Val Ala Ala Thr Phe Ala Arg Lys Phe
        355                 360                 365

Gln Glu Arg Asn Asn Lys Leu Ala
    370                 375

<210> 76
<211> 1451
<212> DNA
<213> Populus deltoides

<220>
<221> CDS
<222> (8)..(1126)

<400> 76
gggattt atg aca ggc aaa aaa caa tgg gag gaa gat ttg cta tca tca      49
        Met Thr Gly Lys Lys Gln Trp Glu Glu Asp Leu Leu Ser Ser
          1               5                  10

gta cgg acc aca gtg ttt aaa gaa tca gaa gct ctt gat ggg aaa tgc      97
Val Arg Thr Thr Val Phe Lys Glu Ser Glu Ala Leu Asp Gly Lys Cys
 15                  20                  25                  30

att aaa att gaa ggt tat gat ttt aat caa gga gtg aac tac tct aag      145
Ile Lys Ile Glu Gly Tyr Asp Phe Asn Gln Gly Val Asn Tyr Ser Lys
                 35                  40                  45

ctt ctc aaa tcc atg gtc tct acc ggg ttt caa gct tcc aac ctt gga      193
Leu Leu Lys Ser Met Val Ser Thr Gly Phe Gln Ala Ser Asn Leu Gly
             50                  55                  60

gat gcc att caa gtt gtt aat aac atg cta gac tgg agg ctt gct gat      241
Asp Ala Ile Gln Val Val Asn Asn Met Leu Asp Trp Arg Leu Ala Asp
         65                  70                  75

gaa gag ata aca gaa gat tgt agt gat gag gag agg gag ttg gcc tat      289
Glu Glu Ile Thr Glu Asp Cys Ser Asp Glu Glu Arg Glu Leu Ala Tyr
     80                  85                  90

aga gag tct gtg aga tgc aaa ctg ttc ttg ggt ttt aca tca aat ctt      337
Arg Glu Ser Val Arg Cys Lys Leu Phe Leu Gly Phe Thr Ser Asn Leu
 95                 100                 105                 110

gtt tct tca ggt gtc aga gat aca att cga tat ctt gtt cag cat cat      385
Val Ser Ser Gly Val Arg Asp Thr Ile Arg Tyr Leu Val Gln His His
                115                 120                 125

atg gtt gat gta gtg gtt aca acg gca ggt ggc ata gaa gaa gat ctt      433
Met Val Asp Val Val Val Thr Thr Ala Gly Gly Ile Glu Glu Asp Leu
            130                 135                 140

ata aaa tgc ctg gca cca aca tac aaa ggt gac ttt tct cta ccc ggg 481
Ile Lys Cys Leu Ala Pro Thr Tyr Lys Gly Asp Phe Ser Leu Pro Gly
        145                 150                 155

gct caa tta cga tca aaa ggg ttg aat cga att ggt aac ttg ttg gta 529
Ala Gln Leu Arg Ser Lys Gly Leu Asn Arg Ile Gly Asn Leu Leu Val
    160                 165                 170

cct aat gac aac tac tgc aaa ttt gag gat tgg atc att cca atc ttt 577
Pro Asn Asp Asn Tyr Cys Lys Phe Glu Asp Trp Ile Ile Pro Ile Phe
175                 180                 185                 190

gac caa atg ttg aag gaa caa att gaa gag aat atc acc tgg aca cct 625
Asp Gln Met Leu Lys Glu Gln Ile Glu Glu Asn Ile Thr Trp Thr Pro
                195                 200                 205

tct aaa tta ata gct cgc atg ggg aaa gaa ata aat aat gag agt tca 673
Ser Lys Leu Ile Ala Arg Met Gly Lys Glu Ile Asn Asn Glu Ser Ser
            210                 215                 220

tac ctt tat tgg gca tat aag aac gac att cca gta ttc tgt cca ggc 721
Tyr Leu Tyr Trp Ala Tyr Lys Asn Asp Ile Pro Val Phe Cys Pro Gly
        225                 230                 235

tta aca gat ggt tct cta ggg gac atg cta tac ttt cat tcc ttc cac 769
Leu Thr Asp Gly Ser Leu Gly Asp Met Leu Tyr Phe His Ser Phe His
    240                 245                 250

aac cct ggt cta att gtt gcc ata gtc caa gat att aga gcc atg aat 817
Asn Pro Gly Leu Ile Val Ala Ile Val Gln Asp Ile Arg Ala Met Asn
255                 260                 265                 270

ggt gaa gct gtc cac gca agt cct aga aaa act ggt atc atc att ctt 865
Gly Glu Ala Val His Ala Ser Pro Arg Lys Thr Gly Ile Ile Ile Leu
                275                 280                 285

gga ggt ggg ctt cct aag cat cat ata tgc aat gcc aat atg atg cgt 913
Gly Gly Gly Leu Pro Lys His His Ile Cys Asn Ala Asn Met Met Arg
            290                 295                 300

aac ggt gca gat tat gct gta ttc atc aat aca gca caa gaa ttt gat 961
Asn Gly Ala Asp Tyr Ala Val Phe Ile Asn Thr Ala Gln Glu Phe Asp
        305                 310                 315

ggg agt gat tct gga gct cat cct gat gag gct gta tcg tgg ggg aaa 1009
Gly Ser Asp Ser Gly Ala His Pro Asp Glu Ala Val Ser Trp Gly Lys
    320                 325                 330

ata cga ggt tct gct aaa act gtt aag gtc cac tgt gat gca act att 1057
Ile Arg Gly Ser Ala Lys Thr Val Lys Val His Cys Asp Ala Thr Ile
335                 340                 345                 350

gct ttt cct ctc cta gtt gct gaa aca ttt gcc cct agg agg aac aga 1105
Ala Phe Pro Leu Leu Val Ala Glu Thr Phe Ala Pro Arg Arg Asn Arg
                355                 360                 365

ttc tgc agc agt act caa agc tagggctgtg tgcagttctt ggccagaaaa 1156
Phe Cys Ser Ser Thr Gln Ser
            370

ttgattcatt tttatttgta ttatgactga acgatccgca ggatgggtag tgggctccat 1216

tgatgccata aacttctttt tttttcccct cagaattaag ggatccgcca gaacacactg 1276

ctctcagccc caaaccattg ttgcctctac tgggagtagc ataaccaatt gaattgcgct 1336

cctccaagca gcgcctctta gttgcgttat ttattgtaag tagcgcaacc aactaaatta 1396

tgctagttcc cacatttatt gactgctatt ttcaaaagaa aaaaaaaaaa aaaaa 1451

<210> 77
<211> 373
<212> PRT
<213> Populus deltoides

<400> 77
Met Thr Gly Lys Lys Gln Trp Glu Glu Asp Leu Leu Ser Ser Val Arg
1               5                   10                  15

Thr Thr Val Phe Lys Glu Ser Glu Ala Leu Asp Gly Lys Cys Ile Lys
            20                  25                  30

Ile Glu Gly Tyr Asp Phe Asn Gln Gly Val Asn Tyr Ser Lys Leu Leu
        35                  40                  45

Lys Ser Met Val Ser Thr Gly Phe Gln Ala Ser Asn Leu Gly Asp Ala
    50                  55                  60

Ile Gln Val Val Asn Asn Met Leu Asp Trp Arg Leu Ala Asp Glu Glu
65                  70                  75                  80

Ile Thr Glu Asp Cys Ser Asp Glu Glu Arg Glu Leu Ala Tyr Arg Glu
                85                  90                  95

Ser Val Arg Cys Lys Leu Phe Leu Gly Phe Thr Ser Asn Leu Val Ser
            100                 105                 110

Ser Gly Val Arg Asp Thr Ile Arg Tyr Leu Val Gln His His Met Val
        115                 120                 125

Asp Val Val Val Thr Thr Ala Gly Gly Ile Glu Glu Asp Leu Ile Lys
    130                 135                 140

Cys Leu Ala Pro Thr Tyr Lys Gly Asp Phe Ser Leu Pro Gly Ala Gln
145                 150                 155                 160

Leu Arg Ser Lys Gly Leu Asn Arg Ile Gly Asn Leu Leu Val Pro Asn
                165                 170                 175

Asp Asn Tyr Cys Lys Phe Glu Asp Trp Ile Ile Pro Ile Phe Asp Gln
            180                 185                 190

Met Leu Lys Glu Gln Ile Glu Glu Asn Ile Thr Trp Thr Pro Ser Lys
        195                 200                 205

Leu Ile Ala Arg Met Gly Lys Glu Ile Asn Asn Glu Ser Ser Tyr Leu
    210                 215                 220

Tyr Trp Ala Tyr Lys Asn Asp Ile Pro Val Phe Cys Pro Gly Leu Thr
225                 230                 235                 240

Asp Gly Ser Leu Gly Asp Met Leu Tyr Phe His Ser Phe His Asn Pro
                245                 250                 255

Gly Leu Ile Val Ala Ile Val Gln Asp Ile Arg Ala Met Asn Gly Glu
            260                 265                 270

Ala Val His Ala Ser Pro Arg Lys Thr Gly Ile Ile Ile Leu Gly Gly
        275                 280                 285

Gly Leu Pro Lys His His Ile Cys Asn Ala Asn Met Met Arg Asn Gly
    290                 295                 300

Ala Asp Tyr Ala Val Phe Ile Asn Thr Ala Gln Glu Phe Asp Gly Ser
305                 310                 315                 320

Asp Ser Gly Ala His Pro Asp Glu Ala Val Ser Trp Gly Lys Ile Arg
                325                 330                 335

Gly Ser Ala Lys Thr Val Lys Val His Cys Asp Ala Thr Ile Ala Phe
            340                 345                 350

Pro Leu Leu Val Ala Glu Thr Phe Ala Pro Arg Arg Asn Arg Phe Cys
        355                 360                 365

Ser Ser Thr Gln Ser
    370

<210> 78
<211> 1488
<212> DNA
<213> Arabidopsis thaliana

<400> 78
acaataaggc tttaaagccc ataaaaccct taaatatatc aaagcccaaa agaaacgcct 60
tttgcgcttt cccgatcgtg gtcaacttcc tctgttacca aaaaatctgt accgcaaaat 120
cctcgtcgaa gctcgctgct gcaaccatgt ccgacgagga gcatcacttt gagtccagtg 180
acgccggagc gtccaaaacc taccctcaac aagctggaac catccgtaag aatggttaca 240
tcgtcatcaa aaatcgtccc tgcaaggttt cgttctcaaa catttctcca ctctcttcct 300
ctgatcttat tagatctgtt cattacttag attcctcaga ttcttttttt tgtcacctcc 360
acgatgttcg actgatattt gttcttgtca tcattgttaa attcacattt tattgcactt 420
ttgttttagc gaaattatta aattggtcat cttcagtttt gttcgattag ataagtttta 480
ggattttttc ttacacaagt tactggatca gctgctaaat gtcattttgt gtcgcaggtt 540
gttgaggttt caacctcgaa gactggcaag catggtcatg ctaaatgtca ttttgtagct 600
attgatatct tcaccagcaa gaaactcgaa gatattgttc cttcttccca caattgtgat 660
gtatgtgaaa aaagctcctt tgatcacttt catttcttgt ttgtttcttt caagtcccat 720
ttgagatttt gtttttgttg aattgggttt caggttcctc atgtcaaccg tactgattat 780
cagctgattg acatttctga agatggatat gtatgtgttc ttaaatagca cttgttcctt 840
tatatggttt agttacttgt tctgttttgt aatcattttg caggtcagtt tgttgactga 900
taacggtagt accaaggatg accttaagct ccctaatgat gacactctgc tccaacaggt 960
taagttttgc atgttcatca cattaaatgt tgctagttaa ttaaaatcaa ctctatgtcg 1020
atttctgaaa atggaagaaa aagtgcagag taatgagtga cctgattgtg ttaatgaaac 1080
agatcaagag tgggtttgat gatggaaaag atctagtggt gagtgtgatg tcagctatgg 1140
gagaggaaca gatcaatgct cttaaggaca tcggtcccaa gtgagactaa caaagcctcc 1200
cctttgttat gagattcttc ttcttcttct gtaggcttcc attactcatc ggagattatc 1260
ttgtttttgg gtgactccta ttttggatat ttaaactttt gttaataatg ccatcttctt 1320
caaccttttc cttctagatg gtttttatac ttcttctaat tgattgattc tttatggttg 1380
tccaagtgtc aaagtgttcc acccatatga ttctaacctt ttgatgagcg aagtctttac 1440
tcgtgcgtta tgtagagacg tagaagcaat accacaaaag agtataat 1488

<210> 79
<211> 1566
<212> DNA
<213> Arabidopsis thaliana

<400> 79
aggataataa tacagtaacc ctagaaaggt ttcctccacc ttcctcttcc cctcctatat 60
aaaaaaaatc gacatcgctt ttgctcactt ctctctctta ggtttttttt cccttctccc 120
aatctcatct tctccgaaaa cctttcttct ctcaaatttc tgtgaaaaca tgtctgacga 180
cgagcaccac tttgaggcca gcgaatccgg agcttccaag acctatcctc aatcagccgg 240
taacatccgt aaaggtggtc acatcgtcat caaaaaccgt ccctgcaagg tctgatttct 300
atttcatcat caaacatcgt tctcgatctc tttttcctga ttctagatct cgtctctgta 360
tagtagctcc ttgattttgt ttttatcctc ggatttgacc tggttctgtt tagtttgaat 420
ttttcttata gatcgctact tagatgaata tgatgaatct tatcctgtta ttttgatggt 480
ggtacctctc tagattcgtg gaattttggg aaatgaaaat gaaaaatgga tagaaatcaa 540
gcaatatcag acgacgcctt ttgtgatttt gaatctaagt agtctattga ttgatttgat 600
ttaaacgttt atggagaaca tagatttgat tttgatattt tggttttgat taggttgttg 660
aggtttcgac ttccaaaact ggcaagcacg gtcacgccaa atgtcacttt gttgctattg 720
atatcttcac tgctaagaag cttgaagata ttgttccatc ttcccacaat tgtgatgtaa 780
gttactacac aaactatgta gattcatttt cacagtattt gatatgattg tgtgatctga 840
ctcaaatatt gttcctttct ctttttttct caggttccac atgtgaaccg tgttgattac 900
cagttgattg atatcactga ggatggcttc gtatgttttt ctttatactc actttcctca 960
tcactccagc tttatttatc tattcttgcc ataacttttg tacttgttta cattataggt 1020
gagccttctc actgacagtg gtggcaccaa ggatgatctc aagcttccca ccgatgatgg 1080
tctcaccgcc caggttattt tcttgtcttt tcatactcgc acacaaatga cttgactttg 1140
tattcatctc tcgaattgtg atattgaaaa cagttgttgt gttttgttaa tgcagatgag 1200
gcttggattc gatgagggaa aggatattgt ggtgtctgtc atgtcttcca tgggagagga 1260
gcagatctgt gccgtcaagg aagttggtgg tggcaagtaa acaagtatca ttcgatatat 1320
tattaccagt ttgacaacgg acgtcaatgt tataagaacc aaaagatgtt tttctttttc 1380
ctaatttaga ccctttgtgt gtgtttcttg ttgcaagaca accatatcta ttggttttgg 1440
attgttggaa aagtttgtgt tgaaacattc aaagtttctt atgagatgtt attcttaaaa 1500
ccactttttg tttgttcact ggatatgttt gttcatgaag cttgttttaa gcaactcttt 1560
acatga 1566

<210> 80
<211> 319
<212> DNA
<213> Artificial Sequence

<220>
<223> Description of Artificial Sequence: Promoter
      sequence

<400> 80
aagacgttcc aaccacgtct tcaaagcaag tggattgatg tgatatctcc actgacgtaa 60
gggatgacgc acaatcccac tatccttcgc aagacccttc ctctatataa ggaagttcat 120
ttcatttgga gaggacacgc tgaaatcacc agtctctctc tcaagcttgg atcctcgagt 180
actagttcag ggagctcgaa ttgatcctct agagctttcg ttcgtatcat cggtttcgac 240
aacgttcgtc aagttcaatg catcagtttc attgcgcaca caccagaatc ctactgagtt 300
tgagtattat ggcattggg 319

<210> 81
<211> 22
<212> DNA
<213> Artificial Sequence

<220>
<223> Description of Artificial Sequence: Primer

<400> 81
gggagggact agtgtgcacg cc                                                22

<210> 82
<211> 38
<212> DNA
<213> Artificial Sequence

<220>
<223> Description of Artificial Sequence: Primer

<400> 82
   gcgaagcggc catggctcga gttttttttt tttttttt                                38

<210> 83
<211> 521
<212> DNA
<213> Arabidopsis thaliana

<400> 83
gggagggact agtgtgcacg ccctgatgaa gctgtgtctt ggggtaaaat taggggttct 60
gctaaaaccg ttaaggtata ctgtgatgct accatagcct tcccattgtt ggttgcagaa 120
acatttgcca caaagagaga ccaaacctgt gagtctaaga cttaagaact gactggttcg 180
tacctctggc ctcatcatcg atgtagtaca agatatcaga gctatgaacg gcgaagctgt 240
ccatgcaaat cctaaaaaga caggcgtttt ggccatggat tcttaaagat cgttgctttt 300
tgattttaca ctggagtgac catataacac tccacattga tgtggctgtg acgcgaattg 360
tcttcttgcg aattgtactt tagtttctct caacctaaaa tgatttgcag attgtgtttt 420
cgtttaaaac acaagagtct tgtagtcaat aatcctttgc cttataaaat tattcagttc 480
caacaaaaaa aaaaaaaaaa ctcgagccat ggccgcttcg c                          521

<210> 84
<211> 497
<212> DNA
<213> Arabidopsis thaliana

<400> 84
ctagtgtgca cgccctgatg aagctgtgtc ttggggtaaa attaggggtt ctgctaaaac 60
cgttaaggta tactgtgatg ctaccatagc cttcccattg ttggttgcag aaacatttgc 120
cacaaagaga gaccaaacct gtgagtctaa gacttaagaa ctgactggtt cgtacctctg 180
gcctcatcat cgatgtagta caagatatca gagctatgaa cggcgaagct gtccatgcaa 240
atcctaaaaa gacaggcgtt ttggccatgg attcttaaag atcgttgctt tttgatttta 300
cactggagtg accatataac actccacatt gatgtggctg tgacgcgaat tgtcttcttg 360
cgaattgtac tttagtttct ctcaacctaa aatgatttgc agattgtgtt ttcgtttaaa 420
acacaagagt cttgtagtca ataatccttt gccttataaa attattcagt tccaacaaaa 480
aaaaaaaaaa aactcga                                                     497

<210> 85
<211> 29
<212> DNA
<213> Artificial Sequence

<220>
<223> Description of Artificial Sequence: Primer

<400> 85
ctcgagaaga ataacatctc ataagaaac                                         29

<210> 86
<211> 29
<212> DNA
<213> Artificial Sequence

<220>
<223> Description of Artificial Sequence: Primer

<400> 86
gagctcggca agtaaacaag tatcattcg                                    29


<210> 87
<211> 906
<212> DNA
<213> Artificial Sequence

<220>
<223> Description of Artificial Sequence: Synthetic
      construct

<400> 87
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc                                                             906


<210> 88
<211> 205
<212> DNA
<213> Artificial Sequence

<220>
<223> Description of Artificial Sequence: Synthetic
      construct

<400> 88
aagaataaca tctcataaga aactttgaat gtttcaacac aaacttttcc aacaatccaa 60
aaccaataga tatggttgtc ttgcaacaag aaacacacac aaagggtcta aattaggaaa 120
aagaaaaaca tcttttggtt cttataacat tgacgtccgt tgtcaaactg gtaataatat 180
atcgaatgat acttgtttac ttgcc                                        205


<210> 89
<211> 662
<212> DNA

<213> Artificial Sequence

<220>
<223> Description of Artificial Sequence: Synthetic
      construct

<400> 89
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtga aatggaaatg gatggagaag agttaatgaa tgatatggtc 240
cttttgttca ttctcaaatt aatattattt gttttttctc ttatttgttg tgtgttgaat 300
ttgaaattat aagagatatg caaacatttt gttttgagta aaaatgtgtc aaatcgtggc 360
ctctaatgac cgaagttaat atgaggagta aaacacttgt agttgtacca ttatgcttat 420
tcactaggca acaaatatat tttcagacct agaaaagctg caaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at                                                                662

<210> 90
<211> 37
<212> PRT
<213> Arabidopsis thaliana

<400> 90
Ala Arg Pro Asp Glu Ala Val Ser Trp Gly Lys Ile Arg Gly Ser Ala
1               5                   10                  15

Lys Thr Val Lys Val Cys Phe Leu Ile Ser Ser His Pro Asn Leu Tyr
            20                  25                  30

Leu Thr Gln Trp Phe
        35

<210> 91
<211> 52
<212> PRT
<213> Lycopersicon esculentum

<400> 91
Gly Ala Arg Pro Asp Glu Ala Val Ser Trp Gly Lys Ile Arg Gly Gly
1               5                   10                  15

Ala Lys Thr Val Lys Val His Cys Asp Ala Thr Ile Ala Phe Pro Ile
            20                  25                  30

Leu Val Ala Glu Thr Phe Ala Ala Lys Ser Lys Glu Phe Ser Gln Ile
        35                  40                  45

Arg Cys Gln Val
    50

<210> 92
<211> 193
<212> PRT

<213> Arabidopsis thaliana

<400> 92
Gly Gly Val Glu Glu Asp Leu Ile Lys Cys Leu Ala Pro Thr Phe Lys
1               5                   10                  15

Gly Asp Phe Ser Leu Pro Gly Ala Tyr Leu Arg Ser Lys Gly Leu Asn
            20                  25                  30

Arg Ile Gly Asn Leu Leu Val Pro Asn Asp Asn Tyr Cys Lys Phe Glu
        35                  40                  45

Asp Trp Ile Ile Pro Ile Phe Asp Glu Met Leu Lys Glu Gln Lys Glu
    50                  55                  60

Glu Asn Val Leu Trp Thr Pro Ser Lys Leu Leu Ala Arg Leu Gly Lys
65                  70                  75                  80

Glu Ile Asn Asn Glu Ser Ser Tyr Leu Tyr Trp Ala Tyr Lys Met Asn
                85                  90                  95

Ile Pro Val Phe Cys Pro Gly Leu Thr Asp Gly Ser Leu Arg Asp Met
            100                 105                 110

Leu Tyr Phe His Ser Phe Arg Thr Ser Gly Leu Ile Ile Asp Val Val
        115                 120                 125

Gln Asp Ile Arg Ala Met Asn Gly Glu Ala Val His Ala Asn Pro Lys
    130                 135                 140

Lys Thr Gly Met Ile Ile Leu Gly Gly Gly Leu Pro Lys His His Ile
145                 150                 155                 160

Cys Asn Ala Asn Met Met Arg Asn Gly Ala Asp Tyr Ala Val Phe Ile
                165                 170                 175

Asn Thr Gly Gln Glu Phe Asp Gly Ser Asp Ser Gly Ala Arg Pro Asp
            180                 185                 190

Glu

<210> 93
<211> 174
<212> PRT
<213> Dianthus caryophyllus

<400> 93
Arg Arg Ser Ile Lys Cys Leu Ala Pro Thr Phe Lys Gly Asp Phe Ala
1               5                   10                  15

Leu Pro Gly Ala Gln Leu Arg Ser Lys Gly Leu Asn Arg Ile Gly Asn
            20                  25                  30

Leu Leu Val Pro Asn Asp Asn Tyr Cys Lys Phe Glu Asp Trp Ile Ile
        35                  40                  45

Pro Ile Leu Asp Lys Met Leu Glu Glu Gln Ile Ser Glu Lys Ile Leu
    50                  55                  60

Trp Thr Pro Ser Lys Leu Ile Gly Arg Leu Gly Arg Glu Ile Asn Asp
65                  70                  75                  80

Glu Ser Ser Tyr Leu Tyr Trp Ala Phe Lys Asn Asn Ile Pro Val Phe
                85                  90                  95

Cys Pro Gly Leu Thr Asp Gly Ser Leu Gly Asp Met Leu Tyr Phe His
            100                 105                 110

Ser Phe Arg Asn Pro Gly Leu Ile Ile Asp Val Val Gln Asp Ile Arg
        115                 120                 125

Ala Val Asn Gly Glu Ala Val His Ala Ala Pro Arg Lys Thr Gly Met
    130                 135                 140

Ile Ile Leu Gly Gly Gly Leu Pro Lys His His Ile Cys Asn Ala Asn
145                 150                 155                 160

Met Met Arg Asn Gly Ala Asp Tyr Ala Val Phe Ile Asn Thr
                165                 170

<210> 94
<211> 22
<212> PRT
<213> Arabidopsis thaliana

<400> 94
Cys Lys Val Val Glu Val Ser Thr Ser Lys Thr Gly Lys His Gly His
1               5                   10                  15

Ala Lys Cys His Phe Val
            20

<210> 95
<211> 22
<212> PRT
<213> Arabidopsis thaliana

<400> 95
Cys Lys Val Val Glu Val Ser Thr Ser Lys Thr Gly Lys His Gly His
1               5                   10                  15

Ala Lys Cys His Phe Val
            20

<210> 96
<211> 22
<212> PRT
<213> Arabidopsis thaliana

<400> 96
Cys Lys Val Val Glu Val Ser Thr Ser Lys Thr Gly Lys His Gly His
1               5                   10                  15

Ala Lys Cys His Phe Val

            20

<210> 97
<211> 22
<212> PRT
<213> Brassica napus

<400> 97
Cys Lys Val Val Glu Val Ser Thr Ser Lys Thr Gly Lys His Gly His
1               5                   10                  15

Ala Lys Cys His Phe Val
            20

<210> 98
<211> 22
<212> PRT
<213> Dianthus caryophyllus

<400> 98
Cys Lys Val Val Glu Val Ser Thr Ser Lys Thr Gly Lys His Gly His
1               5                   10                  15

Ala Lys Cys His Phe Val
            20

<210> 99
<211> 22
<212> PRT
<213> Lycopersicon esculentum

<400> 99
Cys Lys Val Val Glu Val Ser Thr Ser Lys Thr Gly Lys His Gly His
1               5                   10                  15

Ala Lys Cys His Phe Val
            20

<210> 100
<211> 22
<212> PRT
<213> Lycopersicon esculentum

<400> 100
Cys Lys Val Val Glu Val Ser Thr Ser Lys Thr Gly Lys His Gly His
1               5                   10                  15

Ala Lys Cys His Phe Val
            20

<210> 101
<211> 22
<212> PRT
<213> Lycopersicon esculentum

<400> 101

Cys Lys Val Val Glu Val Ser Thr Ser Lys Thr Gly Lys His Gly His
1               5                   10                  15

Ala Lys Cys His Phe Val
            20

<210> 102
<211> 22
<212> PRT
<213> Medicago sativa

<400> 102
Cys Lys Val Val Glu Val Ser Thr Ser Lys Thr Gly Lys His Gly His
1               5                   10                  15

Ala Lys Cys His Phe Val
            20

<210> 103
<211> 22
<212> PRT
<213> Medicago sativa

<400> 103
Cys Lys Val Val Glu Val Ser Thr Ser Lys Thr Gly Lys His Gly His
1               5                   10                  15

Ala Lys Cys His Phe Val
            20

<210> 104
<211> 22
<212> PRT
<213> Medicago sativa

<400> 104
Cys Lys Val Val Glu Val Ser Thr Ser Lys Thr Gly Lys His Gly His
1               5                   10                  15

Ala Lys Cys His Phe Val
            20

<210> 105
<211> 22
<212> PRT
<213> Lactuca sativa

<400> 105
Cys Lys Val Val Glu Val Ser Thr Ser Lys Thr Gly Lys His Gly His
1               5                   10                  15

Ala Lys Cys His Phe Val
            20

<210> 106
<211> 22

<212> PRT
<213> Unknown Organism

<220>
<223> Description of Unknown Organism: Tree sequence
      peptide

<400> 106
Cys Lys Val Val Glu Val Ser Thr Ser Lys Thr Gly Lys His Gly His
  1               5                  10                  15

Ala Lys Cys His Phe Val
             20


<210> 107
<211> 22
<212> PRT
<213> Unknown Organism

<220>
<223> Description of Unknown Organism: Tree sequence
      peptide

<400> 107
Cys Lys Val Val Glu Val Ser Thr Ser Lys Thr Gly Lys His Gly His
  1               5                  10                  15

Ala Lys Cys His Phe Val
             20


<210> 108
<211> 22
<212> PRT
<213> Unknown Organism

<220>
<223> Description of Unknown Organism: Tree sequence
      peptide

<400> 108
Cys Lys Val Val Glu Val Ser Thr Ser Lys Thr Gly Lys His Gly His
  1               5                  10                  15

Ala Lys Cys His Phe Val
             20


<210> 109
<211> 22
<212> PRT
<213> Unknown Organism

<220>
<223> Description of Unknown Organism: Tree sequence
      peptide

<400> 109
Cys Lys Val Val Glu Val Ser Thr Ser Lys Thr Gly Lys His Gly His
  1               5                  10                  15

Ala Lys Cys His Phe Val
            20

<210> 110
<211> 66
<212> DNA
<213> Arabidopsis thaliana

<400> 110
tgcaaggttg ttgaggtttc aacctcgaag actggcaagc atggtcatgc taaatgtcat 60
tttgta                                                            66

<210> 111
<211> 66
<212> DNA
<213> Arabidopsis thaliana

<400> 111
tgcaaggttg ttgaggtttc gacttccaaa actggcaagc acggtcacgc caaatgtcac 60
tttgtt                                                            66

<210> 112
<211> 66
<212> DNA
<213> Arabidopsis thaliana

<400> 112
tgcaaggtgg ttgaggtatc gacttcgaag actgggaagc atggtcacgc caagtgtcac 60
tttgtt                                                            66

<210> 113
<211> 66
<212> DNA
<213> Brassica napus

<400> 113
tgcaaggttg ttgaggtttc gacttcgaag actgggaagc acggtcacgc aaagtgtcac 60
tttgtt                                                            66

<210> 114
<211> 66
<212> DNA
<213> Dianthus caryophyllus

<400> 114
tgcaaggtgg ttgaggtttc tacctccaag actggcaagc acggtcatgc caaatgtcac 60
tttgta                                                            66

<210> 115
<211> 66
<212> DNA
<213> Lycopersicon esculentum

<400> 115

tgcaaggtgg ttgaagtttc aacctccaag acaggcaagc acggtcatgc taaatgtcac 60
ttcgtg 66

<210> 116
<211> 66
<212> DNA
<213> Lycopersicon esculentum

<400> 116
tgcaaggttg tggaagtctc tacatccaaa actggcaagc acggtcacgc caaatgtcat 60
ttcgtt 66

<210> 117
<211> 66
<212> DNA
<213> Lycopersicon esculentum

<400> 117
tgcaaggttg ttgaggtctc cacttccaaa actggcaagc atggacatgc aaaatgtcac 60
tttgtg 66

<210> 118
<211> 66
<212> DNA
<213> Medicago sativa

<400> 118
tgcaaggtgg ttgaagtttc gacttcgaag accgggaagc atggacatgc caagtgtcat 60
tttgtt 66

<210> 119
<211> 66
<212> DNA
<213> Medicago sativa

<400> 119
tgcaaggttg ttgaggtttc tacttcaaaa acaggaaaac atggacatgc aaagtgtcac 60
tttgtt 66

<210> 120
<211> 66
<212> DNA
<213> Medicago sativa

<400> 120
tgcaaggtag ttgaagtttc aacttctaaa actggaaagc atggacatgc aaagtgtcac 60
tttgtt 66

<210> 121
<211> 66
<212> DNA
<213> Lactuca sativa

<400> 121
tgcaaggttg ttgaagtttc tacctccaag actgggaagc atgggcatgc taagtgtcac 60

tttgtc                                                              66

<210> 122
<211> 66
<212> DNA
<213> Unknown Organism

<220>
<223> Description of Unknown Organism: Tree
      oligonucleotide sequence

<400> 122
tgcaaggtcg tggaggtttc aacctctaaa actggcaagc atggccatgc taaatgtcac 60
tttgtt                                                              66

<210> 123
<211> 66
<212> DNA
<213> Unknown Organism

<220>
<223> Description of Unknown Organism: Tree
      oligonucleotide sequence

<400> 123
tgcaaggttg ttgaggtttc cacctcaaag acaggcaagc acggacatgc taagtgccac 60
tttgtg                                                              66

<210> 124
<211> 66
<212> DNA
<213> Unknown Organism

<220>
<223> Description of Unknown Organism: Tree
      oligonucleotide sequence

<400> 124
tgcaaggttg tggaggtttc tacctctaaa actggcaagc acggccatgc caaatgtcac 60
tttgtt                                                              66

<210> 125
<211> 66
<212> DNA
<213> Unknown Organism

<220>
<223> Description of Unknown Organism: Tree
      oligonucleotide sequence

<400> 125
tgcaaggttg ttgaggtttc aacctcaaag acaggcaagc atggacatgc taagtgccac 60
tttgtg                                                              66

<210> 126
<211> 745

<212> DNA
<213> Lycopersicon esculentum

<220>
<221> CDS
<222> (55)..(534)

<400> 126
ttctccacag caaacacaga gaagttcata gcagaagaag agagagattt agct atg 57
Met
1

tct gat gaa gaa cac cat ttt gag tcc aaa gct gat gct ggt gcc tca 105
Ser Asp Glu Glu His His Phe Glu Ser Lys Ala Asp Ala Gly Ala Ser
            5                   10                  15

aaa act tac cct caa caa gct ggt act att cgc aag aat ggt tat ata 153
Lys Thr Tyr Pro Gln Gln Ala Gly Thr Ile Arg Lys Asn Gly Tyr Ile
        20                  25                  30

gtt atc aaa ggc aga cct tgc aag gtt gtt gag gtc tcc act tcc aaa 201
Val Ile Lys Gly Arg Pro Cys Lys Val Val Glu Val Ser Thr Ser Lys
    35                  40                  45

act ggc aag cat gga cat gca aaa tgt cac ttt gtg gca atc gac att 249
Thr Gly Lys His Gly His Ala Lys Cys His Phe Val Ala Ile Asp Ile
50                  55                  60                  65

ttc aat gca aaa aag ctt gaa gat att gtt cct tca tcc cac aat tgt 297
Phe Asn Ala Lys Lys Leu Glu Asp Ile Val Pro Ser Ser His Asn Cys
                70                  75                  80

gat gtg cca cat gtc aat cgt act gac tat cag ctg att gac ata tct 345
Asp Val Pro His Val Asn Arg Thr Asp Tyr Gln Leu Ile Asp Ile Ser
            85                  90                  95

gaa gat ggt ttt gtg tct ctt ctt act gaa aat gga aac acc aaa gac 393
Glu Asp Gly Phe Val Ser Leu Leu Thr Glu Asn Gly Asn Thr Lys Asp
        100                 105                 110

gac ctc aga ctt ccc acc gat gac acc ctg ttg aac cag gtt aaa ggt 441
Asp Leu Arg Leu Pro Thr Asp Asp Thr Leu Leu Asn Gln Val Lys Gly
    115                 120                 125

gga ttt gag gaa gga aag gat ctc gtt ctg tct gtg atg tct gca atg 489
Gly Phe Glu Glu Gly Lys Asp Leu Val Leu Ser Val Met Ser Ala Met
130                 135                 140                 145

ggt gaa gag cag atc tgt gct gtg aag gac att ggt acc aag acc 534
Gly Glu Glu Gln Ile Cys Ala Val Lys Asp Ile Gly Thr Lys Thr
                150                 155                 160

tagttgtgtg cattctgcag cataaataat tgctttttag cgaagacgtt ttatatcttg 594

ttatcgtggt acctttgcaa tctgttttat cgtgaaaaca ccttatatct attggcatgg 654

cttgaatagt tgaaactcta atagtttctg tttggcataa ggcaatgaac tggatttgat 714

agcagaagta atctacatgt cacttttttt t 745

<210> 127
<211> 160
<212> PRT
<213> Lycopersicon esculentum

<400> 127
Met Ser Asp Glu Glu His His Phe Glu Ser Lys Ala Asp Ala Gly Ala
1               5                   10                  15

Ser Lys Thr Tyr Pro Gln Gln Ala Gly Thr Ile Arg Lys Asn Gly Tyr
            20                  25                  30

Ile Val Ile Lys Gly Arg Pro Cys Lys Val Val Glu Val Ser Thr Ser
        35                  40                  45

Lys Thr Gly Lys His Gly His Ala Lys Cys His Phe Val Ala Ile Asp
    50                  55                  60

Ile Phe Asn Ala Lys Lys Leu Glu Asp Ile Val Pro Ser Ser His Asn
65                  70                  75                  80

Cys Asp Val Pro His Val Asn Arg Thr Asp Tyr Gln Leu Ile Asp Ile
                85                  90                  95

Ser Glu Asp Gly Phe Val Ser Leu Leu Thr Glu Asn Gly Asn Thr Lys
            100                 105                 110

Asp Asp Leu Arg Leu Pro Thr Asp Asp Thr Leu Leu Asn Gln Val Lys
        115                 120                 125

Gly Gly Phe Glu Glu Gly Lys Asp Leu Val Leu Ser Val Met Ser Ala
    130                 135                 140

Met Gly Glu Glu Gln Ile Cys Ala Val Lys Asp Ile Gly Thr Lys Thr
145                 150                 155                 160

<210> 128
<211> 25
<212> PRT
<213> Artificial Sequence

<220>
<223> Description of Artificial Sequence: Primer

<400> 128
Gly Ala Ala Gly Cys Thr Cys Gly Ala Gly Gly Cys Thr Gly Cys Ala
1               5                   10                  15

Ala Cys Cys Ala Thr Gly Thr Cys Cys
            20                  25

<210> 129
<211> 26
<212> DNA
<213> Artificial Sequence

<220>
<223> Description of Artificial Sequence: Primer

<400> 129
ggggagctct tgttagtctc acttgg                                          26


<210> 130
<211> 906
<212> DNA
<213> Artificial Sequence

<220>
<223> Description of Artificial Sequence: Synthetic
      construct

<400> 130
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcaccaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc                                                                906


<210> 131
<211> 495
<212> DNA
<213> Artificial Sequence

<220>
<223> Description of Artificial Sequence: Synthetic
      construct

<400> 131
gctgcaacca tgtccgacga ggagcatcac tttgagtcca gtgacgccgg agcgtccaaa 60
acctaccctc aacaagctgg aaccatccgt aagaatggtt acatcgtcat caaaaatcgt 120
ccctgcaagg ttgttgaggt ttcaacctcg aagactggca agcatggtca tgctaaatgt 180
cattttgtag ctattgatat cttcaccagc aagaaactcg aagatattgt tccttcttcc 240
cacaattgtg atgttcctca tgtcaaccgt actgattatc agctgattga catttctgaa 300
gatggatatg tcagtttgtt gactgataac ggtagtacca aggatgacct taagctccct 360
aatgatgaca ctctgctcca acagatcaag agtgggtttg atgatggaaa agatctagtg 420
gtgagtgtaa tgtcagctat gggagaggaa cagatcaatg ctcttaagga catcggtccc 480
aagtgagact aacaa                                                      495


<210> 132
<211> 662
<212> DNA
<213> Artificial Sequence

<220>
<223> Description of Artificial Sequence: Synthetic

construct

<400> 132
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtga aatggaaatg gatggagaag agttaatgaa tgatatggtc 240
cttttgttca ttctcaaatt aatattattt gttttttctc ttatttgttg tgtgttgaat 300
ttgaaattat aagagatatg caaacatttt gttttgagta aaaatgtgtc aaatcgtggc 360
ctctaatgac cgaagttaat atgaggagta aaacacttgt agttgtacca ttatgcttat 420
tcactaggca acaaatatat tttcagacct agaaaagctg caaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at 662

<210> 133
<211> 30
<212> DNA
<213> Artificial Sequence

<220>
<223> Description of Artificial Sequence: Primer

<400> 133
gcgctcgagc tatgtctgat gaagaacacc 30

<210> 134
<211> 28
<212> DNA
<213> Artificial Sequence

<220>
<223> Description of Artificial Sequence: Primer

<400> 134
tttgagctcc agaatgcaca caactagg 28

<210> 135
<211> 906
<212> DNA
<213> Artificial Sequence

<220>
<223> Description of Artificial Sequence: Synthetic
construct

<400> 135
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600

gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc 906

<210> 136
<211> 499
<212> DNA
<213> Artificial Sequence

<220>
<223> Description of Artificial Sequence: Synthetic
      construct

<400> 136
ctatgtctga tgaagaacac cattttgagt ccaaagctga tgctggtgcc tcaaaaactt 60
accctcaaca agctggtact attcgcaaga atggttatat agttatcaaa ggcagacctt 120
gcaaggttgt tgaggtctcc acttccaaaa ctggcaagca tggacatgca aaatgtcact 180
ttgtggcaat cgacattttc aatgcaaaaa agcttgaaga tattgttcct tcatcccaca 240
attgtgatgt gccacatgtc aatcgtactg actatcagct gattgacata tctgaagatg 300
gttttgtgtc tcttcttact gaaaatggaa acaccaaaga cgacctcaga cttcccaccg 360
atgacaccct gttgaaccag gttaaaggtg gatttgagga aggaaaggat ctcgttctgt 420
ctgtgatgtc tgcaatgggt gaagagcaga tctgtgctgt gaaggacatt ggtaccaaga 480
cctagttgtg tgcattctg 499

<210> 137
<211> 662
<212> DNA
<213> Artificial Sequence

<220>
<223> Description of Artificial Sequence: Synthetic
      construct

<400> 137
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtga aatggaaatg gatggagaag agttaatgaa tgatatggtc 240
cttttgttca ttctcaaatt aatattattt gttttttctc ttatttgttg tgtgttgaat 300
ttgaaattat aagagatatg caaacatttt gttttgagta aaaatgtgtc aaatcgtggc 360
ctctaatgac cgaagttaat atgaggagta aaacacttgt agttgtacca ttatgcttat 420
tcactaggca acaaatatat tttcagacct agaaaagctg caaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at 662

<210> 138
<211> 29
<212> DNA
<213> Artificial Sequence

<220>
<223> Description of Artificial Sequence: Primer

<400> 138
gcatgtcgac atgtctgacg aggagcacc                                    29


<210> 139
<211> 906
<212> DNA
<213> Artificial Sequence

<220>
<223> Description of Artificial Sequence: Synthetic
      construct

<400> 139
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc                                                             906


<210> 140
<211> 661
<212> DNA
<213> Artificial Sequence

<220>
<223> Description of Artificial Sequence: Synthetic
      construct

<400> 140
tcgacatgtc tgacgaggag caccacttcg agtccagcga cgccggagct tccaaaacct 60
accctcagca ggctggtaac atccgcaagg gtggtcacat cgtcatcaag ggccgtccct 120
gcaaggttgt tgaggtttcg acttcgaaga ctgggaagca cggtcacgca aagtgtcact 180
ttgttgctat tgacatcttc actgctaaga agctcgagga tattgttccc tcttcccaca 240
attgtgatgt tccccatgtg aaccgtattg actaccagtt gattgatatc tctgagaatg 300
gctttgttag ccttttgacc gacagtggtg gcaccaagga cgacctcaag cttcccaccg 360
atgataatct cagcgctctg atgaagagtg gattcgagga gggaaaggat gtggtggtgt 420
ctgtcatgtc ttccatggga gaggagcaga tctgtgccgt caaggaagtt ggtggtggca 480
agtaaaaccc attctctgag agaggataat cttattacca gtggtcaatg ttataagaac 540
aagaacttgt tttttttcct ttttctaatt tagatcattt gtgttgtgtt tctttgttgc 600
aagacaacca ttatctatta ttggttttgg attgtttaaa aaaaaaaaaa aaaaaaaaaa 660
a                                                                  661


<210> 141
<211> 662
<212> DNA
<213> Artificial Sequence

<220>
<223> Description of Artificial Sequence: Synthetic
      construct

<400> 141
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtga aatggaaatg gatggagaag agttaatgaa tgatatggtc 240
cttttgttca ttctcaaatt aatattattt gttttttctc ttatttgttg tgtgttgaat 300
ttgaaattat aagagatatg caaacatttt gttttgagta aaaatgtgtc aaatcgtggc 360
ctctaatgac cgaagttaat atgaggagta aaacacttgt agttgtacca ttatgcttat 420
tcactaggca acaaatatat tttcagacct agaaaagctg caaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at                                                                662


<210> 142
<211> 325
<212> DNA
<213> Artificial Sequence

<220>
<223> Description of Artificial Sequence: Synthetic
      construct

<400> 142
ggtgcacgcc ctgatgaagc agtgtcttgg ggtaaaataa ggggatctgc taaaactgtc 60
aaggtgtact gtgatgctac catagccttc cctttgttgg ttgctgaaac atttgcctcc 120
aagagagaac aaagctgtga gcacaagacc taagcccaag aaagcttacg tctcttttat 180
cggtttgttc ttccatcttg ttgttgtacc ctttgtcctg ctttacataa cattcatctc 240
taaaacaata ctacctcctt ttgacaaaaa ataaaaaaaa ttggaaaaat ggtttcacaa 300
gaataaaaaa aaaaaaaaaa aaaaa                                       325


<210> 143
<211> 906
<212> DNA
<213> Artificial Sequence

<220>
<223> Description of Artificial Sequence: Synthetic
      construct

<400> 143
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840

ataaggaagt tcatttcatt tggagaggac acgctgaaat caccagtctc tctctaagct 900
tggatc 906

<210> 144
<211> 325
<212> DNA
<213> Artificial Sequence

<220>
<223> Description of Artificial Sequence: Synthetic
      construct

<400> 144
tttttttttt tttttttttt tattcttgtg aaaccatttt tccaattttt tttatttttt 60
gtcaaaagga ggtagtattg ttttagagat gaatgttatg taaagcagga caaagggtac 120
aacaacaaga tggaagaaca aaccgataaa agagacgtaa gctttcttgg gcttaggtct 180
tgtgctcaca gctttgttct ctcttggagg caaatgtttc agcaaccaac aaagggaagg 240
ctatggtagc atcacagtac accttgacag ttttagcaga tccccttatt ttaccccaag 300
acactgcttc atcagggcgt gcacc 325

<210> 145
<211> 662
<212> DNA
<213> Artificial Sequence

<220>
<223> Description of Artificial Sequence: Synthetic
      construct

<400> 145
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtgc aaatggaaat ggatggagaa gagttaatga atgatatggt 240
ccttttgttc attctcaaat taatattatt tgttttttct cttatttgtt gtgtgttgaa 300
tttgaaatta taagagatat gcaaacattt tgttttgagt aaaaatgtgt caaatcgtgg 360
cctctaatga ccgaagttaa tatgaggagt aaaacacttg tagttgtacc attatgctta 420
ttcactaggc aacaaatata ttttcagacc tagaaaagct gaaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at 662

<210> 146
<211> 28
<212> DNA
<213> Artificial Sequence

<220>
<223> Description of Artificial Sequence: Primer

<400> 146
aagctcgaga tgtcggacga agagcacc 28

<210> 147
<211> 28
<212> DNA

<213> Artificial Sequence

<220>
<223> Description of Artificial Sequence: Primer

<400> 147
gtagagctcc accaatacca tctgcagc                                    28


<210> 148
<211> 906
<212> DNA
<213> Artificial Sequence

<220>
<223> Description of Artificial Sequence: Synthetic
      construct

<400> 148
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360
tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc                                                            906


<210> 149
<211> 497
<212> DNA
<213> Artificial Sequence

<220>
<223> Description of Artificial Sequence: Synthetic
      construct

<400> 149
atgtcggacg aagagcacca cttcgaatcc aaggccgatg ccggagcttc aaagacgtat 60
cctcaacaag ctggtactat tcgtaaaggt ggtcacatcg tcataaaaaa tcgtccttgc 120
aaggtggttg aagtttcaac ttccaagaca ggcaagcacg gtcatgctaa atgtcactcg 180
tggcaattga cattttcact ggaaagaaac ttgaggatat tgttccctct tctcacaatt 240
gtgatgttcc tcatgtgaat aggactgatt atcaacttat tgatatctct gaggatggct 300
ttgtgagtct gttgactgaa aatggtaaca ccaaggatga cttgagactc ccaactgatg 360
atactcttct ggctcaggtc aaagatggtt ttgctgaggg gaaagacctg gttctatcag 420
tgatgtctgc catgggagag gagcagattt gtggtatcaa ggacattggc cctaagtagc 480
tgcagatggt attggtg                                                497


<210> 150
<211> 662
<212> DNA

<213> Artificial Sequence

<220>
<223> Description of Artificial Sequence: Synthetic
      construct

<400> 150
gaattgatcc tctagagctt tcgttcgtat catcggtttc gacaacgttc gtcaagttca 60
atgcatcagt ttcattgcgc acacaccaga atcctactga gttcgagtat tatggcattg 120
ggaaaactgt ttttcttgta ccatttgttg tgcttgtaat ttactgtgtt ttttattcgg 180
ttttcgctat cgaactgtga aatggaaatg gatggagaag agttaatgaa tgatatggtc 240
cttttgttca ttctcaaatt aatattattt gttttttctc ttatttgttg tgtgttgaat 300
ttgaaattat aagagatatg caaacatttt gttttgagta aaaatgtgtc aaatcgtggc 360
ctctaatgac cgaagttaat atgaggagta aaacacttgt agttgtacca ttatgcttat 420
tcactaggca acaaatatat tttcagacct agaaaagctg caaatgttac tgaatacaag 480
tatgtcctct tgtgttttag acatttatga actttccttt atgtaatttt ccagaatcct 540
tgtcagattc taatcattgc tttataatta tagttatact catggatttg tagttgagta 600
tgaaaatatt ttttaatgca ttttatgact tgccaattga ttgacaacat gcatcaatcg 660
at                                                                662


<210> 151
<211> 27
<212> DNA
<213> Artificial Sequence

<220>
<223> Description of Artificial Sequence: Primer

<400> 151
cgactcgagc agccatgtct gacgagg                                     27


<210> 152
<211> 29
<212> DNA
<213> Artificial Sequence

<220>
<223> Description of Artificial Sequence: Primer

<400> 152
atcgagctca tcacttgggg ccaatatcc                                   29


<210> 153
<211> 906
<212> DNA
<213> Artificial Sequence

<220>
<223> Description of Artificial Sequence: Synthetic
      construct

<400> 153
cactgaatca aaggccatgg agtcaaagat tcaaatagag gacctaacag aactcgccgt 60
aaagactggc gaacagttca tacagagtct cttacgactc aatgacaaga agaaaatctt 120
cgtcaacatg gtggagcacg acacgcttgt ctacctccaa aaatatcaaa gatacagtct 180
cagaagacca aagggaattg agacttttca acaaagggta atatccggaa acctcctcgg 240
attccattgc ccagctatct gtcactttat tgtgaagata gtggaaaagg aaggtggctc 300
ctacaaatgc catcattgcg ataaaggaaa ggccatcgtt gaagatgcct ctgccgacag 360

tggtcccaaa gatggacccc cacccacgag gagcatcgtg gaaaaagaag acgttccaac 420
cacgtcttca aagcaagtgg attgatgtga taacatggtg gagcacgaca cgcttgtcta 480
cctccaaaaa tatcaaagat acagtctcag aagaccaaag ggaattgaga cttttcaaca 540
aagggtaata tccggaaacc tcctcggatt ccattgccca gctatctgtc actttattgt 600
gaagatagtg gaaaaggaag gtggctccta caaatgccat cattgcgata aaggaaaggc 660
catcgttgaa gatgcctctg ccgacagtgg tcccaaagat ggacccccac ccacgaggag 720
catcgtggaa aaagaagacg ttccaaccac gtcttcaaag caagtggatt gatgtgatat 780
ctccactgac gtaagggatg acgcacaatc ccactatcct tcgcaagacc cttcctctat 840
ataaggaagt tcatttcatt tggagaggac acgctgaaat caccagtctc tctctaagct 900
tggatc 906

<210> 154
<211> 486
<212> DNA
<213> Artificial Sequence

<220>
<223> Description of Artificial Sequence: Synthetic
      construct

<400> 154
cagccatgtc tgacgaggag catcaatttg agtctaaggc tgatgccgga gcatcaaaaa 60
cttaccctca acaagctggt actattcgta agaacggtta tatcgtcatc aaaggccgtc 120
catgcaaggt tgtggaagtc tctacatcca aaactggcaa gcacggtcac gccaaatgtc 180
atttcgttgc tattgacatc ttcactggga agaagcttga ggatattgtc ccctcttcac 240
acaattgtga tgtgccccat gttaatcgta cagattatca gcttattgac atctctgaag 300
atggatttgt gagtctgctt actgacaatg gtaacaccaa ggatgacctc aggcttccta 360
ctgatgaaaa tctgctttca ctgatcaagg acgggtttgc cgagggtaag gacctcgttg 420
tgtctgttat gtcagctatg ggtgaggaac agattaatgc tttgaaggat attggcccca 480
agtgat 486

<210> 155
<211> 662
<212> DNA
<213> Artificial Sequence

<220>
<223> Description of Artificial Sequence: Synthetic
      construct

<400> 155
   gaattgatcc tctagagctt tcgttcgtat catcggtttc gacaacgttc gtcaagttca 60
   atgcatcagt ttcattgcgc acacaccaga atcctactga gttcgagtat tatggcattg 120
   ggaaaactgt ttttcttgta ccatttgttg tgcttgtaat ttactgtgtt ttttattcgg 180
   ttttcgctat cgaactgtga aatggaaatg gatggagaag agttaatgaa tgatatggtc 240
   cttttgttca ttctcaaatt aatattattt gttttttctc ttatttgttg tgtgttgaat 300
   ttgaaattat aagagatatg caaacatttt gttttgagta aaaatgtgtc aaatcgtggc 360
   ctctaatgac cgaagttaat atgaggagta aaacacttgt agttgtacca ttatgcttat 420
   tcactaggca acaaatatat tttcagacct agaaaagctg caaatgttac tgaatacaag 480
   tatgtcctct tgtgttttag acatttatga actttccttt atgtaatttt ccagaatcct 540
   tgtcagattc taatcattgc tttataatta tagttatact catggatttg tagttgagta 600
   tgaaaatatt ttttaatgca ttttatgact tgccaattga ttgacaacat gcatcaatcg 660
   at 662

<210> 156
<211> 22
<212> DNA
<213> Artificial Sequence

<220>
<223> Description of Artificial Sequence: Primer

<400> 156
cactgctcac tagtttgatg gc                                                22


<210> 157
<211> 38
<212> DNA
<213> Artificial Sequence

<220>
<223> Description of Artificial Sequence: Primer

<400> 157
gcgaagcggc catggctcga gttttttttt tttttttt                               38


<210> 158
<211> 413
<212> DNA
<213> Lactuca sativa

<400> 158
cactgctcac tagtttgatg gcagtgattc tggtgctcga cctgatgaag ctgtctcctg 60
ggggaaaata cgtggttctg ctaaatctgt caaggtgcac tgtgatgcaa ctatcgcgtt 120
ccctttactt gttgcagaaa catttgctgc aaagagagag ggggagatga aaaatgttga 180
gtcaaccaaa gctttggttt aaaaaggtgg aacagtgtag gacagggact catttttgat 240
attttgtttg ctaaaaaatg gtctttggaa gaatattgat gcacacaaac aaggagacaa 300
tgttactgat cttggagagt gtaatgtaaa atgtctaaat aatttcaaag cttctcacaa 360
caaatcaaac tttaaaaaaa aaaaaaaaaa aactcgagcc atggccgctt cgc       413


<210> 159
<211> 388
<212> DNA
<213> Lactuca sativa

<400> 159
ctagtttgat ggcagtgatt ctggtgctcg acctgatgaa gctgtctcct gggggaaaat 60
acgtggttct gctaaatctg tcaaggtgca ctgtgatgca actatcgcgt tccctttact 120
tgttgcagaa acatttgctg caaagagaga gggggagatg aaaaatgttg agtcaaccaa 180
agctttggtt taaaaaggtg gaacagtgta ggacagggac tcatttttga tattttgttt 240
gctaaaaaat ggtctttgga agaatattga tgcacacaaa caaggagaca atgttactga 300
tcttggagag tgtacatgta aaatgtctaa ataatttcaa agcttctcac aacaaatcaa 360
actttaaaaa aaaaaaaaaa aaactcga                                   388


<210> 160
<211> 23
<212> DNA
<213> Artificial Sequence

<220>
<223> Description of Artificial Sequence: Primer

<220>
<221> modified_base
<222> (3)

<223> a, t, c or g

<220>
<221> modified_base
<222> (12)
<223> a, t, c or g

<220>
<221> modified_base
<222> (18)
<223> a, t, c or g

<400> 160
ggnttraayc gnathggnaa ytt                                              23


<210> 161
<211> 23
<212> DNA
<213> Artificial Sequence

<220>
<223> Description of Artificial Sequence: Primer

<220>
<221> modified_base
<222> (9)
<223> a, t, c or g

<220>
<221> modified_base
<222> (21)
<223> a, t, c or g

<400> 161
tgrtcgganc crtcraaytc ngc                                              23


<210> 162
<211> 108
<212> DNA
<213> Mycosphaerella fijiensis

<400> 162
cgccaagcta tttaggtgac actatagaat actcaagcta tgcatccaac gcgttgggag 60
ctctcccata tggtcgacct gcaggcggcc gcgaattcac tagtgatt              108


<210> 163
<211> 487
<212> DNA
<213> Mycosphaerella fijiensis

<220>
<221> CDS
<222> (1)..(486)

<400> 163
ggg tta aat cgt att gga aac ttc tta gtg cca aac gac aat tac tgc    48
Gly Leu Asn Arg Ile Gly Asn Phe Leu Val Pro Asn Asp Asn Tyr Cys
  1               5                  10                  15

cgc ttt gaa gac tgg gtg atg cca atc ctc gac aca atg ctc gaa gaa     96
Arg Phe Glu Asp Trp Val Met Pro Ile Leu Asp Thr Met Leu Glu Glu
                20                  25                  30

cag gaa gca tgc aag ggt tcg ggc gaa gca atc cac tgg act ccc agc     144
Gln Glu Ala Cys Lys Gly Ser Gly Glu Ala Ile His Trp Thr Pro Ser
            35                  40                  45

aaa atc atc aac cgg ctt ggc aag gag gtc aac gac gaa tcg tcc gtg     192
Lys Ile Ile Asn Arg Leu Gly Lys Glu Val Asn Asp Glu Ser Ser Val
    50                  55                  60

tac tac tgg gca tgg aag aac gac att cca gtg ttc tgt ccg gcg ctt     240
Tyr Tyr Trp Ala Trp Lys Asn Asp Ile Pro Val Phe Cys Pro Ala Leu
65                  70                  75                  80

act gat ggc agt ctc gga gac atg ctg tac ttc cac acg ttc aaa tcc     288
Thr Asp Gly Ser Leu Gly Asp Met Leu Tyr Phe His Thr Phe Lys Ser
                85                  90                  95

tca ccg cag cag ctt cga gtc gac att gtg gaa gac atc cga aag atc     336
Ser Pro Gln Gln Leu Arg Val Asp Ile Val Glu Asp Ile Arg Lys Ile
            100                 105                 110

aac acc ctc gcc gtc cga gcc aag cgc act ggc atg atc att ctc gga     384
Asn Thr Leu Ala Val Arg Ala Lys Arg Thr Gly Met Ile Ile Leu Gly
        115                 120                 125

ggc ggc att gtc aag cac cac atc gca aat gcc aac ctg atg cgc aat     432
Gly Gly Ile Val Lys His His Ile Ala Asn Ala Asn Leu Met Arg Asn
    130                 135                 140

ggc gcg gaa agc gca gtg tac atc aat acc gcg ccg aat tcg acg gat     480
Gly Ala Glu Ser Ala Val Tyr Ile Asn Thr Ala Pro Asn Ser Thr Asp
145                 150                 155                 160

ccg acc a                                                           487
Pro Thr


<210> 164
<211> 162
<212> PRT
<213> Mycosphaerella fijiensis

<400> 164
Gly Leu Asn Arg Ile Gly Asn Phe Leu Val Pro Asn Asp Asn Tyr Cys
1               5                   10                  15

Arg Phe Glu Asp Trp Val Met Pro Ile Leu Asp Thr Met Leu Glu Glu
                20                  25                  30

Gln Glu Ala Cys Lys Gly Ser Gly Glu Ala Ile His Trp Thr Pro Ser
            35                  40                  45

Lys Ile Ile Asn Arg Leu Gly Lys Glu Val Asn Asp Glu Ser Ser Val
    50                  55                  60

Tyr Tyr Trp Ala Trp Lys Asn Asp Ile Pro Val Phe Cys Pro Ala Leu
65                  70                  75                  80

Thr Asp Gly Ser Leu Gly Asp Met Leu Tyr Phe His Thr Phe Lys Ser
                85                  90                  95

Ser Pro Gln Gln Leu Arg Val Asp Ile Val Glu Asp Ile Arg Lys Ile
            100                 105                 110

Asn Thr Leu Ala Val Arg Ala Lys Arg Thr Gly Met Ile Ile Leu Gly
        115                 120                 125

Gly Gly Ile Val Lys His His Ile Ala Asn Ala Asn Leu Met Arg Asn
    130                 135                 140

Gly Ala Glu Ser Ala Val Tyr Ile Asn Thr Ala Pro Asn Ser Thr Asp
145                 150                 155                 160

Pro Thr

<210> 165
<211> 214
<212> DNA
<213> Mycosphaerella fijiensis

<400> 165
   aatcgaattc ccgcggccgc catggcggcc gggagcatgc gacgtcgggc ccaattcgcc 60
   ctatagtgag tcgtattaca attcactggc cgtcgtttta caacgtcgtg actgggaaaa 120
   cctggcggta cccaacttaa tcgccttgca gcacatcccc ctttcgccag ctggcgtaat 180
   agcgaagagg cccgcaccga tcgcccttcc aaca                              214